# EUROPEAN PATENT APPLICATION

(11) **EP 3 862 006 A1**
(43) Date of publication of application: **11.08.2021**
(21) Application number: 20217410.8
(22) Date of filing: 03.11.2018
(51) Int. Cl.: A61K 31/715, A61K 45/06, A61K 9/00, A23L 5/00, A61P 31/04, A61P 43/00

(54) **GLYCAN PREPARATIONS FOR THE TREATMENT OF INFECTION**

(30) Priority: 03.11.2017 US 201762581517 P; 21.08.2018 US 201862720924 P; 14.09.2018 US 201862731746 P
(62) Divisional of application: 18811987.9
(71) Applicant: Kaleido Biosciences, Inc., Lexington, MA 02421 (US)
(72) Inventor: MAHOWALD, Michael, A., Lexington, MA Massachusetts 02421 (US); GIBSON, Molly, Krisann, Lexington, MA Massachusetts 02421 (US)
(74) Representative: Bühler, Dirk

(57) **Abstract**

Described herein are methods of reducing, preventing, or reducing the risk of, an adverse effect of a pathogen. In some embodiments, the pathogen is a drug resistant pathogen.

## Description

### RELATED APPLICATIONS

This application claims the benefit under 35 U.S.C. §119(e) of U.S. Provisional Application No. 62/581517, entitled "MANAGEMENT OF INFECTIONS", filed November 3, 2017, U.S. Provisional Application No. 62/731746, entitled "MANAGEMENT OF INFECTIONS", filed September 14, 2018, and U.S. Provisional Application No. 62/720924, entitled "OLIGOSACCHARIDE COMPOSITIONS AND METHODS OF USE THEREOF FOR REDUCING AMMONIA LEVELS", filed August 21, 2018, the entire contents of which are incorporated herein by reference.

### BACKGROUND

Hospital-acquired infection by antibiotic-resistant bacteria presents a global health crisis. Infections are generally hard to treat and often lethal. Carbapenem-resistant Enterobacteriaceae (CRE) infections have a mortality rate that exceeds 50% in some populations, despite the best-known efforts to contain the pathogen and the infection. Additionally, recurrent outbreaks are possible. In some instances, "silent" colonization persists for years.

Colonization may be associated with transmission as well as increased risk of infection. CRE and vancomycin-resistant enterococcus (VRE) colonization are associated with increased risk of infection. The relative risk of infection is higher in VRE and CRE colonized patients compared to non-colonized patients. There is also a much higher risk of bloodstream infection (BSI) with colonization by extended-spectrum beta-lactamase (ESBL) producing organisms. Furthermore, colonization is highly associated with spread of hospital acquired infections (HAI). CRE, VRE and C. difficile infections are associated with being acquired after receiving healthcare (e.g., central line-associated bloodstream infections (CLABSI), catheter-associated urinary tract Infection (CAUTI), and C. difficile infections (CDI)).

Existing decolonization regimens do not last and potentially serve to increase resistance. Furthermore, worries about resistance prevents widespread adoption. For some bacteria and some patients, no effective treatment options are available, and some last line of defense antibiotics are associated with unwanted side effects and toxicities. There is a need for additional approaches for clinical management of these infections.

### SUMMARY OF THE INVENTION

Provided herein are methods of treating an adverse effect of, reducing the acquisition of, or reducing the reservoir of a pathogen (e.g., a drug or antibiotic resistant pathogen, or an MDR pathogen) on a facility participant in a facility, In one aspect, the present invention features treating an adverse effect of a pathogen (e.g., a drug or antibiotic resistant pathogen, or an MDR pathogen) on a first facility participant in a facility. In some embodiments, the facility participant is a first facility participant or a second facility participant. In some embodiments, the method comprises administering to one or both, the first facility-participant and a second facility participant, an amount of a glycan preparation effective to reduce, prevent, or reduce the risk of, the adverse effect of the pathogen on the first facility participant, thereby reducing, preventing, or reducing the risk of an adverse effect of a pathogen on the first facility participant.

In another aspect, the present invention features a method of reducing the acquisition by a first facility participant of a pathogen, e.g., a drug or antibiotic resistant pathogen, or an MDR pathogen, in a facility. In some embodiments, the method comprises administering to a second facility participant an amount of a glycan preparation effective to reduce the acquisition of the pathogen by the first facility participant, thereby reducing the acquisition by a first facility participant of the pathogen in a facility.

In yet another aspect, the present invention features a method of reducing the reservoir of a pathogen, e.g., a drug or antibiotic resistant pathogen, or an MDR pathogen, in a facility. In some embodiments, the method comprises administering to a facility-participant an amount of a glycan preparation effective to reduce the acquisition of a pathogen, e.g., a drug or antibiotic resistant pathogen, or an MDR pathogen, by a facility-participant, thereby reducing the reservoir of a MDR organism in a facility.

In still another aspect, the present invention features a method of monitoring or evaluating any of a) pathogen load, b) antibiotic resistance gene load and c) response to a therapeutic preparation in a facility participant comprising: acquiring a value (e.g., by analyzing a suitable sample, e.g., a fecal sample from the facility participant) for a) pathogens, and/or b) antibiotic resistance genes and/or microbiome analysis (e.g. the presence of one or more microbes related to response to a particular therapeutic preparation), thereby monitoring or evaluating the facility participant.

In any and all aspects, in some embodiments, the glycan preparation is administered in an effective amount and/or to a sufficient number of facility-participant(s) to reduce the spread of the pathogen, e.g., from a first facility participant to a second facility participant. In some embodiments, the glycan preparation is administered in an effective amount and/or to a sufficient number of facility participant(s) to reduce the reservoir of pathogen. In some embodiments, the glycan preparation is administered in an effective amount and/or to a sufficient number of facility-participant(s) to reduce the reservoir of drug- or antibiotic-resistance gene, or a MDR gene element. In some embodiments, the glycan preparation is administered in an effective amount and/or to a sufficient number of facility-participant(s) to reduce the spread of drug- or antibiotic-resistance gene, or a MDR element, e.g., from the first facility participant to the second facility participant.

In some embodiments, the glycan preparation is administered in an effective amount and/or to a sufficient number of facility-participant(s) to reduce the rate at which a pathogen causes infection. In some embodiments, the glycan preparation is administered in an effective amount and/or to a sufficient number of facility-participant(s) to reduce the severity of pathogen infection.

In some embodiments, the glycan preparation is administered in an effective amount and/or to a sufficient number of facility-participant(s) to reduce the rate at which a drug- or antibiotic-resistance gene, or an MDR element, is transferred from a donor microbe (e.g., a first pathogen) to a recipient microbe (e.g., a second pathogen or commensal microbe), optionally wherein the microbe is a bacterial taxa. In some embodiments, the glycan preparation is administered in an effective amount and/or to a sufficient number of facility-participant(s) to reduce the expression and/or release by the pathogen of a factor having an adverse effect on the facility participant, e.g., a virulence factor or a toxin, e.g., that causes disease. In some embodiments, the glycan preparation is administered in an effective amount and/or to a sufficient number of facility-participant(s) to reduce dysbiosis of the microbiota in the GI tract (e.g., small intestine, large intestine or colon) of the facility participant by the pathogen.

In some embodiments, the glycan preparation is administered in an effective amount and/or to a sufficient number of facility-participant(s) to reduce the spread of the pathogen, e.g., from a first facility participant to an entity which can harbor the pathogen (e.g., another individual or an inanimate object, e.g., facility built surface (e.g. sink, door handle, toilet, faucet) or medical supply (e.g., a package comprising a dressing or device, or a dressing or device itself). In some embodiments, the glycan preparation is administered in an effective amount and/or to a sufficient number of facility-participant(s) to reduce the spread of the pathogen, e.g., from a second facility participant to an entity which can harbor the pathogen (e.g., another individual or an inanimate object, e.g., a facility built surface (e.g. sink, door handle, toilet, faucet) or medical supply (e.g., a package comprising a dressing or device, or a dressing or device itself).

In some embodiments, the first facility participant is a patient or resident of the facility. In some embodiments, the first facility participant is other than a patient or resident of the facility. In some embodiments, the first facility participant is a patient or resident of the facility and the second facility participant is a patient or resident of the facility. In some embodiments, the first facility participant is a patient or resident of the facility and the second facility participant is other than a patient or resident of the facility. In some embodiments, the first facility participant is other than a patient or resident of the facility and the second facility participant is a patient or resident of the facility. In some embodiments, the first facility participant is other than a patient or resident of the facility and the second facility participant is other than a patient or resident of the facility.

In some embodiments, the entity which can harbor the pathogen is another individual.

In some embodiments, the entity which can harbor the pathogen is an inanimate object, e.g., a facility-built surface (e.g. sink, door handle, toilet, faucet) or a medical supply (e.g., a package comprising a dressing or device, or a dressing or device itself).

In some embodiments, the method comprises administering the effective glycan preparation to the first facility participant. In some embodiments, the method comprises administering the effective glycan preparation to the second facility participant. In some embodiments, the method comprises administering the effective glycan preparation to the first facility participant and to the second facility participant.

In some embodiments, the effective glycan preparation administered to the first facility participant and to the second facility participant is the same. In some embodiments, the effective glycan preparation administered to the first facility participant and to the second facility participant is different, e.g., different in dosage or chemical composition.

In some embodiments, the effective glycan preparation is administered to the first facility participant and to the second facility participant on the same regimen, e.g., for the same number of days. In some embodiments, the effective glycan preparation is administered to the first facility participant and to the second facility participant on a different regimen, e.g., for a different number of days.

In some embodiments, the effective glycan preparation is administered to a facility participant (e.g., a facility participant who is a patient, resident or staff of the facility) prior to entry or admission to the facility. In some embodiments, the effective glycan preparation is administered to a facility participant (e.g., a facility participant who is a patient, resident or staff of the facility) while at the facility. In some embodiments, the effective glycan preparation is administered to a facility participant (e.g., a facility participant who is a patient, resident or staff of the facility) after leaving the facility.

In some embodiments, independently, the first and second facility participant is selected from: a) a patient or resident; b) an individual who is a medical care giver, e.g., a medical practitioner, e.g., a physician or nurse, c) a housekeeping worker; d) a security worker (e.g. a guard); e) a maintenance worker; f) a food preparation worker; g) a laundry worker; h) an administrative worker, e.g., an admissions worker; i) a social worker; j) a visitor or guest; k) a facility employee not of (b)-(i) (e.g., a teacher, a soldier, a sailor, an officer); 1) an individual of b)-k) who has direct contact with the first facility participant of (a), a patient or resident; m) an individual of b)-k) who does not have direct contact with the first facility participant of (a), a patient or resident.

In some embodiments, the method comprises administering a glycan preparation to an individual from a plurality of the classes a-m, e.g., at least 2, 3, 4, 5, 6, 7, 8, 9, 10, 11, 12, or all of classes a-m. In some embodiments, the method comprises at least 50, 60, 70, 80, 90, or 95%, or all of the individuals in a class of a-m are administered a glycan preparation. In some embodiments, the method comprises at least 50, 60, 70, 80, 90, or 95 %, or all of the individuals from a plurality of the classes a-m, e.g., at least 2, 3, 4, 5, 6, 7, 8, 9, 10, 11, 12, or all of classes am are administered a glycan preparation.

In some embodiments, a facility participant, is infected with a pathogen, optionally, wherein the facility participant is asymptomatic (e.g., displays no detectable or diagnosable signs of infection). In some embodiments, the reservoir (e.g., pathogen reservoir or resistance gene reservoir) in the first facility participant is reduced. In some embodiments, the reservoir (e.g., pathogen reservoir or resistance gene reservoir) in the second facility participant is reduced. In some embodiments, the reservoir (e.g., pathogen reservoir or resistance gene reservoir) is reduced in a plurality of facility participants.

In some embodiments, the donor microbe and the recipient microbe are from the same taxa (e.g., genus, species, or strain). In some embodiments, the donor microbe and the recipient microbe are from a different taxa (e.g., genus, species, or strain). In some embodiments, the taxa is one or more of: Enterobacteriaciae (e.g., a genus comprising Plesiomonas, Shigella, or Salmonella), Clostridium (e.g., a genus comprising Clostridium difficile), Enterococcus, and Staphylococcus (e.g., a genus comprising Staphylococcus aureus).

In some embodiments, the recipient microbe is more pathogenic, or has additional or more severe adverse effects on a facility participant (e.g. the first facility participant) compared to the donor microbe. In some embodiments, the donor microbe is more pathogenic, or has additional or more severe adverse effects on a facility participant (e.g. the first facility participant) compared to the recipient microbe.

In some embodiments, the adverse effect (e.g., on the first facility participant) is an infection (e.g. a bacterial infection or bacteremia).

In some embodiments, the infection is a bloodstream infection, a UTI, or a respiratory infection.

In some embodiments, the virulence factor or toxin is one of: Shiga toxin, *E*. *coli* heat labile toxin, and *Clostridium difficile* Toxin A and B.

In some embodiments, the drug- or antibiotic-resistance gene, or an MDR element is one of: MecA, KPC, NDM, OXA, SHV, TIM, CTX-M, VIM, AmpC, VanA, VanB, fluoroquinoline resistance genes (e.g., Qnr), trimethoprim resistance genes (e.g. dihydrofolate reductase), sulfamethoxazole resistance genes (e.g., dihydropteroate synthetase), ciprofloxacin resistance genes, and aminoglycoside resistance genes (e.g., ribosomal methyltransferase).

In some embodiments, the glycan preparation is administered in an amount effective to reduce the level of the pathogen in the gut (e.g., small intestine, large intestine or colon) of a facility-participant.

In some embodiments, the glycan preparation is administered in an amount effective to modulate (e.g. reduce or inhibit) colonization, or modulate (e.g. increase) decolonization, by the pathogen in a facility-participant, e.g., the first and/or second faculty-participant. In some embodiments, treating comprises reducing the risk of an adverse effect of the pathogen on the first facility participant, the second facility participant, or both.

In some embodiments, the glycan preparation is administered in an amount effective to: a) modulate (e.g., reduce) pathogen biomass is modulated (e.g., the number of pathogens and/or the number of drug- or antibiotic-resistance gene or MDR element carriers); b) modulate (e.g., increase) the level of anti-microbial compounds produced by the facility participant (e.g., by the resident gut microbiota and/or the host (e.g., human cells)); c) modulate the environment of the GI tract (e.g., small intestine, large intestine or colon), e.g. reducing the pH (e.g., by increasing production or levels of lactic acid, e.g. produced by the resident gut microbiota); d) modulate (e.g., reduce) the state of competency or a conjugation property of a donor microbe of a drug- or antibiotic-resistance gene or MDR element; e) modulate (e.g., reduce) the number of drug- or antibiotic-resistance gene or MDR element recipients; f) modulate (e.g., reduce) the copy number of a drug- or antibiotic-resistance gene or MDR element (e.g. total copy number, e.g. in a donor microbe); and/or g) modulate (e.g., increase) the fitness deficit (e.g., increase the burden of carrying a drug- or antibiotic-resistance gene or MDR element), in the first and/or second facility participant or a plurality of facility participant(s).

In some embodiments, the glycan preparation is administered in an amount effective to: a) decrease in the resident gut microbiota the abundance (e.g. total number or relative number) of pathogens and/or drug- or antibiotic-resistance gene or MDR element carriers; and/or b) increase in the resident gut microbiota the abundance (e.g. total number or relative number) commensals or beneficial bacteria.

In some embodiments, the glycan preparation is administered in an amount effective to: increase in the resident gut microbiota the abundance (e.g. total number or relative number) commensals or beneficial bacteria, thereby reducing the area of colonizable space for the pathogen. In some embodiments, the glycan preparation is administered in an amount effective to: increase in the resident gut microbiota the abundance (e.g. total number or relative number) commensals or beneficial bacteria, thereby increasing the levels of anti-microbial defense compounds, e.g. bacteriocins, AMPs (anti-microbial peptides), hydrogen peroxide, or acetate (low pH).

In some embodiments, the pathogen is a bacterium. In some embodiments, the pathogen is a drug or antibiotic resistant pathogen, e.g., bacterium. In some embodiments, the pathogen is a multiply drug resistant (MDR) carrying pathogen, e.g., bacterium. In some embodiments, the pathogen is a vancomycin resistant enterococcus (VRE), carbapenem resistant (CRE) Enterobacteriaceae (e.g. *E. coli, Klebsiella, Enterobacter, Proteus*)*.*

In some embodiments, the first and/or second facility participant: i) has received cancer treatment; ii) is a transplant recipient, e.g., a hematopoietic stem cell recipient; iii) has received immunosuppression, and/or iv) has an auto-immune disease (e.g., systemic lupus erythematosus, rheumatoid arthritis, Sjögren's syndrome, or Crohn's disease).

In some embodiments, the first and/or second facility participant has cystic fibrosis.

In some embodiments, the first and/or second facility participant has a defect of the immune system, e.g., i) an acquired defect, e.g., HIV/AIDS, or ii) a hereditary or congenital defect, e.g., SCID, CVID, Bruton's agammaglobulinemia, or an auto-immune disease (e.g., systemic lupus erythematosus, rheumatoid arthritis, Sjögren's syndrome, or Crohn's disease).

In some embodiments, the first and/or second facility participant has a chronic disorder or disease.

In some embodiments, the first and/or second facility participant is i) an infant or ii) an elderly adult. In some embodiments, the facility participant is more than 45, 50, 55, 60, 65, 70, 75, or 80 years of age. In some embodiments, the facility participant is less than 1, 2, 3, 6, 12, 24, or 36 months of age. In some embodiments, the first and/or second facility participant is immune compromised or has received treatment that reduces immune function. In some embodiments, the first and/or second facility participant is, has been, or will be administered dialysis treatment.

In some embodiments, the first and/or second facility participant is, has been, or will be administered treatment that is invasive, e.g., breaks the skin, e.g., surgery or the insertion or implantation of a device (e.g., catheter or stent), or connection to a ventilator, drip line, intrusive monitor or food applicator (orally, rectally, enterically, IV, etc.).

In some embodiments, the facility comprises a hospital, a clinic, a rehabilitation facility, a mental health facility, an intensive care facility (ICU), a neonatal facility, a cancer treatment facility, a nursing facility, a drug-treatment facility, a training facility, a clinical trial facility, an inpatient facility, an outpatient facility. In some embodiments, the facility comprises a long-term care facility, e.g., a home for the elderly. In some embodiments, the facility comprises a prison or other correctional or penal facility. In some embodiments, the facility comprises a ship, e.g., a cruise ship or military vessel. In some embodiments, the facility comprises a military facility, an athletic facility, an educational facility (school, camp), or a leisure facility (e.g., hotel or resort).

In some embodiments, the method further comprises administering to a facility participant, a second treatment, e.g., an antibiotic.

In some embodiments, the glycan preparation is administered to the facility participant prior to working at the facility and/or while working at the facility. In some embodiments, the glycan preparation is administered to the facility participant i) prior to entering the facility, ii) while at the facility, iii) at or after release from the facility, or any combination of (i), (ii), and (iii).

In some embodiments, the method comprises acquiring a level of a pathogen (e.g., a drug or antibiotic resistant pathogen, or an MDR pathogen) from the facility-participant (e.g., by analyzing a sample of the facility participant), optionally, repeating the acquisition two, three, four, or more times.

In some embodiments, the glycan preparation comprises: i) glycan polymers that comprise glucose, galactose, arabinose, mannose, fructose, xylose, fucose, or rhamnose glycan units; ii) the average degree of branching (DB) of the glycan polymers in the glycan preparation is 0, between 0.01 and 0.6, between 0.05 and 0.5, between 0.1 and 0.4, or between 0.15 and 0.4; iii) at least 50% (at least 60%, 65%, 70%, 75%, 80%, or 85%, or less than 50%) of the glycan polymers in the glycan preparation have a degree of polymerization (DP) of at least 3 and less than 30 glycan units, at least 3 and less than 10 glycan units, at least 5 and less than 25 glycan units, or at least 10 and less than 35 glycan units; iv) the average DP (mean DP) of the glycan preparation is between about 5 and 8, between about 8 and 13, between about 13 and 25, between about 5 and 15, between about 5 and 20, or between about 5-15; v) the ratio of alpha- to beta-glycosidic bonds present in the glycan polymers of the glycan preparation is 0, or between about 0.8:1 to about 5:1, between about 1:1 to about 5:1, between about 1:1 to about 3:1, between about 3:2 to about 2:1, or between about 3:2 to about 3:1, vi) the glycan preparation comprises between 15 mol % and 75 mol % (between 20 mol % and 60 mol %, between 25 mol % and 50 mol %, or between 30 mol % and 45 mol %) 1,6 glycosidic bonds; vii) the glycan preparation comprises between 1 mol % and 40 mol % (between 1 mol % and 30 mol %, between 5 mol % and 25 mol %, between 10 mol % and 20 mol %) of at least one, two, or three of 1,2; 1,3; and 1,4 glycosidic bonds; viii) the glycan preparation has a final solubility limit in water of at least about 50 (at least about 60, 70, at least about 75, or less than 50) Brix at 23 °C; or ix) the glycan preparation has a dietary fiber content of at least 50% (at least 60%, 70%, 80%, or at least 90%, or less than 50%), x) any combination of two, three, four, five, six, seven, eight, or nine of i), ii), iii), iv), v), vi), vii), viii), and ix).

In some embodiments, the DB is 0, between 0.01 and 0.05, 0.01 and 0.15, 0.01 and 0.2, 0.05 and 0.2, 0.1 and 0.3, 0.1 and 0.4, 0.1 and 0.5, 0.1 and 0.6, 0.1 and 0.7, 0.2 and 0.5, 0.15 and 0.65, or between 0.4 and 0.75. In some embodiments, the DB is less than 0.35. In some embodiments, the DB is at least 0.35. In some embodiments, the DB is between 0.1 and 0.3. In some embodiments, the DB is between 0.3 and 0.6. In some embodiments, the DB is between 0.01 and 0.1.

In some embodiments, at least 50% of the glycans in the glycan polymer preparation have a DP of at least 3 and less than 30 glycan units. In some embodiments, at least 60%, 65%, 70%, 75%, 80%, or 85% of the glycans in the glycan polymer preparation have a DP of at least 3 and less than 30 glycan units. In some embodiments, less than 50%, of the glycans in the glycan polymer preparation have a DP of at least 3 and less than 30 glycan units. In some embodiments, the DP is at least 3 and less than 30 glycan units, at least 3 and less than 10 glycan units, at least 3 and less than 15 glycan units, at least 3 and less than 20 glycan units, at least 3 and less than 25 glycan units, at least 4 and less than 15 glycan units, at least 4 and less than 20 glycan units, at least 4 and less than 25 glycan units, at least 5 and less than 15 glycan units, at least 5 and less than 20 glycan units, at least 5 and less than 25 glycan units, at least 5 and less than 30 glycan units, at least 6 and less than 15 glycan units, at least 6 and less than 20 glycan units, at least 6 and less than 25 glycan units, at least 6 and less than 30 glycan units, at least 8 and less than 15 glycan units, at least 8 and less than 20 glycan units, at least 8 and less than 25 glycan units, at least 8 and less than 30 glycan units, at least 10 and less than 20 glycan units, or at least 10 and less than 30 glycan units. In some embodiments, the DP is 3-8. In some embodiments, the DP is 8-13. In some embodiments, the DP is 13-25. In some embodiments, the DP is 5-25. In some embodiments, the DP is 10-35.

In some embodiments, the ratio of alpha- to beta-glycosidic bonds present in the glycan polymer preparation is from about 0.8:1 to 5:1. In some embodiments, the ratio of alpha- to beta-glycosidic bonds is between about 0.1:1 to about 4:1, 0.2:1 to about 4:1, 0.3:1 to about 4:1, 0.4:1 to about 4:1, 0.5:1 to about 4:1, 0.6:1 to about 4:1, 0.7:1 to about 4:1, 0.8:1 to about 4:1, 0.9:1 to about 4:1, 1:1 to about 2:1, 1:1 to about 3:1, 1:1 to 4:1, about 2:1 to about 4:1, 2:1 to 5:1, about 3:1 to about 5:1, or 4:1 to about 5:1. In some embodiments, the ratio of alpha- to beta-glycosidic bonds is between about 1:1 to about 5:1. In some embodiments, the ratio of alpha- to beta-glycosidic bonds is between about 1:1 to about 3:1. In some embodiments, the ratio of alpha- to beta-glycosidic bonds is between about 3:2 to about 2:1. In some embodiments, the ratio of alpha- to beta-glycosidic bonds is between about 3:2 to about 3:1.

In some embodiments, the final solubility limit in water of the glycan polymer preparation is at least 50 Brix. In some embodiments, the final solubility limit in water of the glycan polymer preparation is at least about 55, at least about 60, at least about 65, or at least about 70, or at least about 75 Brix at 23 °C. In some embodiments, the final solubility limit in water of the glycan polymer preparation is at least about 70 Brix at 23 °C. In some embodiments, the final solubility limit in water of the glycan polymer preparation is no greater than about 50, no greater than about 40, no greater than about 30, no greater than about 20, no greater than about 10, or no greater than about 5 Brix at 23 °C.

In some embodiments, the glycan polymer preparation has a total dietary fiber content of at least 50%, 60%, 70%, or at least 80% (as measured by the method AOAC 2009.01).

In some embodiments, the method further comprises administering to the first and/or second facility participant a second treatment. In some embodiments, the second treatment comprises administering an antibiotic.

In some embodiments, the facility participant is a worker or prospective worker in the facility, and responsive to the result, evaluating granting the facility participant access to the facility.

In some embodiments, the facility participant is a patient or resident, or prospective patient or resident of the facility, and responsive to the result, evaluating granting the facility participant admission to the facility.

In some embodiments, the facility participant is a patient or resident, or prospective patient or resident of the facility, and responsive to the result evaluating the risk of acquiring a pathogen, optionally, evaluating further treatment and/or changes or adjustments to the treatment regimen or care.

In another aspect, the invention is directed to a method for managing infections in a subject, e.g., a human subject, comprising:
administering a glycan preparation in an amount effective and for a time sufficient to treat the infection, wherein the glycan preparation comprises:
i) glycan polymers that comprise glucose, galactose, arabinose, mannose, fructose, xylose, fucose, or rhamnose glycan units;
ii) the average degree of branching (DB) of the glycan polymers in the glycan preparation is between 0.05 and 0.5;
iii) at least 50% of the glycan polymers in the glycan preparation have a degree of polymerization (DP) of at least 3 and less than 30 glycan units;
iv) the average DP (mean DP) of the glycan preparation is between about 5 and 20;
v) the ratio of alpha- to beta-glycosidic bonds present in the glycan polymers of the glycan preparation is between about 0.8:1 to about 5:1;
vi) the glycan preparation comprises between 15 mol % and 75 mol % 1,6 glycosidic bonds;
vii) the glycan preparation comprises between 1 mol % and 30 mol % of at least one, two, or three of 1,2; 1,3; and 1,4 glycosidic bonds;
viii) the glycan preparation has a final solubility limit in water of at least about 70 Brix at 23 °C; and/or
ix) the glycan preparation has a dietary fiber content of at least 70%.

In some embodiments, the glycan preparation comprises two, three, four, five, six, seven, eight, or nine of the selected properties of i), ii), iii), iv), v), vi), vii), viii), and ix). Optionally, the glycan preparation supports the growth of commensal or probiotic bacteria, *e.g*., in a gut microbiome, further optionally, the glycan preparation does not support the growth of at least one pathogen, *e.g*., does not support the growth of a carbapenem resistant (CRE) Enterobacteriaceae (e.g. *E*. *coli, Klebsiella, Enterobacter, Proteus,* e.g., extended spectrum beta lactamase (ESBL) producing Enterobacteriaciae)), vancomycin resistant enterococcus (VRE), and/or C. difficile taxa, further optionally, the glycan preparation does not support the growth of at least two pathogen, *e.g*., does not support the growth of a CRE, VRE, and/or C. difficile taxa; further optionally, the glycan preparation does not support the growth of CRE; further optionally, the glycan preparation does not support the growth VRE, further optionally, the glycan preparation does not support the growth of C. difficile.

In some embodiments, the human subject is a transplant recipient, e.g., a hematopoietic stem cell (HSCT) recipient or liver transplant recipient. In some embodiments, the human subject is colonized with VRE. In some embodiments, the human subject has received cancer treatment. In some embodiments, the human subject has received immunosuppression. In some embodiments, the human subject is at high-risk for bacterial infection (e.g., due to pre-transplant immune system ablation). In some embodiments, the human subject has end-stage liver disease (ESDL). In some embodiments, the human subject is on a liver transplant waiting list. In some embodiments, the human subject is at risk of being delisted from the transplant list because of an infection. In some embodiments, the human subject develops bacteremia. In some embodiments, the human subject is in an ICU facility.

In some embodiments, the method reduces the rate of infections (e.g., from pathogens that colonize the GI tract), e.g., in critically ill or high-risk subjects. In some embodiments, the method comprises reducing the rate of urinary tract infections. In some embodiments, the method comprises reducing the rate of bloodstream infections. In some embodiments, the method comprises reducing the rate of respiratory tract infections.

In some embodiments, the method comprises managing infections in subjects, e.g. of subject groups with infections (bacteremia): subjects with urinary infections (e.g., infected with Enterococcus, Enterobacteriaciae), subjects with bloodstream infections (e.g., infected with Enterococcus, Enterobacteriaciae), transplant subjects (e.g., bone marrow (e.g., undergoing hematopoietic stem cell transplantation), solid organ (e.g., liver)), intensive care patients (e.g., infected with Carbapenem resistant Enterobacteriaciae and ESBL producing pathogens), pre-transplant liver failure patients (e.g., infected with Vancomycin resistant Enterococcus), post-transplant liver failure patients (e.g., infected with Vancomycin resistant Enterococcus), subjects undergoing chemotherapy with high levels of enteric pathogen bacteremia, *C*. *difficile* infection (CDI)*C*, and chemotherapy-induced diarrhea compared to other subjects (e.g., the general hospital patient population), antibiotic-treated subjects, subjects undergoing or about to undergo a transplant, subjects with cancer, subjects with liver disease (e.g., end-stage renal disease), or subjects with suppressed immune system (*e.g*., immunocompromised subjects), subjects with hepatic encephalopathy (HE). In some embodiments, the method comprises prophylactic treatment, *e.g*., with a glycan preparation, of a subject, *e.g.,* a subject with a high risk of developing an infection. In some embodiments, subjects who are undergoing chemotherapy or antibiotic treatment have reduced diversity of commensal bacteria. In some embodiments, the method comprises managing infections in subjects who are in need of an organ transplant, *e.g.,* a liver or bone marrow transplant. In some embodiments, the method comprises managing infections in subjects immediately, or shortly, before said subject receives an organ transplant, *e.g.,* a liver or bone marrow transplant. In some embodiments, the method comprises managing infections in subjects immediately, or shortly, after said subject receives an organ transplant, *e.g.,* a liver or bone marrow transplant. In some embodiments, the method comprises managing infections in subjects who have, are suspected of having, or at risk of having end-stage liver disease (ESLD). In some embodiments, the method comprises treatment of a subject to reduce the colonization of pathogens, *e.g.,* multidrug resistant pathogens, in a subject. In some embodiments, the method comprises treatment of a subject to reduce the transmission of pathogens, *e.g.,* multidrug resistant pathogens, from a first subject to a second subject. In some embodiments, bacteria that pose a risk of colonization in subjects (or a capable of colonizing the GI tract of subjects) comprise resistant subpopulations of Enterobacteriaceae (e.g., *E*. *cloacae* and Enterococcus), C. difficile (including Nap1 (pandemic hypervirulent) *C*. *difficile* strain), and bacteria that cause infectious diarrhea (e.g., *Campylobacter, Salmonella, Shigella,* enterohemorrhagic *E. coli* (EHEC), enterotoxigenic *E. coli* (ETEC), enteropathogenic *E*. *coli* (EPEC), enteroinvasive *E*. *coli* (EIEC), enteroaggregative *E*. *coli* (EAEC), diffusely adherent *E*. *coli* (DAEC), and uropathogenic E. coli).

In another aspect, the invention is directed to a method for managing infections in a subject, e.g., a human subject, comprising:
administering a glycan preparation in an amount effective and for a time sufficient to treat the infection, wherein the glycan preparation comprises:
i) glycan polymers that comprise glucose, galactose, or mannose glycan units;
ii) the average degree of branching (DB) of the glycan polymers in the glycan preparation is between 0.05 and 0.5;
iii) at least 50% of the glycan polymers in the glycan preparation have a degree of polymerization (DP) at least 3 and less than 30 glycan units;
iv) the average DP (mean DP) of the glycan preparation is between about 5 and 15;
v) the ratio of alpha- to beta-glycosidic bonds present in the glycan polymers of the glycan preparation is between about 0.8:1 to about 5:1;
vi) the glycan preparation comprises between 15 mol % and 75 mol % 1,6 glycosidic bonds;
vii) the glycan preparation comprises between 1 mol % and 30 mol % of at least one, two, or three of 1,2; 1,3; and 1,4 glycosidic bonds;
viii) the glycan preparation has a final solubility limit in water of at least about 70 Brix at 23 °C; and/or
ix) the glycan preparation has a dietary fiber content of at least 70%.

In some embodiments, the glycan preparation comprises two, three, four, five, six, seven, eight, or nine of the selected properties of i), ii), iii), iv), v), vi), vii), viii), and ix).

Optionally, the glycan preparation supports the growth of commensal or probiotic bacteria, *e.g.,* in a gut microbiome, further optionally, the glycan preparation does not support the growth of at least one pathogen, *e.g.,* does not support the growth of a carbapenem resistant (CRE) Enterobacteriaceae (e.g. *E. coli, Klebsiella, Enterobacter, Proteus,* e.g., extended spectrum beta lactamase (ESBL) producing Enterobacteriaciae)), vancomycin resistant enterococcus (VRE), and/or C. difficile taxa, further optionally, the glycan preparation does not support the growth of at least two pathogen, e.g., does not support the growth of a CRE, VRE, and/or C. difficile taxa; further optionally, the glycan preparation does not support the growth of CRE; further optionally, the glycan preparation does not support the growth VRE, further optionally, the glycan preparation does not support the growth of C. difficile.

In some embodiments, the human subject is a transplant recipient, e.g., a hematopoietic stem cell (HSCT) recipient or liver transplant recipient. In some embodiments, the human subject is colonized with VRE. In some embodiments, the human subject has received cancer treatment. In some embodiments, the human subject has received immunosuppression. In some embodiments, the human subject is at high-risk for bacterial infection (e.g., due to pre-transplant immune system ablation). In some embodiments, the human subject has end-stage liver disease (ESDL). In some embodiments, the human subject is on a liver transplant waiting list. In some embodiments, the human subject is at risk of being delisted from the transplant list because of an infection. In some embodiments, the human subject develops bacteremia. In some embodiments, the human subject is in an ICU facility.

In some embodiments, the method reduces the rate of infections (e.g., from pathogens that colonize the GI tract), e.g., in critically ill or high-risk subjects. In some embodiments, the method comprises reducing the rate of urinary tract infections. In some embodiments, the method comprises reducing the rate of bloodstream infections. In some embodiments, the method comprises reducing the rate of respiratory tract infections.

In some embodiments, the method comprises managing infections in subjects, e.g. of subject groups with infections (bacteremia): subjects with urinary infections (e.g., infected with Enterococcus, Enterobacteriaciae), subjects with bloodstream infections (e.g., infected with Enterococcus, Enterobacteriaciae), transplant subjects (e.g., bone marrow (e.g., undergoing hematopoietic stem cell transplantation), solid organ (e.g., liver)), intensive care patients (e.g., infected with Carbapenem resistant Enterobacteriaciae and ESBL producing pathogens), pre-transplant liver failure patients (e.g., infected with Vancomycin resistant Enterococcus), post-transplant liver failure patients (e.g., infected with Vancomycin resistant Enterococcus), subjects undergoing chemotherapy with high levels of enteric pathogen bacteremia, *C. difficile* infection (CDI)C, and chemotherapy-induced diarrhea compared to other subjects (e.g., the general hospital patient population), antibiotic-treated subjects, subjects undergoing or about to undergo a transplant, subjects with cancer, subjects with liver disease (e.g., end-stage renal disease), or subjects with suppressed immune system (*e.g.*, immunocompromised subjects), subjects with hepatic encephalopathy (HE). In some embodiments, the method comprises prophylactic treatment, *e.g.,* with a glycan preparation, of a subject, *e.g.,* a subject with a high risk of developing an infection. In some embodiments, subjects who are undergoing chemotherapy or antibiotic treatment have reduced diversity of commensal bacteria. In some embodiments, the method comprises managing infections in subjects who are in need of an organ transplant, *e.g.*, a liver or bone marrow transplant. In some embodiments, the method comprises managing infections in subjects immediately, or shortly, before said subject receives an organ transplant, *e.g.,* a liver or bone marrow transplant. In some embodiments, the method comprises managing infections in subjects immediately, or shortly, after said subject receives an organ transplant, *e.g.,* a liver or bone marrow transplant. In some embodiments, the method comprises managing infections in subjects who have, are suspected of having, or at risk of having end-stage liver disease (ESLD). In some embodiments, the method comprises treatment of a subject to reduce the colonization of pathogens, *e.g.,* multidrug resistant pathogens, in a subject. In some embodiments, the method comprises treatment of a subject to reduce the transmission of pathogens, *e.g.,* multidrug resistant pathogens, from a first subject to a second subject. In some embodiments, bacteria that pose a risk of colonization in subjects (or a capable of colonizing the GI tract of subjects) comprise resistant subpopulations of Enterobacteriaceae (e.g., *E. cloacae* and Enterococcus), C. difficile (including Napl (pandemic hypervirulent) *C*. *difficile* strain), and bacteria that cause infectious diarrhea (e.g., *Campylobacter, Salmonella, Shigella,* enterohemorrhagic *E. coli* (EHEC), enterotoxigenic *E. coli* (ETEC), enteropathogenic *E. coli* (EPEC), enteroinvasive *E. coli* (EIEC), enteroaggregative *E. coli* (EAEC), diffusely adherent *E*. *coli* (DAEC), and uropathogenicE. coli).

In another aspect, the invention is directed to a method for managing infections in a subject, e.g., a human subject, comprising:
administering a glycan preparation in an amount effective and for a time sufficient to treat the infection, wherein the glycan preparation comprises:
i) glycan polymers that comprise glucose or galactose glycan units;
ii) the average degree of branching (DB) of the glycan polymers in the glycan preparation is between 0.1 and 0.4;
iii) at least 50% of the glycan polymers in the glycan preparation have a degree of polymerization (DP) at least 3 and less than 10 glycan units;
iv) the average DP (mean DP) of the glycan preparation is between about 5 and 8;
v) the ratio of alpha- to beta-glycosidic bonds present in the glycan polymers of the glycan preparation is between about 1:1 to about 3:1;
vi) the glycan preparation comprises between 20 mol % and 60 mol % 1,6 glycosidic bonds;
vii) the glycan preparation comprises between 5 mol % and 25 mol % of at least one, two, or three of 1,2; 1,3; and 1,4 glycosidic bonds;
viii) the glycan preparation has a final solubility limit in water of at least about 70 Brix at 23 °C; and/or
ix) the glycan preparation has a dietary fiber content of at least 70%.

In some embodiments, the glycan preparation comprises two, three, four, five, six, seven, eight, or nine of the selected properties of i), ii), iii), iv), v), vi), vii), viii), and ix).

Optionally, the glycan preparation supports the growth of commensal or probiotic bacteria, *e.g.,* in a gut microbiome, further optionally, the glycan preparation does not support the growth of at least one pathogen, *e.g.,* does not support the growth of a carbapenem resistant (CRE) Enterobacteriaceae (e.g. *E*. *coli, Klebsiella, Enterobacter, Proteus,* e.g., extended spectrum beta lactamase (ESBL) producing Enterobacteriaciae)), vancomycin resistant enterococcus (VRE), and/or C. difficile taxa, further optionally, the glycan preparation does not support the growth of at least two pathogen, e.g., does not support the growth of a CRE, VRE, and/or C. difficile taxa; further optionally, the glycan preparation does not support the growth of CRE; further optionally, the glycan preparation does not support the growth VRE, further optionally, the glycan preparation does not support the growth of C. difficile.

In some embodiments, the human subject is a transplant recipient, e.g., a hematopoietic stem cell (HSCT) recipient or liver transplant recipient. In some embodiments, the human subject is colonized with VRE. In some embodiments, the human subject has received cancer treatment. In some embodiments, the human subject has received immunosuppression. In some embodiments, the human subject is at high-risk for bacterial infection (e.g., due to pre-transplant immune system ablation). In some embodiments, the human subject has end-stage liver disease (ESDL). In some embodiments, the human subject is on a liver transplant waiting list. In some embodiments, the human subject is at risk of being delisted from the transplant list because of an infection. In some embodiments, the human subject develops bacteremia. In some embodiments, the human subject is in an ICU facility.

In some embodiments, the method reduces the rate of infections (e.g., from pathogens that colonize the GI tract), e.g., in critically ill or high-risk subjects. In some embodiments, the method comprises reducing the rate of urinary tract infections. In some embodiments, the method comprises reducing the rate of bloodstream infections. In some embodiments, the method comprises reducing the rate of respiratory tract infections.
In some embodiments, the method comprises managing infections in subjects, e.g. of subject groups with infections (bacteremia): subjects with urinary infections (e.g., infected with Enterococcus, Enterobacteriaciae), subjects with bloodstream infections (e.g., infected with Enterococcus, Enterobacteriaciae), transplant subjects (e.g., bone marrow (e.g., undergoing hematopoietic stem cell transplantation), solid organ (e.g., liver)), intensive care patients (e.g., infected with Carbapenem resistant Enterobacteriaciae and ESBL producing pathogens), pre-transplant liver failure patients (e.g., infected with Vancomycin resistant Enterococcus), post-transplant liver failure patients (e.g., infected with Vancomycin resistant Enterococcus), subjects undergoing chemotherapy with high levels of enteric pathogen bacteremia, *C*. *difficile* infection (CDI)C, and chemotherapy-induced diarrhea compared to other subjects (e.g., the general hospital patient population), antibiotic-treated subjects, subjects undergoing or about to undergo a transplant, subjects with cancer, subjects with liver disease (e.g., end-stage renal disease), or subjects with suppressed immune system (*e.g*., immunocompromised subjects), subjects with hepatic encephalopathy (HE). In some embodiments, the method comprises prophylactic treatment, *e.g*., with a glycan preparation, of a subject, *e.g.,* a subject with a high risk of developing an infection. In some embodiments, subjects who are undergoing chemotherapy or antibiotic treatment have reduced diversity of commensal bacteria. In some embodiments, the method comprises managing infections in subjects who are in need of an organ transplant, *e.g.,* a liver or bone marrow transplant. In some embodiments, the method comprises managing infections in subjects immediately, or shortly, before said subject receives an organ transplant, *e.g.,* a liver or bone marrow transplant. In some embodiments, the method comprises managing infections in subjects immediately, or shortly, after said subject receives an organ transplant, *e.g.,* a liver or bone marrow transplant. In some embodiments, the method comprises managing infections in subjects who have, are suspected of having, or at risk of having end-stage liver disease (ESLD). In some embodiments, the method comprises treatment of a subject to reduce the colonization of pathogens, *e.g.,* multidrug resistant pathogens, in a subject. In some embodiments, the method comprises treatment of a subject to reduce the transmission of pathogens, e.g., multidrug resistant pathogens, from a first subject to a second subject. In some embodiments, bacteria that pose a risk of colonization in subjects (or a capable of colonizing the GI tract of subjects) comprise resistant subpopulations of Enterobacteriaceae (e.g., *E. cloacae* and Enterococcus), C. difficile (including Nap1 (pandemic hypervirulent) *C. difficile* strain), and bacteria that cause infectious diarrhea (e.g., *Campylobacter, Salmonella, Shigella,* enterohemorrhagic *E. coli* (EHEC), enterotoxigenic *E. coli* (ETEC), enteropathogenic *E*. *coli* (EPEC), enteroinvasive *E*. *coli* (EIEC), enteroaggregative *E*. *coli* (EAEC), diffusely adherent *E*. *coli* (DAEC), and uropathogenic E, coli).

In another aspect, the invention is directed to a method for managing infections in a subject, e.g., a human subject, comprising:
administering a glycan preparation in an amount effective and for a time sufficient to treat the infection, wherein the glycan preparation comprises:
i) glycan polymers that comprise glucose, galactose, mannose, rhamnose, fucose, xylose or arabinose glycan units;
ii) the average degree of branching (DB) of the glycan polymers in the glycan preparation is between 0.1 to 0.8 (e.g., 0.1-0.5 or 0.1-0.6);
iii) at least 50% of the glycan polymers in the glycan preparation have a degree of polymerization (DP) of at least 3 and less than 30 glycan units;
iv) the average DP (mean DP) of the glycan preparation is between about DP3 to about DP15 (e.g., mean DP of about DP5 to about DP10, about DP5 to about DP15, about DP4 to about DP 12 or about DP6 to about DP 12);
v) the ratio of alpha- to beta-glycosidic bonds present in the glycan polymers of the glycan preparation is between about 1:1 to about 4:1 (e.g., about 1:1 to about 2:1 or about 1:1 to about 3:1);
vi) the preparation comprising about 50% to about 90% alpha glycosidic bonds (e.g., about 55% to about 75%, or about 50% to about 70% alpha glycosidic bonds),
vii) the preparation comprising about 10% to about 50% beta glycosidic bonds (e.g., about 25% to about 45%, or about 30% to about 50% beta glycosidic bonds),
vii) the glycan preparation comprises between 10-70 mol % (e.g., 30-60 mol %) 1,6-glycosidic bonds (e.g., for xylose, fucose and arabinose containing glycan polymer preparation: 0-60 mol % of 1,6-glycosidic bonds, e.g. 0 mol%),
ix) the glycan preparation comprises between 1-30 mol % (e.g., 3-30 mol %) 1,2-glycosidic bonds; 1-30 mol % (e.g., 3-30 mol %) 1,3-glycosidic bonds, and 1-30 mol % (e.g., 3-30 mol %) 1,4-glycosidic bonds;
x) the glycan preparation has a final solubility limit in water of at least about 70 Brix at 23 °C;
xi) the glycan preparation has a dietary fiber content of at least 70% (as measured by the method AOAC 2009.01);
xii) the glycan preparation has a polydispersity (PD) of between about 1 and 2.8 (e.g., between about 1.1 and about 2.2);
xiii) the glycan preparation has a total furanose content of between about 1 % and about 50% (e.g., between about 5% and 30%, or between about 1% and 15%); or
xiv) any combination of two, three, four, five, six, seven, eight, nine, ten, eleven, twelve, or thirteen of i), ii), iii), iv), v), vi), vii), viii), ix), x), xi), xii), and xiii),
optionally, wherein the glycan preparation is pharmaceutical grade (e.g., manufactured under pharmaceutical GMP); or wherein the glycan polymer preparation is food grade (e.g., manufactured under food GMP); further optionally, wherein the glycan preparation is a powder (e.g., dry powder) or a syrup. In some embodiments, the glycan polymers comprise glucose glycan units. In some embodiments, the glycan preparation is a glu 100. In some embodiments, the glu 100 glycan preparation has the properties of a glu100 described in Table 4a and 4b. In some embodiments, the glycan polymers comprise glucose and galactose glycan units. In some embodiments, the glycan preparation is a glu50gal50. In some embodiments, the glu50gal50 glycan preparation has the properties of a glu50gal50 described in Table 4a and 4b. In some embodiments, the glycan polymers comprise glucose, galactose and mannose glycan units. Optionally, the glycan preparation supports the growth of commensal or probiotic bacteria, *e.g.,* in a gut microbiome, further optionally, the glycan preparation does not support the growth of at least one pathogen, *e.g*., does not support the growth of a carbapenem resistant (CRE) Enterobacteriaceae (e.g. *E. coli, Klebsiella, Enterobacter, Proteus,* e.g., extended spectrum beta lactamase (ESBL) producing Enterobacteriaciae)), vancomycin resistant enterococcus (VRE), and/or C. difficile taxa, further optionally, the glycan preparation does not support the growth of at least two pathogen, *e.g*., does not support the growth of a CRE, VRE, and/or C. difficile taxa; further optionally, the glycan preparation does not support the growth of CRE; further optionally, the glycan preparation does not support the growth VRE, further optionally, the glycan preparation does not support the growth of C. difficile.

In some embodiments, the human subject is a transplant recipient, e.g., a hematopoietic stem cell (HSCT) recipient or liver transplant recipient. In some embodiments, the human subject is colonized with VRE. In some embodiments, the human subject has received cancer treatment. In some embodiments, the human subject has received immunosuppression. In some embodiments, the human subject is at high-risk for bacterial infection (e.g., due to pre-transplant immune system ablation). In some embodiments, the human subject has end-stage liver disease (ESDL). In some embodiments, the human subject is on a liver transplant waiting list. In some embodiments, the human subject is at risk of being delisted from the transplant list because of an infection. In some embodiments, the human subject develops bacteremia. In some embodiments, the human subject is in an ICU facility.

In some embodiments, the method reduces the rate of infections (e.g., from pathogens that colonize the GI tract), e.g., in critically ill or high-risk subjects. In some embodiments, the method comprises reducing the rate of urinary tract infections. In some embodiments, the method comprises reducing the rate of bloodstream infections. In some embodiments, the method comprises reducing the rate of respiratory tract infections.

In some embodiments, the method comprises managing infections in subjects, e.g. of subject groups with infections (bacteremia): subjects with urinary infections (e.g., infected with Enterococcus, Enterobacteriaciae), subjects with bloodstream infections (e.g., infected with Enterococcus, Enterobacteriaciae), transplant subjects (e.g., bone marrow (e.g., undergoing hematopoietic stem cell transplantation), solid organ (e.g., liver)), intensive care patients (e.g., infected with Carbapenem resistant Enterobacteriaciae and ESBL producing pathogens), pre-transplant liver failure patients (e.g., infected with Vancomycin resistant Enterococcus), post-transplant liver failure patients (e.g., infected with Vancomycin resistant Enterococcus), subjects undergoing chemotherapy with high levels of enteric pathogen bacteremia, *C*. *difficile* infection (CDI)*C*, and chemotherapy-induced diarrhea compared to other subjects (e.g., the general hospital patient population), antibiotic-treated subjects, subjects undergoing or about to undergo a transplant, subjects with cancer, subjects with liver disease (e.g., end-stage renal disease), or subjects with suppressed immune system (*e.g*., immunocompromised subjects), subjects with hepatic encephalopathy (HE). In some embodiments, the method comprises prophylactic treatment, *e.g*., with a glycan preparation, of a subject, *e.g.,* a subject with a high risk of developing an infection. In some embodiments, subjects who are undergoing chemotherapy or antibiotic treatment have reduced diversity of commensal bacteria. In some embodiments, the method comprises managing infections in subjects who are in need of an organ transplant, *e.g*., a liver or bone marrow transplant. In some embodiments, the method comprises managing infections in subjects immediately, or shortly, before said subject receives an organ transplant, *e.g.,* a liver or bone marrow transplant. In some embodiments, the method comprises managing infections in subjects immediately, or shortly, after said subject receives an organ transplant, *e.g*., a liver or bone marrow transplant. In some embodiments, the method comprises managing infections in subjects who have, are suspected of having, or at risk of having end-stage liver disease (ESLD). In some embodiments, the method comprises treatment of a subject to reduce the colonization of pathogens, *e.g*., multidrug resistant pathogens, in a subject. In some embodiments, the method comprises treatment of a subject to reduce the transmission of pathogens, *e.g*., multidrug resistant pathogens, from a first subject to a second subject. In some embodiments, bacteria that pose a risk of colonization in subjects (or a capable of colonizing the GI tract of subjects) comprise resistant subpopulations of Enterobacteriaceae (e.g., *E*. *cloacae* and Enterococcus), C. difficile (including Nap1 (pandemic hypervirulent) *C*. *difficile* strain), and bacteria that cause infectious diarrhea (e.g., *Campylobacter, Salmonella, Shigella,* enterohemorrhagic *E*. *coli* (EHEC), enterotoxigenic *E. coli* (ETEC), enteropathogenic *E. coli* (EPEC), enteroinvasive *E. coli* (EIEC), enteroaggregative *E*. *coli* (EAEC), diffusely adherent *E*. *coli* (DAEC), and uropathogenic E. coli).

In another aspect, the invention is directed to a method for managing infections in a subject, e.g., a human subject, comprising:
administering a glycan preparation in an amount effective and for a time sufficient to treat the infection, wherein the glycan preparation comprises:
i) glycan polymers that comprise glucose glycan units;
ii) the average degree of branching (DB) of the glycan polymers in the glycan preparation is between 0.1-0.5;
iii) at least 50% of the glycan polymers in the glycan preparation have a degree of polymerization (DP) of at least 3 and less than 30 glycan units;
iv) the average DP (mean DP) of the glycan preparation is between about DP4 to about DP12 (e.g., about DP5 to about DP 10);
v) the ratio of alpha- to beta-glycosidic bonds present in the glycan polymers of the glycan preparation is between about 1:1 to about 2:1;
vi) the preparation comprising about 50% to about 75% alpha glycosidic bonds,
vii) the preparation comprising about 25% to about 50% beta glycosidic bonds,
vii) the glycan preparation comprises between 30-70 mol % 1,6-glycosidic bonds,
ix) the glycan preparation comprises between 1-30 mol % 1,2-glycosidic bonds; 3-30 mol % 1,3-glycosidic bonds, and 3-30 mol % 1,4-glycosidic bonds;
x) the glycan preparation has a final solubility limit in water of at least about 70 Brix at 23 °C;
xi) the glycan preparation has a dietary fiber content of at least 70% (as measured by the method AOAC 2009.01);
xii) the glycan preparation has a polydispersity (PD) of between about 1.1 and 2.2;
xiii) the glycan preparation has a total furanose content of between about 1% and about 30%; or
xiv) any combination of two, three, four, five, six, seven, eight, nine, ten, eleven, twelve, or thirteen of i), ii), iii), iv), v), vi), vii), viii), ix), x), xi), xii), and xiii),
optionally, wherein the glycan preparation is pharmaceutical grade (e.g., manufactured under pharmaceutical GMP); or wherein the glycan polymer preparation is food grade (e.g., manufactured under food GMP); further optionally, wherein the glycan preparation is a powder (e.g., dry powder) or a syrup. Optionally, the glycan preparation supports the growth of commensal or probiotic bacteria, *e.g*., in a gut microbiome, further optionally, the glycan preparation does not support the growth of at least one pathogen, *e.g*., does not support the growth of a carbapenem resistant (CRE) Enterobacteriaceae (e.g. *E*. *coli, Klebsiella, Enterobacter, Proteus,* e.g., extended spectrum beta lactamase (ESBL) producing Enterobacteriaciae)), vancomycin resistant enterococcus (VRE), and/or C. difficile taxa, further optionally, the glycan preparation does not support the growth of at least two pathogen, *e.g*., does not support the growth of a CRE, VRE, and/or C. difficile taxa; further optionally, the glycan preparation does not support the growth of CRE; further optionally, the glycan preparation does not support the growth VRE, further optionally, the glycan preparation does not support the growth of C. difficile.

In some embodiments, the human subject is a transplant recipient, e.g., a hematopoietic stem cell (HSCT) recipient or liver transplant recipient. In some embodiments, the human subject is colonized with VRE. In some embodiments, the human subject has received cancer treatment. In some embodiments, the human subject has received immunosuppression. In some embodiments, the human subject is at high-risk for bacterial infection (e.g., due to pre-transplant immune system ablation). In some embodiments, the human subject has end-stage liver disease (ESDL). In some embodiments, the human subject is on a liver transplant waiting list. In some embodiments, the human subject is at risk of being delisted from the transplant list because of an infection. In some embodiments, the human subject develops bacteremia. In some embodiments, the human subject is in an ICU facility.

In some embodiments, the method reduces the rate of infections (e.g., from pathogens that colonize the GI tract), e.g., in critically ill or high-risk subjects. In some embodiments, the method comprises reducing the rate of urinary tract infections. In some embodiments, the method comprises reducing the rate of bloodstream infections. In some embodiments, the method comprises reducing the rate of respiratory tract infections.

In some embodiments, the method comprises managing infections in subjects, e.g. of subject groups with infections (bacteremia): subjects with urinary infections (e.g., infected with Enterococcus, Enterobacteriaciae), subjects with bloodstream infections (e.g., infected with Enterococcus, Enterobacteriaciae), transplant subjects (e.g., bone marrow (e.g., undergoing hematopoietic stem cell transplantation), solid organ (e.g., liver)), intensive care patients (e.g., infected with Carbapenem resistant Enterobacteriaciae and ESBL producing pathogens), pre-transplant liver failure patients (e.g., infected with Vancomycin resistant Enterococcus), post-transplant liver failure patients (e.g., infected with Vancomycin resistant Enterococcus), subjects undergoing chemotherapy with high levels of enteric pathogen bacteremia, *C*. *difficile* infection (CDI)C, and chemotherapy-induced diarrhea compared to other subjects (e.g., the general hospital patient population), antibiotic-treated subjects, subjects undergoing or about to undergo a transplant, subjects with cancer, subjects with liver disease (e.g., end-stage renal disease), or subjects with suppressed immune system (*e.g.*, immunocompromised subjects), subjects with hepatic encephalopathy (HE). In some embodiments, the method comprises prophylactic treatment, *e.g*., with a glycan preparation, of a subject, *e.g*., a subject with a high risk of developing an infection. In some embodiments, subjects who are undergoing chemotherapy or antibiotic treatment have reduced diversity of commensal bacteria. In some embodiments, the method comprises managing infections in subjects who are in need of an organ transplant, *e.g*., a liver or bone marrow transplant. In some embodiments, the method comprises managing infections in subjects immediately, or shortly, before said subject receives an organ transplant, *e.g*., a liver or bone marrow transplant. In some embodiments, the method comprises managing infections in subjects immediately, or shortly, after said subject receives an organ transplant, *e.g*., a liver or bone marrow transplant. In some embodiments, the method comprises managing infections in subjects who have, are suspected of having, or at risk of having end-stage liver disease (ESLD). In some embodiments, the method comprises treatment of a subject to reduce the colonization of pathogens, *e.g*., multidrug resistant pathogens, in a subject. In some embodiments, the method comprises treatment of a subject to reduce the transmission of pathogens, *e.g*., multidrug resistant pathogens, from a first subject to a second subject. In some embodiments, bacteria that pose a risk of colonization in subjects (or a capable of colonizing the GI tract of subjects) comprise resistant subpopulations of Enterobacteriaceae (e.g., *E. cloacae* and Enterococcus), C. difficile (including Nap1 (pandemic hypervirulent) *C*. *difficile* strain), and bacteria that cause infectious diarrhea (e.g., *Campylobacter, Salmonella, Shigella,* enterohemorrhagic *E*. *coli* (EHEC), enterotoxigenic *E*. *coli* (ETEC), enteropathogenic *E. coli* (EPEC), enteroinvasive *E. coli* (EIEC), enteroaggregative *E. coli* (EAEC), diffusely adherent *E*. *coli* (DAEC), and uropathogenic E. coli).

In another aspect, the invention is directed to a method for managing infections in a subject, e.g., a human subject, comprising:
administering a glycan preparation in an amount effective and for a time sufficient to treat the infection, wherein the glycan preparation comprises:
i) glycan polymers that comprise glucose and galactose glycan units;
ii) the average degree of branching (DB) of the glycan polymers in the glycan preparation is between 0.1-0.6;
iii) at least 50% of the glycan polymers in the glycan preparation have a degree of polymerization (DP) of at least 3 and less than 30 glycan units;
iv) the average DP (mean DP) of the glycan preparation is between about DP5 to about DP15 (DP6 to about DP12);
v) the ratio of alpha- to beta-glycosidic bonds present in the glycan polymers of the glycan preparation is between about 1:1 to about 3:1;
vi) the preparation comprising about 55% to about 75%,
vii) the preparation comprising about 25% to about 45% beta glycosidic bonds,
vii) the glycan preparation comprises between 10-70 mol % 1,6-glycosidic bonds,
ix) the glycan preparation comprises between 1-30 mol % 1,2-glycosidic bonds; 1-30 mol % 1,3-glycosidic bonds, and 1-30 mol % 1,4-glycosidic bonds;
x) the glycan preparation has a final solubility limit in water of at least about 70 Brix at 23 °C;
xi) the glycan preparation has a dietary fiber content of at least 70% (as measured by the method AOAC 2009.01);
xii) the glycan preparation has a polydispersity (PD) of between about 1.1 and 2.5;
xiii) the glycan preparation has a total furanose content of between about 1% and about 50% (e.g., between about 5% and 30%); or
xiv) any combination of two, three, four, five, six, seven, eight, nine, ten, eleven, twelve, or thirteen of i), ii), iii), iv), v), vi), vii), viii), ix), x), xi), xii), and xiii),
optionally, wherein the glycan preparation is pharmaceutical grade (e.g., manufactured under pharmaceutical GMP); or wherein the glycan polymer preparation is food grade (e.g., manufactured under food GMP); further optionally, wherein the glycan preparation is a powder (e.g., dry powder) or a syrup. Optionally, the glycan preparation supports the growth of commensal or probiotic bacteria, *e.g*., in a gut microbiome, further optionally, the glycan preparation does not support the growth of at least one pathogen, *e.g*., does not support the growth of a carbapenem resistant (CRE) Enterobacteriaceae (e.g. *E*. *coli, Klebsiella, Enterobacter, Proteus,* e.g., extended spectrum beta lactamase (ESBL) producing Enterobacteriaciae)), vancomycin resistant enterococcus (VRE), and/or C. difficile taxa, further optionally, the glycan preparation does not support the growth of at least two pathogen, *e.g*., does not support the growth of a CRE, VRE, and/or C. difficile taxa; further optionally, the glycan preparation does not support the growth of CRE; further optionally, the glycan preparation does not support the growth VRE, further optionally, the glycan preparation does not support the growth of C. difficile.

In some embodiments, the human subject is a transplant recipient, e.g., a hematopoietic stem cell (HSCT) recipient or liver transplant recipient. In some embodiments, the human subject is colonized with VRE. In some embodiments, the human subject has received cancer treatment. In some embodiments, the human subject has received immunosuppression. In some embodiments, the human subject is at high-risk for bacterial infection (e.g., due to pre-transplant immune system ablation). In some embodiments, the human subject has end-stage liver disease (ESDL). In some embodiments, the human subject is on a liver transplant waiting list. In some embodiments, the human subject is at risk of being delisted from the transplant list because of an infection. In some embodiments, the human subject develops bacteremia. In some embodiments, the human subject is in an ICU facility.

In some embodiments, the method reduces the rate of infections (e.g., from pathogens that colonize the GI tract), e.g., in critically ill or high-risk subjects. In some embodiments, the method comprises reducing the rate of urinary tract infections. In some embodiments, the method comprises reducing the rate of bloodstream infections. In some embodiments, the method comprises reducing the rate of respiratory tract infections.

In some embodiments, the method comprises managing infections in subjects, e.g. of subject groups with infections (bacteremia): subjects with urinary infections (e.g., infected with Enterococcus, Enterobacteriaciae), subjects with bloodstream infections (e.g., infected with Enterococcus, Enterobacteriaciae), transplant subjects (e.g., bone marrow (e.g., undergoing hematopoietic stem cell transplantation), solid organ (e.g., liver)), intensive care patients (e.g., infected with Carbapenem resistant Enterobacteriaciae and ESBL producing pathogens), pre-transplant liver failure patients (e.g., infected with Vancomycin resistant Enterococcus), post-transplant liver failure patients (e.g., infected with Vancomycin resistant Enterococcus), subjects undergoing chemotherapy with high levels of enteric pathogen bacteremia, *C*. *difficile* infection (CDI)C, and chemotherapy-induced diarrhea compared to other subjects (e.g., the general hospital patient population), antibiotic-treated subjects, subjects undergoing or about to undergo a transplant, subjects with cancer, subjects with liver disease (e.g., end-stage renal disease), or subjects with suppressed immune system (*e.g.*, immunocompromised subjects), subjects with hepatic encephalopathy (HE). In some embodiments, the method comprises prophylactic treatment, *e.g*., with a glycan preparation, of a subject, *e.g*., a subject with a high risk of developing an infection. In some embodiments, subjects who are undergoing chemotherapy or antibiotic treatment have reduced diversity of commensal bacteria. In some embodiments, the method comprises managing infections in subjects who are in need of an organ transplant, *e.g*., a liver or bone marrow transplant. In some embodiments, the method comprises managing infections in subjects immediately, or shortly, before said subject receives an organ transplant, *e.g*., a liver or bone marrow transplant. In some embodiments, the method comprises managing infections in subjects immediately, or shortly, after said subject receives an organ transplant, *e.g*., a liver or bone marrow transplant. In some embodiments, the method comprises managing infections in subjects who have, are suspected of having, or at risk of having end-stage liver disease (ESLD). In some embodiments, the method comprises treatment of a subject to reduce the colonization of pathogens, *e.g*., multidrug resistant pathogens, in a subject. In some embodiments, the method comprises treatment of a subject to reduce the transmission of pathogens, *e.g*., multidrug resistant pathogens, from a first subject to a second subject. In some embodiments, bacteria that pose a risk of colonization in subjects (or a capable of colonizing the GI tract of subjects) comprise resistant subpopulations of Enterobacteriaceae (e.g., *E. cloacae* and Enterococcus), C. difficile (including Nap1 (pandemic hypervirulent) *C*. *difficile* strain), and bacteria that cause infectious diarrhea (e.g., *Campylobacter, Salmonella, Shigella,* enterohemorrhagic *E*. *coli* (EHEC), enterotoxigenic *E*. *coli* (ETEC), enteropathogenic *E. coli* (EPEC), enteroinvasive *E. coli* (EIEC), enteroaggregative *E. coli* (EAEC), diffusely adherent *E*. *coli* (DAEC), and uropathogenic E. coli).

In another aspect, the invention is directed to a method for managing infections in a subject, e.g., a human subject, comprising:
administering a glycan preparation in an amount effective and for a time sufficient to treat the infection, wherein the glycan preparation comprises:
i) glycan polymers that comprise glucose, galactose, and mannose glycan units;
ii) the average degree of branching (DB) of the glycan polymers in the glycan preparation is between 0.1-0.6;
iii) at least 50% of the glycan polymers in the glycan preparation have a degree of polymerization (DP) of at least 3 and less than 30 glycan units;
iv) the average DP (mean DP) of the glycan preparation is between about DP5 to about DP15 (DP6 to about DP12);
v) the ratio of alpha- to beta-glycosidic bonds present in the glycan polymers of the glycan preparation is between about 1:1 to about 3:1;
vi) the preparation comprising about 55% to about 75%,
vii) the preparation comprising about 25% to about 45% beta glycosidic bonds,
vii) the glycan preparation comprises between 10-70 mol % 1,6-glycosidic bonds,
ix) the glycan preparation comprises between 1-30 mol % 1,2-glycosidic bonds; 1-30 mol % 1,3-glycosidic bonds, and 1-30 mol % 1,4-glycosidic bonds;
x) the glycan preparation has a final solubility limit in water of at least about 70 Brix at 23 °C;
xi) the glycan preparation has a dietary fiber content of at least 70% (as measured by the method AOAC 2009.01);
xii) the glycan preparation has a polydispersity (PD) of between about 1.1 and 2.5;
xiii) the glycan preparation has a total furanose content of between about 1% and about 50% (e.g., between about 5% and 30%);
   or
xiv) any combination of two, three, four, five, six, seven, eight, nine, ten, eleven, twelve, or thirteen of i), ii), iii), iv), v), vi), vii), viii), ix), x), xi), xii), and xiii),
optionally, wherein the glycan preparation is pharmaceutical grade (e.g., manufactured under pharmaceutical GMP); or wherein the glycan polymer preparation is food grade (e.g., manufactured under food GMP); further optionally, wherein the glycan preparation is a powder (e.g., dry powder) or a syrup. Optionally, the glycan preparation supports the growth of commensal or probiotic bacteria, *e.g*., in a gut microbiome, further optionally, the glycan preparation does not support the growth of at least one pathogen, *e.g*., does not support the growth of a carbapenem resistant (CRE) Enterobacteriaceae (e.g. *E*. *coli, Klebsiella, Enterobacter, Proteus, e.g*., extended spectrum beta lactamase (ESBL) producing Enterobacteriaciae)), vancomycin resistant enterococcus (VRE), and/or C. difficile taxa, further optionally, the glycan preparation does not support the growth of at least two pathogen, *e.g*., does not support the growth of a CRE, VRE, and/or C. difficile taxa; further optionally, the glycan preparation does not support the growth of CRE; further optionally, the glycan preparation does not support the growth VRE, further optionally, the glycan preparation does not support the growth of C. difficile.

In some embodiments, the human subject is a transplant recipient, e.g., a hematopoietic stem cell (HSCT) recipient or liver transplant recipient. In some embodiments, the human subject is colonized with VRE. In some embodiments, the human subject has received cancer treatment. In some embodiments, the human subject has received immunosuppression. In some embodiments, the human subject is at high-risk for bacterial infection (e.g., due to pre-transplant immune system ablation). In some embodiments, the human subject has end-stage liver disease (ESDL). In some embodiments, the human subject is on a liver transplant waiting list. In some embodiments, the human subject is at risk of being delisted from the transplant list because of an infection. In some embodiments, the human subject develops bacteremia. In some embodiments, the human subject is in an ICU facility.

In some embodiments, the method reduces the rate of infections (e.g., from pathogens that colonize the GI tract), e.g., in critically ill or high-risk subjects. In some embodiments, the method comprises reducing the rate of urinary tract infections. In some embodiments, the method comprises reducing the rate of bloodstream infections. In some embodiments, the method comprises reducing the rate of respiratory tract infections.

In some embodiments, the method comprises managing infections in subjects, e.g. of subject groups with infections (bacteremia): subjects with urinary infections (e.g., infected with Enterococcus, Enterobacteriaciae), subjects with bloodstream infections (e.g., infected with Enterococcus, Enterobacteriaciae), transplant subjects (e.g., bone marrow (e.g., undergoing hematopoietic stem cell transplantation), solid organ (e.g., liver)), intensive care patients (e.g., infected with Carbapenem resistant Enterobacteriaciae and ESBL producing pathogens), pre-transplant liver failure patients (e.g., infected with Vancomycin resistant Enterococcus), post-transplant liver failure patients (e.g., infected with Vancomycin resistant Enterococcus), subjects undergoing chemotherapy with high levels of enteric pathogen bacteremia, *C. difficile* infection (CDI)C, and chemotherapy-induced diarrhea compared to other subjects (e.g., the general hospital patient population), antibiotic-treated subjects, subjects undergoing or about to undergo a transplant, subjects with cancer, subjects with liver disease (e.g., end-stage renal disease), or subjects with suppressed immune system (*e.g.,* immunocompromised subjects), subjects with hepatic encephalopathy (HE). In some embodiments, the method comprises prophylactic treatment, *e.g*., with a glycan preparation, of a subject, *e.g*., a subject with a high risk of developing an infection. In some embodiments, subjects who are undergoing chemotherapy or antibiotic treatment have reduced diversity of commensal bacteria. In some embodiments, the method comprises managing infections in subjects who are in need of an organ transplant, *e.g*., a liver or bone marrow transplant. In some embodiments, the method comprises managing infections in subjects immediately, or shortly, before said subject receives an organ transplant, *e.g*., a liver or bone marrow transplant. In some embodiments, the method comprises managing infections in subjects immediately, or shortly, after said subject receives an organ transplant, *e.g*., a liver or bone marrow transplant. In some embodiments, the method comprises managing infections in subjects who have, are suspected of having, or at risk of having end-stage liver disease (ESLD). In some embodiments, the method comprises treatment of a subject to reduce the colonization of pathogens, *e.g*., multidrug resistant pathogens, in a subject. In some embodiments, the method comprises treatment of a subject to reduce the transmission of pathogens, *e.g*., multidrug resistant pathogens, from a first subject to a second subject. In some embodiments, bacteria that pose a risk of colonization in subjects (or a capable of colonizing the GI tract of subjects) comprise resistant subpopulations of Enterobacteriaceae (e.g., *E. cloacae* and Enterococcus), C. difficile (including Napl (pandemic hypervirulent) *C*. *difficile* strain), and bacteria that cause infectious diarrhea (e.g., *Campylobacter, Salmonella, Shigella,* enterohemorrhagic *E*. *coli* (EHEC), enterotoxigenic *E*. *coli* (ETEC), enteropathogenic *E. coli* (EPEC), enteroinvasive *E. coli* (EIEC), enteroaggregative *E. coli* (EAEC), diffusely adherent *E*. *coli* (DAEC), and uropathogenic E. coli).

In another aspect, the invention is directed to a method for managing infections in a subject, e.g., a human subject, comprising:
administering a glycan preparation in an amount effective and for a time sufficient to treat the infection, wherein the glycan preparation comprises:
i) glycan polymers that comprise glucose glycan units;
ii) the average degree of branching (DB) of the glycan polymers in the glycan preparation is between 0.1 and 0.4;
iii) 45% to 55% of the glycan polymers in the glycan preparation have a degree of polymerization (DP) of at least 3 and less than or equal to 10 glycan units;
iv) the average DP (mean DP) of the glycan preparation is between about 5 and 8;
v) the ratio of alpha- to beta-glycosidic bonds present in the glycan polymers of the glycan preparation is between about 1:1 to about 1.5:1;
vi) the glycan preparation comprises between 20 mol % and 60 mol % 1,6 glycosidic bonds;
vii) the glycan preparation comprises between 5 mol % and 25 mol % of at least one, two, or three of 1,2; 1,3; and 1,4 glycosidic bonds;
viii) the glycan preparation has a final solubility limit in water of at least about 70 Brix at 23 °C; and/or
ix) the glycan preparation has a dietary fiber content of at least 70%. In another aspect, the invention is directed to a method for managing infections in a subject, e.g., a human subject, comprising:
   administering a glycan preparation in an amount effective and for a time sufficient to treat the infection, wherein the glycan preparation comprises:
   i) glycan polymers that comprise glucose or galactose glycan units;
   ii) the average degree of branching (DB) of the glycan polymers in the glycan preparation is between 0.1 and 0.4;
   iii) at least 50% of the glycan polymers in the glycan preparation have a degree of polymerization (DP) at least 3 and less than 10 glycan units;
   iv) the average DP (mean DP) of the glycan preparation is between about 5 and 8;
   v) the ratio of alpha- to beta-glycosidic bonds present in the glycan polymers of the glycan preparation is between about 1:1 to about 3:1;
   vi) the glycan preparation comprises between 20 mol % and 60 mol % 1,6 glycosidic bonds;
   vii) the glycan preparation comprises between 5 mol % and 25 mol % of at least one, two, or three of 1,2; 1,3; and 1,4 glycosidic bonds;
   viii) the glycan preparation has a final solubility limit in water of at least about 70 Brix at 23 °C; and/or
   ix) the glycan preparation has a dietary fiber content of at least 70%.
In some embodiments, the glycan preparation comprises two, three, four, five, six, seven, eight, or nine of the selected properties of i), ii), iii), iv), v), vi), vii), viii), and ix); optionally, wherein the glycan preparation is pharmaceutical grade (e.g., manufactured under pharmaceutical GMP); or wherein the glycan polymer preparation is food grade (e.g., manufactured under food GMP); further optionally, wherein the glycan preparation is a powder (e.g., dry powder) or a syrup. Optionally, the glycan preparation supports the growth of commensal or probiotic bacteria, *e.g*., in a gut microbiome, further optionally, the glycan preparation does not support the growth of at least one pathogen, *e.g*., does not support the growth of a carbapenem resistant (CRE) Enterobacteriaceae (e.g. *E*. *coli, Klebsiella, Enterobacter, Proteus,* e.g., extended spectrum beta lactamase (ESBL) producing Enterobacteriaciae)), vancomycin resistant enterococcus (VRE), and/or C. difficile taxa, further optionally, the glycan preparation does not support the growth of at least two pathogen, *e.g*., does not support the growth of a CRE, VRE, and/or C. difficile taxa; further optionally, the glycan preparation does not support the growth of CRE; further optionally, the glycan preparation does not support the growth VRE, further optionally, the glycan preparation does not support the growth of C. difficile.

In some embodiments, the human subject is a transplant recipient, e.g., a hematopoietic stem cell (HSCT) recipient or liver transplant recipient. In some embodiments, the human subject is colonized with VRE. In some embodiments, the human subject has received cancer treatment. In some embodiments, the human subject has received immunosuppression. In some embodiments, the human subject is at high-risk for bacterial infection (e.g., due to pre-transplant immune system ablation). In some embodiments, the human subject has end-stage liver disease (ESDL). In some embodiments, the human subject is on a liver transplant waiting list. In some embodiments, the human subject is at risk of being delisted from the transplant list because of an infection. In some embodiments, the human subject develops bacteremia. In some embodiments, the human subject is in an ICU facility.

In some embodiments, the method reduces the rate of infections (e.g., from pathogens that colonize the GI tract), e.g., in critically ill or high-risk subjects. In some embodiments, the method comprises reducing the rate of urinary tract infections. In some embodiments, the method comprises reducing the rate of bloodstream infections. In some embodiments, the method comprises reducing the rate of respiratory tract infections.

In some embodiments, the method comprises managing infections in subjects, e.g. of subject groups with infections (bacteremia): subjects with urinary infections (e.g., infected with Enterococcus, Enterobacteriaciae), subjects with bloodstream infections (e.g., infected with Enterococcus, Enterobacteriaciae), transplant subjects (e.g., bone marrow (e.g., undergoing hematopoietic stem cell transplantation), solid organ (e.g., liver)), intensive care patients (e.g., infected with Carbapenem resistant Enterobacteriaciae and ESBL producing pathogens), pre-transplant liver failure patients (e.g., infected with Vancomycin resistant Enterococcus), post-transplant liver failure patients (e.g., infected with Vancomycin resistant Enterococcus), subjects undergoing chemotherapy with high levels of enteric pathogen bacteremia, *C*. *difficile* infection (CDI)C, and chemotherapy-induced diarrhea compared to other subjects (e.g., the general hospital patient population), antibiotic-treated subjects, subjects undergoing or about to undergo a transplant, subjects with cancer, subjects with liver disease (e.g., end-stage renal disease), or subjects with suppressed immune system *(e.*g*.,* immunocompromised subjects), subjects with hepatic encephalopathy (HE). In some embodiments, the method comprises prophylactic treatment, *e.g*., with a glycan preparation, of a subject, *e.g*., a subject with a high risk of developing an infection. In some embodiments, subjects who are undergoing chemotherapy or antibiotic treatment have reduced diversity of commensal bacteria. In some embodiments, the method comprises managing infections in subjects who are in need of an organ transplant, *e.g*., a liver or bone marrow transplant. In some embodiments, the method comprises managing infections in subjects immediately, or shortly, before said subject receives an organ transplant, *e.g*., a liver or bone marrow transplant. In some embodiments, the method comprises managing infections in subjects immediately, or shortly, after said subject receives an organ transplant, *e.g*., a liver or bone marrow transplant. In some embodiments, the method comprises managing infections in subjects who have, are suspected of having, or at risk of having end-stage liver disease (ESLD). In some embodiments, the method comprises treatment of a subject to reduce the colonization of pathogens, *e.g*., multidrug resistant pathogens, in a subject. In some embodiments, the method comprises treatment of a subject to reduce the transmission of pathogens, *e.g*., multidrug resistant pathogens, from a first subject to a second subject. In some embodiments, bacteria that pose a risk of colonization in subjects (or a capable of colonizing the GI tract of subjects) comprise resistant subpopulations of Enterobacteriaceae (e.g., *E. cloacae* and Enterococcus), C. difficile (including Nap1 (pandemic hypervirulent) *C. difficile* strain), and bacteria that cause infectious diarrhea (e.g., *Campylobacter, Salmonella, Shigella*, enterohemorrhagic *E*. *coli* (EHEC), enterotoxigenic *E*. *coli* (ETEC), enteropathogenic *E*. *coli* (EPEC), enteroinvasive *E*. *coli* (EIEC), enteroaggregative *E*. *coli* (EAEC), diffusely adherent *E*. *coli* (DAEC), and uropathogenic E. coli).

In another aspect, the invention is directed to a method for managing infections in a subject, e.g., a human subject, comprising:
administering a glycan preparation in an amount effective and for a time sufficient to treat the infection, wherein the glycan preparation comprises:
   i) glycan polymers that comprise glucose and galactose glycan units;
   ii) the average degree of branching (DB) of the glycan polymers in the glycan preparation is between 0.1 and 0.4;
   iii) 45% to 55% of the glycan polymers in the glycan preparation have a degree of polymerization (DP) of at least 3 and less than or equal to 10 glycan units;
   iv) the average DP (mean DP) of the glycan preparation is between about 5 and 8;
   v) the ratio of alpha- to beta-glycosidic bonds present in the glycan polymers of the glycan preparation is between about 2:1 to about 3:1;
   vi) the glycan preparation comprises between 20 mol % and 60 mol % 1,6 glycosidic bonds;
   vii) the glycan preparation comprises between 5 mol % and 25 mol % of at least one, two, or three of 1,2; 1,3; and 1,4 glycosidic bonds;
   viii) the glycan preparation has a final solubility limit in water of at least about 70 Brix at 23 °C; and/or
   ix) the glycan preparation has a dietary fiber content of at least 70%. In another aspect, the invention is directed to a method for managing infections in a subject, e.g., a human subject, comprising:
administering a glycan preparation in an amount effective and for a time sufficient to treat the infection, wherein the glycan preparation comprises:
   i) glycan polymers that comprise glucose or galactose glycan units;
   ii) the average degree of branching (DB) of the glycan polymers in the glycan preparation is between 0.1 and 0.4;
   iii) at least 50% of the glycan polymers in the glycan preparation have a degree of polymerization (DP) at least 3 and less than 10 glycan units;
   iv) the average DP (mean DP) of the glycan preparation is between about 5 and 8;
   v) the ratio of alpha- to beta-glycosidic bonds present in the glycan polymers of the glycan preparation is between about 1:1 to about 3:1;
   vi) the glycan preparation comprises between 20 mol % and 60 mol % 1,6 glycosidic bonds;
   vii) the glycan preparation comprises between 5 mol % and 25 mol % of at least one, two, or three of 1,2; 1,3; and 1,4 glycosidic bonds;
   viii) the glycan preparation has a final solubility limit in water of at least about 70 Brix at 23 °C; and/or
   ix) the glycan preparation has a dietary fiber content of at least 70%.
   In some embodiments, the glycan preparation comprises two, three, four, five, six, seven, eight, or nine of the selected properties of i), ii), iii), iv), v), vi), vii), viii), and ix); optionally, wherein the glycan preparation is pharmaceutical grade (e.g., manufactured under pharmaceutical GMP); or wherein the glycan polymer preparation is food grade (e.g., manufactured under food GMP); further optionally, wherein the glycan preparation is a powder (e.g., dry powder) or a syrup. Optionally, the glycan preparation supports the growth of commensal or probiotic bacteria, *e.g*., in a gut microbiome, further optionally, the glycan preparation does not support the growth of at least one pathogen, *e.g*., does not support the growth of a carbapenem resistant (CRE) Enterobacteriaceae (e.g. *E. coli, Klebsiella, Enterobacter, Proteus,* e.g.. extended spectrum beta lactamase (ESBL) producing Enterobacteriaciae)), vancomycin resistant enterococcus (VRE), and/or C. difficile taxa, further optionally, the glycan preparation does not support the growth of at least two pathogen, *e.g*., does not support the growth of a CRE, VRE, and/or C. difficile taxa; further optionally, the glycan preparation does not support the growth of CRE; further optionally, the glycan preparation does not support the growth VRE, further optionally, the glycan preparation does not support the growth of C. difficile.

In some embodiments, the human subject is a transplant recipient, e.g., a hematopoietic stem cell (HSCT) recipient or liver transplant recipient. In some embodiments, the human subject is colonized with VRE. In some embodiments, the human subject has received cancer treatment. In some embodiments, the human subject has received immunosuppression. In some embodiments, the human subject is at high-risk for bacterial infection (e.g., due to pre-transplant immune system ablation). In some embodiments, the human subject has end-stage liver disease (ESDL). In some embodiments, the human subject is on a liver transplant waiting list. In some embodiments, the human subject is at risk of being delisted from the transplant list because of an infection. In some embodiments, the human subject develops bacteremia. In some embodiments, the human subject is in an ICU facility.

In some embodiments, the method reduces the rate of infections (e.g., from pathogens that colonize the GI tract), e.g., in critically ill or high-risk subjects. In some embodiments, the method comprises reducing the rate of urinary tract infections. In some embodiments, the method comprises reducing the rate of bloodstream infections. In some embodiments, the method comprises reducing the rate of respiratory tract infections.

In some embodiments, the method comprises managing infections in subjects, e.g. of subject groups with infections (bacteremia): subjects with urinary infections (e.g., infected with Enterococcus, Enterobacteriaciae), subjects with bloodstream infections (e.g., infected with Enterococcus, Enterobacteriaciae), transplant subjects (e.g., bone marrow (e.g., undergoing hematopoietic stem cell transplantation), solid organ (e.g., liver)), intensive care patients (e.g., infected with Carbapenem resistant Enterobacteriaciae and ESBL producing pathogens), pre-transplant liver failure patients (e.g., infected with Vancomycin resistant Enterococcus), post-transplant liver failure patients (e.g., infected with Vancomycin resistant Enterococcus), subjects undergoing chemotherapy with high levels of enteric pathogen bacteremia, *C. difficile* infection (CDI)C, and chemotherapy-induced diarrhea compared to other subjects (e.g., the general hospital patient population), antibiotic-treated subjects, subjects undergoing or about to undergo a transplant, subjects with cancer, subjects with liver disease (e.g., end-stage renal disease), or subjects with suppressed immune system (*e.g.,* immunocompromised subjects), subjects with hepatic encephalopathy (HE). In some embodiments, the method comprises prophylactic treatment, *e.g*., with a glycan preparation, of a subject, *e.g*., a subject with a high risk of developing an infection. In some embodiments, subjects who are undergoing chemotherapy or antibiotic treatment have reduced diversity of commensal bacteria. In some embodiments, the method comprises managing infections in subjects who are in need of an organ transplant, *e.g*., a liver or bone marrow transplant. In some embodiments, the method comprises managing infections in subjects immediately, or shortly, before said subject receives an organ transplant, *e.g*., a liver or bone marrow transplant. In some embodiments, the method comprises managing infections in subjects immediately, or shortly, after said subject receives an organ transplant, *e.g*., a liver or bone marrow transplant. In some embodiments, the method comprises managing infections in subjects who have, are suspected of having, or at risk of having end-stage liver disease (ESLD). In some embodiments, the method comprises treatment of a subject to reduce the colonization of pathogens, *e.g*., multidrug resistant pathogens, in a subject. In some embodiments, the method comprises treatment of a subject to reduce the transmission of pathogens, *e.g*., multidrug resistant pathogens, from a first subject to a second subject. In some embodiments, bacteria that pose a risk of colonization in subjects (or a capable of colonizing the GI tract of subjects) comprise resistant subpopulations of Enterobacteriaceae (e.g., *E. cloacae* and Enterococcus), C. difficile (including Napl (pandemic hypervirulent) *C*. *difficile* strain), and bacteria that cause infectious diarrhea (e.g., *Campylobacter, Salmonella, Shigella,.* enterohemorrhagic *E. coli* (EHEC), enterotoxigenic *E. coli* (ETEC), enteropathogenic *E*. *coli* (EPEC), enteroinvasive *E. coli* (EIEC), enteroaggregative *E. coli* (EAEC), diffusely adherent *E*. *coli* (DAEC), and uropathogenic E. coli).

In some embodiments, a glycan preparation comprising any of the aforementioned properties is provided. In some embodiments, a glycan preparation obtainable by (or producible from) any of the aforementioned method (or process) is provided.

The details of one or more aspects and embodiments of the invention are set forth herein. Other features, objects, and advantages of the invention will be apparent from the Detailed Description, the Figures, the Examples, and the Claims.

### BRIEF DESCRIPTION OF THE DRAWINGS

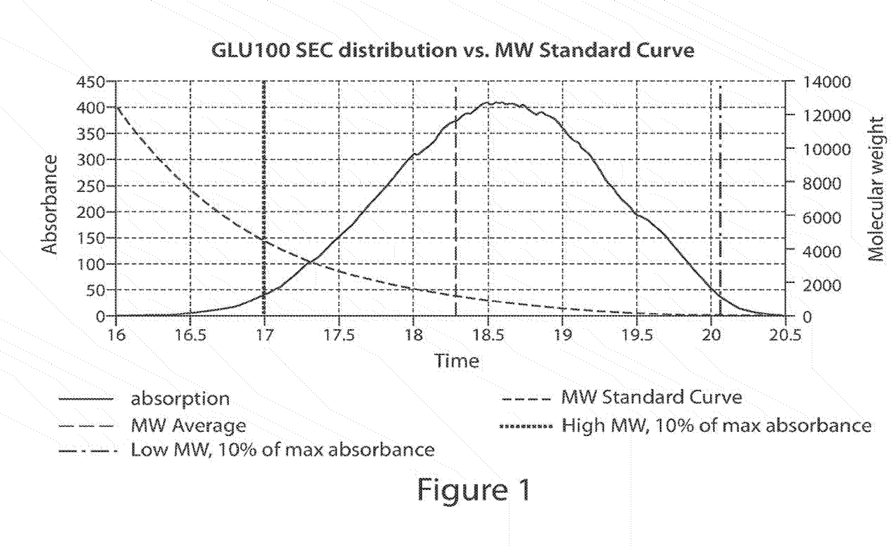

**Figure 1** is a representative SEC curve between 16 and 20.5 minutes of a glu100 sample showing the average MW and the MW at 10% of maximum absorption on both the leading and trailing edges of the curve.

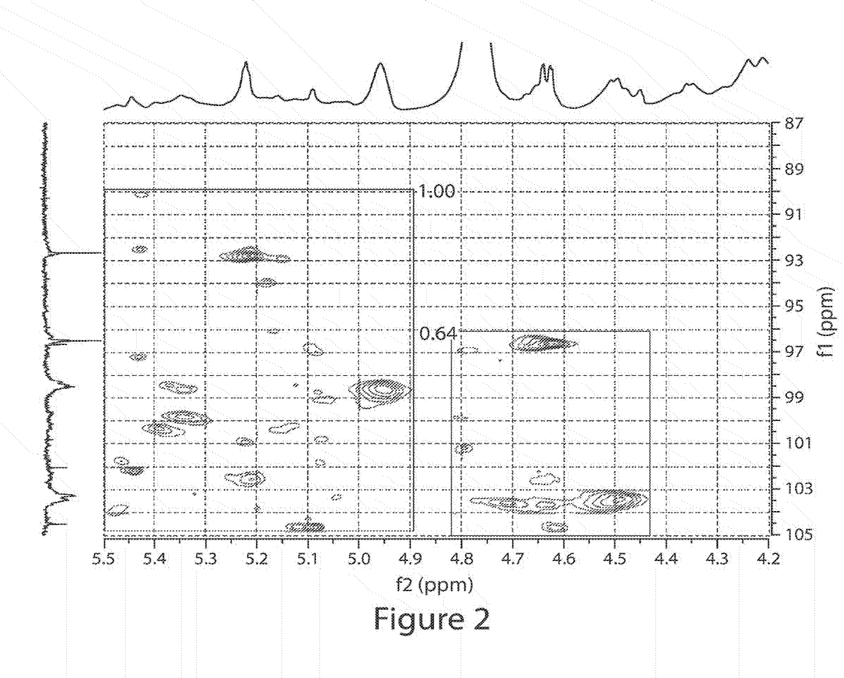

**Figure 2** is a representative anomeric region of an ¹H-¹³C HSQC spectrum of a glu100 sample showing the signal distribution of alpha- and beta-glycosidic bonds.

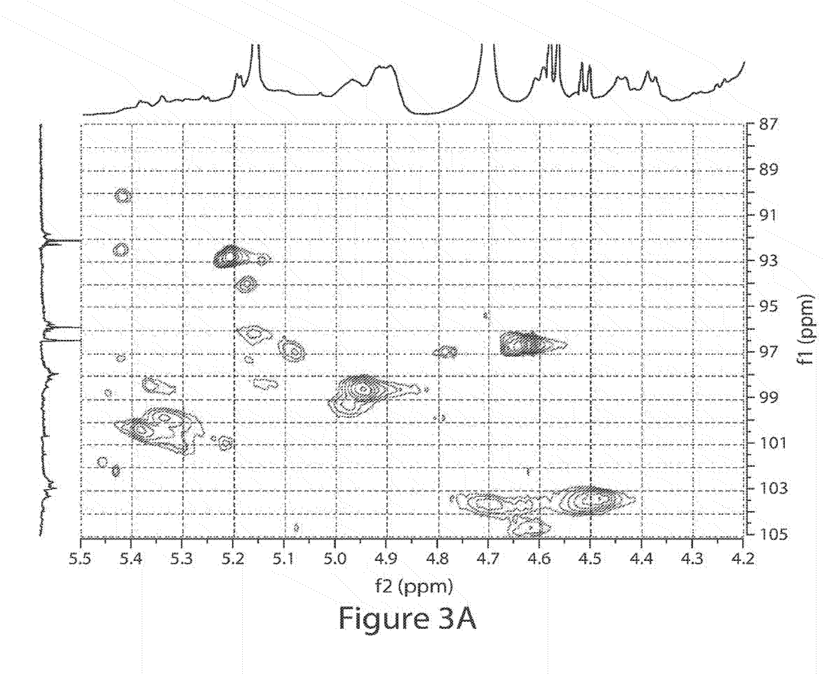

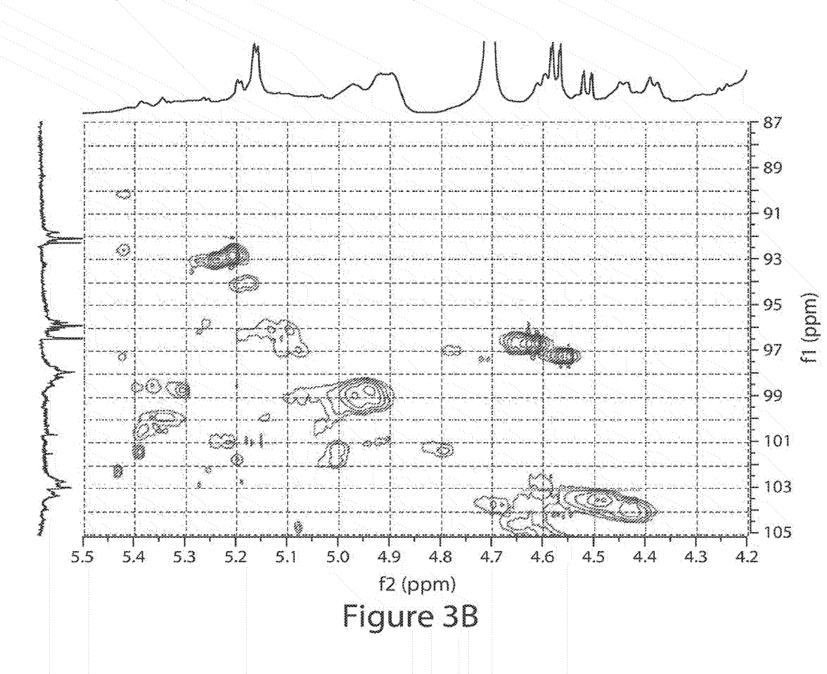

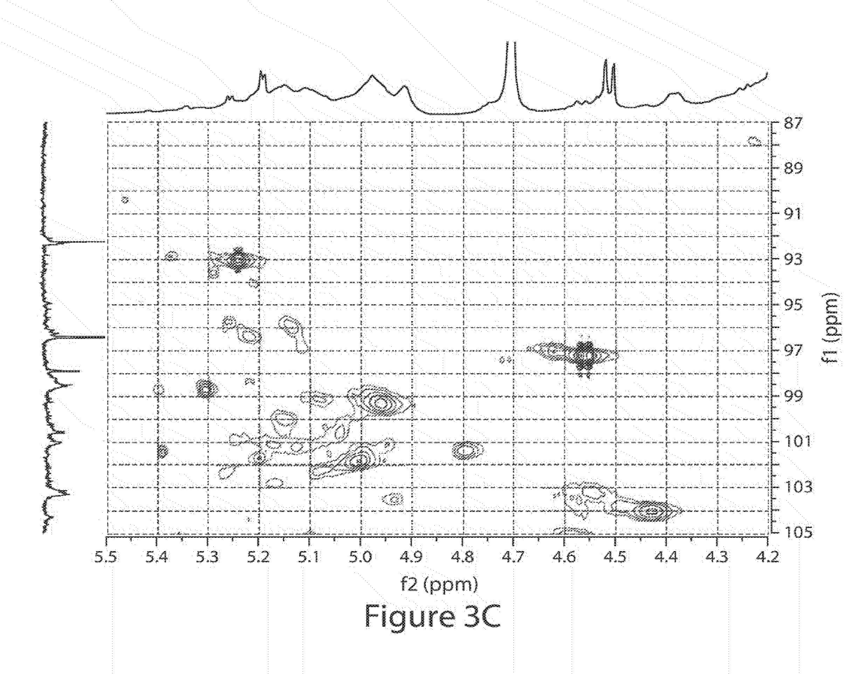

**Figures 3A****,** **3B****, and** **3C** are a series of representative anomeric regions of an ¹H-¹³C HSQC spectrum of glu100 (Fig. 3A), glu50gal50 (Fig. 3B), and gal100 (Fig. 3C) samples, demonstrating the additive effect of the fingerprint peaks.

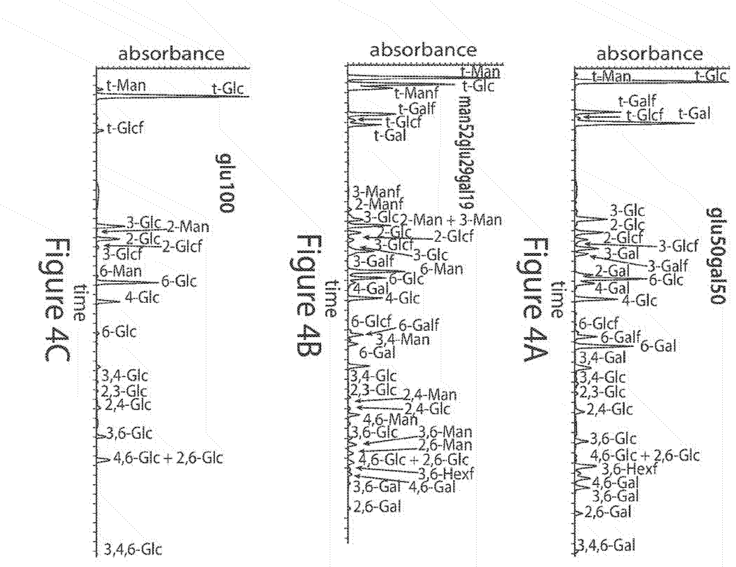

**Figures 4A, 4B, and 4C** are representative GC chromatograms of three representative permethylated and hydrolyzed glycans, glu50gal50 (Fig. 4A), man52glu29gal19 (Fig. 4B), and glu100 (Fig. 4C), showing distribution of regio-chemistry as assigned by comparison to known standards.

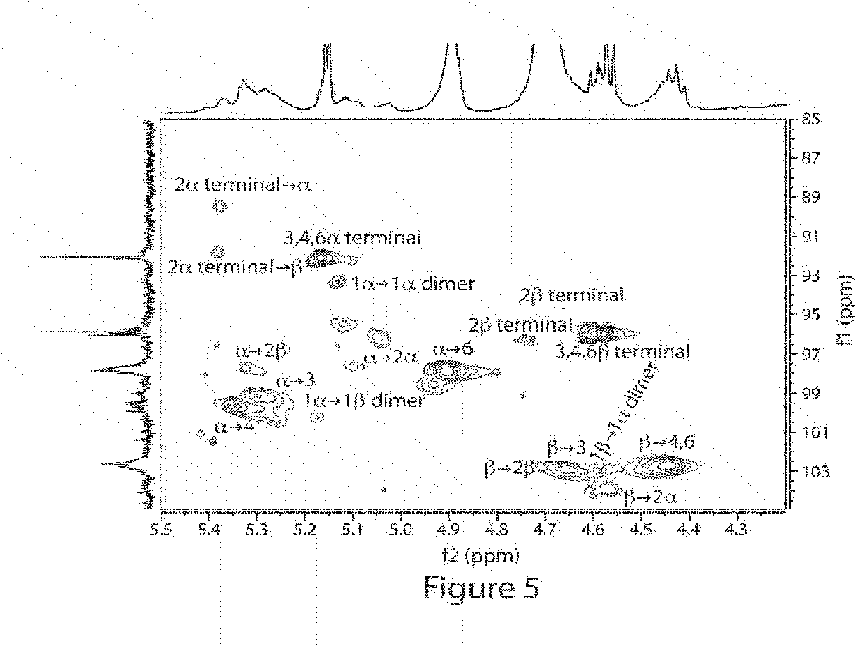

**Figure 5** is a representative partial assignment of the peaks in the anomeric region of a glu100 sample ¹H-¹³C HSQC spectrum showing the separation between alpha and beta isomers in the ¹H axis, with alpha isomers downfield (¹H >4.8 ppm in this case) and beta isomers upfield (¹H <4.8 ppm in this case). In addition, terminal and internal sugars can be distinguished in the ¹³C axis with terminal sugars upfield (¹³C<94 ppm for alpha and ¹³C<100 ppm for beta in this case) and internal sugars downfield (¹³C>94 ppm for alpha and ¹³C>100 ppm for beta in this case).

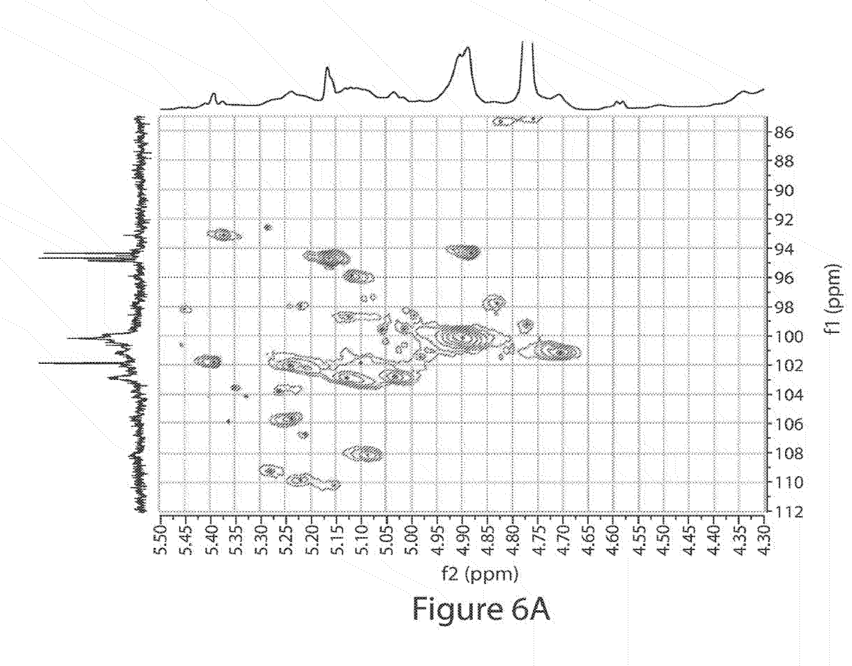

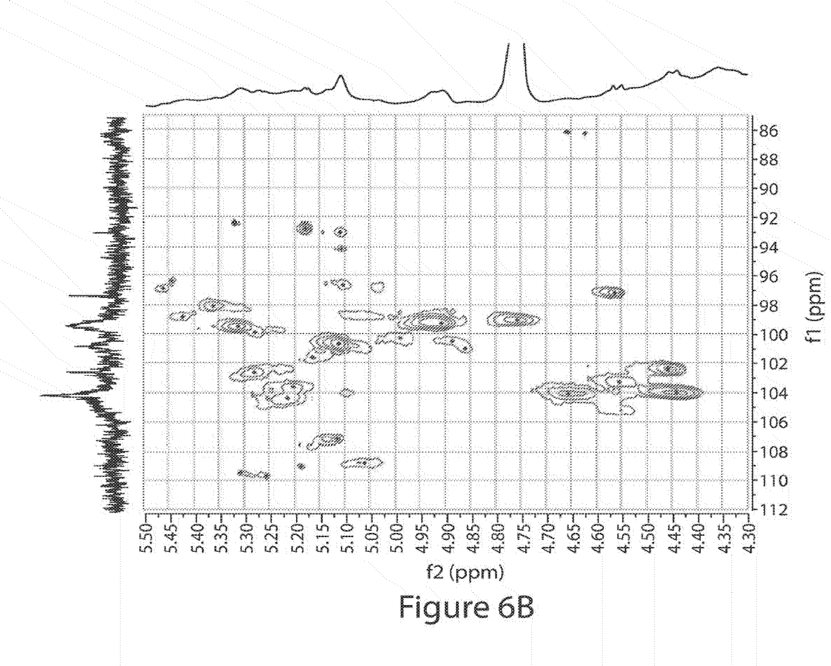

**Figures 6A** **and** **6B** are a series of anomeric region of the 1H-13C HSQC spectrum of man100 (Figure 6A) and xyl100 (Figure 6B).

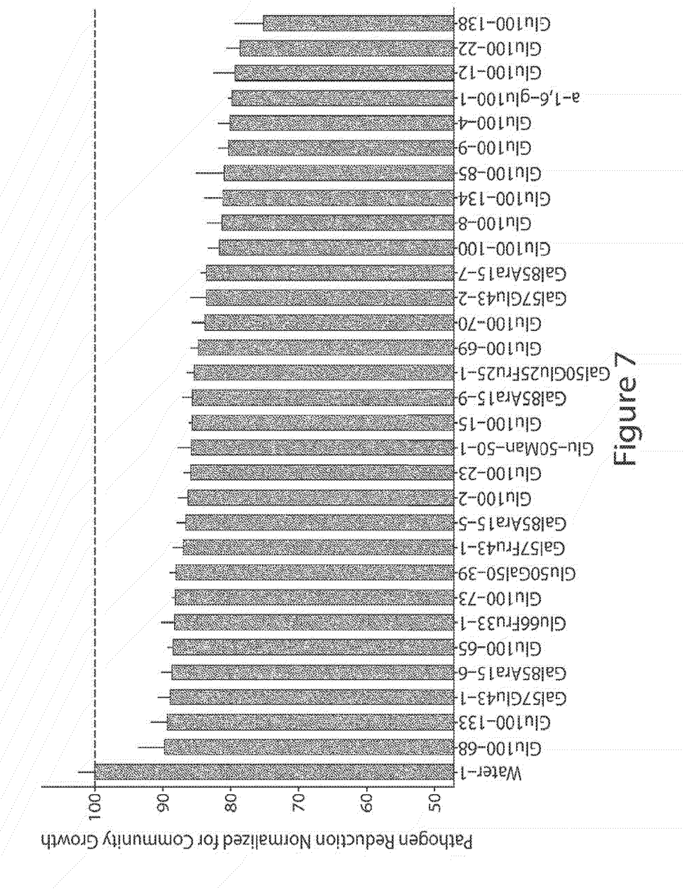

**Figure 7** is a bar graph depicting Carbapenem-resistant Enterobacteriaceae (CRE) *Escherichia coli* growth, grown in the presence of glycans for 24 hours, normalized to control with no glycan added. Glycans that reduce growth of CRE *E*. *coli* by >10% are plotted. Shown are average index (1-scaled community growth*scaled pathogen growth) normalized to control ± st.dev.

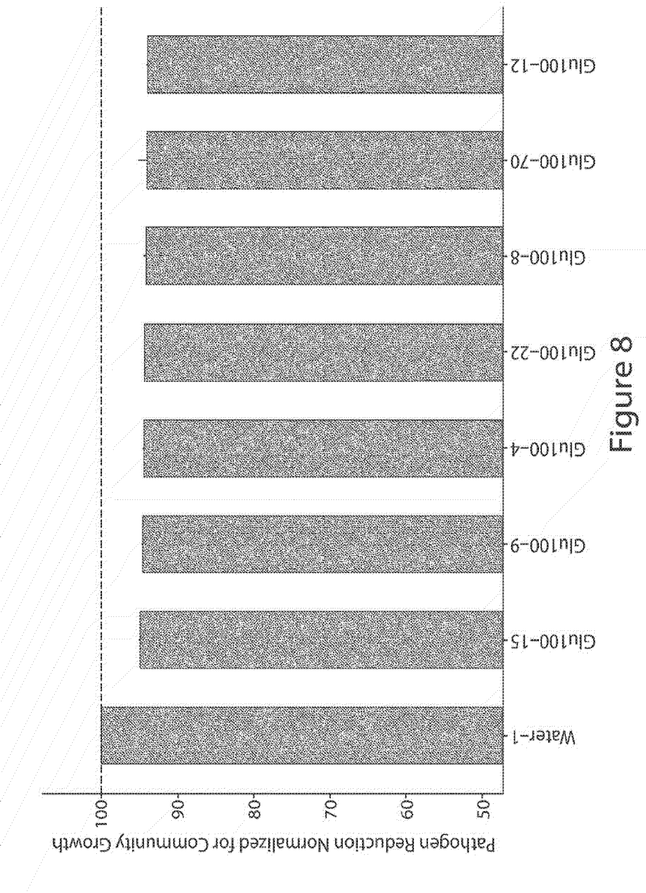

**Figure 8** is a bar graph depicting Vancomycin-resistant Enterococcus (VRE) *Enterococcus faecium* growth, grown in the presence of glycans for 24 hours, normalized to control with no glycan added. Glycans that reduce growth of VRE *E. faecium* by >5% are plotted. Shown are average index (1-scaled community growth*scaled pathogen growth) normalized to control ± st.dev.

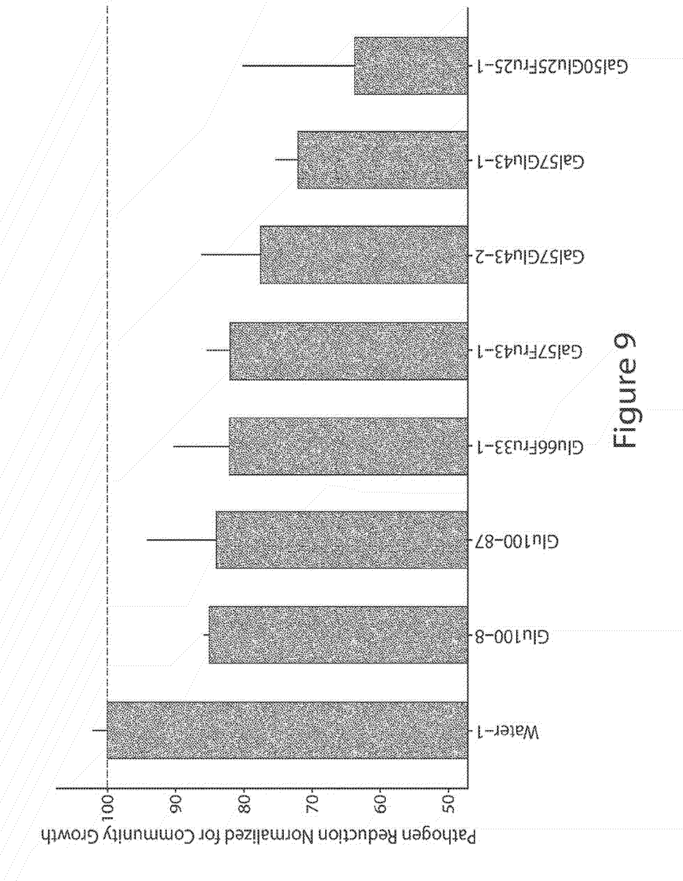

**Figure 9** is a bar graph depicting Carbapenem-resistant Enterobacteriaceae (CRE) *Klebsiella pneumoneae* growth, grown in the presence of glycans for 24 hours, normalized to control with no glycan added. Glycans that reduce growth of CRE *K. pneumoneae* by > 10% are plotted. Shown are average index (1-scaled community growth*scaled pathogen growth) normalized to control ± st.dev.

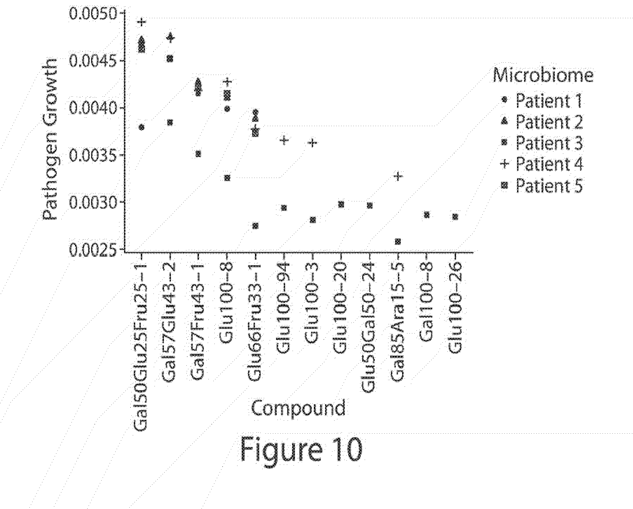

**Figure 10** is a graph depicting reduction in Carbapenem-resistant Enterobacteriaceae (CRE) *Klebsiella pneumoneae* growth when added to fecal samples from five stem cell transplant patients. All data represented are in the top 80 percentile of commensal community growth and ordered by pathogen growth reduction. Shown are 1/time to midlog for aerobic growth following anaerobic phase (pathogen growth).

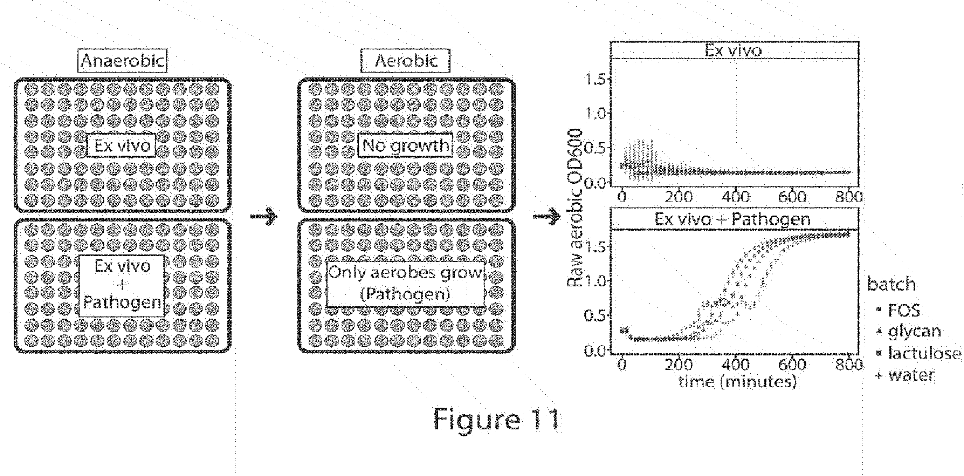

**Figure 11** is an illustration of a high-throughput screening method for identification of glycan preparations that reduce the growth rate of pathogens. Fecal slurries from healthy donors are grown in 96-well microplates under anaerobic conditions with or without pathogens (left). Then, samples are transferred to selective media (middle) and aerobic growth curves are measured (right).

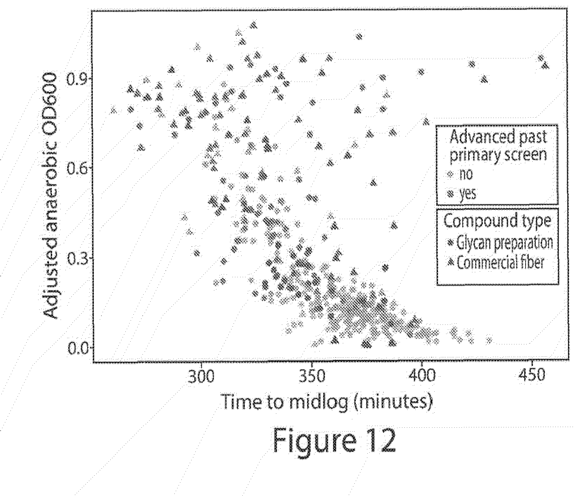

**Figure 12** is a graph providing the results from high-throughput screening efforts. Anaerobic OD600 and time to midlog, calculated from aerobic growth curves, allow selection of glycan preparations that support commensal growth over pathogen growth (e.g, selection for glycan preparations exhibiting high anaerobic OD600 and longtime to midlog).

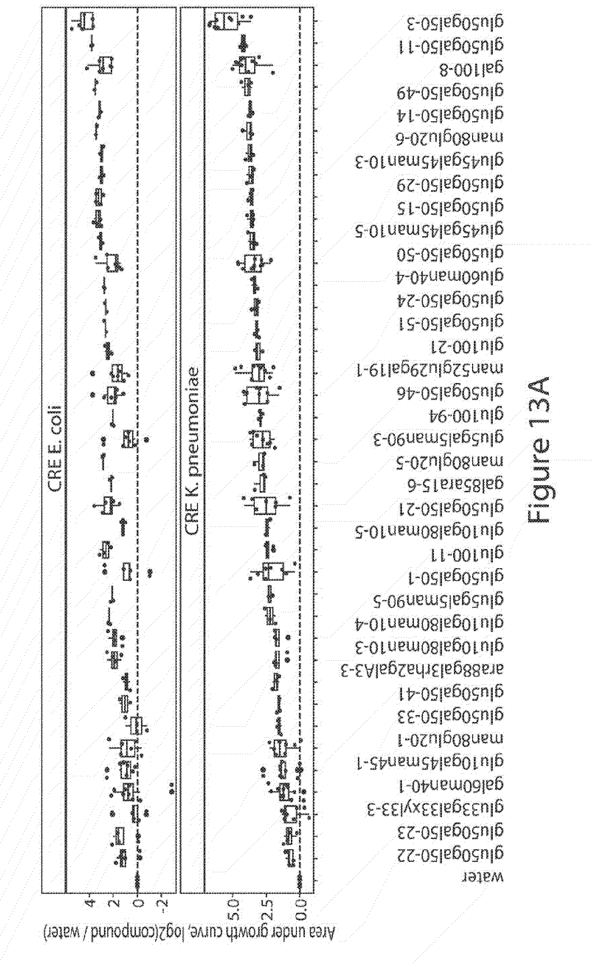

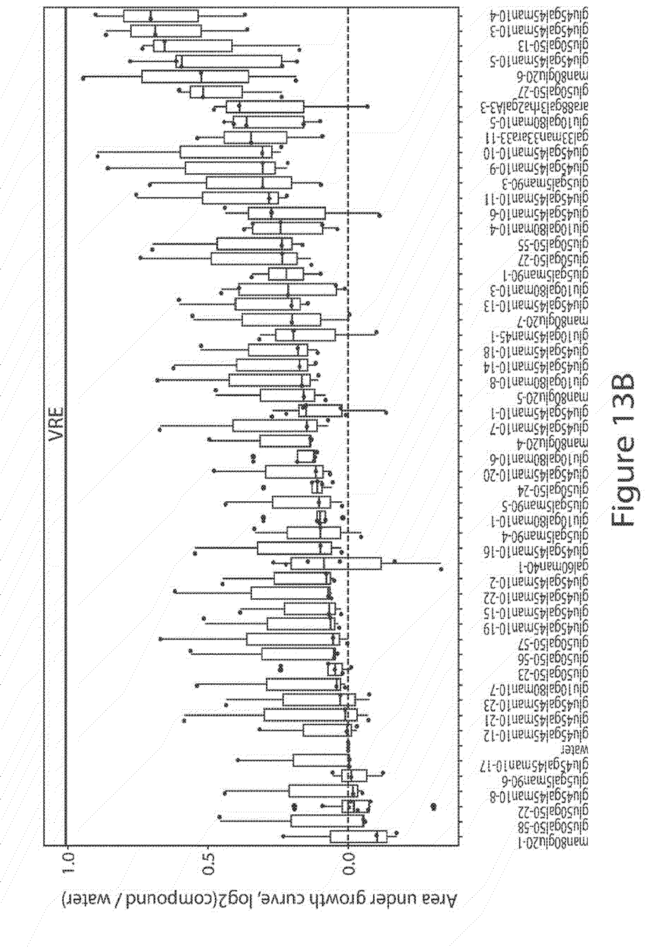

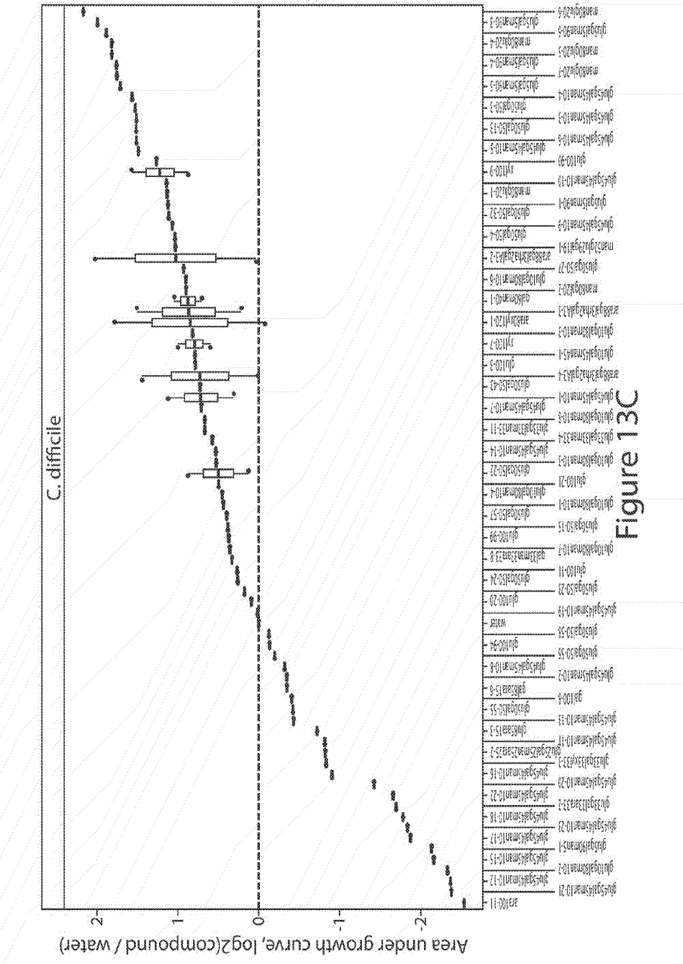

**Figures 13A-13C** are graphs showing that selected glycan preparations do not support pathogen growth (VRE and CRE). Each data point represents the area under the growth curve for a single pathogen strain grown in the presence of a glycan preparation, relative to water. Each glycan preparation was tested for growth of seven Escherichia coli (CRE E. coli) strains (Fig. 13A, upper panel), ten Klebsiella pneumoniae (CRE K. pneumoniae) strains (Fig. 13A, lower panel), and eleven Enterococcus faecium (VRE) strains (Fig. 13B), respectively. Additionally, each glycan preparation was tested for growth of two C. difficile strains (Fig. 13C).

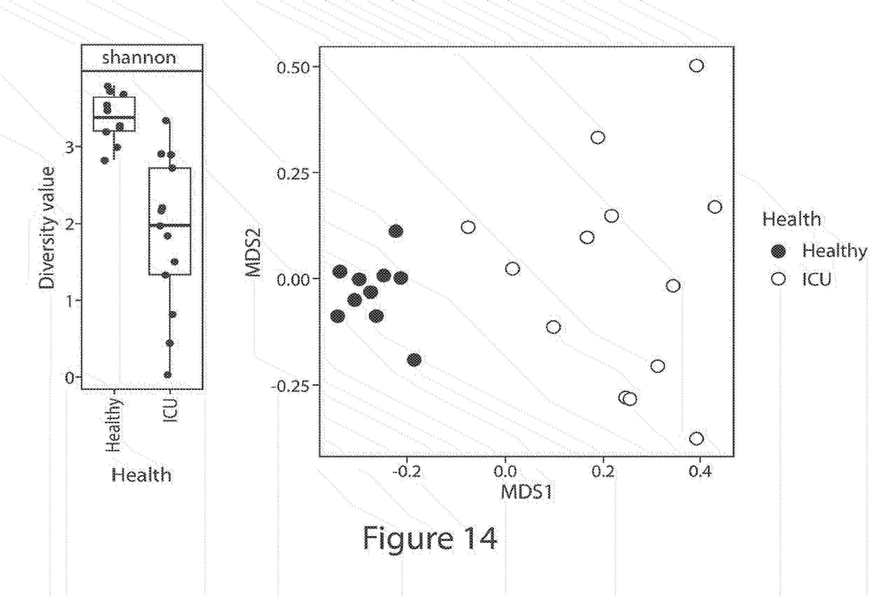

**Figure 14** provides graphs of the relative alpha-diversity of microbiome samples from healthy subjects and patients receiving antibiotics in an ICU. Notably, the microbiomes of patients receiving antibiotics in an ICU are much less diverse than those of healthy subjects (left panel) and the bacterial composition of ICU patients is highly divergent from that of healthy subjects (which are tightly clustered to the left of the PC plot) (right panel).

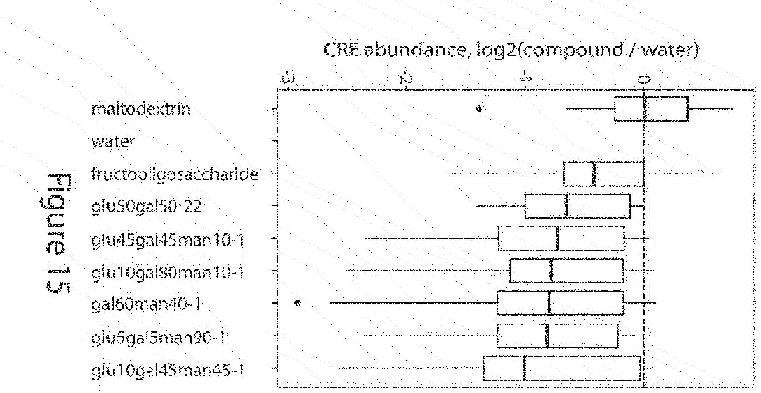

**Figure 15** is a graph showing the fold-change in relative abundance of CRE pathogens in microbiome samples from 13 patients receiving antibiotics in an ICU following incubation of the sample with selected glycan preparations and commercially available fibers, relative to control (water).

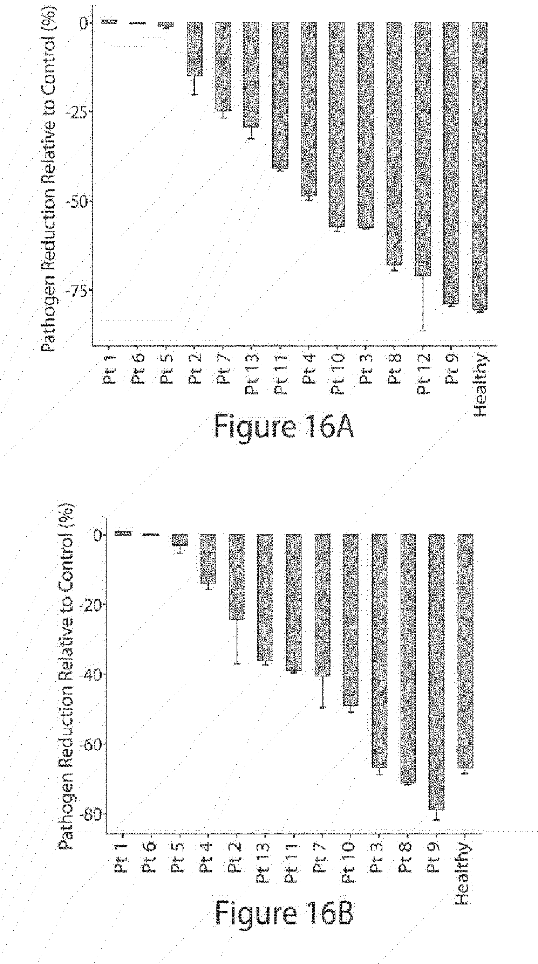

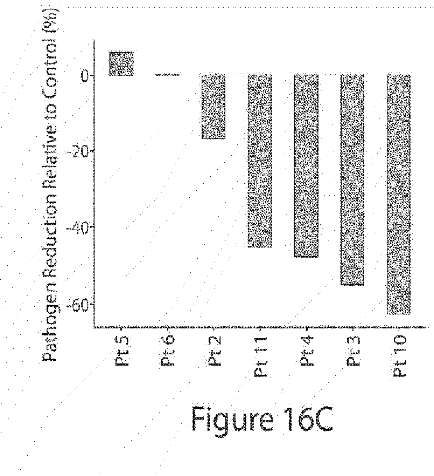

**Figures 16A-16C** are graphs showing the relative abundance of CRE pathogens in individual microbiome samples from 13 patients (Pt) receiving antibiotics in an ICU following incubation and a healthy patient following incubation of the sample with Glu45Gal45Man10 **(****Figure 16A****),** Glu10Gal80Man10 **(****Figure 16B****)** and Glu50Gal50 **(****Figure 16C****),** relative to control (water).

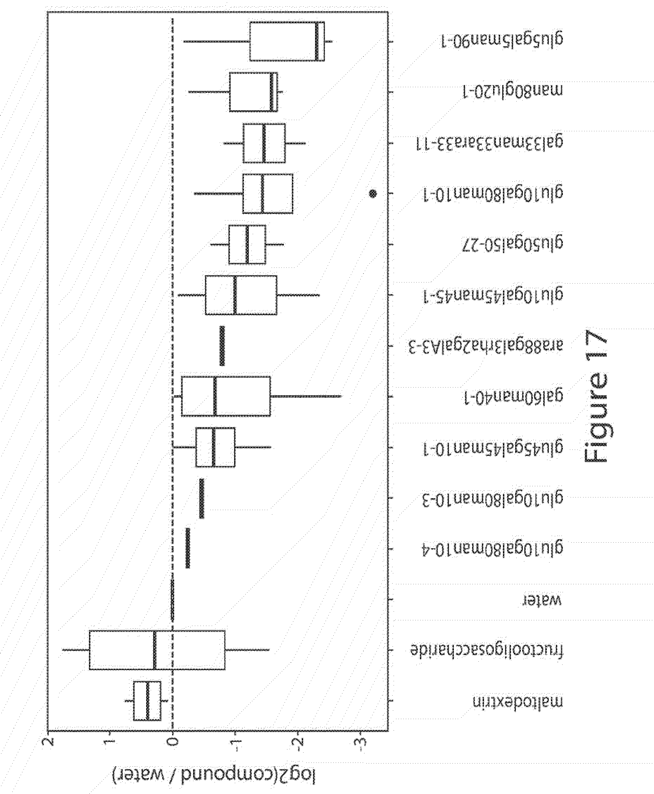

**Figure 17** is a graph showing the fold-change in relative abundance of VRE pathogens in microbiome samples from 13 patients receiving antibiotics in an ICU following incubation of the sample with selected glycan preparations and commercially available fibers, relative to control (water).

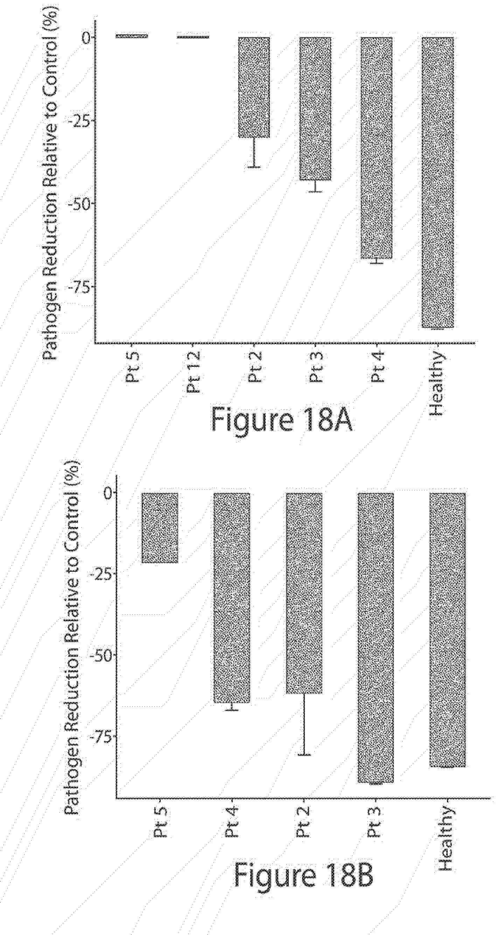

**Figures 18A-18B** are graphs showing the relative abundance of VRE pathogens in individual microbiome samples from 13 patients (Pt) receiving antibiotics in an ICU following incubation and a healthy patient following incubation of the sample with Glu45Gal45Man10 **(****Figure 18A****),** and Glu10Gal80Man10 **(****Figure 18B****)** relative to control (water).

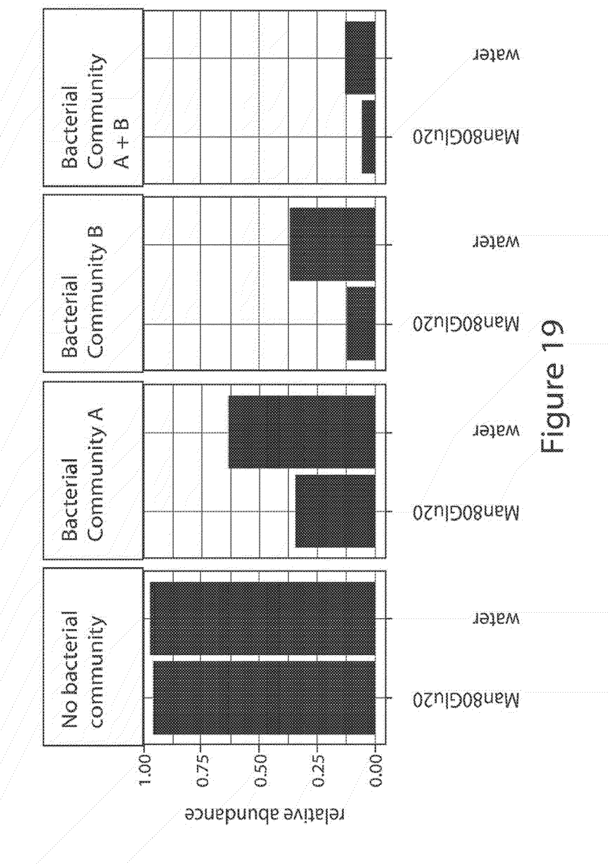

**Figure 19** is a graph showing a synbiotic effect of combining glycans with commensal bacteria to reduce the abundance of pathogens in a sample from an ICU patient with pathogen levels previously non-responsive to administration by glycan preparations.

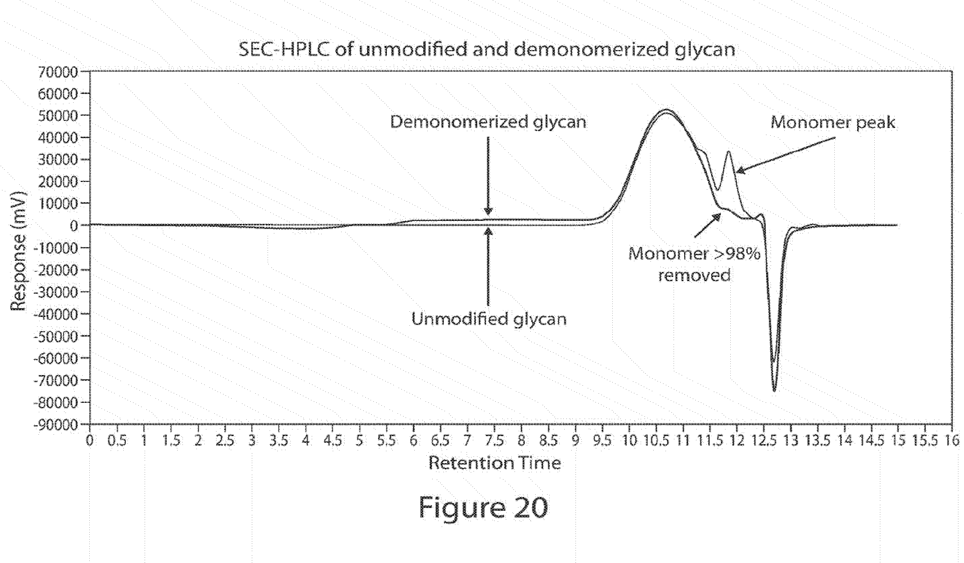

**Figure 20** depicts overlaid SEC-HPLC chromatograms of an unmodified glycan preparation and a glycan preparation that has been demonomerized by amine column flash chromatography, as described in Example 18.

### DETAILED DESCRIPTION OF THE INVENTION

Described herein are methods of modulating (the presence or level of) a pathogen in a subject (such as a facility participant), e.g., reducing or preventing colonization of a pathogen, reducing the level of a pathogen, or reducing the risk of an adverse effect (e.g., an infection) of a pathogen in a subject, by administration of a glycan preparation (e.g., a glycan preparation described herein). In some embodiments, the pathogen is a drug-resistant pathogen (e.g., an antibiotic-resistant pathogen, or a multidrug-resistant (MDR) pathogen). The methods provided are of particular utility in a facility, e.g., a healthcare facility, (e.g., an intensive care unit (ICU), a long-term (LT) care facility, and the like) or other facilities where subjects dwell in close quarters e.g., i) prison or other correctional facilities, ii) schools, university campus, camps or other educational facilities, iii) resorts, hotels, cruise ships or other travel and leisure facilities, iv) athletic facilities, e.g., athletic clubs, spas, locker rooms, team transportation, etc. or v) other facilities where drug resistant pathogens are of particular concern. In some embodiments, administration of the glycan preparation comprises administration to one or both of a first subject and a second subject. In some embodiments, the first subject and second subject comprise a first facility participant or a second facility participant. A subject as described herein (e.g., a first subject or a second subject, e.g., a first facility participant or a second facility participant) may comprise: a) a patient or resident in a healthcare facility, such as a hospital, clinic or inpatient facility; b) an individual who is a medical care giver, e.g., a medical practitioner, e.g., a physician, nurse, or physician assistant, technician or technologist; c) a housekeeping worker; d) a security worker; e) a maintenance worker; f) a food preparation worker; g) a laundry worker; h) an administrative worker, e.g., an admissions worker; i) a social worker; j) a visitor; k) a facility employee; 1) a member of, resident at, worker or staff member at any of an alternative location (i-v, e.g., correctional, educational, leisure, athletic facility) listed above, m) an individual of b)-1) who has direct contact with the subject; or n) an individual of b)-l) who does not have direct contact with the subject.

In some embodiments, the first subject (e.g., facility participant) or second subject (e.g., facility participant) comprises a patient. In some embodiments, the patient is a high-risk patient. In some embodiments, the patient is immunosuppressed. In some embodiments, the patient currently or previously has had cancer or an organ or tissue transplant. In some embodiments, the patient is located close to or is exposed to an infected or colonized patient. In some embodiments, the patient is an adult (e.g., an elderly adult). In some embodiments, the patient is a child. In some embodiments, the patient is an infant.

In some embodiments, described herein is an administration of a glycan preparation to a subject prior to entering a facility (e.g., an ICU, LT care facility, or nursing home). In some embodiments, the subject is a healthcare worker (e.g., a physician, nurse), auxiliary facility staff member (e.g., food preparation staff, cleaning staff, security staff, maintenance staff), or a patient (e.g., an incoming or prospective patient). In some embodiments, provided is a method of monitoring one or more of a) pathogen load; b) antibiotic resistance gene load; or c) response to a therapeutic preparation in a healthcare worker (of an ICU/LT care facility), an incoming/prospective patient, or a resident patient comprising: analyzing a suitable sample, e.g., a fecal sample from the subject (e.g., facility participant) for a) a pathogen, and/or b) an antibiotic resistance gene and/or microbiome analysis (e.g., the presence of one or more microbes that are known to response to a particular therapeutic preparation).

In some embodiments, provided is a method of administration of an amount of a glycan preparation effective to reduce colonization with, prevent colonization with, or reduce the risk of an adverse effect of a pathogen to one or both of a first facility-participant and a second facility participant, thereby reducing, preventing, or reducing the risk of an adverse effect of a pathogen on a facility participant. In some embodiments, provided is a method of decolonizing the gastrointestinal tract (e.g., all of the GI tract or part of the GI tract, e.g. the small intestine or the large intestine) from a pathogen or an antibiotic resistance gene carrier. In some embodiments, the method comprises shifting the microbial community in the gastrointestinal tract toward a commensal population, e.g., thereby replacing (e.g. outcompeting) a pathogen or an antibiotic resistance gene carrier.

In some embodiments, administration of the glycan preparation is to all facility participants, or a subset of thereof. In some embodiments, administration of the glycan preparation is to facility participants deemed at high risk of an adverse event such as infection or colonization. In some embodiments, administration of the glycan preparation is provided to reduce the spread of pathogen to other untreated participants. In some embodiments, the glycan preparation is administered in an effective amount and/or to a sufficient number of facility participant(s), that the spread of the pathogen, e.g., from a first facility participant to a second facility participant, is reduced. Such reduction might be measured by any of the methods described herein, from any of the samples described herein, derived from both first facility participant environments as well as those associated with the second facility participant.

In some embodiments, the glycan preparation is administered in an effective amount and/or to a sufficient number of facility-participant(s) to reduce the spread of the pathogen, e.g., from a first facility participant to an entity which can harbor the pathogen (e.g., another individual or an inanimate object, e.g., facility built surface (e.g. sink, door handle, toilet, faucet) or medical supply (e.g., a package comprising a dressing or device, or a dressing or device itself). In some embodiments, the entity is another individual. In some embodiments, the entity is an inanimate object (e.g., a facility built surface (e.g. sink, door handle, toilet, faucet) or medical supply (e.g., a package comprising a dressing or device, or a dressing or device itself).

In some embodiments, the glycan preparation is administered in an effective amount and/or to a sufficient number of facility-participant(s) to reduce the spread of the pathogen, e.g., from a second facility participant to an entity which can harbor the pathogen (e.g., another individual or an inanimate object, e.g., a facility built surface (e.g. sink, door handle, toilet, faucet) or medical supply (e.g., a package comprising a dressing or device, or a dressing or device itself). In some embodiments, the entity is another individual. In some embodiments, the entity is an inanimate object (e.g., a facility built surface (e.g. sink, door handle, toilet, faucet) or medical supply (e.g., a package comprising a dressing or device, or a dressing or device itself).

In some embodiments, provided is a method of reducing a pathogen reservoir in a subject (e.g., facility participant) by administering a glycan preparation to the subject, e.g., in an effective amount and/or to a sufficient number of subjects that the pathogen reservoir is reduced. In some embodiments, the pathogen reservoir is reduced by about 1%, about 2%, about 3%, about 4%, about 5%, about 10%, about 15%, about 20%, about 30%, about 35%, about 40%, about 45%, about 50%, about 55%, about 60%, about 65%, about 70%, about 75%, about 80%, about 85%, about 90%, about 95%, about 99%, or about 100%, e.g., relative to a reference standard. In some embodiments, the pathogen reservoir comprises the pathogen biomass.

In some embodiments, provided is a method of modulating the biomass of a pathogen or an antibiotic resistance gene carrier. In some embodiments, the modulating comprises increasing or decreasing, e.g., the biomass of a pathogen or an antibiotic resistance gene carrier. In some embodiments, the glycan preparation is administered in an effective amount and/or to a sufficient number of facility participants), that the reservoir or biomass of a pathogen is reduced. In some embodiments, the glycan preparation is administered in an effective amount that pathogen biomass is modulated, e.g., reduced (e.g., the number of pathogens and/or the number of drug- or antibiotic-resistance gene or MDR element carriers is modulated). In some embodiments, provided is a method of modulating the number of pathogens or antibiotic resistance gene carriers (e.g., in a population, e.g., a microbial population).

Exemplary pathogens include Enterobacteriaciae (e.g., a genus comprising Plesiomonas, Shigella, or Salmonella), Clostridium (e.g., a genus comprising Clostridium difficile), Enterococcus, Staphylococcus (e.g., a genus comprising Staphylococcus aureus), Campylobacter, Vibrio, Aeromonas, Norovirus, Astrovirus, Adenovirus, Sapovirus, or Rotavirus.

In some embodiments, the pathogen includes Enterobacteriaciae (e.g., a genus comprising Plesiomonas, Shigella, or Salmonella). In some embodiments, the pathogen includes Clostridium (e.g., a genus comprising Clostridium difficile). In some embodiments, the pathogen includes Enterococcus. In some embodiments, the pathogen includes Staphylococcus.

In some embodiments, the method comprises reducing the spread of a pathogen by administering to a subject (e.g., facility participant) a glycan preparation, e.g., in an effective amount and/or to a sufficient number of subjects that the spread of the pathogen is reduced. In some embodiments, the spread of a pathogen is reduced by about 1%, about 2%, about 3%, about 4%, about 5%, about 10%, about 15%, about 20%, about 30%, about 35%, about 40%, about 45%, about 50%, about 55%, about 60%, about 65%, about 70%, about 75%, about 80%, about 85%, about 90%, about 95%, about 99%, or about 100%, e.g., relative to a reference standard. In some embodiments, the spread of a pathogen comprises the spread from a first subject (e.g., a first facility participant) to a second subject (e.g., a second facility participant). In some embodiments, the spread of a pathogen comprises the spread from a first subject (e.g., a first facility participant) or a second subject (e.g., a second facility participant) to an entity which can harbor the pathogen (e.g., another individual or an inanimate object, e.g., facility built surface (e.g. sink, door handle, toilet, faucet) or medical supply (e.g., a package comprising a dressing or device, or a dressing or device itself). In some embodiments, the glycan preparation is administered in an effective amount and/or to a sufficient number of facility participant(s), that the spread of drug- or antibiotic-resistance gene, or a MDR element, e.g., from a first facility participant to a second facility participant, is reduced. This reduction might be measured by any of the methods described herein.

In some embodiments, provided is a method of reducing a drug-resistance gene reservoir (e.g., an antibiotic resistance gene reservoir or MDR gene reservoir) in a subject by administering a glycan preparation to the subject (e.g., facility participant), e.g., in an effective amount and/or to a sufficient number of subjects that the drug-resistance gene reservoir (e.g., antibiotic resistance gene reservoir or MDR gene reservoir) is reduced. In some embodiments, a drug-resistance gene reservoir (e.g., an antibiotic resistance gene reservoir or MDR gene reservoir) is reduced by about 1%, about 2%, about 3%, about 4%, about 5%, about 10%, about 15%, about 20%, about 30%, about 35%, about 40%, about 45%, about 50%, about 55%, about 60%, about 65%, about 70%, about 75%, about 80%, about 85%, about 90%, about 95%, about 99%, or about 100%, e.g., relative to a reference standard. Exemplary antibiotic resistance genes include penicillin resistance genes, MecA (conferring methicillin, penicillin and other penicillin-like antibiotic resistance) and other genes that encode the protein PBP2A (penicillin binding protein 2A), carbapenemase resistance genes (e.g., Klebsiella pneumonia carbapenemase (KPC)), betalactamase resistance genes (e.g., New Delhi betalactamase (NDM), OXA, SHV, TIM, CTX-M, VIM), vancomycin resistance genes (e.g., VanA, VanB, vancomycin resistance genes in Enterococcus), AmpC (carbapenem and beta lactam resistance genes in Enterobacteriaceae), fluoroquinoline resistance genes (e.g., Qnr), trimethoprim resistance genes (e.g. dihydrofolate reductase), sulfamethoxazole resistance genes (e.g., dihydropteroate synthetase), ciprofloxacin resistance genes, and aminoglycoside resistance genes (e.g., ribosomal methyltransferase). The reduction of a drug-resistance gene reservoir (e.g., an antibiotic resistance gene reservoir or MDR gene reservoir) may be assessed using any technique described herein, e.g., a technique described for the assessment of a pathogen reservoir.

In some embodiments, provided is a method of reducing the spread of a drug-resistance gene (e.g., an antibiotic resistance gene or MDR gene) comprising administering a glycan preparation to a subject (e.g., facility participant), e.g., in an effective amount and/or to a sufficient number of subjects that the spread of the drug-resistance gene (e.g., antibiotic resistance gene or MDR gene) is reduced. In some embodiments, the spread of an antibiotic resistance gene is reduced by about 1%, about 2%, about 3%, about 4%, about 5%, about 10%, about 15%, about 20%, about 30%, about 35%, about 40%, about 45%, about 50%, about 55%, about 60%, about 65%, about 70%, about 75%, about 80%, about 85%, about 90%, about 95%, about 99%, or about 100%, e.g., relative to a reference standard. In some embodiments, the spread of a drug-resistance gene (e.g., an antibiotic resistance gene or MDR gene) comprises the spread from a first subject (e.g., a first facility participant) to a second subject (e.g., a second facility participant). In some embodiments, the glycan preparation is administered in an effective amount and/or to a sufficient number of facility participant(s), that the rate at which a drug- or antibiotic-resistance gene, or an MDR element, is transferred from a first pathogen to a second pathogen is reduced. This transfer might be measured by showing the presence of a similar gene or toxin, identified by any of the methods described herein, present in a second pathogen distinct from the first pathogen. This distinction can be at the level of organism identification (e.g., metabolite production, species identity, or susceptibility to antibiotics), or by molecular methods to show other differences, such as any of those described herein.

In some embodiments, provided is a method of reducing the rate at which a pathogen causes infection (e.g., in a subject, e.g., facility participant) by administering a glycan preparation to the subject, e.g., in an effective amount and/or to a sufficient number of subjects that the rate of infection is reduced. In some embodiments, the glycan preparation is administered in an effective amount and/or to a sufficient number of facility participant(s), that the rate at which a pathogen causes infection, or the severity of pathogen infection, as indicated by assessment of symptoms associated with infection, is reduced. In some embodiments, the rate of infection is reduced by about 1%, about 2%, about 3%, about 4%, about 5%, about 10%, about 15%, about 20%, about 30%, about 35%, about 40%, about 45%, about 50%, about 55%, about 60%, about 65%, about 70%, about 75%, about 80%, about 85%, about 90%, about 95%, about 99%, or about 100%, e.g., relative to a reference standard.

Reduction in the rate of infection using a method described herein may be prospective or retrospective, e.g., relative to an infection. In some embodiments, the method described herein comprises monitoring a subject (e.g., facility participant) or a population of subjects for a similar infection, e.g., through observation of similar symptoms or similar features to those known to be caused by or identified with a pathogen of interest. Rather than, or in addition to using clinical characteristics, any of the methods described herein might be used to more specifically determine the type of the pathogen involved, and its relationship -if any- to spread or a reservoir.

In some embodiments, provided is a method of modulating the gastrointestinal tract (e.g., all of the GI tract or a part thereof, e.g., the small intestine, the large intestine, the colon, and the like) of a subject (e.g., facility participant). In some embodiments, the method comprises modulating the environment (e.g., chemical or physical environment) of the gastrointestinal tract of a subject to make the gastrointestinal tract (and the microbial community therein) less selective or less receptive for a pathogen or an antibiotic resistance gene carrier. In some embodiments, the method further comprises administering a second agent in combination with a glycan preparation, e.g., charcoal or an antibiotic-degrading enzyme (e.g., beta-lactamase), or a synbiotic (e.g., an engineered beta-lactamase (e.g., a non-infectious beta-lactamase).

In some embodiments, provided is a method of reducing the transfer of a drug-resistance gene (e.g., an antibiotic resistance gene or an MDR gene) from one organism (e.g., bacterial taxa, e.g., taxa containing the antibiotic resistance gene or an MDR gene) to another organism (e.g., taxa that do not contain the antibiotic resistance gene or an MDR gene) by administering a glycan preparation to a subject (e.g., facility participant), e.g., in an effective amount and/or to a sufficient number of subjects that the transfer of the drug-resistance gene (e.g., an antibiotic resistance gene or MDR gene) is reduced. In some embodiments, the method comprises reducing the transfer of a drug-resistance gene (e.g., an antibiotic resistance gene or an MDR gene) to an organism with increased pathogenic potential. In some embodiments, the method comprises reducing the number of recipient bacteria, (e.g., commensal bacterial strains), capable of taking up an antibiotic resistance gene, in a subject (e.g., facility participant). In some embodiments, the method comprises reducing the probability of a pathogen or an antibiotic resistance gene carrier to spread or transfer an antibiotic resistance gene. In some embodiments, the method comprises reducing the ability of a pathogen or an antibiotic resistance gene carrier to reach a state of competency. Competency refers to the bacteria's ability to take up genes (e.g., antibiotic resistance genes) from the environment (von Wintersdorff et al. Front. Microbiol. (2016); 7: 173). In some embodiments, the method comprises reducing exchange of gene material (e.g., conjugation-based) in a pathogen or an antibiotic resistance gene carrier. In some embodiments, the method comprises reducing the level of free nucleic acid (e.g. microbial DNA, e.g., comprising an antibiotic resistance gene cassette), in a pathogen or antibiotic resistance gene carrier, e.g., after the pathogen or antibiotic resistance gene carrier reaches competency. In some embodiments, the method comprises increasing microbial metabolism of a nucleic acid (e.g. microbial DNA, e.g., comprising an antibiotic resistance gene cassette). In some embodiments, the method comprises use of a nucleic acid binding molecule as a scavenger, e.g., for binding to a pathogen-derived or antibiotic resistance gene carrier-derived nucleic acid.

In some embodiments, provided is a method of reducing pathogen infectivity, as determined by the incidence of the number of pathogens in a population. In some embodiments, the glycan preparation is administered in an effective amount to reduce the number of pathogen cells that can transmit a drug or antibiotic resistance gene, or MDR element, to another organism. In some embodiments, the glycan preparation is administered in an effective amount to reduce the number of organisms (e.g. bacteria) that can receive a drug or antibiotic resistance gene, or MDR element. In some embodiments, the glycan preparation is administered in an effective amount to reduce the ability of a pathogen cell to enter the state in which it can donate a drug or antibiotic resistance gene, or MDR element, to another organism. In some embodiments, the glycan preparation is administered in an effective amount to reduce the ability of a pathogen cell to enter the state in which it can receive a drug or antibiotic resistance gene, or MDR element, from another organism.

In some embodiments, provided is a method of reducing the presence of a drug or antibiotic resistance gene, or MDR element in a microbe (e.g., a pathogen, e.g. a bacterial pathogen) or microbial population. In some embodiments, the glycan preparation is administered in an effective amount to reduce the copy number of a drug or antibiotic resistance gene, or MDR element, in a microbe (e.g. a bacterial pathogen, on a cell-by-cell basis) or reduce the total number in a microbial population. In some embodiments, the glycan preparation is administered in an effective amount to increase the population of a gut microbe that is not a (potential) host for a drug or antibiotic resistance gene, or MDR element. In some embodiments, the glycan preparation is administered in an effective amount to reduces the competence of a pathogen, e.g., *Streptococcus,* to take up a drug or antibiotic resistance gene, or MDR element. In some embodiments, the glycan preparation is administered in an effective amount to reduces the ability of a bacterial cell (e.g., a pathogen), e.g., a gram-negative organism, e.g., *E. coli* or *Klebsiella,* to take up a drug or antibiotic resistance gene, or MDR element.

In some embodiments, the glycan preparation is administered in an effective amount to shift the microbial community of a facility participant to displace or inhibit a pathogen, an organism that can donate a drug or antibiotic resistance gene, or MDR element (donor microbes), or an organism that can receive a drug or antibiotic resistance gene, or MDR element (recipient microbes). In some embodiments, the glycan preparation is administered in an effective amount to reduce the probability of donor microbes to spread a drug or antibiotic resistance gene, or MDR element.

In some embodiments, provided is a method of managing an infection by a pathogen. In some embodiments, managing an infection by a pathogen comprises treating, preventing, and/or reducing the risk of developing an infection by a pathogen. In some embodiments, treating an infection by a pathogen comprises administering a glycan preparation to a subject (e.g., facility participant) or population upon detection of a pathogen. In some embodiments, preventing an infection by a pathogen comprises administering a glycan preparation to a subject (e.g., facility participant) or population at risk of developing an infection. The subject or population may include those who may have been exposed to the pathogen directly and/or infected individuals. In some embodiments, reducing the risk of developing an infection by a pathogen comprises administering a glycan preparation to a subject or population that may become exposed to a pathogen.

In some embodiments, provided is a method to reduce the expression or release (e.g., by a pathogen) of a factor having an adverse effect on a subject (e.g., facility participant) such as a virulence factor or toxin. In some embodiments, the factor causes a disease. In some embodiments, a glycan preparation is administered in an effective amount and/or to a sufficient number of facility participants), that the expression or release by a microbe (e.g., a pathogen) of a factor having an adverse effect on a facility participant, e.g., a virulence factor or a toxin, e.g., that causes disease, is reduced. In some embodiments, the expression of a factor (e.g., a virulence factor) is reduced by about 1%, about 2%, about 3%, about 4%, about 5%, about 10%, about 15%, about 20%, about 30%, about 35%, about 40%, about 45%, about 50%, about 55%, about 60%, about 65%, about 70%, about 75%, about 80%, about 85%, about 90%, about 95%, about 99%, or about 100%, e.g., relative to a reference standard. An adverse effect in a subject (e.g., facility participant), by such a factor, includes causing a disease, delaying diagnosis of a disease, or reducing the effectiveness of a disease treatment.

Exemplary virulence factors include Shiga toxin, *E. coli* heat labile toxin, and *Clostridium difficile* Toxin A and B, and similar toxins that may cause a disease. Additional factors having an adverse effect include those mediating resistance to antibiotics, which might delay response to treatment or the identification of an effective treatment. Some common resistance genes are markers such as, e.g., MecA (methicillin resistance in Staphylococcus), KPC, NDM, OXA, SHV, TIM, CTX-M, VIM and AmpC (carbapenem and beta lactam resistance in Enterobacteriaceae), VanA and VanB (vancomycin resistance in Enterococcus). Reduction in these markers might be assessed by culturing on selective media (e.g., containing antibiotics), such as ChromID VRE or ChromID Carba, or media supplemented with cephalosporins or other beta lactams. Alternatively, it might be assessed by antibiotic susceptibility testing of strains isolated in the course of normal clinical care or surveillance of participants, healthcare workers, or the healthcare environment, thereby determining the presence of resistance or a toxin. Additionally, it may be assessed by molecular methods to quantify (or determine the presence or absence of) specific genes, for instance by PCR, quantitative PCR, digital droplet PCR, hybridization, or similar methods. Such methods are in widespread use and some available methods are have received FDA aproval for diagnostic use.

In some embodiments, provided is a method of modulating the number of gene donors (donor microbes) in a population (e.g., the state of competency/conjugation). In some embodiments, provided is a method of modulating the number of gene recipients (recipient microbes) in a population. In some embodiments, a glycan preparation is administered in an effective amount to reduce the number of donor microbes (e.g., microbes that carry drug- or antibiotic-resistance genes or MDR elements). In some embodiments, a glycan preparation is administered in an effective amount to reduce the number of recipient microbes. For example, a glycan preparation may affect bacterial activity such that it reduces the frequency of transfer of toxins or determinants of antibiotic resistance between strains or species. This may be accomplished, for instance by transformation, conjugation, phage production or transduction. plasmid release or plasmid replication, such that fewer pathogens are able to access these toxins or resistance determinants. This in turn may reduce the availability of those markers to new pathogens. Such a reduction might be accomplished by modulating a state of competency or conjugation property.

In some embodiments, provided is a method of modulating the copy number of a resistance gene in a population. In some embodiments, the glycan preparation is administered in an effective amount that the copy number of a drug- or antibiotic-resistance gene, toxin or virulence factor is reduced. For example, when fewer copies of the same genetic element are present there is generally a decline in its expression. Thus, a glycan preparation resulting in decreased copy number of a drug or antibiotic resistance gene, toxin or virulence factor, might be expected to increase susceptibility to an antibiotic, reduce adverse effects of a pathogen, or reduce the availability of the gene, toxin or virulence factor to other microbial recipients. This might occur, for instance, due to reduced activity of or expression of addiction modules or other elements of importance for maintaining the copy number of the gene, toxin or resistance marker.

In some embodiments, provided is a method of modulating the fitness deficit (e.g., increase the burden of carrying a drug- or antibiotic-resistance gene or MDR element) of a population. In some embodiments, the modulating comprises increasing the burden of carrying a resistance gene. In some embodiments, the glycan preparation is administered in an effective amount that the fitness deficit is increased. A glycan preparation that increases the fitness deficit (e.g., caused by carrying or expressing a toxin, virulence factor or antibiotic resistance determinant) reduces the number of microbes (e.g., bacterial pathogens) carrying it, or their ability to persist in particular subjects (e.g., facility participants). In some embodiments, the glycan preparation alters the ecology of the GI tract (or a subset thereof, e.g., small intestine, large intestine, or colon) such that nitrogen sources is in short supply. This in turn can increase the cost of maintaining additional genetic elements by nucleic acid synthesis. In some embodiments, the fitness deficit results from enhanced recognition or response by the host (e.g., a human subject). For example, some factors, such as bacterial lipopolysaccharide (LPS) are directly recognized by human cells, resulting in immune responses.

Some pathogens (e.g., viruses and bacteria, e.g., Vibrio cholerae and Norovirus) have been shown to have glycan receptors, or glycan moieties that are necessary to infect gut cells (Holmer, et al. FEBS Letters 584 (2010) 2548-2555). In some embodiments, provided is a method of decreasing the binding of a pathogen to a cell, decreasing the activity of a pathogen on or in a cell, decreasing the entry of a pathogen into or onto a cell, or decreasing the effect of a pathogen on a cell, wherein the method comprises administering of a glycan preparation in an effective amount and/or to a sufficient number of subjects to decrease the binding of a pathogen, its activity, entry into, or effect on a cell. In some embodiments, the cell is a human cell. In some embodiments, the glycan preparation binding to a pathogen prevents said pathogen or another pathogen from reaching and entering a cell. In other embodiments, without being bound by theory, a glycan preparation may directly or indirectly induce a modification of the gut lining or the mucous membrane, or affect another property such that pathogen entry into a cell, pathogen effect on a cell, the ability of a pathogen to persist within a cell or avoid antibody or immune recognition.

In some embodiments, provided is a method of modulating the anti-microbial output (e.g., immune response) of a subject. For example, a glycan preparation is administered to increase mucus production, or antibody production or secretion, or the production of antimicrobial peptides (e.g., such as RegIIIγ) thereby increasing resistance to pathogens. RegIIIγ is an antimicrobial protein that binds intestinal bacteria via interactions with peptidoglycan carbohydrate (Cash et al., Science. (2006) August 25; 313(5790): 1126-1130).

In some embodiments, provided is a method of modulating the microbial community composition and/or the metabolic output of the microbial community, e.g. modulating the environment, e.g., to modulate (e.g., reduce) pathogen growth. In some embodiments, a glycan preparation is administered in an effective amount to modulate the microbial community and alter the environment of the GI tract, (e.g., altering pH, altering lactic acid, altering microbial density, etc.). In some embodiments, the method comprises outcompeting a pathogen or an antibiotic resistance gene carrier for space or nutrients in the gastrointestinal tract. In some embodiments, a glycan preparation is administered in an effective amount to reduce the "space" for a pathogen to colonize, e.g., physical space. In some embodiments, the method comprises malting non-pathogenic bacteria fitter (e.g., providing a more selective food source or encouraging growth of fitter (e.g., faster) growing species/strains). In some embodiments, the method comprises outcompeting a pathogen or an antibiotic resistance gene carrier by increasing the population of a commensal bacterial strain, or by increasing an anti-microbial defense mechanism in a commensal bacterial strain, e.g., production of a bacteriocin, anti-microbial peptide, hydrogen peroxide, or low pH (e.g., through increased level of an acid (e.g., acetate, butyrate, and the like).

In some embodiments, provided are methods of reducing the spread of pathogens. In some embodiments, pathogens include bacterial pathogens (e.g., Abiotrophia spp., (e.g., A. defective), Achromobacter spp., Acinetobacter spp., (e.g., A. baumanii), Actinobaculum spp., (e.g., A. schallii), Actinomyces spp., (e.g., A. israelii), Aerococcus spp., (e.g., A. urinae), Aeromonas spp., e.g., A. hydrophila, Aggregatibacter spp., e.g. A. aphrophilus, Bacillus anthracis, Bacillus cereus group, Bordetella spp., Brucella spp., e.g. B. henselae, Burkholderia spp., e.g., B. cepaciae, Campylobacter spp., e.g., C. jejuni, Chlamydia spp., Chlamydophila spp., Citrobacter spp., e.g., C. freundii, Clostridium botulinum, Clostridium difficile, Clostridium perfringens, Corynebacterium spp., e.g., C. amycolatum, Cronobacter, e.g., C. sakazakii, Enterobacteriaceae, including many of the genera below, Ehrlichia spp., Enterobacter spp., e.g., E. cloacae, Enterococcus spp., e.g. E. faecium, Escherichia spp., including enteropathogenic, uropathogenic, and enterohemorrhagic strains of E. coli, Francisella spp., e.g. F. tularensis, Fusobacterium spp., e.g. F. necrophorum, Gemella spp., e.g. G. mobilloram, Granulicatella spp., e.g. G. adiaciens, Haemophilus spp., e.g. H. influenza, Helicobacter spp., e.g. H. pylori, Kingella spp., e.g. K. kingae, Klebsiella spp., e.g. K. pneumoniae, Legionella spp., e.g. L. pneumophila, Leptospira spp., Listeria spp., e.g. L. monocytogenes, Morganella spp., e.g. M. morganii, Mycobacterium spp., e.g. M. abcessus, Neisseria spp., e.g. N. gonorrheae, Nocardia spp., e.g. N. asteroids, Ochrobactrum spp., e.g. O. anthropic, Pantoea spp., e.g. P. agglomerans, Pasteurella spp., e.g. P. multocida, Pediococcus spp., Plesiomonas spp., e.g. P. shigelloides, Proteus spp., e.g. P. vulgaris, Providencia spp., e.g. P. stuartii, Pseudomonas spp., e.g. P. aeruginosa, Raoultella spp., e.g. R. ornithinolytica, Rothia spp., e.g. R. mucilaginosa, Salmonella spp., e.g. S. enterica, Serratia spp., e.g. S. marcesens, Shigella spp., e.g. S. flexneri, Staphylococcus aureus, Staphylococcus lugdunensis, Staphylococcus pseudintermedius. Staphylococcus saprophyticus, Stenotrophomonas spp., e.g. S. maltophilia, Streptococcus agalactiae, Streptococcus anginosus, Streptococcus constellatus. Streptococcus dysgalactiae, Streptococcus intermedius, Streptococcus milleri, Streptococcus pseudopneumoniae, Streptococcus pyogenes, Streptooccus pneumoniae, Treponema spp., Ureaplasma ureolyticum, Vibrio spp., e.g. V. cholerae, and Yersinia spp., (e.g., Y. enterocolitica)); viral pathogens (e.g., Adenovirus, Astrovirus, Cytomegalovirus, Enterovirus, Norovirus, Rotavirus, and Sapovirus); and gastrointestinal pathogens (e.g., Cyclospora spp., Cryptosporidium spp., Entamoeba histolytica, Giardia lamblia, and Microsporidia, (e.g., Encephalitozoon canaliculi)).

In some embodiments, the method comprises reducing the spread of antibiotic resistant organisms. Antibiotic resistant organisms include: Beta-lactamase producing Enterobacteriaceae (including extended spectrum beta lactamase and carbapenemase producers, possessing genes such as TIM, OXA, VIM, SHV, CTX-M, KPC. NDM or AmpC); Vancomycin-resistant Enterococcus (e.g., possessing genes such as VanA or VanB); Fluoroquinolone-resistant Enterobacteriaceae (e.g., with genes such as Qnr); Carbapenem-resistant and multidrug resistant Pseudomonas; Methicillin-resistant Staphylococcus aureus and Streptococcus pneumoniae (e.g., possessing the MecA gene); Multidrug resistant Acinetobacter (often containing beta lactamase); Trimethoprim resistant organisms (e.g., dihydrofolate reductase); Sulfamethoxazole resistant organisms (e.g., dihydropteroate synthetase); and Aminoglycoside resistant organisms (e.g., ribosomal methyltransferase).

In some embodiments, provided is a method to manage an infection by a pathogen comprising, administering to a first and/or second facility participant, a second treatment. In some embodiments, the second treatment comprises administering charcoal, or other adsorbing agent. In this embodiment, the adsorbing agent might serve to reduce the presence of antibiotic within the GI tract (e.g., small intestine, large intestine, colon), so as to reduce the selective pressure of maintaining a resistance determinant, thereby allowing its reservoir, level, spread or adverse effect to be reduced. Alternatively, the adsorbing agent might increase the beneficial effect of the glycan preparation. In some embodiments, the second treatment comprises administering a nonabsorbable antibiotic such as a beta lactam, or a beta lactamase inhibitor to a subject (e.g., facility participant). In some embodiments, the second treatment comprises administering an antibiotic-degrading enzyme, e.g., beta-lactamase enzyme.

In some embodiments, the subject is critically ill and/or a transplant patient. Critically ill subjects and/or transplant patients are prone to infections (e.g. have a high rate of infections), such as bloodstream infections. In some embodiments, infectious microbes are carried in the gut (e.g., can be acquired through colonization) and include *E. coli, Klebsiella,* other Enterobacteriaceae, and *Enterococcus.* In some embodiments, the microbes (e.g., pathogens) are drug resistant (e.g. carbapenem-resistant Enterobacteriaceae, vancomycin-resistant Enterococcus).

In some embodiments, assessment of colonization (e.g., with pathogens) is used to predict the risk of infection (e.g., bloodstream infection, urinary tract infection (UTI), or respiratory infection, bacteremia), e.g., by correlating levels of colonization (e.g., by assessing a suitable sample for presence or absence of predetermined bacterial taxa and/or assessing pathogen load) with risk of infection, wherein evidence of colonization is correlated with an increased risk of infection, wherein culture-negative subjects are at lower risk of infection. In some embodiments, higher levels of bacteria lead to higher rates of infection. In some embodiments, intestinal colonization (e.g. by a pathogen, e.g. VRE) precedes infection in other tissues (e.g., bloodstream). Examples of gastrointestinal tract-colonizing pathogens may include: Enterobacteriaceae (e.g. *E. coli, Klebsiella, Enterobacter, Proteus)* and Enterococcus. In some embodiments, gastrointestinal tract-colonizing pathogens further include multidrug resistant bacteria (e.g., Carbapenem resistant Enterobacteriaceae, Vancomycin resistant Enterococcus).

In some embodiments, the outcome of screening subject populations for pathogen status determines the course of bloodstream infection management. In some embodiments, screening methods comprise stool sampling (e.g. by rectal swab) of subjects. In some embodiments, the method comprises assessing the presence/absence (abundance) of drug/antibiotic resistant pathogens (e.g., VRE) in the stool. In some embodiments, the level of pathogens within the gut is correlated with infection risk. In some embodiments, intensive care unit (ICU) subjects, transplant subjects, chemotherapy-receiving subjects, and antibiotic-receiving subjects have a higher risk of having pathogen colonization from antibiotic resistant bacteria such as carbapenem resistant Enterobacteriaciae and Vancomycin-resistant Enterococcus. In some embodiments, reducing the level of pathogens within the gut reduces risk (e.g., by administering a glycan preparation if desired in combination with an antibiotic). In some embodiments, if the drug resistant pathogen is absent, the subject is administered a glycan preparation to prevent infection (e.g., bloodstream infection) or bacteremia. In some embodiments, if the drug resistant pathogen is present, the subject is administered a glycan preparation to reduce infection (e.g., bloodstream infection) or bacteremia.

In some embodiments, provided is a method to reduce the colonization level or prevalence of antibiotic resistant pathogens carried in the GI tract of high-risk subjects (e.g. facility participants). Exemplary antibiotic resistant pathogens include Carbapenem-resistant Enterobacteriaciae (e.g., extended spectrum beta lactamase (ESBL) producing Enterobacteriaciae) and Vancomycin-resistant Enterococcus.

In some embodiments, provided is a method to reduce the rate of infections (e.g., from pathogens that colonize the GI tract) in critically ill or high-risk subjects (e.g. facility participants). In some embodiments, the method comprises reducing the rate of urinary tract infections. In some embodiments, the method comprises reducing the rate of bloodstream infections. In some embodiments, the method comprises reducing the rate of respiratory tract infections.

In some embodiments, the method comprises managing infections in subjects. Examples of subject groups with infections (bacteremia) include: subjects with urinary infections (e.g., infected with Enterococcus, Enterobacteriaciae), subjects with bloodstream infections (e.g., infected with Enterococcus, Enterobacteriaciae), transplant subjects (e.g., bone marrow (e.g., undergoing hematopoietic stem cell transplantation), solid organ (e.g., liver)), intensive care patients (e.g., infected with Carbapenem resistant Enterobacteriaciae and ESBL producing pathogens), pre-transplant liver failure patients (e.g., infected with Vancomycin resistant Enterococcus), post-transplant liver failure patients (e.g., infected with Vancomycin resistant Enterococcus). Subjects undergoing chemotherapy experience high levels of enteric pathogen bacteremia, *C*. *difficile* infection (CDI)C, and chemotherapy-induced diarrhea compared to other subjects (e.g., the general hospital patient population). In some embodiments, antibiotic-treated subjects comprise higher pathogen loads, including antibiotic resistant pathogens. In some embodiments, subjects undergoing or about to undergo a transplant, subjects with cancer, subjects with liver disease (e.g., end-stage renal disease), or subjects with suppressed immune system (*e.g*., immunocompromised subjects) may have high risk of developing infections, *e.g*., gut-derived infections. In some embodiments, the method comprises prophylactic treatment, *e.g*., with a glycan preparation, of a subject, *e.g*., a subject with a high risk of developing an infection. In some embodiments, subjects who are undergoing chemotherapy or antibiotic treatment have reduced diversity of commensal bacteria. In some embodiments, the method comprises treatment of a subject to reduce the colonization of pathogens, e.g., multidrug resistant pathogens, in a subject, *e.g*., subjects in a facility, *e.g*., a hospital or long-term care facility. In some embodiments, the method comprises treatment of a subject to reduce the transmission of pathogens, *e.g*., multidrug resistant pathogens, from a first subject to a second subject, *e.g*., subjects in a facility, *e.g*., a hospital or long-term care facility. In some embodiments, bacteria that pose a risk of colonization in subjects (or a capable of colonizing the GI tract of subjects) comprise resistant subpopulations of Enterobacteriaceae (e.g., *E*. *cloacae* and Enterococcus), C. difficile (including Nap1 (pandemic hypervirulent) *C*. *difficile* strain), and bacteria that cause infectious diarrhea (e.g., *Campylobacter, Salmonella, Shigella,* enterohemorrhagic *E. coli* (EHEC), enterotoxigenic *E. coli* (ETEC), enteropathogenic *E*. *coli* (EPEC), enteroinvasive *E*. *coli* (EIEC), enteroaggregative *E*. *coli* (EAEC), diffusely adherent *E*. *coli* (DAEC), and uropathogenic E. coli).

In some embodiments, the method comprises managing infections in subjects with other disorders. In some embodiments, the method comprises managing infections in subjects with excess ammonia levels. In some embodiments, subjects with hepatic encephalopathy (HE) may be treated according to the methods provided herein. In some embodiments, the subject has HE as a consequence of liver cirrhosis. In some embodiments, such subjects display hepatic multiple adverse neurological symptoms that occur when the liver is unable to remove toxic substances such as ammonia from the blood. In some embodiments, the subjects have or are suspected of having minimal HE. In some embodiments, the subjects have or are suspected of having overt HE. Standard-of-care treatments for overt HE include lactulose, lactitol, and antibiotics (e.g., rifaximin or neomycin). Treatments may also include dietary modifications and probiotics. In some embodiments, such methods result in decreased incidence of future episodes of ammonia crisis, or, in subjects at risk of HE, by decreased occurrence of an initial episode of ammonia crisis.

In some embodiments, the method comprises managing infections in subjects who are in need of an organ transplant, *e.g*., a liver or bone marrow transplant. In some embodiments, the method comprises managing infections in subjects immediately, or shortly, before said subject receives an organ transplant, *e.g*., a liver or bone marrow transplant. In some embodiments, the method comprises managing infections in subjects immediately, or shortly, after said subject receives an organ transplant, *e.g*., a liver or bone marrow transplant. In some embodiments, the method comprises managing infections in subjects who have, are suspected of having, or at risk of having end-stage liver disease (ESLD).

In some embodiments, efficacy of a treatment provided herein may be assessed by resolution of the symptoms or diagnostic criteria listed above (e.g., reduction in serum ammonia levels). In some embodiments, one or both of the following is reduced: i) the number of ammonia crises over a period of 1 year (e.g., by at least 1, 2, or at least 3 crises), ii) the severity of complications from ammonia crises, including neurodevelopmental delays and/or cognitive declines (e.g., compared to a suitable control group not receiving the oligosaccharide composition). In some embodiments, the time period between ammonia crises is increased, e.g., by at least 15%, 30%, 60%, 100%, or 200% (e.g., compared to a suitable control group not receiving the oligosaccharide composition).

In some embodiments, the method reduces the abundance of pathogens and increases the relative of abundance of commensal bacteria, e.g., in a subject, e.g., in the gastrointestinal tract of the subject (e.g. the colon). In some embodiments, the method increases the alpha-diversity (*e.g.,* a high degree of diversity) of a microbial community (e.g., a community of commensal bacteria), *e.g*., of the gut of a subject.

In some embodiments, glycan preparations are substantially fermented or consumed by commensal bacteria and are not fermented or consumed by pathogens. In some embodiments, a glycan preparation that is substantially consumed by commensal bacteria may increase the diversity and biomass of the commensal microbiota and lead to a reduction in the relative abundance of a pathogen(s), such as a bacterial pathogen (e.g., a pathogenic taxa). In some embodiments, a glycan preparation is substantially non- fermented or not consumed by VRE or CRE species. In some embodiments, a glycan preparation is substantially non-fermented or not consumed by *C. difficile.*

In some embodiments, a glycan preparation supports the growth of commensal or probiotic bacteria, *e.g*., in a gut microbiome. In some embodiments, a glycan preparation does not support the growth of at least one pathogen, *e.g*., does not support the growth of a CRE, VRE, and/or C. difficile species.

In some embodiments, administration of a glycan preparation may increase the concentration,amount or relative abundance of commensal bacteria relative to pathogenic bacteria in the microbiome of a subject (e.g., a human patient). In some embodiments, administration of a glycan preparation and a population of viable commensal or probiotic bacteria may increase the concentration, amount, or relative abundance of commensal bacteria relative to pathogenic bacteria in the microbiome of a subject (e.g., a human patient). In some embodiments, administration of a glycan preparation that supports the growth of commensal or probiotic bacteria, *e.g*., in a gut microbiome, may increase the concentration, amount or relative abundance of commensal bacteria relative to pathogenic bacteria in the microbiome of a subject (e.g., a human patient). In some embodiments, administration of a glycan preparation that does not support the growth of at least one pathogen, *e.g*., does not support the growth of a CRE, VRE, and/or C. difficile species, *e.g*., in a gut microbiome, may increase the concentration, amount or relative abundance of commensal bacteria relative to pathogenic bacteria in the microbiome of a subject (e.g., a human patient). In some embodiments, administration of a glycan preparation that supports the growth of commensal or probiotic bacteria and does not support the growth of at least one pathogen, *e.g*., does not support the growth of a CRE, VRE, and/or C. difficile species, *e.g*., in a gut microbiome, may increase the concentration, amount or relative abundance of commensal bacteria relative to pathogenic bacteria in the microbiome of a subject (e.g., a human patient).

### Methods of Assessment

The pathogen reservoir (e.g., in a subject, e.g., a facility participant) may be measured by any known method in the art, such as PCR (see e.g., PCR-based tests for carbapenem-resistant Enterobacteriaciae such as the Cepheid GeneXpert, Carba-R [www.cepheid.com/us/cepheid-solutions/clinical-ivd-tests/healthcare-associated-infections/xpert-carba-r] or VanA [www.cepheid.com/us/cepheid-solutions/clinical-ivd-tests/healthcare-associated-infections/xpert-vana] or C. difficile [www.cepheid.com/us/cepheid-solutions/clinical-ivd-tests/healthcare-associated-infections/xpert-c-difficile]), or selective media used to identify subjects colonized with multidrug resistant pathogens, such as BioMerieux ChromID Carba, VRE, MRSA and C. difficile (www.biomerieux-usa.com/clinical/chromid-culture-media).

The pathogen reservoir may be assessed by direct subject screening, such as by analysis of a sample from the rectum, colon, stool, skin, or other region of the body of a subject (e.g., facility participant). Alternatively, the pathogen reservoir may be assessed through environmental sampling, e.g., through analysis of samples taken from a subject's home (including a room in a care facility) or possessions, e.g., clothing, accessories, or surfaces in proximity to the subject or other facility participants, e.g., a laundry, sink, drain, door, doorknob, or air-handling device, or objects used in the treatment or care of a subject, such as a linen, towel, sheet, glove, dressing gown, mask, endoscope, laryngoscope, bandage, catheter, bed, stretcher, or other objects.

The pathogen reservoir may also be assessed by measurement of the rate of infection of a subject, e.g. first facility participant, or neighbors of the first facility participant, e.g., a second facility participant, in the facility. This may be determined, for example, by analysis of a pathogen cultured in a particular facility or area of a facility, e.g., in the course of standard care of a subject, or by surveillance of a certain anatomical site on or within a subject, using standard clinical diagnostic procedures.

In some embodiments, a reduction in the pathogen reservoir is the result of fewer subjects having said pathogen (e.g., about 1%, about 2%, about 3%, about 4%, about 5%, about 10%, about 15%, about 20%, about 30%, about 35%, about 40%, about 45%, about 50%, about 55%, about 60%, about 65%, about 70%, about 75%, about 80%, about 85%, about 90%, about 95%, or about 99% fewer subjects having said pathogen) or of each subject having less total pathogen overall (e.g., each subject having about 1%, about 2%, about 3%, about 4%, about 5%, about 10%, about 15%, about 20%, about 30%, about 35%, about 40%, about 45%, about 50%, about 55%, about 60%, about 65%, about 70%, about 75%, about 80%, about 85%, about 90%, about 95%, or about 99% less total pathogen).

The spread of a pathogen may be assessed using any technique described herein, e.g., a technique described for the assessment of the pathogen reservoir. The spread of a pathogen may be further assessed through a determination of whether or not the pathogen or component thereof (e.g., a virulence factor or resistance determinant, e.g., a carbapenemase gene or toxin) derived from a first subject (e.g., first facility participant) is similar to a pathogen or component thereof derived from a second subject (e.g., second facility participant). This type of determination may be carried out using any known method in the art, including nucleic acid sequencing, microsatellite sequencing, PCR, pulse-field gel electrophoresis, or comparison of characteristics of pathogens (e.g., pathogen identification, biochemical characteristics, or antibiotic susceptibility profiles). These determinations are routinely conducted as part of public health surveillance. In some embodiments, a pathogen or a component thereof derived from a first subject has about a 1%, about 2%, about 3%, about 4%, about 5%, about 10%, about 15%, about 20%, about 30%, about 35%, about 40%, about 45%, about 50%, about 55%, about 60%, about 65%, about 70%, about 75%, about 80%, about 85%, about 90%, about 95%, or about 99%) similarity to a pathogen or component thereof derived from a second subject.

The reduction of a drug-resistance gene (e.g., an antibiotic resistance gene or MDR gene) may be assessed using any technique described herein, e.g., a technique described for the assessment of a pathogen reservoir.

In some embodiments, a reduction in a drug-resistance gene is assessed by culturing microbes on selective media (e.g., containing antibiotics), such as ChromID VRE or ChromID Carba, or media supplemented with a cephalosporin or another beta lactam. In some embodiments, a reduction in a drug-resistance gene is assessed by antibiotic susceptibility testing of a strain isolated in the course of standard clinical care or surveillance of a subject (e.g., facility participant), or the environment at large to determine the presence of drug-resistance gene or a toxin.

In some embodiments, provided is a method of reducing the rate at which a pathogen causes infection (e.g., in a subject, e.g., facility participant) by administering a glycan preparation to the subject, e.g., in an effective amount and/or to a sufficient number of subjects that the rate of infection is reduced. In some embodiments, the rate of infection is reduced by about 1%, about 2%, about 3%, about 4%, about 5%, about 10%, about 15%, about 20%, about 30%, about 35%, about 40%, about 45%, about 50%, about 55%, about 60%, about 65%, about 70%, about 75%, about 80%, about 85%, about 90%, about 95%), about 99%, or about 100%, e.g., relative to a reference standard.

In some embodiments, reducing the rate of infection may comprise implementation of a screening program, e.g., involving monitoring for a similar infection, e.g., through observation of similar symptoms or similar features, to measure the rate at which an infection occurs. In said screening program, a reduction in rate may be assessed by comparing the rate of an infection prior to, during, or after administration of a glycan preparation. Facility participants in a screening program may be matched by a known risk factor for infection, or by another characteristic (e.g., medical history, age, or health status).

In some embodiments, provided is a method of monitoring or evaluating any of a) pathogen load, b) antibiotic resistance gene load and c) response to a therapeutic preparation in a facility participant comprising: analyzing a suitable sample, e.g., a fecal sample from the subject (e.g., facility participant) for a) pathogens, and/or b) antibiotic resistance genes and/or microbiome analysis (e.g., the presence of one or more microbes related to response to a particular therapeutic preparation), thereby monitoring or evaluating the facility participant.

In some embodiments, transfer of a drug-resistance gene (e.g., an antibiotic resistance gene or an MDR gene) may be measured using any of the available methods known in the art. For example, the transfer may be assessed by evaluating the presence of a gene in a first pathogen compared with the presence of a gene in a second pathogen. In some embodiments, the transfer of a drug-resistance gene (e.g., an antibiotic resistance gene or an MDR gene) is reduced by about 1%, about 2%, about 3%, about 4%, about 5%, about 10%, about 15%, about 20%, about 30%, about 35%, about 40%, about 45%, about 50%, about 55%, about 60%, about 65%, about 70%, about 75%, about 80%, about 85%, about 90%, about 95%, about 99%, or about 100%, e.g., relative to a reference standard.

### Glycan compositions and manufacture thereof

Glycan compositions can comprise the glycans described herein, dietary fibers, such as, e.g., FOS (fructo-oligosaccharide), other sugars (e.g., monomers, dimers, such as, e.g., lactulose) and sugar alcohols, and optionally other components, such as, e.g., polyphenols, fatty acids, peptides, micronutrients, etc., such as those described in WO 2016/172658, "MICROBIOME REGULATORS AND RELATED USES THEREOF", and microbes, such as bacteria. Glycan preparations described in WO 2016/122889 "GLYCAN THERAPEUTICS AND RELATED METHODS THEREOF" and WO 2016/172657, "GLYCAN THERAPEUTICS AND METHODS OF TREATMENT", which in their entirety are hereby incorporated by reference, are suitable for in the methods and compositions described herein. Preparations comprising glycans can be generated using a non-enzymatic catalyst, e.g., the polymeric catalyst described in WO 2012/118767, "POLYMERIC ACID CATALYSTS AND USES THEREOF" or by other suitable methods. Methods to prepare the polymeric and solid-supported catalysts described herein can be found in WO 2014/031956, "POLYMERIC AND SOLID-SUPPORTED CATALYSTS, AND METHODS OF DIGESTING CELLULOSIC MATERIALS USING SUCH CATALYSTS." The glycans generated, e.g., by using the catalyst, for example as described in WO 2016/007778, "OLIGOSACCHARIDE COMPOSITIONS AND METHODS FOR PRODUCING THEREOF" are suitable for the methods and compositions described herein. All patent applications are incorporated herein by reference in their entirety.

In some embodiments, glycans are made using solid-phase oligosaccharide synthesis, e.g., using a variety of protection groups to accomplish glycan synthesis. Exemplary methods are described in "Solid-Phase Oligosaccharide Synthesis and Combinatorial Carbohydrate Libraries", Peter H. Seeberger and Wilm-Christian Haase, American Chemical Society, 2000; and "Opportunities and challenges in synthetic oligosaccharide and glycoconjugate research", Thomas J. Boltje et al., Nat Chem. 2009 November 1; 1(8): 611-622.

In some embodiments, glycans may be synthesized using an enzyme catalyst (e.g., a glycosidase or glycosyltransferase, either isolated or expressed in bacteria) to synthesize the glycans by a polymerization reaction that creates oligomers from individual glycan subunits that are added to the reaction. Exemplary methods are described in "Synthesis and Purification of Galacto-Oligosaccharides: State of the Art", Carlos Vera et al., World J. Microbiol Biotechnol. 2016; 32:197; "Synthesis of Novel Bioactive Lactose-Derived Oligosaccharides by Microbial Glycoside Hydrolases", Marina Diez-Municio et al., Microbial Biotechnol. 2014; 7(4), 315-331; and "Methods of Improving Enzymatic Trans-Glycosylation for Synthesis of Human Milk Oligosaccharide Biomimetics", Birgitte Zeuner et al., J. Agric. Food Chem. 2014, 62, 9615-9631, WO 2005/003329 "NOVEL GALACTOOLIGOSACCHARIDE COMPOSITION AND THE PREPARATION THEREOF", all of which are hereby incorporated by reference.

In some embodiments, glycan preparations may be prepared using glycan polymers, such as starch and other fibers, such as dietary fibers (such as described herein) and subject them to a catalyst (e.g., an acid catalyst, a solid or polymeric catalyst, an enzyme catalyst) to change one or more glycan (or fiber) properties, e.g., degree of polymerization (e.g. depolymerization), degree of branching (e.g. debranching), or glycosidic bond distribution (e.g., by adding new types of glycosidic bonds or removing existing bonds). An exemplary method for corn syrup is described in U.S. Patent Publication No. 2016/0007642, Example 101, which is incorporated by reference. Other methods, such as those used for preparation of resistant starch (e.g., described in M.G. Sajilata et al., "Resistant Starch - A Review," Comprehensive Reviews in Food Science and Food Safety - Vol. 5, 2006, and U.S. Patent Publication No. 2006/0257977, "Slowly digestible starch"), such as, e.g., heat treatment, enzymic treatment, chemical treatment, or a combination thereof, may be used to produce glycan preparations described herein.

Glycan compositions and glycan preparations described herein may have the properties of any one of rows 3-55 of Table 4A and 4B. In some embodiments, a glycan compomposition and/or glycan preparation has the properties of Glu5Gal5Man90-2, Glu10Gal10Man80-1, Glu20Gal20Man20Xy120Ara20-1, Glu20Gal20Man20Xyl20Ara20-2, Gal33Man33Ara33-8, Gal57Glu43-1, Glu100-87, Gal57Glu43-2, Glu50Gal50-11, Glu50Gal50-32, Glu50Gal50-14, Glu50Gal50-27, Glu50Gal50-23, Glu50Gal50-2, Glu100-129, Glu100-136, Glu100-17, Glu100-64, Glu100-76, Glu100-131, Glu100-83, Glu100-139, Glu100-84, Glu100-74, Glu100-98, Glu100-141, Glu100-29, Glu100-18, Glu100-99, Glu100-72, Glu100-82, Glu100-130, Glu100-78, Glu100-66, Glu100-89, Glu100-133, Glu100-68, Glu100-90, Glu100-94, Glu100-5, 3-Obn Glu100-1, Gal100-30. Glu33Gal33Fuc33-3, Ara100-12, Xyl100-8, Xy175Ara25-3, Glu80Man20-2, Glu60Man40-5, Man80Glu20-2, Man60Glu40-2, Man52Glu29Gal19-2, Man52Glu29Gal19-3, or Man100-17, as described in Table 4A and 4B.

### Glycan preparation properties

Glycan may have any one or more of the characteristics and properties disclosed in WO2016/122889, WO2016/172657, WO 2016/007778, and WO2016/172658, each of which is incorporated herein by reference in its entirety, and any characteristics and properties disclosed herein.

The glycans produced by the methods described herein may comprise oligosaccharides. In some embodiments, the glycans comprise homo-oligosaccharides (or homoglycans), wherein all the monosaccharides in a polymer are of the same type.

In some embodiments, the glycans comprise hetero-oligosaccharides (or heteroglycans), wherein more than one type of monosaccharide is present in the polymer. In some embodiments, the glycans have one or more of the properties described herein. In some embodiments, the glycan preparation has one or more of the bulk properties described herein.

### Degree of polymerization (DP)

In some embodiments, glycan preparations are produced, e.g., using a method described herein, that are polydisperse, exhibiting a range of degrees of polymerization.

Optionally, the preparations may be fractionated, e.g. representing 60%, 65%, 70%, 75%, 80%, 85%, 90%, 95%, 97%, 98%, or greater than 98% short (about DP1-2), medium (about DP3-10), long (about DP11-18), or very long (about DP>18) species. In one embodiment, a polydisperse, fractionated glycan preparation is provided comprising at least 85%, 90%, or at least 95% medium-length species with a DP of about 3-10. In one embodiment, a polydisperse, fractionated glycan preparation is provided comprising at least 85%, 90%, or at least 95% long-length species with a DP of about 11-18. In one embodiment, a polydisperse, fractionated glycan preparation is provided comprising at least 85%, 90%, or at least 95% very long-length species with a DP of about 18-30.

Optionally, the preparations may be fractionated, e.g. representing 60%, 65%, 70%, 75%, 80%, 85%, 90%, 95%, 97%, 98%, or greater than 98% short (about DP1-2) or medium (about DP3-10) glycans in the preparation. Alternatively, or in addition to fractionation, the small DP fraction (e.g. monomers and dimers) are subjected to enzymatic fermentation, e.g. with suitable yeasts to break down these sugars. In one embodiment, a polydisperse, fractionated glycan preparation is prepared using a method described herein, comprising at least 85%, 90%, or at least 95% of glycans with a DP of about 3-10.

In some embodiments, about 55%, 60%, 65%, 70%, 75%, 80%, 85%, 90%, 95%, or about 97% of the glycans of the glycan preparation have a DP of at least DP3, DP4, DP5, DP6 or DP7. In some embodiments, about 55%, 60%, 65%, 70%, 75%, 80%, 85%, 90%, 95%, or about 97% of the glycans of the glycan preparation have a DP from about DP3 to about DP10, from about DP3 to about DP8, from about DP3 to about DP6, from about DP3 to about DP5, from about DP3 to about DP4, from about DP2 to about DP4, from about DP2 to about DP5, from about DP2 to about DP6, from about DP2 to about DP8, or from about DP2 to about DP10. In some embodiments, less than 1%, 2%, 3%, 5%, 10%, 15%, 20%, 25%, 30%, 40%, or less than 50% of the glycans of the glycan preparation have a DP of DP2 or less.

In some embodiments, about 55%, 60%, 65%, 70%, 75%, 80%, 85%, 90%, 95%, or about 97% of the glycan preparation has a DP of between 2 and 25, between 3 and 25, between 4 and 25, between 5 and 25, between 6 and 25, between 7 and 25, between 8 and 25, between 9 and 25, between 10 and 25, between 2 and 30, between 3 and 30, between 4 and 30, between 5 and 30, between 6 and 30, between 7 and 30, between 8 and 30, between 9 and 30, or between 10 and 30. In one embodiment, the glycan preparation has a degree of polymerization (DP) of at least 3 and less than 30 glycan units.

In some embodiments, about 55%, 60%, 65%, 70%, 75%, 80%, 85%, 90%, 95%, or about 97% of the glycan preparation has a DP of at least 5 and less than 30 glycan units. In some embodiments, about 55%, 60%, 65%, 70%, 75%, 80%, 85%, 90%, 95%, or about 97% of the glycan preparation has a DP of at least 8 and less than 30 glycan units. In some embodiments, about 55%, 60%, 65%, 70%. 75%, 80%, 85%, 90%, 95%, or about 97% of the glycan preparation has a DP of at least 10 and less than 30 glycan units. In some embodiments, about 55%, 60%, 65%, 70%, 75%, 80%, 85%, 90%, 95%, or about 97% of the glycan preparation has a DP of between 3, 4, 5, 6, 7, 8 and 10, 11, 12, 13, 14, 15, 16, 17, 18, 19, 20 glycan units. In some embodiments, about 55%, 60%, 65%, 70%, 75%, 80%, 85%, 90%, 95%, or about 97% of the glycan preparation has a DP of between 10, 11, 12, 13, 14, 15, 16, 17, 18, 19 and 20, 21, 22, 23, 24, 25, 26, 27, 28, 29, 30 glycan units. In some embodiments, about 55%, 60%, 65%, 70%, 75%, 80%, 85%, 90%, 95%, or about 97% of the glycan preparation has a DP of between 3, 4, 5, 6, 7, 8, 9, 10 and 20, 21, 22, 23, 24, 25, 26, 27, 28 glycan units. In one embodiment, about 55%, 60%, 65%, 70%, 75%, 80%, 85%, 90%, 95%, or about 97% of the glycan preparation has a DP of at least 2. In one embodiment, about 55%, 60%, 65%, 70%, 75%, 80%, 85%, 90%, 95%, or about 97% of the glycan preparation has a DP of at least 3.

### Average DP

In some embodiments, the glycan preparation has an average degree of polymerization (average DP) of about DP2, DP3, DP4, DP5, DP6, DP7, DP8, or DP9. In some embodiments, the glycan preparation has an average degree of polymerization (average DP) of between about 2 and about 10, between about 2 and about 8, between about 2 and about 6, between about 2 and about 4, between about 3 and about 10, between about 3 and about 8, between about 3 and about 6, or between about 3 and about 4.

In some embodiments, about 55%, 60%, 65%, 70%, 75%, 80%, 85%, 90%, 95%, or about 97% of the glycan preparation has an average degree of polymerization (DP) of about DP5, DP6, DP7, DP8, DP9, DP10, DP11, or DP12. In some embodiments, the average DP of the glycan preparation is between about DP5 and DP10, between about DP6 and DP10, between about DP6 and DP12, between about DP6 and DP14, between about DP8 and DP12, between about DP8 and DP14, between about DP8 and DP16, between about DP10 and DP16 between about DP10 and DP18, between about DP4 and DP18, between about DP6 and DP18, or between about DP8 and DP18.

### Average Molecular weight

In some embodiments, about 55%, 60%, 65%, 70%, 75%, 80%, 85%, 90%, 95%, or about 97% of the glycans of the preparation have an average molecular weight of about 200, 250, 300, 350, 400, 450, 500, 550, 600, 650, 700, 750, 800, 850. 900, 950, 1000, 1050, 1100, 1150, 1200, 1250, 1300, 1350, 1400, 1450, 1500, 1550, 1600, 1650, 1700, 1750, 1800 g/mol and less than 400, 500, 600, 700, 800, 900, 1000, 1100, 1200, 1300, 1400, 1500, 1600, 1700, 1800, 1900, 2000, 2100, 2200, 2300,2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, and 5000 g/mol.

### Degree of branching (DB)

In some embodiments, the glycan preparations range in structure from linear to branched. Branched glycans may contain at least one glycan subunit being linked via an alpha or a beta glycosidic bond so as to form a branch. The branching rate or degree of branching (DB) may vary, such that the glycans of a preparation comprise at least 1, at least 2, at least 3, at least 4, at least 5, or at least about 6 branching points in the glycan. In some embodiments, the glycans of the glycan preparation are unbranched (DB=0).

In some embodiments, the glycan preparations (e.g. oligo- or polysaccharides) range in structure from linear to highly branched. Unbranched glycans may contain only alpha linkages or only beta linkages. Unbranched glycans may contain at least one alpha and at least one beta linkage. Branched glycans may contain at least one glycan unit being linked via an alpha or a beta glycosidic bond so as to form a branch. The branching rate or degree of branching (DB) may vary, such that about every 2^{nd}, 3^{rd} , 4^{4th} , 5^{th} , 6^{th} , 7^{th} , 8^{th} , 9^{th}, 10^{th}, 15^{th}, 20^{th}, 25^{th}, 30^{th}, 35^{th}, 40^{th}, 45^{th}, 50^{th}, 60^{th}, or 70^{th} unit comprises at least one branching point. For example, animal glycogen contains a branching point approximately every 10 units.

In some embodiments, preparations of glycan are provided, wherein the preparation comprises a mixture of branched glycans, wherein the average degree of branching (DB, branching points per residue) is 0 (unbranched), 0.01. 0.02, 0.03, 0.04, 0.05, 0.06, 0.07, 0.08, 0.09, 0.1, 0.2, 0.3, 0.4, 0.5, 0.6, 0.7, 0.8, 0.9, 0.95, 0.99, 1, or 2. In some embodiments, preparations of glycans are provided, wherein the average degree of branching is at least 0.01, 0.05, 0.1, 0.2, 0.3, or at least 0.4. In some embodiments, preparations of glycans are provided, wherein the average degree of branching is between about 0.01 and 0.1, 0.01 and 0.2, 0.01 and 0.3, 0.01 and 0.4, 0.01 and 0.5, 0.01 and 0.6, or between about 0.01 and 0.7. In some embodiments, preparations of glycans are provided, wherein the average degree of branching is between about 0.05 and 0.1, 0.05 and 0.2, 0.05 and 0.3, 0.05 and 0.4, 0.05 and 0.5, 0.05 and 0.6, or between about 0.05 and 0.7. In some embodiments, preparations of glycans are provided, wherein the average degree of branching is not 0. In some embodiments, preparations of glycans are provided, wherein the average degree of branching is not between at least 0.1 and less than 0.4 or at least 0.2 and less than 0.4. In some embodiments, the preparations of glycans comprise linear glycans. In some embodiments, the preparations of glycans comprise glycans that exhibit a branched or branch-on-branch structure.

In some embodiments, preparations of glycans are provided wherein the average degree of branching (DB) is not 0, but is at least 0.01, 0.05, 0.1, or at least 0.2, or ranges between about 0.01 and about 0.2 or between about 0.05 and 0.1.

### Glycosidic bonds and linkages

Linkages between the individual glycan subunits found in preparations of glycans may include alpha 1->2, alpha 1->3, alpha 1->4, alpha 1->5, alpha 1->6. alpha 2->1, alpha 2->3, alpha 2->4, alpha 2->6, beta 1->2, beta 1->3, beta 1->4, beta 1->5, beta 1->6, beta 2->1, beta 2->3, beta 2->4, and beta 2->6.

In some embodiments, the glycan preparations comprise only alpha linkages. In some embodiments, the glycans comprise only beta linkages. In some embodiments, the glycans comprise mixtures of alpha and beta linkages.

In some embodiments, the alpha:beta glycosidic bond ratio in a preparation is about 1:1, 2:1, 3:1, 4:1, or 5:1.. In some embodiments, the beta:alpha glycosidic bond ratio in a preparation is about 1:1, 2:1, 3:1, 4:1, or 5:1.

In some embodiments, the alpha:beta glycosidic bond ratio in a preparation is about 0.1:1, 0.2:1, 0.3:1, 0.4:1, 0.5:1, 0.6:1, 0.7:1, 0.8:1, 0.9:1, 1:1, 1.2:1, 1.5:1, 1.7:1, 2:1, 2.2:1, 2.5:1, 2.7:1, 3:1, 4:1, 5:1, 6:1, 7:1, 8:1, 9:1, or about 10:1.

In some embodiments, the glycans of the glycan preparation comprise both alpha- and beta-glycosidic bonds selected from the group consisting of 1->2 glycosidic bond, a 1->3 glycosidic bond, a 1->4 glycosidic bond, a 1->5 glycosidic bond and a 1->6 glycosidic bond. In some embodiments, the glycan preparation comprises at least two or at least three alpha and beta 1->2 glycosidic bonds, alpha and beta 1->3 glycosidic bonds, alpha and beta 1->4 glycosidic bonds, alpha and beta 1->5 glycosidic bonds, and/or alpha and beta 1->6 glycosidic bonds.

In some embodiments, the glycans of the glycan preparation comprise substantially all alpha- or beta configured glycan subunits, optionally comprising about 1%, 2%, 3%, 4% 5%, 6%, 7%, 8%, 9%. 10%, 11%, 12%, 13%, 14%, 15%, 16%, 17%, 18%, 19%, or 20% of the respective other configuration.

In some embodiments, the preparations of glycans comprise at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 98%, 99%, at least 99.9% or even 100% glycans with alpha glycosidic bonds. In some embodiments, the preparations of glycans comprise at least 1%, 2%, 3%, 4%, 5%, 6%, 7%. 8%, 9%, 10%, 15%, 20%, 25%, 30%, 35%, 40%, 45%, 50%, 55%, 60%, 65%, 70%, 75%, 80%, 85%, 90%, 95%, 97%, 98%, 99%, at least 99.9% or even 100% glycans with beta glycosidic bonds. In some embodiments, preparations of glycans are provided, wherein at least 10%, 15%, 20%, 25%, 30%, 35%, 40%, 45%, 50%, 55%, 60%, 65%, 70%, 75%, 80%, or at least 85% of glycans with glycosidic bonds that are alpha glycosidic bonds, at least 10%, 15%, 20%, 25%, 30%, 35%, 40%, 45%, 50%, 55%, 60%, 65%, 70%, 75%, 80%, or at least 85% of glycans with glycosidic bonds that are beta glycosidic bonds, and wherein the percentage of alpha and beta glycosidic bonds does not exceed 100%.

In some embodiments, preparations of glycans are provided, wherein at least 1%, 2%, 3%, 4%, 5%, 6%. 7%, 8%, 9%, 10%, 15%, 20%, 25%, 30%, 35%, 40%, 45%, 50%, 55%, 60%, 65%, 70%, 75%, 80%, 85%, 90%, 95%, 97%, 98%, 99%, at least 99.9% or even 100% of glycan glycosidic bonds are one or more of: 1->2 glycosidic bonds, 1->3 glycosidic bonds, 1->4 glycosidic bonds, and 1->6 glycosidic bonds. In some embodiments, preparations of glycans are provided, wherein at least 1%, 2%, 3%, 4%, 5%, 6%, 7%, 8%, 9%, 10%, 15%, at least 20%, or 25% each of glycan glycosidic bonds are 1->2, 1->3, 1->4, and 1->6 glycosidic bonds.

Optionally, the preparations of glycans further comprise at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, or at least 85% of glycan glycosidic bonds that are selected from the group consisting of : alpha 2->1, alpha 2->3, alpha 2->4, alpha 2->6, beta 2->1, beta 2->3, beta 2->4, and beta 2->6, glycosidic bonds.

In some embodiments, the glycans of the glycan preparation comprise at least two glycosidic bonds selected from the group consisting of alpha 1->2 and alpha 1->3, alpha 1->2 and alpha 1->4, alpha 1->2 and alpha 1->6, alpha 1->2 and beta 1->2, alpha 1->2 and beta 1->3, alpha 1->2 and beta 1->4, alpha 1->2 and beta 1->6, alpha 1->3 and alpha 1->4, alpha 1->3 and alpha 1->6, alpha 1->3 and beta 1->2, alpha 1->3 and beta 1->3, alpha 1->3 and beta 1->4, alpha 1->3 and beta 1->6, alpha 1->4 and alpha 1->6, alpha 1->4 and beta 1->2, alpha 1->4 and beta 1->3, alpha 1->4 and beta 1->4, alpha 1->4 and beta 1->6, alpha 1->6 and beta 1->2, alpha 1->6 and beta 1->3, alpha 1->6 and beta 1->4, alpha 1->6 and beta 1->6, beta 1->2 and beta 1->3, beta 1->2 and beta 1->4, beta 1->2 and beta 1->6, beta 1->3 and beta 1->4, beta 1->3 and beta 1->6, and beta 1->4 and beta 1->6.

### L- and D-forms

In some embodiments, preparations of glycans are provided, wherein at least one glycan subunit is a sugar in L-form. In some embodiments, preparations of glycans are provided, wherein at least one glycan subunit is a sugar in D-form. In some embodiments, preparations of glycans are provided, wherein the glycan subunits are sugars in L- or D-form as they naturally occur or are more common (e.g. D-glucose, D-xylose, L-arabinose).
In some embodiments, the preparation of glycans (e.g. oligosaccharides and polysaccharides) comprises a desired mixture of L- and D-forms of glycan subunits, e.g. of a desired ratio, such as: 1:1, 1:2, 1:3, 1:4, 1:5 L- to D-fonns or D- to L-forms.

In some embodiments, the preparation of glycans comprises a desired mixture of L- and D-forms of glycan units, e.g. of a desired ratio, such as: 1:1, 1:2, 1:3, 1:4, 1:5, 1:6, 1:7, 1:8, 1 :9, 1:10, 1:12, 1:14, 1:16, 1:18, 1:20, 1:25, 1:30, 1:35, 1:40, 1:45, 1:50, 1:55, 1:60, 1:65, 1:70, 1:75, 1:80, 1:85, 1:90, 1:100, 1:150 L- to D-forms or D- to L-forms.
In some embodiments, the preparation of glycans comprises glycans with substantially all L- or D-forms of glycan subunits, optionally comprising about 1%, 2%, 3%, 4% 5%, 6%, 7%, 8%, 9%, 10%, 11%, 12%, 13%, 14%, 15%, 16%, 17%, 18%, 19%, or 20% of the respective other form.

### Glycan unit content

In some embodiments, preparations of glycans are provided, wherein at least one glycan subunit is a tetrose, a pentose, a hexose, or a heptose. Optionally, the glycan subunits involved in the formation of the glycans of the glycan preparation are varied. Examples of monosaccharide glycan subunits include hexoses, such as glucose, galactose, and fructose, and pentoses, such as xylose. Monosaccharides generally have the chemical formula: Cₓ(H₂O)_{y}, where conventionally x ≥ 3. Monosaccharides can be classified by the number x of carbon atoms they contain, for example: diose (2) triose (3) tetrose (4), pentose (5), hexose (6), and heptose (7). The monosaccharide glycan subunits may exist in an acyclic (open-chain) form. Open-chain monosaccharides with same molecular graph may exist as two or more stereoisomers. The monosaccharides may also exist in a cyclic form through a nucleophilic addition reaction between the carbonyl group and one of the hydroxyls of the same molecule. The reaction creates a ring of carbon atoms closed by one bridging oxygen atom. In these cyclic forms, the ring usually has 5 (furanoses) or 6 atoms (pyranoses).

In some embodiments, the preparation of glycans comprises a desired mixture of different monosaccharide glycan subunits, such as a mixture of a diose (2), a triose (3), tetrose (4), pentose (5), hexose (6), or heptose (7). In some embodiments, the glycans of the glycan preparation comprise a desired mixture of a pentose (5) and a hexose (6).

In some embodiments, the preparation of glycans comprises a desired mixture of two, three, four or five different glycan subunits, such as a mixture of, e.g., i) one or more glycan subunits selected from monosaccharides, selected from glucose, a galactose, an arabinose, a mannose, a fructose, a xylose, a fucose, and a rhamnose; ii) one or more glycan subunits selected from disaccharides selected from acarviosin, n-acetyllactosamine, allolactose, cellobiose, chitobiose, glactose-alpha-1,3-galactose, gentiobiose, isomalt, isomaltose, isomaltulose, kojibiose, lactitol, lactobionic acid, lactose, lactulose, laminaribiose, maltitol, maltose, mannobiose, melibiose, melibiulose, neohesperidose, nigerose, robinose, rutinose, sambubiose, sophorose, sucralose, sucrose, sucrose acetate isobutyrate, sucrose octaacetate, trehalose, turanose, vicianose, and xylobiose; iii) one or more glycan subunits selected from amino sugars selected from acarbose, N-acetylemannosamine, N-acetylmuramic acid, N-acetylnueraminic acid, N-acetyletalosaminuronic acid, arabinopyranosyl-N-methyl-N-nitrosourea, D-fructose-L-histidine, N-glycolyneuraminic acid, ketosamine, kidamycin, mannosamine, 1B-methylseleno-N-acetyl-D-galactosamine, muramic acid, muramyl dipeptide, phosphoribosylamine, PUGNAc. sialyl-Lewis A, sialyl-Lewis X, validamycin, voglibose, N-acetylgalactosamine, N-acetylglucosamine, aspartylglucosamine, bacillithiol, daunosamine, desosamine, fructosamine, galactosamine, glucosamine, meglumine, and perosamine; iv) one or more glycan subunits selected from deoxy sugars selected from 1-5-abydroglucitol, cladinose, colitose, 2-deoxy-D-glucose, 3-deoxyglucasone, deoxyribose, dideoxynucleotide, digitalose, fludeooxyglucose, sarmentose, and sulfoquinovose; v) one or more glycan subunits selected from imino sugars selected from castanospermine, 1-deoxynojirimycin, iminosugar, miglitol, miglustat, and swainsonine; one or more glycan subunits selected from sugar acids selected from N-acetylneuraminic acid, N-acetyltalosamnuronic acid, aldaric acid, aldonic acid, 3-deoxy-D-manno-oct-2-ulosonic acid, glucuronic acid, glucosaminuronic acid, glyceric acid, N-glycolylneuraminic acid, iduronic acid, isosaccharinic acid, pangamic acid, sialic acid, threonic acid, ulosonic acid, uronic acid, xylonic acid, gluconic acid, ascorbic acid, ketodeoxyoctulosonic acid, galacturonic acid, galactosaminuronic acid, mannuronic acid, mannosaminuronic acid, tartaric acid, mucic acid, saccharic acid, lactic acid, oxalic acid, succinic acid, hexanoic acid, fumaric acid, maleic acid, butyric acid, citric acid, glucosaminic acid, malic acid, succinamic acid, sebacic acid, and capric acid; vi) one or more glycan subunits selected from short-chain fatty acids selected from formic acid, acetic acid, propionic acid, butryic acid, isobutyric acid, valeric acid, and isovaleric acid; and vii) one or more glycan subunits selected from sugar alcohols selected from methanol, ethylene glycol, glycerol, erythritol, threitol, arabitol, ribitol, xylitol, mannitol, sorbitol, galactitol, iditol, volemitol, fucitol, inositol, maltotritol, maltotetraitol, and polyglycitol.

Exemplary glycans are described by a three-letter code representing the monomeric sugar component followed by a number out of one hundred reflecting the percentage of the material that monomer constitutes. Thus, 'glu100' is ascribed to a glycan generated from a 100% D-glucose (glycan unit) input and 'glu50gal50' is ascribed to a glycan generated from 50% D-glucose and 50% D-galactose (glycan units) input or, alternatively from a lactose dimer (glycan unit) input. As used herein: xyl = D-xylose; ara = L-arabinose; gal = D-galactose; glu = D-glucose; rha = L-rhamnose; fuc = L-fucose; man = D-mannose; sor = D-sorbitol; gly = D-glycerol; neu = NAc-neuraminic acid.

In some embodiments, the preparation of glycans comprises one glycan unit A selected from i) to vii) above, wherein glycan unit A comprises 100% of the glycan unit input. For example, in some embodiments, the glycan preparation is selected from the homo-glycans xyl100, rha100, ara100, gal100, glu100, and man100. In some embodiments, the glycan preparation is selected from the homo-glycans fuc 100 and fru100.

In some embodiments, the preparation of glycans comprises a mixture of two glycan units A and B selected independently from i) to vii) above, wherein A and B may be selected from the same or a different group i) to vii) and wherein A and B may be selected in any desired ratio (e.g. anywhere from 1-99% A and 99-1% B, not exceeding 100%).

For example, in some embodiments, the glycan preparation is selected from the hetero-glycans ara50gal50, ara50gal50, xyl75gal25, ara80xyl20, ara60xyl40, ara50xyl50, glu80man20, glu60man40, man80glu20, man60glu40, xyl75ara25, ga175xyl25, Man80gal20, gal75xyl25, Man66gal33, Man75gal25, glu80gal20, glu60gal40, glu40gal60, glu20gal80, gal80man20, ga160man40, gal40man60, glu80xyl20. glu60xyl40, glu40xyl60, glu20xyl80, glu80ara20, glu60ara40, glu40ara60, glu20ara80, gal80xyl20, gal60xyl40, gal40xy160, gal20xyl80, gal80ara20, gal60ara40, gal40ara60, gal20ara80, man80xyl20, man60xyl40, man40xyl60, man20xyl80, man80ara20, man60ara40, man40ara60, man20ara80, xyl80ara20, xyl60ara40, glu50gal50. and man62glu38.

In some embodiments, the preparation of glycans comprises a mixture of three glycan units A, B and C selected independently from i) to vii) above, wherein A, B and C may be selected from the same or a different group i) to vii) and wherein A, B and C may be selected in any desired ratio (e.g. anywhere from 1-99% A, 1-99% B, 1-99% C, not exceeding 100%). For example, in some embodiments, the glycan preparation is selected from the hetero-glycans xyl75glu12gal12, xyl33glu33gal33, xyl75glu12gal12, glu33gal33fuc33, glu33gal33nman33, glu33gal33xyl33, glu33gal33ara33, gal33man33xyl33, gal33man33ara33, man52glu29gal19, Glu33Man33Xyl33, Glu33Man33Ara33, Glu33Xyl33Ara33, Gal33Man33Xyl33, Gal33Man33Ara33, Gal33Xyl33Ara33, Man33Xyl33Ara33, Glu90Gal5Man5, Glu80Gal110Man10, Gln60Gal20Man20, Glu40Gal30Man30, Glu20Gal40Man40, Glul0Gal45Man45, Glu5Ga190Man5, Glu10Gal80Man10, Glu20Gal60Man20, Glu30Gal40Man30, Glu40Gal20Man40, Glu45Gal10Man45, Glu5Gal5Man90, Glu10Gal10Man80, Glu20Gal20Man60, Glu30Gal30Man40, Glu40Gal40Man20, and Glu45Gal45Man10.

In some embodiments, the preparation of glycans comprises a mixture of four glycan units A, B. C and D selected independently from i) to vii) above, wherein A, B, C and D may be selected from the same or a different group i) to vii) and wherein A, B, C and D may be selected in any desired ratio (e.g. anywhere from 1-99% A, 1-99% B, 1-99% C, 1-99% D, not exceeding 100%).

In some embodiments, the preparation of glycans comprises a mixture of five glycan units A, B, C, D and E selected independently from i) to vii) above, wherein A, B, C, D and E may be selected from the same or a different group i) to vii) and wherein A, B, C, D and E may be selected in any desired ratio (e.g. anywhere from 1-99% A, 1-99% B, 1-99% C, 1-99% D, 1-99% E, not exceeding 100%).

Provided herein are glycan preparations (as described herein, e.g., having any DP, DB, alpha:beta glycosidic bond ratio, number of glycosidic bonds, bond regiochemistry and bond stereochemistry, and other characteristics (e.g., solubility, fermentability, viscosity, sweetness, etc.) described herein), comprising glycans comprising:
1) a glucose glycan unit, optionally wherein the glycan preparation comprises any amount of glucose between 1% and 100%, further optionally wherein the glycan preparation comprises a second, third, fourth or fifth glycan unit (optionally, independently selected from xylose, arabinose, galactose, mannose, rhamnose, fructose, or fucose), further optionally, wherein the glycan preparation is one of: gal50glu25fru25, gal57glu43, gal57glu43, glu100, glu10gal10man80, glu10gal45man45, glu10gal80man10, glu20ara80, glu20gal20man20xyl20ara20, glu20gal20man60, glu20gal40man40, glu20gal60man20, glu20gal80, glu20xyl80, glu25gal25man25ara25, glu25gal25man25xyl25, glu25gal25xyl25ara25, glu25man25xyl25ara25, glu30gal30man40, glu30gal40man30, glu33gal33ara33, glu33gal33fuc33, glu33gal33man33, glu33gal33xyl33, glu33man33ara33, glu33man33xyl33, glu33xyt33ara33, glu40ara60, glu40gal20man40, glu40gal30man30, glu40gal40man20, glu40gal60, glu40xyl60, glu45gal10man45, glu45gal45man10, glu50gal50, glu5gal5man90, glu5gal90man5, glu60ara40, glu60gal20man20, glu60gal40, glu60man40, glu60xyl40, glu66fru33, glu80ara20, glu80gal10man10, glu80ga120, glu80man20, glu80man20, glu80xyl20, glu90gal5man5, man52glu29gal19, man60glu40, man62glu38, man80glu20, xyl33glu33gal33, or xyl75glu12gal12;
2) a galactose glycan unit, optionally wherein the glycan preparation comprises any amount of galactose between 1% and 100%, further optionally wherein the glycan preparation comprises a second, third, fourth or fifth glycan unit (optionally, independently selected from xylose, arabinose, glucose, mannose, rhamnose, fructose, or fucose), further optionally, wherein the glycan preparation is one of: ara50gal50, gal100, gal20ara80, gal20xyl80, gal25man25xyl25ara25, gal33man33ara33, gal33man33xyl33, gal33xyl33ara33, gal40ara60, gal40man60, gal40xyl60, gal50glu25fru25, gal57fru43, gal57glu43, gal60ara40, gal60man40, gal60xyl40, gal75xyl25, gal80ara20, gal80man20, gal80xyl20, glu10gal10man80, glu10gal45man45, glu10gal80man10, glu20gal20man20xyl20ara20, glu20gal20man60, glu20gal40man40, glu20gal60man20, glu20gal80, glu25gal25man25ara25, glu25gal25man25xyl25, glu25gal25xyl25ara25, glu30gal30man40, glu30gal40man30, glu33gal33ara33, glu33gal33fuc33, glu33gal33man33, glu33gal33xyl33, glu40gal20man40, glu40gal30man30, glu40gal40man20, glu40gal60, glu45gal10man45, glu45gal45man10, glu50gal50, glu5gal5man90, glu5gal90man5, glu60gal20man20, glu60gal40, glu80gal10man10, glu80gal20, glu90gal5man5, man52glu29gal19, man66gal33, man75gal25, man80gal20, xyl33glu33gal33, xyl75ga125, or xyl75glu12gal12;
3) a mannose glycan unit, optionally wherein the glycan preparation comprises any amount of mannose between 1% and 100%, further optionally wherein the glycan preparation comprises a second, third, fourth or fifth glycan unit (optionally, independently selected from xylose, arabinose, glucose, galactose, rhamnose, fructose, or fucose), further optionally, wherein the glycan preparation is one of: gal25man25xyl25ara25, gal33man33ara33, gal33man33xyl33, gal40man60, gal60man40, gal80man20, glu10gal110man80, glu10gal45man45, glu10gal80man10, glu20gal20man20xyl20ara20, glu20gal20man60, glu20gal40man40, glu20gal60man20, glu25gal25man25ara25, glu25gal25man25xyl25, glu25man25xyl25ara25, glu30gal30man40, glu30gal40man30, glu33gal33man33, glu33man33ara33, glu33man33xyl33, glu40gal20man40, glu40gal30man30, glu40gal40man20, glu45gal10man45, glu45gal45man10, glu5gal5man90, glu5gal90man5, glu60gal20man20, glu60man40, glu80gal10man10, glu80man20, glu80man20, glu90gal5man5, man100, man20ara80, man20xyl80, man33xyl33ara33, man40ara60, man40xyl60, man52glu29gal19, man60ara40, man60glu40, man60xyl40, man62glu38, man66gal33, man75gal25, man80ara20, man80gal20, man80glu20, or man80xyl20;
4) an arabinose glycan unit, optionally wherein the glycan preparation comprises any amount of arabinose between 1% and 100%, further optionally wherein the glycan preparation comprises a second, third, fourth or fifth glycan unit (optionally, independently selected from xylose, glucose, galactose, mannose, rhamnose, fructose, or fucose), further optionally, wherein the glycan preparation is one of: ara100, ara50gal50, ara50xyl50, ara60xyl40, ara80xyl20, gal20ara80, gal25man25xyl25ara25, gal33man33ara33, gal33xyl33ara33, gal40ara60, gal60ara40, gal80ara20, glu20ara80, glu20gal20man20xyl20ara20, glu25gal25man25ara25, glu25gal25xyl25ara25, glu25man25xyl25ara25, glu33gal33ara33, glu33man33ara33, glu33xyl33ara33, glu40ara60, glu60ara40, glu80ara20, man20ara80, man33xyl33ara33, man40ara60, man60ara40, man80ara20, xyl60ara40, xyl75ara25, or xyl80ara20;
5) a xylose glycan unit, optionally wherein the glycan preparation comprises any amount of xylose between 1% and 100%, further optionally wherein the glycan preparation comprises a second, third, fourth or fifth glycan unit (optionally, independently selected from arabinose, glucose, galactose, mannose, rhamnose, fructose, or fucose), further optionally, wherein the glycan preparation is one of: ara50xyl50, ara60xyl40, ara80xyl20, gal20xyl80, gal25man25xyl25ara25, gal33man33xyl33, gal33xyl33ara33, gal40xyl60, gal60xyl40, gal75xyl25, gal80xyl20, glu20gal20man20xy120ara20, glu20xyl80, glu25gal25man25xyl25, glu25gal25xyl25ara25, glu25man25xyl25ara25, glu33gal33xyl33, glu33man33xyl33, glu33xyl33ara33, glu40xy160, glu60xyl40, glu80xy120, man20xyl80, man33xyl33ara33, man40xyl60, man60xyl40, man80xyl20, xyl100, xyl33glu33gal33, xyl60ara40, xyl75ara25, xyl75ga125, xyl75glu12gal12, or xyl80ara20;
6) a fructose glycan unit, optionally wherein the glycan preparation comprises any amount of fructose between 1% and 100%, further optionally wherein the glycan preparation comprises a second, third, fourth or fifth glycan unit (optionally, independently selected from xylose, arabinose, glucose, galactose, mannose, rhamnose, or fucose), further optionally, wherein the glycan preparation is one of: fru100, gal50glu25fru25, gal57fru43, or glu66fru33;
7) a fucose glycan unit, optionally wherein the glycan preparation comprises any amount of fucose between 1% and 100%, further optionally wherein the glycan preparation comprises a second, third, fourth or fifth glycan unit (optionally, independently selected from xylose, arabinose, glucose, galactose, mannose, rhamnose, or fructose), further optionally, wherein the glycan preparation is one of: glu33gal33fuc33;
8) a rhamnose glycan unit, optionally wherein the glycan preparation comprises any amount of rhamnose between 1% and 100%, further optionally wherein the glycan preparation comprises a second, third, fourth or fifth glycan unit (optionally, independently selected from xylose, arabinose, glucose, galactose, mannose, fructose, or fucose), further optionally, wherein the glycan preparation is rha100; and
   further, optionally, wherein the glycan preparation comprises one or more (e.g., two, three, four, five, six, seven, eight, or nine) of the following properties (including bulk properties):
   i) the glycan preparation comprises glycans that comprise glucose, galactose, arabinose, mannose, fructose, xylose, fucose, or rhamnose glycan units;
   ii) the average degree of branching (DB) of the glycans in the glycan preparation is 0, between 0.01 and 0.6, between 0.05 and 0.5, between 0. 1 and 0.4, or between 0.15 and 0.4;
   iii) at least 50% (at least 60%, 65%, 70%, 75%, 80%, or 85%, or less than 50%) of the glycans in the glycan preparation have a degree of polymerization (DP) of at least 3 and less than 30 glycan units, at least 2 and less than 10 glycan units, at least 5 and less than 25 glycan units, or at least 10 and less than 35 glycan units (optionally, wherein the glycan unit is a monomer, e.g., a monosugar);
   iv) the average DP (mean DP) of the glycan preparation is between about 2 and 5, between about 5 and 8, between about 8 and 13, between about 13 and 25, between about 5 and 15, between about 5 and 20, or between about 5-15;
   v) the ratio of alpha- to beta-glycosidic bonds present in the glycans of the glycan preparation is 0, or between about 0.8:1 to about 5:1, between about 1:1 to about 5:1, between about 1:1 to about 3:1, between about 3:2 to about 2:1, or between about 3:2 to about 3:1,
   vi) the glycan preparation comprises between 15 mol % and 75 mol % (between 20 mol % and 60 mol %, between 25 mol % and 50 mol %, or between 30 mol % and 45 mol %) 1,6 glycosidic bonds;
   vii) the glycan preparation comprises between 1 mol % and 40 mol % (between 1 mol % and 30 mol %, between 5 mol % and 25 mol %, between 10 mol % and 20 mol %) of at least one, two, or three of 1,2; 1,3; and 1,4 glycosidic bonds;
   viii) the glycan preparation has a final solubility limit in water of at least about 50 (at least about 60, 70, at least about 75, or less than 50) Brix at 23 °C; or
   ix) the glycan preparation has a dietary fiber content (e.g., a,s measured by AOAC 2009.01) of at least 50% (at least 60%, 70%, 80%, or at least 90%, or less than 50%),
   x) any combination of:
      - two of: i), ii), iii), iv), v), vi), vii), viii), and ix);
      - three of: i), ii), iii), iv), v), vi), vii), viii), and ix);
      - four of: i), ii), iii), iv), v), vi), vii), viii), and ix);
      - five of: i), ii), iii), iv), v), vi), vii), viii), and ix);
      - six of: i), ii), iii), iv), v), vi), vii), viii), and ix);
      - seven of: i), ii), iii), iv), v), vi), vii), viii), and ix);
      - eight of: i), ii), iii), iv), v), vi), vii), viii), and ix); or
      - all of: i), ii), iii), iv), v), vi), vii), viii), and ix).

In some embodiments, preparations of glycans are provided, wherein at least one glycan subunit is selected from the group consisting of a glucose, a galactose, an arabinose, a mannose, a fructose, a xylose, a fucose, and a rhamnose. In one embodiment, glycan preparations are provided, wherein at least one glycan subunit is glucose. In one embodiment, glycan preparations are provided, comprising at least 90%, 95%, at least 99% or 100% glycans consisting of glucose. In some embodiments, glycan preparations comprise glu100. In some embodiments, glycan preparations comprise α-1,6-glu100. In some embodiments, glycan preparations comprise arabinogalactan. In some embodiments, glycan preparations comprise ga150glu25fru25. In some embodiments, glycan preparations comprise gal57glu43. In some embodiments, glycan preparations comprise glu66fru33. In some embodiments, glycan preparations comprise gal85ara15.

In some embodiments, the preparation of glycans comprises a desired mixture of two different monosaccharide glycan subunits, such as a mixture of, e.g., glucose and galactose, glucose and arabinose, glucose and mannose, glucose and fructose, glucose and xylose, glucose and fucose, glucose and rhamnose, galactose and arabinose, galactose and mannose, galactose and fructose, galactose and xylose, galactose and fucose, and galactose and rhamnose, arabinose and mannose, arabinose and fructose, arabinose and xylose, arabinose and fucose, and arabinose and rhamnose, mannose and fructose, mannose and xylose, mannose and fucose, and mannose and rhamnose, fructose and xylose, fructose and fucose, and fructose and rhamnose, xylose and fucose, xylose and rhamnose, and fucose and rhamnose, e.g. in a ratio of 1:1, 1:2, 1:3, 1:4, or 1:5 or the reverse ratio thereof, or a in a ratio of 1:1, 1:2, 1:3, 1:4, 1:5, 1:6, 1:7, 1:8, 1:9, 1:10, 1:12, 1:14, 1:16, 1:18, 1:20, 1:25, 1:30, 1:35, 1:40, 1:45, 1:50, 1:55, 1:60, 1:65, 1:70, 1:75, 1:80, 1:85, 1:90, or 1:100 or the reverse ratio thereof.

In some embodiments, the preparation of glycans comprises a desired mixture of three different monosaccharide glycan subunits, such as a mixture of, e.g. for glucose-containing glycan preparations, glucose, galactose and arabinose; glucose, galactose and mannose; glucose, galactose and fructose; glucose, galactose and xylose; glucose, galactose and fucose, glucose, galactose and rhamnose; glucose, arabinose, and mannose; glucose, arabinose and fructose; glucose, arabinose and xylose; glucose, arabinose and fucose; glucose, arabinose and rhamnose; glucose, mannose and fructose; glucose, mannose and xylose; glucose, mannose and fucose; glucose, mannose rhamnose; glucose, fructose and xylose; glucose, fructose and fucose; glucose, fructose and rhamnose; glucose, fucose and rhamnose, e.g. in a ratio of 1:1:1, 1:2:1, 1:3:1., 1:4:1, 1:5:1, 1:1:2, 1:2:2, 1:3:2, 1:4:2, 1:1:3, 1:2:3, 1:3:3, 1:1:4, 1:2:4, 1:1:5, 1:2:5, , etc., or . a in a ratio of 1:1:1, 1:2:1, 1:3:1, 1:4:1, 1:5:1, 1:6:1, 1:7:1, 1:8:1, 1:9:1, 1:10:1, 1:12:1, 1:14:1, 1:16:1, 1:18:1, 1:20:1, 1:1:2, 1:2:2, 1:3:2, 1:4:2, 1:5:2, 1:6:2, 1:7:2, 1:8:2, 1:9:2, 1:10:2, 1:1:3, 1:2:3, 1:3:3, 1:4:3, 1:5:3, 1:6:3, 1:7:3, 1:8:3, 1:9:3, 1:10:3, 1:1:4, 1:2:4, 1:3:4, 1:4:4, 1:5:4, 1:6:4, 1:7:4, 1:8:4, 1:9:4, 1:10:4, 1:1:5, 1:2:5, 1:3:5, 1:4:5, 1:5:5, 1:6:5, 1:7:5, 1:8:5, 1:9:5, 1:10:5, etc.

In some embodiments, the preparation of glycans does not comprise N-acetylgalactosamine or N-acetylglucosamine. In some embodiments, the preparation of glycans does not comprise sialic acid. In some embodiments, the preparation of glycans does not comprise a lipid and fatty acid. In some embodiments, the preparation of glycans does not comprise an amino acid.

### Furanose: Pyranose

In some embodiments, preparations of glycans are provided, wherein at least one glycan subunit is a furanose sugar. In some embodiments, preparations of glycans are provided, wherein at least one glycan subunit is a pyranose sugar. In some embodiments, glycans comprise mixtures of furanose and pyranose sugars. In some embodiments, the furanose: pyranose sugar ratio in a preparation is about 0.1:1, 0.2:1, 0.3:1, 0.4:1, 0.5:1, 0.6:1, 0.7:1, 0.8:1, 0.9:1, 1:1, 1.2:1, 1.5:1, 1.7:1, 2:1, 2.2:1, 2.5:1, 2.7:1, 3:1, 4:1, 5:1, or about 6:1 or the furanose: pyranose sugar ratio in a preparation is about 7:1, 8:1, 9:1, or about 10:1..

In some embodiments, the preparation of glycans comprises substantially all furanose or pyranose sugar, optionally comprising 1%, 2%, 3%, 4% 5%, 6%, 7%, 8%, 9%, 10%, 11%, 12%, 13%. 14%, 15%, 16%, 17%, 18%, 19%, or 20% of the respective other sugar.

In some embodiments, the preparation of glycans comprises substantially all pyranose sugar and no more than about 0.1%, 02%, 0.5%, 1%, 2%, 3%, 4%, or no more than 5% of glycan units in the preparation in furanose form. In some embodiments, no more than 3%, 2% or no more than 1% of monomeric glycan units in the preparation are in furanose form.

### Salts

In some embodiments, the preparation of glycans comprises a glycan subunit or plurality of glycan subunits present in a salt form (e.g., a pharmaceutically acceptable salt form), such as, e.g., a hydrochlorate, hydroiodate, hydrobromate, phosphate, sulfate, methanesulfate, acetate, formate, tartrate, malate, citrate, succinate, lactate, gluconate, pyruvate, fumarate, propionate, aspartate, glutamate, benzoate, ascorbate salt.

### Derivatization

If desired, the monosaccharide or oligosaccharide glycan subunits of the glycans are further substituted or derivatized, e.g., hydroxyl groups can be etherified or esterified. For example, the glycans (e.g. oligo- or polysaccharide) can contain modified saccharide units, such as 2'-deoxyribose wherein a hydroxyl group is removed, 2'-fluororibose wherein a hydroxyl group is replaced with a fluorine, or N- acetylglucosamine, a nitrogen-containing form of glucose (e.g., 2'-fluororibose, deoxyribose, and hexose). The degree of substitution (DS, average number of hydroxyl groups per glycosyl unit) can be 1, 2, or 3, or another suitable DS. In some embodiments, 1%, 2%, 3%, 4% 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 80%, 85%, 90%, 95%, 96%, 97%, 98%, 99%, or 100% of glycan subunits are substituted or derivatized. In some embodiments, the degree of substitution varies between subunits, e.g., a certain percentage is not derivatized, exhibits a DS of 1, exhibits a DS of 2, or exhibits a DS of 3. Any desired mixture can be generated, e.g. 0-99% of subunits are not derivatized, 0-99% of subunits exhibit a DS of 1, 0-99% of subunits exhibit a DS of 2, and 0-99% of subunits exhibit a DS of 3, with the total making up 100%. The degree of substitution can be controlled by adjusting the average number of moles of substituent added to a glycosyl moiety (molar substitution (MS)). The distribution of substituents along the length of the glycan oligo- or polysaccharide chain can be controlled by adjusting the reaction conditions, reagent type, and extent of substitution. In some embodiments, the monomeric subunits are substituted with one or more of an acetate ester, sulfate half-ester, phosphate ester, or a pyruvyl cyclic acetal group.

### Solubility

In some embodiments, the glycans in a preparation are highly soluble. In some embodiments, glycan preparations can be concentrated to at least to 55 Brix, 65 Brix, 60 Brix, 65 Brix, 70 Brix, 75 Brix, 80 Brix, or at least 85 Brix without obvious solidification or crystallization at 23 °C (final solubility limit). In some embodiments, glycan preparations are concentrated to at least about 0.5 g/ml, 1 g/ml, 1.5 g/ml, 2 g/ml, 2.5 g/ml, 3 g/ml, 3.5 g/ml or at least 4 g/ml without obvious solidification or crystallization at 23 °C (final solubility limit). In some embodiments, the glycan preparations (e.g. oligosaccharides) are branched, e.g. have an average DB of at least 0.01, 0.05, or 0.1 and has a final solubility limit in water of at least about 70 Brix, 75 Brix, 80 Brix, or at least about 85 Brix at 23 °C or is at least about 1 g/ml, 2 g/ml or at least about 3 g/ml.

In some embodiments, the preparation of glycans has a final solubility limit of at least 0.001 g/L, 0.005 g/L, 0.01 g/L, 0.05 g/L, 0.1 g/L, 0.2 g/L, 0.3 g/L, 0.4 g/L, 0.5 g/L, 0.6 g/L, 0.7 g/L, 0.8 g/L, 0.9 g/L, 1g/L, 5 g/L, 10 g/L, 20 g/L, 30 g/L, 40 g/L, 50 g/L, 100 g/L, 200 g/L, 300 g/L, 400 g/L, 500 g/L, 600 g/L, 700 g/L, 800 g/L, 900 g/L, 1000 g/L in deionized water, or in a suitable buffer such as, e.g., phosphate-buffered saline, pH 7.4 or similar physiological pH) and at 20 °C. In some embodiments, the preparation of glycans is greater than 50%, greater than 60%, greater than 70%, greater than 80%, greater than 90%, greater than 95%, greater than 96%), greater than 97%, greater than 98%, greater than 99%, or greater than 99.5% soluble with no precipitation observed at a concentration of greater than 0.001 g/L, 0.005 g/L, 0.01 g/L, 0.05 g/L, 0.1 g/L, 0.2 g/L, 0.3 g/L, 0.4 g/L, 0.5 g/L, 0.6 g/L, 0.7 g/L, 0.8 g/L, 0.9 g/L, 1g/L, 5 g/L, 10 g/L, 20 g/L, 30 g/L, 40 g/L, 50 g/L. 100 g/L, 200 g/L, 300 g/L, 400 g/L, 500 g/L, 600 g/L, 700 g/L, 800 g/L, 900 g/L, 1000 g/L in deionized water, or in a suitable buffer such as, e.g., phosphate-buffered saline, pH 7.4 or similar physiological pH) and at 20 °C.

### Sweetness

In some embodiments, the preparation of glycans has a desired degree of sweetness. For example, sucrose (table sugar) is the prototype of a sweet substance. Sucrose in solution has a sweetness perception rating of 1, and other substances are rated relative to this (e.g., fructose, is rated at 1.7 times the sweetness of sucrose). In some embodiments, the sweetness of the preparation of glycans ranges from 0.1 to 500,000 relative to sucrose. In some embodiments, the relative sweetness is 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40,45, 50, 55, 60, 65, 70. 80, 90, 100, 150, 200, 250, 300, 350, 400, 450, 500, 550, 600, 650, 700, 750, 800, 850, 900, 950, 1000, 2000, 3000, 4000, 5000, 6000, 7000, 8000, 9000, 10000, 25000, 50000, 75000, 100000, 150000, 200000, 250000, 300000, 350000, 40000, 450000, 500000, or more than 500,000 relative to sucrose (with sucrose scored as one). In some embodiments, the preparation of glycans is mildly sweet, or both sweet and bitter.

In some embodiments, the preparation of glycans, e.g. a preparation that is substantially DP2+ or DP3+ (e.g. at least 80%, 90%, or at least 95%, or a fractionated preparation of DP2+ or DP3+), is substantially imperceptible as sweet and the relative sweetness is about 0, 0.0001, 0.001, 0.005, 0.01, 0.05, 0.1, 0.2, 0.3, 0.4, 0.5, 0.6, 0.7, or about 0.8 relative to sucrose (with sucrose scored as one).

Glycan preparations can be characterized by any suitable methods including those described in WO2016/122889, WO2016/172657, WO 2016/007778, and WO2016/172658, incorporated herein by reference.
In embodiments, glycan compositions and glycan preparations may comprise one or more (e.g., two, three, four, five, six or more) of the following properties (including bulk properties):
a) the glycan comprising at least one of glucose, galactose, arabinose, mannose, fructose, xylose, fucose, or rhamnose,
b) a high degree of polymerization (DP), e.g. at least about 50%, 60%, 70%, 80%, 90%, 95%, 98%, 99% of polymers range in DP from about 30-100,000, about 30-50,000, about 30-10,000, about 30-5,000, about 30-1,000, about 30-500, about 30-200, about 30-100, or about 3-50,
c) a low degree of polymerization, e.g. at least about 50%, 60%, 70%, 80%, 90%, 95%, 98%, 99% of polymers range in DP from about 2-29, about 2-25, about 2-20, about 2-15, about 2-10, about 2-8, about 2-6, about 3-8, or about 4-8,
d) a high viscosity e.g., ranging from about 100-10,000 mPas, 100-5,000 mPas, 100-1,000 mPas, 100-500 mPas, in water at 20° C,
e) a low viscosity, e.g., ranging from about 1-99 mPas, 1-50 mPas, 1-10 mPas, 1-5 mPas, 25-75 mPas, or 10-50 mPas, in water at 20° C,
f) a high final solubility limit in water of at least about 60, 70, or at least about 75 Brix at 23° C,
g) a low final solubility limit in water of no more than 5, 10, 20, 30, 40, 50 Brix at 23° C, or insolubility (e.g. no more than 0.1 Brix)
h) a caloric value of about 0.1 cal/g to 3 cal/g, 0.1 cal/g to 2 cal/g, 0.1 cal/g to 1.5 cal/g, 0.1 cal/g to 1 cal/g, 0.1 cal/g to 0.5 cal/g,
i) a non-caloric value (e.g., about 0 cal/g to 0.09 cal/g, 0 cal/g to 0.05 cal/g or about 0 cal/g to 0.01 cal/g
j) a low degree of digestibility, wherein no more than about 30%, 20%, 10%, 5%, 1%, 0.5% of the glycan is digestible by a human glycosidase (e.g., alpha-amylase)
k) a high degree of digestibility, wherein at least 50%, 60%, 70%, 80%, 90%, 95% of the glycan is digestible by a human glycosidase (e.g., alpha-amylase)
l) a low degree of fermentability, wherein no more than about 40%, 30%, 20%, 10%, 5%, 1%, 0.5% of the glycan is fermentable by a human (e.g., colonic) microbial community or a single bacterial strain,
m) a high degree of fermentability, wherein at least 50%, 60%, 70%, 80%, 90%, 95% of the glycan is fermentable by a human (e.g. colonic) microbial community or a single bacterial strain,
n) a slow rate of fermentation, wherein no more than about 0.5%, 1%, 2%, 5%, 10%, or 15% of the glycan is fermented by a human (e.g., colonic) microbial community or a single bacterial strain in 12-24 hours,
o) a fast rate of fermentation, wherein at least about 15%, 20%, 30%, 40%, or 50% of the glycan is fermented by a human (e.g. colonic) microbial community or a single bacterial strain in 12-24 hours,
p) a high degree of gastrointestinal tolerance (e.g., is tolerated by a subject in high daily doses, e.g. at least about 5 g/day, 10 g/day, 15 g/day, 20 g/day, 30 g/day, 40 g/day, 50 g/day, 60 g/day, or 70 g/day without substantial side effects, e.g. such as bloating, excess gas, GI discomfort, diarrhea or constipation);
q) any combination of:
   - two of: a), b), c), d), e), f), g), h), i), j), k), l), m), n), o), p);
   - three of: a), b), c), d), e), f), g), h), i), j), k), l), m), n), o), p);
   - four of: a), b), c), d), e), f), g), h), i), j), k), l), m), n), o), p);
   - five of: a), b), c), d), e), f), g), h), i), j), k), l), m), n), o), p);
   - six of: a), b), c), d), e), f), g), h), i), j), k), 1), m), n), o), p);
   - seven of: a), b), c), d), e), f), g), h), i), j), k), l), m), n), o), p);
   - eight of: a), b), c), d), e), f), g), h), i), j), k), l), m), n), o), p);
   - nine of: a), b), c), d), e), f), g), h), i), j), k), l), m), n), o), p);
   - ten of: a), b), c), d), e), f), g), h), i), j), k), l), m), n), o), p); or
   - all of: a), b), c), d), e), f), g), h), i), j), k), l), m), n), o), p).

In embodiments, glycan compositions and glycan preparations may comprise one or more (e.g., two, three, four, five, six or more) of the following properties (including bulk properties):
i) the glycan preparation comprises glycans that comprise glucose, galactose, arabinose, mannose, fructose, xylose, fucose, or rhamnose glycan units;
ii) the average degree of branching (DB) of the glycans in the glycan preparation is 0, between 0.01 and 0.6, between 0.05 and 0.5, between 0.1 and 0.4, or between 0.15 and 0.4;
iii) at least 50% (at least 60%, 65%, 70%, 75%, 80%, or 85%, or less than 50%) of the glycans in the glycan preparation have a degree of polymerization (DP) of at least 3 and less than 30 glycan units, at least 2 and less than 10 glycan units, at least 5 and less than 25 glycan units, or at least 10 and less than 35 glycan units (optionally, wherein the glycan unit is a monomer, e.g., a monosugar);
iv) the average DP (mean DP) of the glycan preparation is between about 2 and 5, between about 5 and 8, between about 8 and 13, between about 13 and 25, between about 5 and 15, between about 5 and 20, or between about 5-15;
v) the ratio of alpha- to beta-glycosidic bonds present in the glycans of the glycan preparation is 0, or between about 0.8:1 to about 5:1, between about 1:1 to about 5:1, between about 1:1 to about 3:1, between about 3:2 to about 2:1, or between about 3:2 to about 3:1,
vi) the glycan preparation comprises between 15 mol % and 75 mol % (between 20 mol % and 60 mol %, between 25 mol % and 50 mol %, or between 30 mol % and 45 mol %) 1,6 glycosidic bonds;
vii) the glycan preparation comprises between 1 mol % and 40 mol % (between 1 mol % and 30 mol %, between 5 mol % and 25 mol %, between 10 mol % and 20 mol %) of each at least one, two, or three of 1,2; 1,3; and 1,4 glycosidic bonds;
viii) the glycan preparation has a final solubility limit in water of at least about 50 (at least about 60, 70, at least about 75, or less than 50) Brix at 23 °C; or
ix) the glycan preparation has a dietary fiber content (as measured by AOAC 2009.01) of at least 50% (at least 60%, 70%, 80%, or at least 90%, or less than 50%),
x) any combination of two, three, four, five, six, seven, eight, or nine of i), ii), iii), iv), v), vi), vii), viii), and ix).

Glycan compositions described herein can comprise one or more sugars and/or sugar alcohols._Compositions can comprise a simple sugar (such as a monosaccharide, a disaccharide, a trisaccharide, a tetrasacchaaride or a pentasaccharide), a sugar alcohol, or any combination thereof. In some embodiments, composition comprises a metabolizable sugar or metabolizable sugar alcohol, wherein the sugar or sugar alcohol is metabolized in the gastrointestinal tract of the host. The sugars, and sugar alcohols disclosed in WO 2016/172658, which is hereby incorporated by reference, are suitable for use in methods and compositions described herein. In embodiments, a composition described herein, e.g., glycan composition described herein, can comprise polyphenols, fatty acids (e.g., short chain fatty acids), amino acids, peptides, and micronutrients, e.g., as described herein and in WO 2016/172658 hereby incorporated by reference and in Table A.

**Table A. Exemplary constituents of glycan compositions: Sugars, Sugar Alcohols, Amino Acids, Vitamins, Minerals, Fatty Acids, and Polyphenols**

| Compound | Examples |
|---|---|
| Sugar | glucose, galactose, N-acetylglucosamine, N-acetylgalactosamine, fructose, fucose, mannose, N-acetylmannosamine, glucuronic acid, N-acetylglucuronic acid, galactosuronic acid, N-acetylgalactosuronic acid, xylose, arabinose, rhamnose, ribose, sucrose, sorbose, lactose, maltose, lactulose, tagatose, kojibiose, nigerose, isomaltose, trehalose, sophorose, laminaribiose, gentiobiose, turanose, maltulose, palatinose, gentiobiulose, mannobiose, melibiulose, rutinulose, xylobiose |
| Sugar Alcohol | sorbitol, mannitol, lactitol, erythritol, glycerol, arabitol, maltitol, xylitol, ribitol, threitol, galactitol, fucitol, iditol, inositol |
| Amino Acid | alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, valine |
| Vitamin | pantothenate, thiamine, riboflavin, niacin, pyridoxol, biotin, folate, 4-aminobenzoate, cobinamide, phenyolyl cobamide, 5-methylbenzimidazolyl cobamide, cobalamin, pyridoxine, pyridoxamine, ergadenylic acid, cyanocobalamin, choline, retinol, a carotenoid, zeaxanthin |
| Element/Mineral | chloride, sodium, calcium, magnesium, nitrogen, potassium, manganese, iron, zinc, nickel, copper, cobalt |
| Fatty Acid | acetic acid, propionic acid, butryic acid, isobutyric acid, valeric acid, isovaleric acid, hexanoic acid, octanoic acid, formic acid, oxalic acid, glyoxylic acid, glycolic acid, acrylic acid, malonic acid, pyruvic acid, lactic acid, succinic acid, acetoacetic acid, fumaric acid, maleic acid, oxaloacetic acid, malic acid, tartaric acid, crotonic acid, glutaric acid, alpha-ketoglutaric acid, caproic acid, adipic acid, citric acid, aconitic acid, isocitric acid, sorbic acid, enanthic acid, pimelic acid, benzoic acid, salicylic acid, caprylic acid, phthalic acid, pelargonic acid, trimesic acid, cinnamic acid, capric acid, sebacic acid, stearic acid, oleic acid, linoleic acid, α-linolenic acid, γ-linolenic acid, stearidonic acid |
| Polyphenol | Anthocyanins, Chalcones, Dihydro-chalcones, Dihydro-flavonols, Flavanols, Flavanones, Flavones, Flavonols, Isoflavonoids, Lignans, Non-phenolic metabolites, Alkylmethoxy-phenols, Alkylphenols,, Betacyanins, Capsaicinoids, Curcuminoids, Dihydro-capsaicins, Furano-coumarins, Hydroxy-benzaldehydes, Hydroxy-benzoketones, Hydroxycinnam-aldehydes, Hydroxy-coumarins, Hydroxyphenyl-alcohols, Hydroxy-phenylpropenes, Methoxyphenols, Naphtoquinones, Phenolic terpenes, Tyrosols, Hydroxybenzoic acids, Hydroxy-cinnamic acids, Hydroxyphenylacetic acids, Hydroxy-phenylpropanoic acids, Hydroxy-phenylpentanoic acids, Stilbenes, catechin, ellagitannin, isoflavone, flavonol, flavanone, anthocyanin, lignin, alkylmethoxyphenol, alkylphenol, curcuminoid, furanocoumarin, hydroxybenzaldehyde, hydroxybenzoketone, hydroxycinnamaldehyde, hydroxycoumarin, hydroxyphenylpropene, methoxyphenol, naphtoquinone, phenolic terpenes, tyrosols |

### Probiotics

In embodiments, a composition described herein, e.g., glycan composition described herein, can comprise commensal or probiotic bacterial taxa, e.g., those described in Tables 6-8, and bacteria that are generally recognized as safe (GRAS) or known commensal or probiotic microbes. In embodiments, a composition described herein, e.g., glycan composition described herein, can comprise bacterial taxa described in Tables 6-8. In some embodiments, probiotic or commensal bacterial taxa (or preparations thereof) may be administered to a subject (e.g., facility participant) receiving the glycan preparations.

In some embodiments, the composition further comprises at least about 1 % (w/w) of a probiotic or commensal bacterium or a combination thereof (e.g., at least about 2%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or more).

Probiotic microorganisms may also be included in the glycan compositions, or used in combination with a glycan composition described herein. A probiotic microorganism is also referred to a probiotic. Probiotics can include the metabolites generated by the probiotic microorganisms during fermentation. These metabolites may be released to the medium of fermentation, e.g., into a host organism (e.g., subject), or they may be stored within the microorganism. Probiotic microorganism includes bacteria, bacterial homogenates, bacterial proteins, bacterial extracts, bacterial ferment supernatants and combinations thereof, which perform beneficial functions to the host animal, e.g., when given at a therapeutic dose.

Useful probiotic microorganisms include at least one lactic acid and/or acetic acid and/or propionic acid producing bacteria, e.g., microbes that produce lactic acid and/or acetic acid and/or propionic acid by decomposing carbohydrates such as glucose and lactose. Preferably, the probiotic microorganism is a lactic acid bacterium. In embodiments, lactic acid bacteria include Lactobacillus, Leuconostoc, Pediococcus, Streptococcus, and Bifidobacterium. Suitable probiotic microorganisms can also include other microorganisms which beneficially affect a host by improving the hosts intestinal microbial balance, such as, but not limited to yeasts such as Saccharomyces, Debaromyces, Candida, Pichia and Torulopsis, molds such as Aspergillus, Rhizopus, Mucor, and Penicillium and Torulopsis, and other bacteria such as but not limited to the genera Bacteriodes, Clostridium, Fusobacterium, Melissococcus, Propionibacterium, Enterococcus, Lactococcus, Staphylococcus, Peptostreptococcus, Bacillus, Pediococcus, Micrococcus, Leuconostoc, Weissella, Aerococcus, and Oenococcus, and combinations thereof. Non-limiting examples of lactic acid bacteria useful in the disclosure herein include strains of Streptococcus lactis, Streptococcus cremoris, Streptococcus diacetylactis, Streptococcus thermophilus, Lactobacillus bulgaricus, Lactobacillus acidophilus, Lactobacillus helveticus, Lactobacillus bifidus, Lactobacillus casei, Lactobacillus lactis, Lactobacillus plantarum, Lactobacillus rhamnosus, Lactobacillus delbruekii, Lactobacillus thermophilus, Lactobacillus fermentii, Lactobacillus salivarius, Lactobacillus paracasei, Lactobacillus brevis, Bifidobacterium longum, Bifidobacterium infantis, Bifidobacterium bifidum, Bifidobcterium animalis, Bifidobcterium lactis, Bifidobcterium breve, Bifidobcterium adolescentis, and Pediococcus cerevisiae and combinations thereof, in particular Lactobacillus, Bifidobacterium, and combinations thereof

Probiotic microorganisms which are particularly useful in the present disclosure include those which (for human administration) are of human origin (or of the origin of the mammal to which the probiotic microorganism is being administered), are non-pathogenic to the host, resist technological processes (i.e. can remain viable and active during processing and in delivery vehicles), are resistant to gastric acidity and bile toxicity, adhere to gut epithelial tissue, have the ability to colonize the gastrointestinal tract, produce antimicrobial substances, modulate immune response in the host, and influence metabolic activity (e.g. cholesterol assimilation, lactase activity, vitamin production).

The probiotic microorganism can be included in the glycan preparations as a single strain or a combination of multiple strains, wherein the total number of bacteria in a dose of probiotic microorganism is from about 1x 10³ to about 1 x 10¹⁴, or from about 1x 10 to about 1 x 10¹², or from about 1 x 10⁷ to about 1 x 10¹¹ CFU per dose.

The probiotic microorganisms can be incorporated into the glycan preparations while the probiotic microorganism is alive but in a state of "suspended animation" or somnolence. Once freeze-dried, the viable cultures(s) of probiotic microorganism are handled so as to minimize exposure to moisture that would reanimate the cultures because, once reanimated, the cultures can experience high rates of morbidity unless soon cultured in a high moisture environment or medium. Additionally, the cultures are handled to reduce possible exposure to high temperatures (particularly in the presence of moisture) to reduce morbidity.

The probiotic microorganisms can be used in a powdered, dry form. The probiotic microorganisms can also be administered in the glycan preparation or in a separate glycan preparation, administered at the same time or different time as the glycan preparations. Examples of probiotics include, but are not limited to, those that acidify the colon such as those from the genera Lactobacillus or Bifidobacterium, which are thought to maintain a healthy balance of intestinal microbiota by producing organic acids (lactic & acetic acids), hydrogen peroxide, and bacteriocins which are documents to inhibit enteric pathogens.

Other Lactobacillus bacteria which can be employed include, but are not limited to, L. crispatus, L. casei, L. rhamnosus, L. reuteri, L. fermentum, L. plantarum, L. sporogenes, and L. bulgaricus.

Other probiotic bacteria suitable for the glycan compositions include Bifidobacterium lactis, B. animalis, B. bifidum, B. longum, B. adolescentis, and B. infantis. In embodiments, a commensal bacterial taxa that can be used in and/or in combination with a composition described herein comprises Akkermansia, Anaerococcus, Bacteroides, Bifidobacterium (including Bifidobacterium lactis, B. animalis, B. bifidum, B. longum, B. adolescentis, B. breve, and B. infantis), Blautia, Clostridium, Corynebacterium, Dialister, Eubacterium, Faecalibacterium, Finegoldia, Fusobacterium, Lactobacillus (including, L. acidophilus, L. helveticus, L. bifidus, L. lactis, L. fermentii, L. salivarius, L. paracasei, L. brevis, L. delbruekii, L. thermophiles, L. crispatus, L. casei, L. rhamnosus, L. reuteri, L. fermentum, L. plantarum, L. sporogenes, and L. bulgaricus), Peptococcus, Peptostreptococcus, Peptoniphilus, Prevotella, Roseburia, Ruminococcus, Staphylococcus, and/or Streptococcus (including S. lactis, S. cremoris, S. diacetylactis, S. thermophiles).

In embodiments, a commensal bacterial taxa, e.g., GRAS strain, that can be used in and/or in combination with a composition described herein comprises Bacillus coagulans GBI-30, 6086; Bifidobacterium animalis subsp. Lactis BB-12; Bifidobacterium breve Yakult; Bifidobacterium infantis 35624; Bifidobacterium animalis subsp. Lactis UNO 19 (DR10); Bifidobacterium longum BB536; Escherichia coli M-17; Escherichia coli Nissle 1917; Lactobacillus acidophilus DDS-1; Lactobacillus acidophilus LA- 5; Lactobacillus acidophilus NCFM; Lactobacillus casei DN 114-001 (Lactobacillus casei Inimumtas(s)/Defensis); Lactobacillus casei CRL431; Lactobacillus casei F19; Lactobacillus paracasei Stl 1 (or NCC2461); Lactobacillus johnsonii Lai (Lactobacillus LCI, Lactobacillus johnsonii NCC533); Lactococcus lactis L1A; Lactobacillus plantarum 299V; Lactobacillus reuteri ATTC 55730 (Lactobacillus reuteri SD2112); Lactobacillus rhamnosus ATCC 53013; Lactobacillus rhamnosus LB21; Saccharomyces cerevisiae (boulardii) lyo; mixture of Lactobacillus rhamnosus GR-1 and Lactobacillus reuteri RC-14; mixture of Lactobacillus acidophilus NCFM and Bifidobacterium lactis BB-12 or BL-04; mixture of Lactobacillus acidophilus CL1285 and Lactobacillus casei; and a mixture of Lactobacillus helveticus R0052, Lactobacillus rhamnosus R0011, and/or Lactobacillus rhamnosus GG (LGG).

In some embodiments, the method comprises the administration of a glycan preparation and the administration of a commensal or probiotic bacterial species. In some embodiments, the combined administration of glycan preparations and commensal bacteria may be used to benefit patients with depleted microbiomes (e.g., patients with few or no detectable commensal bacteria), e.g., patients who are undergoing chemotherapy or receiving antibiotics. In some embodiments, a subject or patient may have a gut microbiome devoid of any detectable commensal bacteria. In some embodiments, the method comprises combined administration of glycan preparations and commensal bacteria to a subject or patient who has a gut microbiome devoid of any detectable commensal bacteria.

### Synbiotics

Provided herein are combinations of microbes (e.g., bacterial taxa) with glycan compositions disclosed herein which can, e.g., be utilized by the microbes as their substrate for growth. Exogenously introduced microbes can provide a number of beneficial effects, such as, e.g., those described in Tables 6-8. This may occur by promoting the growth of the microbes (using the glycans), thereby allowing the microbes to outgrow other bacteria at the site of colonization.

Methods provided herein include administering one or more (e.g., one or more, two or more, three or more, four or more, and so on) bacterial taxa, such as those listed in Tables 6-8 to a subject (e.g., facility participant) in combination with a glycan composition. Such a combination can increase, suppress, and/or alter certain bacterial taxa. Methods are provided herein to modulate the processing of an exogenous substance described herein, comprising administering one or more (e.g., one or more, two or more, three or more, four or more, and so on) bacterial taxa to a subject in combination with a glycan described herein to a subject. The subject can include a subject that has taken, is taking or will be taking an antibiotic. The subject can include a subject that is not taking or has not taken an antibiotic.

### Prebiotics

In some embodiments, the glycan compositions comprise a prebiotic substance. In some embodiments, prebiotics may be administered to a subject (e.g., facility participant) receiving the glycan preparations. Prebiotics are substantially non-digestible substances by the host that when consumed may provide a beneficial physiological effect on the host by selectively stimulating the favorable growth or activity of a limited number of indigenous bacteria in the gut (Gibson G R, Roberfroid M B. J Nutr. (1995) 125:1401-12.). A prebiotic such as a dietary fiber or prebiotic oligosaccharide (e.g. crystalline cellulose, wheat bran, oat bran, cone fiber, soy fiber, beet fiber and the like) may further encourage the growth of probiotic and/or commensal bacteria in the gut by providing a fermentable dose of carbohydrates to the bacteria and increase the levels of those microbial populations (e.g. lactobacilli and bifidobacteria) in the gastrointestinal tract. Prebiotics may include, but are not limited to, various galactans and carbohydrate based gums, such as psyllium, guar, carrageen, gellan, lactulose, and konjac. In some embodiments, the prebiotic is one or more of galacto-oligosaccharides (GOS), lactulose, raffinose, stachyose, lactosucrose, fructo-oligosaccharides (FOS, e.g. oligofructose or oligofructan), inulin, isomaltooligosaccharide, xylo-oligosaccharides (XOS), paratinose oligosaccharide, isomaltose oligosaccharides (IMOS), transgalactosylated oligosaccharides (e.g. transgalacto-oligosaccharides), transgalactosylate disaccharides, soybean oligosaccharides (e.g. soyoligosaccharides), chitosan oligosaccharide (chioses), gentiooligosaccharides, soy- and pectin-oligosaccharides, glucooligosaccharides, pecticoligosacch arides, palatinose polycondensates, difructose anhydride III, sorbitol, maltitol, lactitol, polyols, polydextrose, linear and branched dextrans, pullulan (e.g. HMW or LMW), hemicelluloses, reduced paratinose, cellulose, beta-glucose, beta-galactose, beta-fructose, verbascose, galactinol, xylan, inulin, chitosan, beta-glucan, guar gum, gum arabic, pectin, high sodium alginate, and lambda carrageenan, or mixtures thereof. In some embodiments, the prebiotic is one or more of glu100, α-1,6-glu100, arabinogalactan, gal50glu25fru25, gal57glu43, glu66fru33, gal85ara15, or mixtures thereof.

Prebiotics can be found in certain foods, e.g. chicory root, Jerusalem artichoke, Dandelion greens, garlic, leek, onion, asparagus, wheat bran, wheat flour, banana, milk, yogurt, sorghum, burdock, broccoli, Brussels sprouts, cabbage, cauliflower, collard greens, kale, radish and rutabaga, and miso. In some embodiments, the glycan preparations described herein are administered to a subject (e.g., facility participant) in conjunction with a diet that includes foods rich in prebiotics. Suitable sources of soluble and insoluble fibers are commercially available. In some embodiments, a glycan composition comprises at least about 1% (w/w) of a prebiotic substance (e.g., at least about 2%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or more). In embodiments, the glycan composition comprises FOS. In embodiments, the glycan composition comprises lactulose.

Changes in bacterial populations can be measured by the "prebiotic index." The prebiotic index considers increases in the growth rate of Bifidobacteria, Eubacteria, and Lactobacilli as positive effects and increases in Clostridia, Bacteroides, sulfate-reducing bacteria, and Escherichia coli as negative effects. The prebiotic index (PI) relates to the sum of: (Bifidobacteria/total bacteria) + (Lactobacilli/total bacteria) - (Bacteroides/total bacteria) - (Clostridia/total bacteria), (see Palframan et al, 2003, Lett Appl Microbiol 37:281-284). In embodiments, administration of the glycan composition to a subject may result in an increased prebiotic index. Administration of a glycan composition to a subject may result in an increase in: Bacteroides, Blautia, Clostridium, Fusobacterium, Eubacterium, Ruminococcus, Peptococcus, Peptostreptococcus, Akkermansia, Faecalibacterium, Roseburia, Prevotella, Bifidobacterium, Lactobacilli, Christensenella minuta, or a Christensenellaceae.

In some embodiments, the glycan composition comprises an antibiotic, an antifungal agent, an antiviral agent, or an anti-inflammatory agent (e.g. a cytokine, hormone, etc.).

In some embodiments, the glycan compositions further comprise a second therapeutic agent or preparation thereof, such as a drug.

Pharmaceutical compositions, medical foods, supplements (e.g., dietary supplements) and unit dosage forms suitable for use in the methods and compositions described herein can be found in WO 2016/122889, WO 2016/172657, and WO 2016/172658, which are hereby incorporated by reference. Provided herein are also food supplements, food ingredients and nutraceuticals.

In some embodiments, the glycan compositions do not contain a prebiotic substance. In some embodiments glycan compositions do not contain a probiotic bacterium. In some embodiments, glycan compositions comprise one or more of glycan preparations described herein.

The glycan preparations described herein may be formulated into any suitable dosage form, e.g. for nasal, oral, rectal or gastric administration. In some embodiments, the glycan preparations described herein may be formulated for enteral administration. In some embodiments, the glycan preparations described herein may be formulated for tube feeding (e.g. naso-gastric, oral-gastric or gastric feeding). The dosage forms described herein can be manufactured using processes that are known to those of skill in the art.

The dosage form may be a packet, such as any individual container that contains a glycan preparation in the form of, e.g., a liquid (e.g., a beverage), a gel, a cream, an ointment, a powder, a tablet, a pill, a capsule, a sachet, a gummy, a suppository, a single-use applicator or medical device (e.g. a syringe). For example, provided is also an article of manufacture, such as a container comprising a unit dosage form of the glycan preparation, and a label containing instructions for use of such glycan.

Forms of the compositions that can be used orally include tablets, push-fit capsules and soft, sealed capsules. Compressed tablets can be prepared by compressing in a suitable machine the active ingredient in a free-flowing form such as a powder or granules, optionally mixed with binders (e.g., povidone, gelatin, hydroxypropylmethyl cellulose), inert diluents, preservative, antioxidant, disintegrant (e.g., sodium starch glycolate, cross-linked povidone, cross-linked sodium carboxymethyl cellulose) or lubricating, surface active or dispersing agents. The tablets can optionally be coated or scored and can be formulated so as to provide slow or controlled release of the active ingredient therein. The push-fit capsules can contain the active ingredients in admixture with filler such as lactose, binders such as starches, and/or lubricants such as talc or magnesium stearate and, optionally, stabilizers. In soft capsules, the active compounds and/or other agents (e.g., prebiotics or probiotics) can be dissolved or suspended in suitable liquids, such as fatty oils, liquid paraffin, or liquid polyethylene glycols. In one embodiment, a provided glycan preparation includes a softgel formulation. A softgel can contain a gelatin based shell that surrounds a liquid fill. The shell can be made of gelatin, plasticizer (e.g., glycerin and/or sorbitol), modifier, water, color, antioxidant, or flavor. In addition, stabilizers can be added.

Dragee cores are provided with suitable coatings. For this purpose, concentrated sugar solutions can be used, which can optionally contain gum arabic, talc, polyvinyl pyrrolidone, carbopol gel, polyethylene glycol, or titanium dioxide, lacquer solutions, and suitable organic solvents or solvent mixtures. Dyestuffs or pigments can be added to the tablets or dragee coatings for identification or to characterize different combinations of active compound doses. Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethylene glycol or an oil medium, for example peanut oil, liquid paraffin, or olive oil.

Solid formulations for oral use may comprise an enteric coating, which may control the location at which a glycan preparation is absorbed in the digestive system. For example, an enteric coating can be designed such that a glycan preparation does not dissolve in the stomach but rather travels to the small intestine, where it dissolves. An enteric coating can be stable at low pH (such as in the stomach) and can dissolve at higher pH (for example, in the small intestine). Material that can be used in enteric coatings includes, for example, alginic acid, cellulose acetate phthalate, plastics, waxes, shellac, and fatty acids (e.g., stearic acid, palmitic acid).

Liquid preparations can be in the form of, for example, aqueous or oily suspensions, solutions, emulsions, syrups or elixirs, or can be presented as a dry product (e.g. sachet) for reconstitution with water or other suitable aqueous vehicle before use. Such liquid preparations can contain conventional additives, such as suspending agents, for example sorbitol, methyl cellulose, glucose syrup, gelatin, hydroxyethyl cellulose, carboxymethyl cellulose, aluminum stearate gel or hydrogenated edible fats, emulsifying agents, for example lecithin, sorbitan monooleate, acacia; non-aqueous vehicles (which can include edible oils), for example almond oil, oily esters such as glycerine, propylene glycol, or ethyl alcohol; preservatives, for example methyl or propyl p-hydoxyhenzoate or sorbic acid, and, if desired, conventional flavoring or coloring agents. In some embodiments, liquid formulations can comprise, for example, an agent in water-in-solution and/or suspension form; and a vehicle comprising polyethoxylated castor oil, alcohol, and/or a polyoxyethylated sorbitan mono-oleate with or without flavoring. Each dosage form may comprise an effective amount of a glycan and can optionally comprise pharmaceutically inert agents, such as conventional excipients, vehicles, fillers, binders, disintegrants, pH adjusting substances, buffer, solvents, solubilizing agents, sweeteners, coloring agents, and any other inactive agents that can be included in pharmaceutical dosage forms for administration. Examples of such vehicles and additives can be found in Remington's Pharmaceutical Sciences, 17th edition (1985).

The pharmaceutical compositions provided herein can be in unit-dosage forms or multiple-dosage forms. A unit-dosage form, as used herein, refers to physically discrete unit suitable for administration to human in need thereof. In an embodiment, the unit-dosage form is provided in a package. Each unit-dose can contain a predetermined quantity of an active ingredient(s) sufficient to produce the desired therapeutic effect, in association with other pharmaceutical carriers or excipients. Examples of unit-dosage forms include, but are not limited to, ampoules, syringes, and individually packaged tablets and capsules. Unit-dosage forms can be administered in fractions or multiples thereof. A multiple-dosage form is a plurality of identical unit-dosage forms packaged in a single container, which can be administered in segregated unit-dosage form. Examples of multiple-dosage forms include, but are not limited to, vials, bottles of tablets or capsules, or bottles of pints or gallons. In another embodiment, the multiple dosage forms comprise different pharmaceutically active agents. For example, a multiple dosage form can be provided which comprises a first dosage element comprising a composition comprising a glycan and a second dosage element comprising a prebiotic, a therapeutic agent and/or a probiotic, which can be in a modified release form. In this example a pair of dosage elements can make a single unit dosage. In one embodiment, a kit is provided comprising multiple unit dosages, wherein each unit comprises a first dosage element comprising a composition comprising a glycan preparation and a second dosage element comprising probiotic, a pharmaceutical agent, a prebiotic or a combination thereof, which can be in a modified release form. In another embodiment, the kit further comprises a set of instructions. In some embodiments, the unit-dosage form comprises between about 1 mg to about 100 g of the glycan preparation (e.g., a glycan disclosed herein). For example, the unit-dosage form may comprise about 50 mg to about 50 g, about 500 mg to about 50 g, about 5 g to about 50 g, about 100 mg to about 100 g, about 1 g to about 100 g, about 10 g to about 100 g, about 1 g to about 10 g, about 1 g to about 20 g, about 1 g to about 30 g, about 1 g to about 40 g, about 1 g to about 50 g, about 1 g to about 60 g, about 1 g to about 70 g, about 1 g to about 80 g, about 1 g to about 90 g, about 1 g to about 100 g, about 1 g to about 150 g, about 1 g to about 200 g of the glycan. In other embodiments, the unit-dosage form comprises between about 0.001 mL to about 1000 mL of the glycan (e.g., a glycan disclosed herein). For example, the unit-dosage form may comprise about 0.001 mL to about 950 mL, about 0.005 mL to about 900 mL, about 0.01 mL to about 850 mL, about 0.05 mL to about 800 mL, about 0.075 mL to about 750 mL, about 0. 1 mL to about 700 mL, about 0.25 mL to about 650 mL, about 0.5 mL to about 600 mL, about 0.75 mL to about 550 mL, about 1 mL to about 500 mL, about 2.5 mL to about 450 mL, about 5 mL to about 400 mL, about 7.5 mL to about 350 mL, about 10 mL to about 300 mL, about 12.5 mL to about 250 mL, about 15 mL to about 200 mL, about 17.5 mL to about 150 mL, about 20 mL to about 100 mL, or about 25 mL to about 75 mL of the glycan.

In certain embodiments, the unit-dosage form comprises about 0.001 mL to about 10 mL, about 0.005 mL to about 7.5 mL, about 0.01 mL to about 5 mL, about 0.05 mL to about 2.5 mL, about 0.1 mL to about 1 mL, about 0.25 mL to about 1 mL, or about 0.5 mL to about 1 mL of the glycan. In other embodiments, the unit-dosage form comprises about 0.01 mL to about 10 mL, about 0.025 mL to about 7.5 mL, about 0.05 mL to about 5 mL, or about 0.1 mL to about 2.5 mL of the glycan. In other embodiments, the unit-dosage form comprises about 0.1 mL to about 10 mL, about 0.25 mL to about 7.5 mL, about 0.5 mL to about 5 mL, about 0.5 mL to about 2.5 mL, or about 0.5 mL to about 1 mL of the glycan.

In some embodiments, the unit-dosage form, e.g., a tablet, capsule (e.g., a hard capsule, push-fit capsule, or soft capsule), or softgel, has a body length of between about 0.1 inches to about 1.5 inches (e.g., about 0.5 inches and about 1 inch), or about 5 mm to about 50 mm (e.g., about 10 mm to about 25 mm). In some embodiments, the unit-dosage form. e.g., a tablet, capsule (e.g., a hard capsule, push-fit capsule, or soft capsule), or softgel, has an external diameter of about 0.05 inches to about 1 inch (e.g., about 0.1 inches to about 0.5 inches), or about 1 mm to about 25 mm (e.g., about 5 mm to about 10 mm).

Each unit-dosage form of the glycan may have a caloric value of between about 0.01 kcal and about 1000 kcal. For example, the unit-dosage form may have a caloric value of about 0.01 kcal to about 100 kcal, about 0.05 kcal to about 50 kcal, about 0.1 kcal to about 10 kcal, about 0.25 kcal to about 2.5 kcal, about 0.5 kcal to about 5 kcal, about 0.75 kcal to about 7.5 kcal, about 1 kcal to 10 kcal, about 5 kcal to about 50 kcal, or about 10 kcal to about 100 kcal. In certain embodiments, the unit-dosage form of the glycan has a caloric value of between 10 kcal to about 500 kcal.. In certain embodiments, the unit-dosage form of the glycan has a caloric value of between 1 kcal to about 100 kcal. In certain embodiments, the unit-dosage form of the glycan has a caloric value of between 0. 1 kcal to about 10 kcal.

In still other embodiments, the unit-dosage form may have a caloric value of about 0.001 kcal to about 10 kcal, about 0.005 kcal to about 10 kcal, about 0.01 kcal to about 10 kcal, about 0.025 kcal to about 25 kcal, about 0.05 kcal to about 50 kcal, about 0.075 kcal to about 75 kcal, about 0. 1 kcal to 100 kcal, about 0.25 kcal to about 10 kcal, about 0.5 kcal to about 5 kcal, about 0.25 kcal to about 25 kcal, or about 0.1 kcal to about 1 kcal.

The unit-dosage form of the glycan may be formulated to dissolve in an aqueous solution (e.g., water, milk, juice, and the like) and is orally administered as a beverage, syrup, solution, or suspension. For example, the unit-form dosage of the glycan may comprise a cube, packet, lozenge, pill, tablet, capsule, candy, powder, elixir, or concentrated syrup formulated for dissolving into an aqueous solution prior to oral administration. In other embodiments, the unit-dosage form of the glycan may comprise a cube, packet, lozenge, pill, tablet, capsule, candy, powder, elixir, or concentrated syrup formulated to dissolve in vivo, e.g., in the mouth, stomach, intestine, or colon of the subject upon oral administration.
In some embodiments, the glycan preparation is administered enterically. This preferentially includes oral administration, or by an oral or nasal tube (including nasogastric, nasojejunal, oral gastric, or oral jejunal). In other embodiments, administration includes rectal administration (including enema, suppository, or colonoscopy).

The dosage forms described herein can be manufactured using processes that are known to those of skill in the art. For example, for the manufacture of tablets, an effective amount of a prebiotic can be dispersed uniformly in one or more excipients or additives, for example, using high shear granulation, low shear granulation, fluid bed granulation, or by blending for direct compression. Excipients and additives include diluents, binders, disintegrants, dispersants, lubricants, glidants, stabilizers, surfactants, antiadherents, sorbents, sweeteners, and colorants, or a combination thereof. Diluents, also termed fillers, can be used to increase the bulk of a tablet so that a practical size is provided for compression. Non-limiting examples of diluents include lactose, cellulose, microcrystalline cellulose, mannitol, dry starch, hydrolyzed starches, powdered sugar, talc, sodium chloride, silicon dioxide, titanium oxide, dicalcium phosphate dihydrate, calcium sulfate, calcium carbonate, alumina and kaolin. Binders can impart cohesive qualities to a tablet formulation and can be used to help a tablet remain intact after compression.

Non-limiting examples of suitable binders include starch (including corn starch and pregelatinized starch), gelatin, sugars (e.g., glucose, dextrose, sucrose, lactose and sorbitol), celluloses, polyethylene glycol, alginic acid, dextrin, casein, methyl cellulose, waxes, natural and synthetic gums, e.g., acacia, tragacanth, sodium alginate, gum arabic, xantan gum, and synthetic polymers such as polymethacrylates, polyvinyl alcohols, hydroxypropylcellulose, and polyvinylpyrrolidone. Lubricants can also facilitate tablet manufacture; non-limiting examples thereof include magnesium stearate, calcium stearate, stearic acid, glyceryl behenate, and polyethylene glycol. Disintegrants can facilitate tablet disintegration after administration, and non-limiting examples thereof include starches, alginic acid, crosslinked polymers such as, e.g., crosslinked polyvinylpyrrolidone, croscarmellose sodium, potassium or sodium starch glycolate, clays, celluloses (e.g., carboxymethylcelluloses (e.g., carboxymethylcellulose (CMC), CMC-Na, CMC-Ca)), starches, gums and the like. Non-limiting examples of suitable glidants include silicon dioxide, talc, and the like. Stabilizers can inhibit or retard drug decomposition reactions, including oxidative reactions. Surfactants can also include and can be anionic, cationic, amphoteric or nonionic. Exemplary sweeteners may include stevia extract, aspartame, sucrose, alitame, saccharin, and the like. If desired, the tablets can also comprise nontoxic auxiliary substances such as pH buffering agents, preservatives, e.g., antioxidants, wetting or emulsifying agents, solubilizing agents, coating agents, flavoring agents (e.g., mint, cherry, anise, peach, apricot, licorice, raspberry, vanilla), and the like. Additional excipients and additives may include aluminum acetate, benzyl alcohol, butyl paraben, butylated hydroxy toluene, calcium disodium EDTA, calcium hydrogen phosphate dihydrate, dibasic calcium phosphate, tribasic calcium phosphate, candelilla wax, carnuba wax, castor oil hydrogenated, cetylpyridine chloride, citric acid, colloidal silicone dioxide, copolyvidone, corn starch, cysteine HCl, dimethicone, disodium hydrogen phosphate, erythrosine sodium, ethyl cellulose, gelatin, glycerin, glyceryl monooleate, glyceryl monostearate, glycine, HPMC pthalate, hydroxypropylcellulose, hydroxyl propyl methyl cellulose, hypromellose, iron oxide red or ferric oxide, iron oxide yellow, iron oxide or ferric oxide, magnesium carbonate, magnesium oxide, magnesium stearate, methionine, methacrylic acid copolymer, methyl paraben, silicified microcrystalline cellulose, mineral oil, phosphoric acid, plain calcium phosphate, anhydrous calcium phosphate, polaxamer 407, polaxamer 188, plain polaxamer, polyethylene oxide, polyoxyl40 stearate, polysorbate 80, potassium bicarbonate, potassium sorbate, potato starch, povidone, propylene glycol, propylene paraben, propyl paraben, retinyl palmitate, saccharin sodium, selenium, silica, silica gel, fumed silica, sodium benzoate, sodium carbonate, sodium citrate dihydrate, sodium crossmellose, sodium lauryl sulfate, sodium metabisulfite, sodium propionate, sodium starch, sodium starch glycolate, sodium stearyl fumarate, sorbic acid, sorbitol, sorbitan monooleate, pregelatinized starch, succinic acid, triacetin, triethyl citrate, vegetable stearin, vitamin A, vitamin E, vitamin C, or a combination thereof. The amounts of these excipients and additives can be properly selected based on their relation to other components and properties of the preparation and production method.

Immediate-release formulations of an effective amount of a glycan preparation can comprise one or more combinations of excipients that allow for a rapid release of a pharmaceutically active agent (such as from 1 minute to 1 hour after administration).Controlled-release formulations (also referred to as sustained release (SR), extended-release (ER, XR, or XL), time-release or timed-release, controlled-release (CR), or continuous-release) refer to the release of a glycan preparation from a dosage form at a particular desired point in time after the dosage form is administered to a subject.

In one embodiment a controlled release dosage form begins its release and continues that release over an extended period of time. Release can occur beginning almost immediately or can be sustained. Release can be constant, can increase or decrease over time, can be pulsed, can be continuous or intermittent, and the like. In one embodiment, a controlled release dosage refers to the release of an agent from a composition or dosage form in which the agent is released according to a desired profile over an extended period of time. In one aspect, controlled-release refers to delayed release of an agent from a composition or dosage form in which the agent is released according to a desired profile in which the release occurs after a period of time.

Pharmaceutical carriers or vehicles suitable for administration of the compounds provided herein include all such carriers known to those skilled in the art to be suitable for the particular mode of administration. In addition, the compositions can one or more components that do not impair the desired action, or with components that supplement the desired action, or have another action.

In a further aspect, the dosage form can be an effervescent dosage form. Effervescent means that the dosage form, when mixed with liquid, including water and saliva, evolves a gas. Some effervescent agents (or effervescent couple) evolve gas by means of a chemical reaction which takes place upon exposure of the effervescent disintegration agent to water or to saliva in the mouth. This reaction can be the result of the reaction of a soluble acid source and an alkali monocarbonate or carbonate source. The reaction of these two general compounds produces carbon dioxide gas upon contact with water or saliva. An effervescent couple (or the individual acid and base separately) can be coated with a solvent protective or enteric coating to prevent premature reaction. Such a couple can also be mixed with previously lyophilized particles (such as a glycan). The acid sources can be any which are safe for human consumption and can generally include food acids, acid and hydrite antacids such as, for example: citric, tartaric, amalic, fumeric, adipic, and succinics. Carbonate sources include dry solid carbonate and bicarbonate salt such as sodium bicarbonate, sodium carbonate, potassium bicarbonate and potassium carbonate, magnesium carbonate and the like. Reactants which evolve oxygen or other gasses and which are safe for human consumption are also included. In one embodiment citric acid and sodium bicarbonate are used.

In another aspect, the dosage form can be in a candy form (e.g., matrix), such as a lollipop or lozenge. In one embodiment an effective amount of a glycan is dispersed within a candy matrix. In one embodiment the candy matrix comprises one or more sugars (such as dextrose or sucrose). In another embodiment the candy matrix is a sugar-free matrix. The choice of a particular candy matrix is subject to wide variation. Conventional sweeteners (e.g., sucrose), sugar alcohols suitable for use with diabetic subjects (e.g., sorbitol or mannitol), or other sweeteners (e.g., sweeteners described herein) may be employed. The candy base can be very soft and fast dissolving or can be hard and slower dissolving. Various forms will have advantages in different situations.

A candy mass composition comprising an effective amount of the glycan can be orally administered to a subject in need thereof so that an effective amount of the glycan will be released into the subject's mouth as the candy mass dissolves and is swallowed. A subject in need thereof includes a human adult or child.

The dosage forms described herein can also take the form of pharmaceutical particles manufactured by a variety of methods, including but not limited to high-pressure homogenization, wet or dry ball milling, or small particle precipitation. Other methods useful to make a suitable powder formulation are the preparation of a solution of active ingredients and excipients, followed by precipitation, filtration, and pulverization, or followed by removal of the solvent by freeze-drying, followed by pulverization of the powder to the desired particle size. In one embodiment, the pharmaceutical particles have a final size of 3-1000 microns, such as at most 3, 4, 5, 6, 7, 8, 9, 10, 20, 30, 40, 50, 60, 70, 80, 90, 100, 150, 200, 250, 300, 350, 400, 450, 500, 550, 600, 650, 700, 750, 800, 850, 900, 950, 1000 microns. In another embodiment, the pharmaceutical particles have a final size of 10-500 microns. In another embodiment, the pharmaceutical particles have a final size of 50-600 microns. In another embodiment, the pharmaceutical particles have a final size of 100-800 microns.

In another aspect, the disclosure provides a method of making a unit-dosage form described herein, comprising providing a glycan (e.g., a glycan described herein); formulating the glycan into a unit-dosage form (e.g., a unit-dosage form described herein), packaging the unit-dosage form, labelling the packaged unit-dosage form, and/or selling or offering for sale the packaged and labeled unit-dosage form.

The unit-dosage forms described herein may also be processed. In one embodiment, the processing comprises one or more of: processing the dosage form into a pharmaceutical composition, e.g., formulating, combining with a second component, e.g., an excipient or buffer; portioning into smaller or larger aliquots; disposing into a container, e.g., a gas or liquid tight container; packaging; associating with a label; shipping or moving to a different location. In one embodiment, the processing comprises one or more of: classifying, selecting, accepting or discarding, releasing or withholding, processing into a pharmaceutical composition, shipping, moving to a different location, formulating, labeling, packaging, releasing into commerce, or selling or offering for sale, depending on whether the predetermined threshold is met. In some embodiments, the processed dosage forms comprise a glycan described herein.

In some embodiments, the processing comprises one or more of: processing the dosage form into a pharmaceutical composition, e.g., formulating, combining with a second component, e.g., an excipient or buffer; portioning into smaller or larger aliquots; disposing into a container, e.g., a gas or liquid tight container; packaging; associating with a label; shipping or moving to a different location. In one embodiment, the processing comprises one or more of: classifying, selecting, accepting or discarding, releasing or withholding, processing into a pharmaceutical composition, shipping, moving to a different location, formulating, labeling, packaging, releasing into commerce, or selling or offering for sale, depending on the determination.

In another embodiment, an oral dosage form is provided comprising a glycan preparation, wherein the oral dosage form is a syrup. The syrup can comprise about 1%, 5%, 10%, 15%, 20%, 25%, 30%, 35%, 40%, 45%, 50%, 55%, 60%, 65%, 70%, 75%, 80%, or 85% solid. The syrup can comprise about 15%, 20%, 25%, 30%, 35%, 40%, 45%, or 50% liquid, for example, water. The solid can comprise a glycan preparation. The solid can be, for example, about 1-96%, 10-96%, 20-96%, 30-96%, 40-96%, 50-96%, 60-96%, 70-96%, 80-96%, or 90-96% glycan preparation. In another embodiment, a glycan preparation is formulated as a viscous fluid.

In one embodiment, the composition comprises a foaming component, a neutralizing component, or a water-insoluble dietary fiber. A foaming component can be at least one member selected from the group consisting of sodium hydrogen carbonate, sodium carbonate, and calcium carbonate. In one embodiment a neutralizing component can be at least one member selected from the group consisting of citric acid, L-tartaric acid, fumaric acid, L-ascorbic acid, DL-malic acid, acetic acid, lactic acid, and anhydrous citric acid. In one embodiment a water-insoluble dietary fiber can be at least one member selected from the group consisting of crystalline cellulose, wheat bran, oat bran, cone fiber, soy fiber, and beet fiber. The formulation can contain a sucrose fatty acid ester, powder sugar, fruit juice powder, and/or flavoring material.

In some embodiments, the dosage forms are formulated to release the pharmaceutical compositions comprising glycan preparations in a specific region(s) of the GI tract, such as the small or the large intestine. In some embodiments, the dosage forms are formulated to release the pharmaceutical compositions comprising glycan preparations in a specific region(s) of the GI tract, such as the cecum, ascending colon, transverse colon, descending colon, sigmoid colon, and/or rectum.

In some embodiments, the dosage form for the glycan preparations described herein is an enzyme-responsive delivery system. For example, trypsin responsive polymers can be made using hydrogels that are crosslinked by peptides that are degraded by trypsin. Trypsin is active in the small intestine. Trypsin-responsive delivery systems can be used to target delivery of the glycan preparations to the small intestine. In another example, enzyme-digestible hydrogels consisting of poly(vinyl pyrrolidone) crosslinked with albumin are degraded in the presence of pepsin.

In some embodiments, the dosage form for the glycan preparations described herein is a delivery device that enables prolonged retention at a specific site in the GI tract. For example, a gastroretentive delivery system enables prolonged release of the glycan preparations to the stomach. Gastroretentive delivery may be used for the glycan preparations that modulate bacteria in the stomach or in the upper small intestine.
In some embodiments, the dosage form for the glycan preparations described herein is a mucoadhesive delivery system that adheres to the mucosal surfaces of the stomach. They are typically composed of polymers with numerous hydrogen-bonding groups, e.g., cross-linked polyacrylic acids, sodium carboxymethyl cellulose, sodium alginate, carrageenan, Carbopol 934P, or thiolated polycarbophil.

In some embodiments, the dosage form for the glycan preparations described herein is an expanding delivery system that rapidly increases in size in the stomach, which slows its passage through the pylorus. Such systems include systems that unfold in the stomach. For example, geometric shapes such as tetrahedrons, rings, disks, etc. can be packed into a gelatin capsule. When the capsule dissolves, the shape unfolds. The systems can be composed of one or more erodible polymer (e.g., hydroxy-propyl cellulose), one or more non-erodible polymer (e.g., polyolefins, polyamides, polyurethanes). The glycan may then be dispersed within the polymer matrix. The retention times can be fine-tuned by the polymer blend. Alternatively, devices made from elastic polymers that are stable in the acidic pH of the stomach but dissolve in the neutral/alkaline conditions further along the GI tract can be used. Such polymer formulations can prevent intestinal obstruction when the device exits the stomach. Supramolecular polymer gels crosslinked by hydrogen bonds between carboxyl groups may also be used, e.g. composed of poly(acryloyl 6-aminocaproic acid) (PA6ACA) and poly(methacrylic acid-co-ethyl acrylate) (EUDRAGIT L 100-55). Other systems include swellable excipients, such as collagen sponges.

For example, a hydrogel matrix (e.g. a swellable core: polyvinyl pyrrolidone XL, Carbopol 934P, calcium carbonate) swells 2-50 times in the stomach. Superporous hydrogel composites swell to hundreds of times their original volume in a few minutes. Some systems exploit gas generation to achieve expansion, e.g. carbon dioxide-generating, expandable systems that are surrounded by a hydrophilic membrane.

In some embodiments, the dosage form for the glycan preparations described herein is a density-controlled delivery system. These systems are designed to either float or sink in gastric fluids, which delays their emptying from the stomach. For example, high-density systems enable the device to settle to the bottom of the stomach, below the pylorus, and thus avoid stomach emptying. Other systems are low-density/floating systems. Such devices may, e.g., comprise entrapped air in hollow chambers or may incorporate low-density materials like fats, oils, or foam powder. Low density may be achieved through swelling, e.g. hydrocolloid containing capsules dissolve upon contacting gastric fluid and the hydrocolloids swell to form a mucous body. Alternative polymers include: chitosans, sodium alginate, and glycerol monooleate matrix. Low density may be achieved through gas generation. For example, tablets loaded with carbonate and optionally citric acid generate carbon dioxide after contact with acidic aqueous media. The carbon dioxide generated is entrapped within the gelling hydrocolloid causing the system to float. Hydrocolloids include hydroxypropyl methylcellulose and Carbopol 934P. In some embodiments, the dosage form for the glycan preparations described herein employs a design to retain a device in the small or large intestine. The location-specific nature of the device is provided by a specific triggering method, e.g. pH, enzyme, etc. These include systems designed for mucoadhesion and also microneedle pills. Microneedle pills comprise a drug reservoir spiked with microneedles that is encapsulated in a pH-responsive coating. When the pill reaches the desired location in the GI tract and the coating dissolves, the microneedles enable the pill to become stuck to the lining of the GI tract. In other embodiments, the microneedle pills comprise a capsule that consists of two chemical compartments filled with citric acid and sodium bicarbonate, respectively. As the pill dissolves in the digestive system, barriers between the two substances erode, allowing them to mix and create a chemical reaction that pushes micro-needles of saccharides through the outer layer of the capsule and into the lining of the small intestine. The saccharide needles can be filled with drugs that are delivered into nearby blood vessels as the saccharide is absorbed.

In some embodiments, the dosage form for the glycan preparations described herein employs a pH sensitive polymer coating. For example, pH-dependent polymers (bi- or tri-phasic) can be insoluble at low pH levels (e.g. acid resistance in the stomach, pH 1-2) and become increasingly soluble as pH rises, e.g. to about 5.5 - 6.2 in the duodenum, to about pH 5.7 in the ascending colon, to about pH 6.4 in the cecum, to about pH 6.6 in the transverse colon, to about pH 7.0 in the descending colon, to about 7.2 - 7.5 in the ileum, or to about pH 7.5 in the distal small intestine. In one example, TARGIT™ technology may be used for site-specific delivery of the glycan preparations in the gastrointestinal (GI) tract. The system employs pH-sensitive coatings onto injection-molded starch capsules to target the terminal ileum and colon.

In some embodiments, the dosage form for the glycan preparations described herein is a delayed release system or time controlled release system. Such systems usually employ enteric coatings that may be combined with pH sensitive and time release functions. For example, ETP (enteric coated time-release press coated) tablets may be used that are composed of three components: a glycan-containing core tablet (rapid release function), a press-coated, swellable hydrophobic polymer layer (e.g. hydroxypropyl cellulose layer (HPC), and a time release function. The duration of lag phase can be controlled either by weight or composition of polymer layer and an enteric coating layer (acid resistance function).

In some embodiments, the dosage form for the glycan preparations described herein employs Eudragit® enteric coatings of tablets and capsules. Other suitable synthetic polymers include: Shellac, ethyl cellulose, cellulose acetate phthalate, hydroxypropylmethyl cellulose, polyvinyl acetate phthalate and poly glutamic acid coatings, such as poly-γ-glutamic acid (γ-PGA). These coatings combine both mucoadhesive and pH-dependent release strategies. To enhance colon targeted delivery Eudragits® are methacrylic co-polymers with varying side group compositions that alter the pH at which they are soluble. For example, for Eudragit®-coated systems no significant drug release occurs in the stomach (e.g. at pH 1.4) and in the small intestine (e.g. at pH 6.3), while significant drug release can be seen at pH 7.8 in the ileocaecal region.

In some embodiments, the dosage form for the glycan preparations described herein is a microbial-triggered system, such as a polysaccharide based delivery system. Polysaccharide based delivery systems contain biodegradable and mucoadhesive polymer coatings, including coatings of chitosan and pectin. Other suitable natural polymers include, e.g., guar gum, inulin, cyclodextrin, dextran, amylase, chondrotin sulphate, and locust bean gum. These delivery systems can be used to target the glycan to the small intestine. Coatings with naturally occurring polysaccharides like guar gum, xanthan gum, chitosan, alginates, etc. are degraded by colonic gut microbiota, e.g. enzymes such as, xylosidase, arabinosidase, galactosidase etc. For example, CODES™ technology may be used to deliver the glycan preparations. This system combines the polysaccharide coating with a pH-sensitive coating. In some embodiments, the system consists of a core tablet coated with three layers of polymer coatings: The outer coating is composed of Eudragit L. This coating gets dissolved in the duodenum and exposes the next coating. The next coating is composed of Eudragit E. This layer allows the release of lactulose present in the inner core. The lactulose gets metabolized into short chain fatty acids that lower the surrounding pH where the Eudragit E layer dissolves. The dissolving of Eudragit E results in the exposure of the glycan. The bacteria present in the colon are responsible for the degradation of polysaccharides that are released from the core tablet. The degradation of polysaccharides may result in organic acids formation that lowers the pH of the contents surrounding the tablet.
In some embodiments, the dosage form for the glycan preparations described herein is a pressure-controlled delivery system. The system employs the fact that higher pressures are encountered in the colon than in the small intestine. For example, for ethylcellulose systems that are insoluble in water, the release of glycans occurs following disintegration of a water-insoluble polymer capsule as a result of pressure in the lumen of the colon. The release profile may be adjusted by varying the thickness of the ethylcellulose, the capsule size and/or density of the capsule.

In some embodiments, the dosage form for the glycan preparations described herein is a pulsatile colon targeted delivery system. For example, the system can be a pulsincap system. The capsule which is employed comprises a plug that is placed in the capsule that controls the release of the glycan. A swellable hydrogel (e.g. hydroxyl propyl methyl cellulose (HPMC), poly methyl methacrylate or polyvinyl acetate) seals the drug content. When the capsule gets in contact with a fluid the plug is pushed off from the capsule and the glycan is released. The release profile can be controlled by varying the length and/or point of intersection of the plug with the capsule body. Another system is a port system. The capsule body is enclosed in a semi-permeable membrane. The insoluble plug consists of an osmotically active agent and the glycan. When the capsule gets in contact with a fluid the semi-permeable membrane permits inflow of the fluid which increases pressure in the capsule body. This leads to an expelling of the plug and release of the glycan.

In some embodiments, the dosage form for the glycan preparations described herein is an osmotically controlled colon targeted delivery system. An exemplary system, OROS-CT, consists of osmotic units (up to 5 or 6 push pull units) encapsulated in a hard gelatin capsule. The push pull units are bi-layered with outer enteric impermeable membrane and inner semi-permeable membrane. The internal, central part of the push pull consists of the drug layer and push layer. The glycan is released through the semi-permeable membrane. The capsule body enclosing the push pull units is dissolved immediately after administration. In the GI tract the enteric impermeable membrane prevents water absorption. The enteric coating is dissolved in small intestine (higher pH, >7), water enters the unit through the semi-permeable membrane causing push layer to swell and force out the glycan.

In some embodiments, the dosage form for the glycan preparations described herein is "smart pill" which can be used to release the glycan just before reaching the ileocecal valve. In some embodiments, the dosage form for the glycan preparations described herein is a rectally administered formulation. For example, enemas introduce a glycan preparation in liquid formulation into the rectum. The volume administered is typically less than 10 mL. Suppositories introduce a glycan preparation into the rectum. Suppositories are solid dosage forms that melt or dissolve when inserted into the rectum, releasing the glycans. Typical excipients for suppository formulations include cocoa butter, polyethylene glycols, and agar.

### Kits

Kits also are contemplated. For example, a kit can comprise unit dosage forms of the glycan preparation, and a package insert containing instructions for use of the glycan in treatment of a gastrointestinal disorder or condition. The kits include a glycan preparation in suitable packaging for use by a subject in need thereof. Any of the compositions described herein can be packaged in the form of a kit. A kit can contain an amount of a glycan preparation (optionally additionally comprising a prebiotic substance, a probiotic bacterium, and/or a second therapeutic agent) sufficient for an entire course of treatment, or for a portion of a course of treatment. Doses of a glycan preparation can be individually packaged, or the glycan preparation can be provided in bulk, or combinations thereof. Thus, in one embodiment, a kit provides, in suitable packaging, individual doses of a glycan preparation that correspond to dosing points in a treatment regimen, wherein the doses are packaged in one or more packets.

In one embodiment, the glycan preparation can be provided in bulk in a single container, or in two, three, four, five, or more than five containers. For example, each container may contain enough of a glycan preparation for a particular week of a treatment program that runs for a month. If more than one bulk container is provided, the bulk containers can be suitably packaged together to provide sufficient glycan preparation for all or a portion of a treatment period. The container or containers can be labeled with a label indicating information useful to the subject in need thereof or the physician performing the treatment protocol, such as, e.g. dosing schedules.

The glycan preparation can be packaged with other suitable substances, such as probiotic bacteria, prebiotic substances or other substances, as described herein. The other substance or substances can be packaged separately from the glycan preparation, or mixed with the glycan preparation, or combinations thereof. Thus, in one embodiment, kits include a dosage form containing all the ingredients intended to be used in a course of treatment or a portion of a course of treatment, e.g., a glycan preparation and optionally buffers, excipients, etc., a probiotic, prebiotic or a polymer agent. In one embodiment, a glycan preparation is packaged in one package or set of packages, and additional components, such as probiotic bacteria, prebiotics, and therapeutic agents are packaged separately from the glycan preparation.

Kits can further include written materials, such as instructions, expected results, testimonials, explanations, warnings, clinical data, information for health professionals, and the like. In one embodiment, the kits contain a label or other information indicating that the kit is only for use under the direction of a health professional. The container can further include scoops, syringes, bottles, cups, applicators or other measuring or serving devices.

### Medical Food

Also provided herein are preparations of glycans formulated as a medical food. Any glycan preparation described herein may be formulated as a medical food as well as pharmaceutical compositions that comprise glycan preparations.

A medical food is defined in section 5(b)(3) of the Orphan Drug Act (21 U.S.C. 360ee(b)(3)). Medical food is formulated to be consumed (oral intake) or administered enterally (e.g. feeding/nasogastric tube) under medical supervision, e.g. by a physician. It is intended for the specific dietary management of a disease or condition, such as, e.g. dysbiosis or a GI-tract disease. Medical foods as used herein do not include food that is merely recommended by a physician as part of an overall diet to manage the symptoms or reduce the risk of a disease or condition. Medical foods comprising a preparation of glycans are foods that are synthetic (e.g., formulated and/or processed products, such as, being formulated for the partial or exclusive feeding of a subject by oral intake or enteral feeding by tube) and not naturally occurring foodstuff used in a natural state.

In some embodiments, the subject (e.g., facility participant) has limited or impaired capacity to ingest, digest, absorb, or metabolize ordinary foodstuffs or certain nutrients. In other embodiments, the subject has other special medically determined nutrient requirements, the dietary management of which cannot be achieved by the modification of the normal diet alone. Medical foods comprising a preparation of glycans are administered to a subject in need thereof under medical supervision (which may be active and ongoing) and usually, the subject receives instructions on the use of the medical food. Medical foods may comprise one or more food additives, color additives, GRAS excipients and other agents or substances suitable for medical foods. Medical food preparations may be nutritionally complete or incomplete formulas.

### Dietary Supplements

Any glycan preparation described herein may be formulated as a dietary supplement, e.g, for use in a method described herein. Dietary supplements are regulated under the Dietary Supplement Health and Education Act (DSHEA) of 1994. A dietary supplement is a product taken by mouth that contains a "dietary ingredient" intended to supplement the diet. The "dietary ingredients" in these products may include, in addition to a glycan preparation described herein, one or more of: vitamins, minerals, herbs or other botanicals, amino acids, and substances such as enzymes, organ tissues, glandulars, and metabolites. Dietary supplements can also be extracts or concentrates, and may be found in many forms such as tablets, capsules, softgels, gelcaps, liquids, or powders. They can also be in other forms, such as a bar, but if they are, information on their label must not represent the product as a conventional food or a sole item of a meal or diet. DSHEA requires that every supplement be labeled a dietary supplement and not as a general food.

### Food Ingredient

Any glycan preparation described herein may be formulated as a food ingredient or food additive, e.g., for use in a method described herein. Food ingredients may be generally recognized as safe (GRAS) or may require FDA authorization. Glycan preparations can be added to any desirable food, e.g. beverages (incl., e.g., fruit juices), dairy products (e.g., milk, yogurt, cheese), cereals (any grain products), bread, spreads, etc.

A glycan preparation may be formulated as a food. The term "food" as defined in the Federal Food, Drug and Cosmetic Act (21 U.S.C. Section 321(a)(f)) refers to articles used for food or drink for man or other animals, chewing gum, and articles used for components of any such article. Food is formulated to be consumed (oral intake). Foods may comprise, in addition to a glycan preparation, one or more food additives, color additives, GRAS excipients and other agents or substances suitable for foods. Food preparations may be nutritionally complete or incomplete formulas. Food products can be, e.g., a beverage, a powdered beverage mix, a bar, a candy, a dairy product, confection, baked good, a gummy, and the like.

### Methods of Modulating Microbial Taxa

The compounds and compositions provided herein may be used in methods to modulate bacterial taxa (e.g. 1, 2, 3, 4, 5 or more taxa) present in the microbiota of a subject (e.g., facility participant). In some embodiments, modulation comprises a change in the structure of the microbiota, such as a change in the relative composition of a taxa or a change in the relative abundance of a taxa, e.g., relative to another taxa or relative to what would be observed in the absence of the modulation. In other embodiments, modulation comprises a change in a function of the microbiota, such as a change in gene expression, level of a gene product (e.g., RNA or protein), or metabolic output of the microbiota, or a change in a functional pathway of the host (e.g., a change in gene expression, level of a gene product, or metabolic output of a host cell or host process). Methods of modulating microbial taxa disclosed in WO 2016/122889 and WO 2016/172657 which are hereby incorporated by reference, are suitable for use in methods described herein.

The methods describe herein include administering to a subject (e.g., facility participant) a composition described herein, e.g., comprising a glycan composition described herein, in an amount effective to modulate taxa. In some embodiments, the abundance of a bacterial taxa may increase relative to other taxa (or relative from one point in time to another) when the composition is administered and the increase can be at least a 5%, 10%, 25% 50%, 75%, 100%, 250%, 500%, 750% increase or at least a 1000% increase. The abundance of a bacterial taxa may also decrease relative to other taxa (or relative from one point in time to another) when the composition is administered and the decrease can be at least a 5%, 10%, 25% 50%, 75%, 85%, 90%, 95%, 96%, 97%, 98%, 99% decrease, or at least a 99.9% decrease. Administration of the composition can modulate the abundance of the desired and/or non-desired bacterial taxa in the subject's gastrointestinal microbiota.

In some embodiments, the composition described herein, e.g., comprising a glycan composition described herein, modulates (e.g. increases or decreases) the growth of one or more bacterium, such as, e.g., those that belong to genera Bacteroides, Odoribacter, Parabacteroides, Alistipes, Blautia, Clostridium, Coprococcus, Dorea, Eubacterium, Lachnospira, Roseburia, Ruminococcus, Faecalibacterium, Oscillospiri, and Subdoligranulum which can be found in the GI tract. In some embodiments, the composition described herein, e.g., comprising a glycan composition described herein, modulates (e.g. increases or decreases) the growth of one or more bacterium, such as, e.g., of the genus Akkermansia, Anaerofilum, Bacteroides, Blautia, Bifidobacterium, Butyrivibrio, Clostridium, Coprococcus , Dialister, Dorea, Fusobacterium, Eubacterium, Faecalibacterium, Lachnospira, Lactobacillus, Phascolarctobacterium, Peptococcus, Peptostreptococcus, Prevotella, Roseburia, Ruminococcus, and Streptococcus, and/or one or more of the species Akkermansia municiphilia, Christensenella minuta, Clostridium coccoides, Clostridium leptum, Clostridium scindens, Dialister invisus, Eubacterium rectal, Eubacterium eligens, Faecalibactelium prausnitzii. Streptococcus salivarius, and Streptococcus thermophilus.

In some embodiments, the composition described herein, e.g., comprising a glycan composition described herein modulates (e.g., increases or decreases) the growth of at least two bacterial taxa selected from Prevotella, Akkermansia, Bacteroides, Clostridium (Erysipelotrichaceae), Clostridium (Clostridiaceae), Bifidobacterium, Aggregatibacter, Clostridium (Peptostreptococcaveae), Parabacteroides, Lactobacillus, and Enterococcus. In some embodiments, a composition described herein, e.g., comprising a glycan composition described herein, modulates (e.g. increases or decreases) the growth of one or more bacterial taxa residing in the GI tract, such as, e.g., those that belong to genera Bacteroides, Odoribacter, Parabacteroides, Alistipes, Blautia, Clostridium, Coprococcus, Dorea, Eubacterium, Lachnospira, Roseburia, Ruminococcus, Faecalibacterium, Oscillospira, and Subdoligranulum which can be found in the GI tract. In some embodiments, a composition described herein, e.g., comprising a glycan composition described herein, modulates (e.g. increases or decreases) the growth of one or more bacterial taxa, such as those that are thought to be associated with a healthy gastrointestinal state, e.g., one or more of the genus Akkermansia, Anaerofilum, Bacteroides, Blautia, Bifidobacterium, Butyrivibrio, Clostridium, Coprococcus , Dialister, Dorea, Fusobacterium, Eubacterium, Faecalibacterium, Lachnospira, Lactobacillus, Phascolarctobacterium, Peptococcus, Peptostreptococcus, Prevotella, Roseburia, Ruminococcus, and Streptococcus, and/or one or more of the species Akkermansia municiphilia, Christensenella minuta, Clostridium coccoides, Clostridium leptum, Clostridium scindens, Dialister invisus, Eubacterium rectal, Eubacterium eligens, Faecalibacterium prausnitzii, Streptococcus salivarius, and Streptococcus thermophilus. In some embodiments, the composition described herein, e.g., comprising a glycan composition described herein, modulates (e.g. increases or decreases) the growth of one or more bacterial taxa, such as taxa of the phylum Verrucomicrobia, e.g., those of the genus Akkermansia.

In some embodiments, a composition described herein, e.g., comprising a glycan composition described herein, modulates (e.g. increases or decreases) the growth of one or more bacterial taxa predominantly residing in the small intestine. For example, the composition described herein, e.g., comprising a glycan composition described herein, modulates one or more (2, 3, 4, 5, 6, 7, 8, 9, 10 or more) bacterial taxa that reside predominantly in the small intestine, such as, e.g. Actinobacteria, Firmicutes (Bacilli, Clostridia), and Proteobacteria (Alphaproteobacteria, Betaproteobacteria). In some embodiments, a composition described herein, e.g., comprising a glycan composition described herein, modulates one or more (2, 3, 4 5, 6, 7, 8, 9, 10 or more) bacterial taxa that reside predominantly in the small intestine selected from the genera: Cryocola, Mycobacterium, Enterococcus, Lactococcus, Streptococcus, Turicibacter, Blautia, Coprococcus, Holdemania, Pseudoramibacter Eubacterium, Agrobacterium, Sphingomonas, Achromobacter, Burkholderia, and Ralstonia.

In some embodiments, a composition described herein, e.g., comprising a glycan composition described herein, modulates (e.g., increases or decreases) the growth of one or more bacterial taxa predominantly residing in the large intestine. For example, a composition described herein, e.g., comprising a glycan composition described herein, modulates one or more (2, 3, 4, 5, 6, 7, 8, 9, 10 or more) bacterial taxa that reside predominantly in the large intestine, such as, e.g. Bacteroidetes, Firmicutes (Clostridia), Verrucomicrobia, and Proteobacteria (Deltaproteobacteria). In some embodiments, a composition described herein, e.g., comprising a glycan composition described herein, modulates one or more (2, 3, 4, 5, 6, 7, 8, 9, 10 or more) bacterial taxa that reside predominantly in the large intestine selected from the genera: Bacteroides, Butyricimonas, Odoribacter, Parabacteroides, Prevotella, Anaerotruncus, Phascolarctobacterium, Ruminococcus, Bilophila, and Akkermansia.

In some embodiments, the composition described herein, e.g., comprising a glycan composition described herein, modulates (e.g. increases or decreases) the growth of one or more bacterial taxa predominantly residing in the cecum, such as, e.g. Actinobacteria, Bacteroides, Bacilli, Clostridia, Mollicutes, Alpha Proteobacteria, and Verrucomicrobia.
In some embodiments, a composition described herein, e.g., comprising a glycan composition described herein, modulates (e.g., increases or decreases) the growth of one or more bacterial taxa predominantly residing in the ascending colon, such as, e.g. Actinobacteria, Bacteroides, Bacilli, Clostridia, Fusobacteria, Beta Proteobacteria, Delta/Epsilon Proteobacteria, Gamma Proteobacteria, and Verrucomicrobia.

In some embodiments, the composition described herein, e.g., comprising a glycan composition described herein, modulates (e.g. increases or decreases) the growth of one or more bacterial taxa predominantly residing in the traverse colon, such as, e.g. Actinobacteria, Bacteroides, Clostridia, Mollicutes, Fusobacteria, and Gamma Proteobacteria.
In some embodiments, a composition described herein, e.g., comprising a glycan composition described herein, modulates (e.g. increases or decreases) the growth of one or more bacterial taxa predominantly residing in the descending colon, such as, e.g. Bacteroides, Clostridia, Mollicutes, Fusobacteria, Delta/Epsilon Proteobacteria and Verrucomicrobia.

In some embodiments, a composition described herein, e.g., comprising a glycan composition described herein, modulates (e.g., increases or decreases) the growth of one or more bacterial taxa predominantly residing in the sigmoid colon, such as, e.g. Actinobacteria, Bacteroides, Bacilli, Clostridia, Mollicutes, Alpha Proteobacteria, Beta Proteobacteria, and Verrucomicrobia.

In some embodiments, a composition described herein, e.g., comprising a glycan composition described herein, modulates (e.g. increases or decreases) the growth of one or more bacterial taxa predominantly residing in the rectum, such as, e.g. Bacteroides, Clostridia, Mollicutes, Alpha Proteobacteria, Gamma Proteobacteria, and Verrucomicrobia.

In some embodiments, a composition described herein, e.g., comprising a glycan composition described herein, modulates (e.g., stimulate/increase or suppress/decrease) the growth of one or more (e.g. 1, 2, 3, 4, 5, 6, 7, 8, 9, 10, or more) bacterial taxa of genera including, e.g. Alistipes, Akkermansia, Anaerofilum, Bacteroides, Blautia, Bifidobacterium, Butyrivibrio, Clostridium, Coprococcus , Dialister, Dorea, Fusobacterium, Eubacterium, Faecalibacterium, Lachnospira, Lactobacillus,, Odoribacter, Oscillospira, Parabacteroides, Phascolarctobacterium, Peptococcus, Peptostreptococcus, Prevotella, Roseburia, Ruminococcus, and Streptococcus and Subdoligranulum and of the species Akkermansia municiphilia, Christensenella minuta, Clostridium coccoides, Clostridium leptum, Clostridium scindens, Dialister invisus, Eubacterium rectal, Eubacterium eligens, Faecalibacterium prausnitzii, Streptococcus salivarius, and Streptococcus thermophilus.

In some embodiments, a composition described herein, e.g., comprising a glycan composition described herein, modulates (e.g. substantially increase or substantially decrease) the growth (and the total number) of (or substantially increase or substantially decrease the relative representation/abundance in the total (gastrointestinal) community) of one or more of (e.g. 1, 2, 3, 4, 5, 6, 7, 8, 9, 10, or more) bacterial taxa listed in Tables 6-8.

In some embodiments, a composition described herein, e.g., comprising a glycan composition described herein, substantially increases the growth, e.g. the total number or the relative representation/abundance in the total (gastrointestinal) community) of one or more of (e.g. 1, 2, 3, 4, 5, 6, 7, 8, 9, 10, or more) bacterial taxa listed in Tables 6-8.

In some embodiments, a composition described herein, e.g., comprising a glycan composition described herein, substantially decreases the growth, e.g. the total number or the relative representation/abundance in the total (gastrointestinal) community) of one or more of (e.g. 1, 2, 3, 4, 5, 6, 7, 8, 9, 10, or more) bacterial taxa listed in Tables 6-8.

In some embodiments, a composition described herein, e.g., comprising a glycan composition described herein, substantially increases and decreases the growth, e.g. the total number or the relative representation/abundance in the total (gastrointestinal) community) of one or more of (e.g. 1, 2, 3, 4, 5, 6, 7, 8, 9, 10, or more) bacterial taxa listed in Tables 6-8.

In certain embodiments, the ratio of certain bacterial taxa or their relative abundance may be shifted. Such shifts may be measured with respect to the ratio present in the subject prior to administration of the pharmaceutical glycan composition, or to a control group not taking the pharmaceutical glycan composition.

In some embodiments, a glycan preparation that is capable of reducing the adverse effects of a pathogen may be identified using a high-throughput screening effort of a library of glycan preparations. In some embodiments, a high-throughput screen may comprise i) spiking, or adding to a bacterial fecal community apathogen and propagating the community anaerobically; optionally, ii) contacting the fecal community with a test glycan composition; iii) measuring bacterial growth (e.g., by optical density, e.g., OD600); iv) transferring the community into aerobic conditions; v) measuring pathogen growth, e.g., time period to reach mid-log phase; vi) selecting a glycan composition based on high anaerobic growth (e.g., representative of growth of commensal bacteria (which are substantially anaerobes), e.g., measured by optical density, e.g., OD600) and/or slow aerobic growth (representative of growth of pathogenic bacteria (which grow under aerobic conditions)), e.g., measuring the time interval to mid-log growth phase; and/or subjecting a sample of the community to 16S sequencing before and after addition of the glycan composition for a period of time to determine relative abundance of pathogen and commensal bacteria in the community.

### Proteomic Analysis of Microbial Populations

Preparations of glycan compositions may be selected based on their ability to modulate the expression of microbial proteins, e.g., enzymes, associated with the processing of exogenous substances. Suitable methods for proteomic analysis of microbial populations can be found in WO 2016/122889 and WO 2016/172657, which are hereby incorporated by reference. In some embodiments, proteomic analysis can be performed following protocols described in e.g., Cordwell, Exploring and exploiting bacterial proteomes, Methods in Molecular Biology, 2004, 266:115.

### Identification of microbial (e.g. bacterial) constituents

Microbial modulation (e.g., of representation/abundance of a taxa) by the glycan compositions described herein, e.g., occurring in vivo in the GI tract can be analyzed using any number of methods known in the art and described herein. Suitable methods can be found in WO 2016/122889, WO 2016/172657, and WO 2016/172658, which are hereby incorporated by reference. In some embodiments, quantitative PCR (qPCR) can be used as a method to determine whether a glycan composition can result in a shift of the population of bacteria in the GI tract. In some embodiments, microbial constituents can be identified by characterizing the DNA sequence of microbial 16S small subunit ribosomal RNA gene (16S rRNA gene). In other embodiments, a microbial composition can be identified by characterizing nucleotide markers or genes, in particular highly conserved genes (e.g., "house-keeping" genes), or a combination thereof, or whole genome shotgun sequence (WGS).

### Administration to a subject

The glycan compositions, pharmaceutical compositions and therapeutic agents described herein can be administered to a subject (e.g., facility participant) in need thereof by any appropriate means. In some embodiments, the glycan composition is administered enterically. This includes oral administration, or by an oral or nasal tube (including nasogastric, nasojejunal, oral gastric, or oral jejunal). In other embodiments, administration includes rectal administration (including enema, suppository, or colonoscopy). Methods of administering to a subject suitable for use with methods and compositions described herein can be found in WO 2016/122889, WO 2016/172657, and WO 2016/172658, which in their entirety, are hereby incorporated by reference.

Active compounds and pharmaceutical agents, e.g., prebiotic substances, probiotic bacteria or drugs, may be administered separately, e.g., prior to, concurrent with or after administration of the glycan compositions and not as a part of the pharmaceutical composition or medical food or dietary supplement (e.g. as a co-formulation) of glycan compositions. In some embodiments, pharmaceutical compositions or medical foods comprising preparations of glycan compositions are administered in combination with a recommended or prescribed diet, e.g. a diet that is rich in probiotic and/or prebiotic-containing foods, such as it may be determined by a physician or other healthcare professional.

### Definitions

"Abundance" of a microbial taxa as used herein is a relative term and refers to the relative presence of a microbial taxa to other taxa in a community in a defined microbial niche, such as the GI tract, or in the entire host organism (e.g., a human or a laboratory animal model of disease).

"Acquire" or "acquiring" as the terms are used herein, refer to obtaining possession of a value, e.g., a numerical value, or image, or a physical entity (e.g., a sample), by "directly acquiring" or "indirectly acquiring" the value or physical entity. "Directly acquiring" means performing a process (e.g., performing a synthetic or analytical method or protocol) to obtain the value or physical entity. "Indirectly acquiring" refers to receiving the value or physical entity from another party or source (e.g., a third-party laboratory that directly acquired the physical entity or value). Directly acquiring a value or physical entity includes performing a process that includes a physical change in a physical substance or the use of a machine or device. Examples of directly acquiring a value include obtaining a sample from a human subject (e.g., facility participant). Directly acquiring a value includes performing a process that uses a machine or device, e.g., an NMR spectrometer to obtain an NMR spectrum.

As used herein, "digestibility" is a value for the caloric value of a glycan or glycan preparation, e.g., in the gastrointestinal tract of a subject. Glycan preparations can have varied caloric value, depending on how well, if at all, a host, e.g., host enzyme, can digest it. For example, glycan polymer preparations that are indigestible by a host (e.g., mammal, e.g., human, enzyme) contain minimal caloric value (e.g., have no caloric value and are non-caloric). Caloric value as used herein, does not refer to the caloric value determined in a bomb calorimeter or similar device but to the caloric value usable by the subject. In some examples, glycan preparations that are indigestible are not absorbed and thus not assimilated or utilized for energy in the human body. Caloric value refers to usable caloric value, e.g., calories that are assimilated or utilized for energy in the human body. Digestibility may be measured as described in McCleary (AOAC Method 2009.01, also referred to as AACC International Approved Method 32-45.01) (McCleary et al. (2010) J. AOAC Int., 93(1), 221-233), e.g., using pancreatic α-amylase and conditions close to physiological (pH 6, 37°C) for the enzymatic incubation step. In other embodiments, digestibility can be measured as described in McCleary et al., (2012) J. AOAC Int., 95 (3), 824-844, e.g., using AOAC 201 1 .25 (Integrated Total Dietary Fiber Assay).

"Distinct" as used herein, e.g. with reference to a species in a glycan, is meant to denote that it is chemically and/or structurally different from another. For example, two sugars are "distinct" if they are chemically different, e.g. a fucose and a xylose, or structurally different, e.g. cyclic vs. acyclic, L- vs. D-form. Two dimers are distinct if they consist of the same two monomers but one pair contains alpha-1,4 bond and the other contains a beta-1,6 bond. Distinct entities may have any other suitable distinguishing characteristic or property that can be detected by methods known in the art and/or described herein.

As used herein, a "dosage regimen", "dosing regimen", or "treatment regimen" is a modality of drug administration that achieves a therapeutic objective. A dosage regimen includes definition of one, two, three, or four of: a route of administration, a unit dose, a frequency of dosage, and a length of treatment.

"Dysbiosis" refers to an imbalanced state of the microbiota, e.g., within the GI tract, in which the normal diversity, proportion of a first bacterial taxa to a second bacterial taxa and/or function (e.g. the production of a metabolite) of the ecological network is disrupted or disturbed. This undesired, e.g., unhealthy, state can be due to a number of factors including, but not limited to, a decrease or increase in the diversity of the microbiota (e.g. bacterial taxa), the overgrowth of one or more pathogens or pathobionts, or the shift to an ecological microbial community that no longer provides an essential function to the host subject, and, in an embodiment, therefore no longer promotes health or, which is associated with unwanted symptoms in the subject. In one embodiment, the production of a metabolite is modulated so as to contribute to the development of a disease or disorder.

By the terms "effective amount" and "therapeutically effective amount" of a composition (such as, e.g., a pharmaceutical composition) or a drug agent is meant a sufficient amount of the composition or agent to provide the desired effect. In some embodiments, a physician or other health professional decides the appropriate amount and dosage regimen. An effective amount also refers to an amount of a composition (such as, e.g., a pharmaceutical composition) or a drug agent that prevents the development or relapse of a medical condition.

"Facility" as used herein, refers to any type of location where subjects spend time for rest, recreation, education, correction, rehabilitation, or medical treatment. Types of facilities include hospitals, doctor's offices, clinics, medical center (e.g., urgent care clinic, hospital emergency room), nursing homes, or other rehabilitative, restorative, and/or skilled medical/nursing care to patients or residents; correctional facilities (e.g., prisons, jails, detention centers), long term care facilities, rehabilitation centers, urgent care clinics, ambulatory surgical center, doctor's offices, medical centers, day care, pre-schools, elementary schools, middle schools, high schools, private schools, preparatory schools, colleges, universities, community colleges, vocational schools, camps, health clubs (e.g., gyms), parks, amusement parks, theme parks, theatres, arenas, stadiums, sports entertainment complexes, concert halls, auditoriums, shopping centers (e.g., malls, strip malls, plazas), military bases, military vessels, resorts, cruise ships, hostels, bed-and-breakfasts, motels, and hotels.

As used herein, "fermentability" is a value for how readily a glycan or glycan polymer preparation can be used for fermentation by a microbe, microbial community, or microbiome (e.g., in the gastroinstestinal tract of a subject). In some examples, "non-fermentable" refers to glycan polymer preparations that have a relatively low fermentability, e.g., of less than 40% by weight, e.g., less than 40%, 35%, 30%, 20%, 15%, or less (by weight). In some examples, "fermentable" refers to glycan polymer preparations which have a relatively high fermentability, e.g., at least 60% by weight, e.g., at least 60%, 65%, 70%), 75%, 80%, 85% by weight, or higher. Fermentability can be determined by methods described in "Fermentability of Various Fiber Sources by Human Fecal Bacteria In Vitro", at AMERICAN JOURNAL CLINICAL NUTRITION, 1991, 53 1418-1424; or US Patent 5,085,883, both of which are incorporated herein by reference.

"Microbial engraftment" or simply "engraftment" refers to the establishment (e.g. growth) of microbial taxa in a target niche (e.g. the human gut, such as the colon or intestines) that are either underrepresented (e.g. relative to a healthy reference subject) or absent (e.g. undetectable) in a human subject prior to engraftment (e.g. by administering the microbial taxa to the subject, e.g. in form of a synbiotic described herein). Engrafted microbial taxa can establish for a transient period, or demonstrate long-term stability in the microbiota that populates the subject post engraftment of the microbial taxa. In some embodiments, the engrafted microbial taxa can induce an environmental shift in the target niche representing a shift from dysbiosis to a health state.

As used herein, a "glycan preparation" (also referred to as a "preparation of glycans", "glycan preparation", "glycan polymer preparation", "glycan polymer composition", "glycan composition", "oligosaccharide preparation", "oligosaccharide composition" or "glycan") is a preparation comprising glycans that exhibits a desired effect (e.g. a therapeutic effect). In some embodiments, preparations of glycans do not contain one or more naturally occurring oligosaccharide, including: glucooligosaccharide, mannanoligosaccharide, inulin, lychnose, maltotretraose, nigerotetraose, nystose, sesemose, stachyose, isomaltotriose, nigerotriose, maltotriose, melezitose, maltotriulose, raffinose, kestose, fructooligosaccharide, 2'-fucosyllactose, galactooligosaccharide, glycosyl, idraparinux, isomaltooligosaccharide, maltodextrin, xylooligosaccharide, agar, agarose, alginic acid, alguronic acid, alpha glucan, amylopectin, amylose, arabioxylan, beta-glucan, callose, capsulan, carrageenan, cellodextrin, cellulin, cellulose, chitin, chitin nanofibril, chitin-glucan complex, chitosan, chrysolaminarin, curdlan, cyclodextrin, alpha-cylcodextrin, dextran, dextrin, dialdehyde starch, ficoll, fructan, fucoidan, galacloglucomannan, galactomannan, galactosamineogalactan, gellan gum, glucan, glucomannan, glucoronoxyland, glycocalyx, glycogen, hemicellulose, hypromellose, icodextrin, kefiran, laminarin, lentinan, levan polysaccharide, lichenin, mannan, mucilage, natural gum, paramylon, pectic acid, pectin, pentastarch, phytoglycogen, pleuran, poligeenan, polydextrose, porphyran, pullulan, schizophyllan, sepharose, sinistrin, sizofiran, sugammadex, welan gum, xantham gum, xylan, xyloglucan, zymosan, and the like. In some embodiments, a glycan exists as a salt, e.g., a pharmaceutically acceptable salt.

A "glycan unit" as used herein refers to the individual unit of a glycan disclosed herein, e.g., the building blocks from which the glycan is made. In an embodiment, a glycan subunit is a monomer. In an embodiment, a glycan subunit is a dimer. In an embodiment, a glycan subunit is a monosaccharide. In an embodiment, a glycan subunit is a disaccharide. In some embodiments, the glycan subunit is a carbohydrate and may be selected from a sugar alcohol, a short-chain fatty acid, a sugar acid, an imino sugar, a deoxy sugar, and an amino sugar. In some embodiments, the glycan subunit is erythrose, threose, erythulose, arabinose, lyxose, ribose, xylose, ribulose, xylulose, allose, altrose, galactose, glucose, gulose, idose, mannose, talose, fructose, psicose, sorbose, tagatose, fucose, fuculose, rhamnose, mannoheptulose, sedoheptulose, and the like. In some embodiments, the glycan subunit is glucose, galactose, arabinose, mannose, fructose, xylose, fucose, or rhamnose. In embodiments, a glycan comprises distinct glycan subunits, e.g., a first and a second monosaccharide, or a first and a second disaccharide. In embodiments, a glycan comprises distinct glycan subunits, e.g., a first, a second, a third, a fourth, and/or a fifth distinct glycan subunit.

As used herein, the term "hepatic encephalopathy (HE)" refers to a class of disease that includes multiple adverse neurological symptoms which occur as a results of advanced liver disease or otherwise when the liver is unable to remove toxic substances such as ammonia from the blood. In some embodiments, HE arises as a consequence of or in conjunction with liver cirrhosis. In some embodiments, a subject having hepatic encephalopathy has elevated levels of ammonia relative to a normal, healthy subject. In some embodiments, a subject having hepatic encephalopathy may have mild HE, severe HE, or overt HE. Standard-of-care treatments for HE include lactulose, lactitol, and antibiotics (e.g., rifaximin or neomycin). Treatments may also include dietary modifications and probiotics. Treatment efficacy may be assessed by resolution of the symptoms above (e.g., reduction in serum ammonia levels), decreased incidence of future episodes of HE, or, in subjects at risk of HE, by decreased occurrence of an initial episode of HE. In some embodiments, patients who have experienced an extended duration of hyperammonemia or high peak ammonia, particularly patients with liver damage (e.g., patients with liver cirrhosis, e.g., elderly patients), may develop hepatic encephalopathy (HE) associated with profound and chronic neurologic morbidity, including severe intellectual disability, deficits in executive function impacting daily activities, e.g., such as, confusion, forgetfulness, anxiety or excitation, sudden changes in personality or behavior, changes in sleep patterns, disorientation, sweet or musty smelling breath, slurred speech, and/or difficulty controlling motor functions.

As used herein, the term "hyperammonemia" refers to a condition in a subject (e.g., a human subject) associated with elevated levels of ammonia. Generally, a subject having hyperammonemia has elevated levels of ammonia in circulation, e.g., in blood. In some embodiments, the duration and severity of hyperammonemia is positively correlated with brain damage. In some embodiments, patients who have experienced an extended duration of hyperammonemia or high peak ammonia, particularly pediatric patients, may develop profound and chronic neurologic morbidity, including developmental delay, severe intellectual disability, deficits in executive function impacting daily activities, cerebral palsy, and seizure disorder.

As used herein, an "isolated" or "purified" glycan preparation is substantially pure and free of contaminants, e.g. pathogens, enzymes or otherwise unwanted biological material, or toxic or otherwise unwanted organic or inorganic compounds. In some embodiments, pure or isolated compounds, compositions or preparations may contain traces of solvents and/or salts (such as less than 10%, 9%, 8%, 7%, 6%, 5%, 4%, 3%, 2%, 1%, less than 0.5% or 0.1% by w/w, w/v, v/v or molar %). Purified compounds or preparations contain at least about 60% (by w/w, w/v, v/v or molar %), at least about 75%, at least about 90%, at least about 95%, at least about 97%, at least about 98%, or at least about 99% by w/w, w/v, v/v or molar % the compound(s) of interest. For example, a purified (substantially pure) or isolated preparation of glycans is one that is at least 80%, 85%, 90%, 91%, 92%, 93%, 94%, 95%, 98%, 99%, 99.5%, 99.8%, 99.9% or 100% of the glycan by w/w, w/v, v/v or molar % (e.g., not including any solvent, such as e.g. water, in which the glycan preparation may be dissolved) and separated from the components that accompany it, e.g. during manufacture, extraction/purification and/or processing (e.g. such that the glycan is substantially free from undesired compounds). Purity may be measured by any appropriate standard method, for example, by column chromatography (e.g., size-exclusion chromatography (SEC)), thin layer chromatography (TLC), gas chromatography (GC), high-performance liquid chromatography (HPLC) or nuclear magnetic resonance (NMR) spectroscopy. Purified or purity may also define a degree of sterility that is safe for administration to a human subject, e.g., lacking viable infectious or toxic agents.

"Microbiome" as used herein refers to the genetic content of the communities of microbes ("microbiota") that live in and on a subject (e.g. a human subject), both sustainably and transiently, including eukaryotes, archaea, bacteria, and viruses (including bacterial viruses (e.g., phage)), wherein "genetic content" includes genomic DNA, RNA such as ribosomal RNA and messenger RNA, the epigenome, plasmids, and all other types of genetic information, In some embodiments, microbiome specifically refers to genetic content of the communities of microorganisms in a niche.

"Microbiota" as used herein refers to the community of microorganisms that occur (sustainably or transiently) in and on a subject (e.g. a human subject), including eukaryotes, archaea, bacteria, and viruses (including bacterial viruses, e.g. phage). In some embodiments, microbiota specifically refers to the microbial community in a niche.

A "participant" in a facility refers to members of, residents at, workers, patients at, visitors at, or staff members of a facility. Types of participants include but are not limited to a patient (e.g., long term care facility resident, out-patient); a medical practitioner (e.g., a physician, nurse, or physician assistant); a technician or technologist; a housekeeping worker; a security worker; a maintenance worker; a food preparation worker; a laundry worker; an administrative worker; a social worker; a visitor; and/or a facility employee. Patients may include long term care facility residents; patients frequently receiving antibiotics; patients with recurrent urinary tract infections; catheterized patients; patients with urinary tract hardware (e.g., Foley catheters, stents); immunosuppressed patients (e.g., patients with HIV/AIDS); patients receiving chemotherapy (e.g., cytotoxic chemotherapy and/or hematopoietic stem cell transplant); patients receiving solid organ transplants; patients with end stage organ failure; congenital immunosuppressed patients (e.g., patients with SCID, CVID, Bruton's agammaglobulinemia); cystic fibrosis patients; and patients with chronic conditions requiring frequent hospitalization.

As used herein, a "pharmaceutical composition" is a composition or preparation, having pharmacological activity or other direct effect in the mitigation, treatment, or prevention of disease, and/or a finished dosage form or formulation thereof and is for human use. A pharmaceutical composition is typically produced under good manufacturing practices (GMP) conditions. Pharmaceutical compositions may be sterile or non-sterile. If non-sterile, such pharmaceutical compositions typically meet the microbiological specifications and criteria for non-sterile pharmaceutical products as described in the U.S. Pharmacopeia (USP) or European Pharmacopoeia (EP). Pharmaceutical compositions may further comprise or may be co-administered with additional active agents, such as, e.g. additional therapeutic agents. Pharmaceutical compositions may also comprise e.g. additional therapeutic agents, polyphenols, prebiotic substances, probiotic bacteria, pharmaceutically acceptable excipients, solvents, carriers or any combination thereof. Any glycan preparation described herein may be formulated as a pharmaceutical composition.

The term "subject" as used herein refers to any human subject. In some embodiments, the subject is a facility participant. The term does not denote any particular age or gender. Subjects may include pregnant women. Subjects may include a newborn (a preterm newborn, a full-term newborn), an infant up to one year of age, young children (e.g., 1 year to 12 years), teenagers, (e.g., 13-19 years), adults (e.g., 20-64 years), and elderly adults (65 years and older).

A "substantial decrease" as used herein (e.g. with respect to a biomarker or metabolite) is a decrease of 5%, 10%, 20%, 30%, 40%, 50%, 60%, 70%, 80%, 90%, 95%, 97%, 98%, 99%, 99.9% or 100%.

A "substantial increase" as used herein (e.g. with respect to a biomarker or metabolite) is an increase of 10%, 20%, 30%, 40%, 50%, 60%, 70%, 80%, 90%, 100%, 150%, 200%, 250%, 300%, 350%, 400%, 450%, 500%, 550%, 600%, 650%, 700%, 750%, 800%, 850%, 900%, 950%, 1000%, or more than 1000%.

"Synthetic" as used herein refers to a man-made compound or preparation, such as a glycan preparation, that is not naturally occurring. In one embodiment, a catalyst (e.g. a non-enzymatic catalyst (e.g., a polymeric or solid acid catalyst) or an enzyme catalyst (e.g., a glycosidase or glycosyltransferase) is used to synthesize the glycans of the preparation under suitable reaction conditions, e.g. by a polymerization reaction that creates oligomers from individual glycan subunits that are added to the reaction. In some embodiments, the catalyst acts as a hydrolysis agent and can break glycosidic bonds. In other embodiments, the catalyst can form glycosidic bonds. In one embodiment, solid-phase oligosaccharide synthesis is used to synthesize the glycans of the preparation under suitable reaction conditions, e.g. by a polymerization reaction that creates oligomers from individual glycan subunits that are added to the reaction. Synthetic glycan preparations may also include glycans that are not isolated from a natural oligo- or polysaccharide source. It is to be understood that while the glycan preparation is not isolated from a natural oligo- or polysaccharide source, the glycan subunits making up the glycan can be and often are isolated from natural oligo- or polysaccharide sources, including those listed herein, or are synthesized de novo.

The terms "treating" and "treatment" as used herein refer to the administration of an agent or composition to a subject (e.g., a symptomatic subject afflicted with an adverse condition, disorder, or disease) so as to affect a reduction in severity and/or frequency of a symptom, preventing or reducing the risk of (having) an adverse event, eliminate a symptom and/or its underlying cause, and/or facilitate improvement or remediation of damage, and/or preventing an adverse condition, disorder, or disease in an asymptomatic subject who is susceptible to a particular adverse condition, disorder, or disease, or who is suspected of developing or at risk of developing the condition, disorder, or disease.

As used herein, "pathogen load" refers to the amount, in relative or absolute terms, of a pathogen associated with a facility participant, in colony forming units per mass, or per volume, or per surface area, or in relative composition, for instance making up 0.1% of the bacteria within the gut. Pathogen load is synonymous with pathogen biomass.
As used herein, "antibiotic resistance gene load" refers to the quantity of resistance determinants, in either relative terms compared to the total genetic material from microbes, or in absolute terms, in terms of copies of the resistance determinant per mass, per volume, per surface area, or per person.

As used herein, "pathogen reservoir" refers to a quantity of pathogen, in a specific site or sites within or associated with a healthcare facility, or a facility participant, that harbors a pathogen in the absence of clinically noticeable symptoms of infection.
As used herein, "fitness deficit" refers to the relative disadvantage of a population of microbes such that they are not able to maintain numbers as high as another population, or are unable to persist over time.

As used herein, "addiction module" refers to a mechanism by which a transmitted genetic element such as a plasmid or transposon can ensure that it is copied and transmitted to subsequent generations of daughter cells. These involve mechanisms such as toxin-antitoxin systems, whereby the genetic element encodes a long-lived toxin, and short-lived antitoxin. When the genetic material is present, continued production of the antitoxin ensures the continued ability of the organism to grow, but upon loss of the genetic material, the antitoxin is lost, and the toxin halts growth or kills the resulting cell. On a population level this ensures that only cells maintaining the genetic material are able to persist.

As used herein, "resistance determinant" refers to the gene, mutation, or group of genes or genetic material necessary to confer resistance to a given antibiotic.

As used herein, "pathogenic potential" refers to the potential of a particular pathogen to cause symptoms of disease in a given group of facility participants.

As used herein, "pathogenic infectivity" refers to the likelihood that a given pathogen, with its own pathogenic potential, will cause a symptomatic infection or disease in a given facility participant. The severity of a pathogen infection is measured in many ways, such as by clinical examination, including physical examination, vital signs, laboratory values, length of hospital stay, organ systems involved, necessary maneuvers or procedures to treat, potential or actual sequelae, illness progression, length of hospital stay, and need for critical care or rehabilitation.

All publications, patents, and patent applications cited or referenced in this specification are herein incorporated by reference to the same extent as if each independent publication or patent publication was specifically and individually indicated to be incorporated by reference.

### EXAMPLES

The invention is further illustrated by the following examples. The examples are provided for illustrative purposes only, and are not to be construed as limiting the scope or content of the invention in any way. The practice of the present invention will employ, unless otherwise indicated, conventional methods of protein chemistry, biochemistry, recombinant DNA techniques and pharmacology, within the skill of the art. Such techniques are explained fully in the literature. See, e.g., T.E. Creighton, Proteins: Structures and Molecular Properties (W.H. Freeman and Company, 1993); Green & Sambrook et al., Molecular Cloning: A Laboratory Manual, 4th Edition (Cold Spring Harbor Laboratory Press, 2012); Colowick & Kaplan, Methods In Enzymology (Academic Press); Remington: The Science and Practice of Pharmacy, 22nd Edition (Pharmaceutical Press, 2012); Sundberg & Carey, Advanced Organic Chemistry: Parts A and B, 5th Edition (Springer, 2007).

### Example 1. Glycan Preparations

To a round bottom flask equipped with an overhead stirrer and a jacketed short-path condenser was added one or more mono- or disaccharides along with 3-20% by dry weight of one or more of the catalysts e.g. acid, ionic, ionic/acid containing catalysts such as, e.g. described in U.S. patent No. 9,079,171 and WO 201.61007778, which are incorporated herein by reference in their entirety. Water or another compatible solvent (zero to 10 equiv.) was added to the dry mixture and the slurry was combined at approximately 100 rpm using a paddle sized to match the contours of the selected round bottom flask as closely as possible. The mixture was then heated to 80-185° C. Once the solids achieved a molten state, the vessel was placed under 10-1000 mbar vacuum pressure. The reaction was stirred for 30 minutes to 8 hours, constantly removing water from the reaction. Reaction progress was monitored by HPLC. When sufficient oligomerization had occurred, the stirrer was shut off, the reaction was cooled to room temperature and vented to atmospheric pressure, and the product, either as a solid or syrup, was dissolved in a volume of water sufficient to create a solution of approximately 50 Brix (grams sugar per 100 g solution). Once dissolution was complete, solid catalyst was removed by filtration and the oligomer solution was concentrated to approximately 50-75 Brix by rotary evaporation. In cases in which an organic solvent has been used, water immiscible solvents can be removed by biphasic extraction and water miscible solvents can be removed by rotary evaporation concomitant to the concentration step.

Among others, the following glycans were made in multiple batches and tested in various assays described herein:
Single glycan unit (homo-glycans): ara100, fru100, gal100, galA100, glcNac100, glu100, gluA100, Lglu100, man100, rha100, xyl100.

Two glycan units (hetero-glycans): Ara60Xyl40, Ara80Xyl20, Gal20Ara80, Gal20Xyl80, Gal40Ara60, Gal40Man60, Gal40Xyl60, Gal57Glu43, Gal60Ara40, Gal60Man40, Gal60Xyl40, Gal80Ara20, Gal80Man20, Gal80Xyl20, Glu20Ara80, Glu20Xyl80, Glu40Ara60, Glu40Gl60, Glu40Xyl60, Glu50Gal50, Glu50Lglu50, Glu60Ara40, Glu60Gal20Man20, Glu60Gal40, Glu60Man40, Glu60Xyl40, Glu66Fru33, Glu75Gala25, Glu75GluA25, Glu75GluN25, Glu80Ara20, Glu80Gal20, Glu80Lglu20, Glu80Man20, Glu80Xyl20, Glu90LGlu10, Man20Ara80, Man20Xyl80, Man40Ara60, Man40Xyl60, Man60Ara40, Man60Glu40, Man60Xyl40, Man75Gal25, Man80Ara20, Man80Gal20, Man80Glu21, Man80Xyl20, Xyl60Ara40, Xyl75Ara25, Xyl80Ara20, and the hybrid glycans glu90sor10 and glu90gly10.

Three glycan units (hetero-glycans): Ga15Xyl5Ara90, Gal5Xyl90Ara5, Gal10Xyl110Ara8O, Gal10Xyl45Ara45, Gal10Xyl80Ara10, Gal20Xyl20Ara60, Ga120Xyl40Ara40. Gal20Xyl60Ara20, Gal30Xyl30Ara40, Gal30Xyl40Ara30, Gal33Man33Ara33, Gal33Man33Xyl33, Gal33Xyl33Ara33, Gal45Xyl10Ara45, Gal45Xyl45Ara10, Gal50Glu25Fru25, Gal40Xyl20Ara40, Gal40Xyl30Ara30, Gal40Xyl40Ara20, Gal60Xyl20Ara20, Gal80Xyl10Ara10, Gal90Xyl5Ara5, Glu5Gal5Man90, Glu5Gal90Man5, Glu5Xy15Ara90, Glu5Xyl90Ara5, Glu10Gal10Man80, Glu10Gal45Man45, Glu10Gal80Man10, Glu10Xyl10Ara80, Glu10Xyl45Ara45, Glu10Xyl80Ara10, Glu20Gal20Man60, Glu20Gal40Man40, Glu20Gal60Man20, Glu20Gal80, Glu20Xyl20Ara60, Glu20Xyl40Ara40, Glu20Xyl60Ara20, Glu30Gal30Man40, Glu30Gal40Man30, Glu30Xyl30Ara40, Glu30Xyl40Ara30, Glu33Gal33Ara33, Glu33Gal33Fuc33, Glu33Gal33Man33, Glu33Gal33Xyl33, Glu33Man33Ara33, Glu33Man33Xyl33, Glu33Xyl33Ara33, Glu40Gal20Man40, Glu40Gal30Man30, Glu40Gal40Man20, Glu40Xyl20Ara40, Glu40Xyl30Ara30, Glu40Xy140Ara20, Glu45Gal10Man45, Glu45Gal45Man10, Glu45Xyl10Ara45, Glu45Xyl45Ara10, Glu60Xyl20Ara20, Glu75GluNAc2S, Glu80Gal10Man10, Glu80Xyl10Ara10, Glu90Gal5Man5, Glu90Xyl5Ara5, Man33Xyl33Ara33, Man52Glu29Gal19.

Four glycan units (hetero-glycans): Gal25Man25Xyl25Ara25, Glu25Gal25Man25Ara25, Glu25Gal25Man25Xyl25, Glu25Gal25Xyl25Ara25, Glu25Man25Xy125Ara25.

Five glycan units (hetero-glycans): Glu20Gal20Man20Xyl20Ara20.

Glycans are described by a three- to six-letter code representing the monomeric sugar component followed by a number out of one hundred reflecting the percentage of the material that monomer constitutes. Thus, 'glu100' is ascribed to a glycan generated from a 100% D-glucose (glycan unit) input and 'glu50gal50' is ascribed to a glycan generated from 50% D-glucose and 50% D-galactose (glycan units) input or, alternatively from a lactose dimer (glycan unit) input. As used herein: xyl = D-xylose; ara = L-arabinose; gal = D-galactose; glu = D-glucose; rha = L-rhamnose; fuc = L-fucose; man = D-mannose; sor = D-sorbitol; gly = D-glycerol; neu = NAc-neuraminic acid; Lglu = L-glucose; gluA = D-glucuronic acid; gluN = D-glucosamine; gluNAc = N-acetyl-D-glucosamine; galA = D-galacturonic acid.

### Example 2. Purification

Oligo- and polysaccharides were dissolved in deionized water to a final concentration of 25-50 Brix. The material was then exposed to at least 2 mass equivalents of Dowex Monosphere 88 ion exchange resin. Exposure may occur by swirling in a flask at 120-170 rpm or by filtration through a wet slurry packed column as long as the residence time is sufficient for the solution to achieve a final pH between 3 and 5. The oligomer solution was isolated by filtration (as in the case of swirled reactions) or elution (as in the case of column filtration) and the process was repeated with Dowex Monosphere 77 ion exchange resin in an analogous fashion until the solution pH was above 5.5. Finally, the solution was exposed to Dowex Optipore SD-2 Adsorbent decolorizing resin until the solution was sufficiently clarified and filtered through a 0.2 micron filter to remove residual resin and resin fines. The final solution was then concentrated to 50-85 Brix by rotary evaporation or to a solid by lyophilization.

### Example 3. High-throughput preparation at small scale

The oligomers and polymers were synthesized in a parallel fashion in 24-, 48-, or 96-well plates or similarly sized arrays of 1 dram vials housed in aluminum heating blocks. In this example, all liquid transfers were handled by a programmable robot or manually using calibrated pipettes. To each vial or well was added 20-100% by dry weight of one or more catalysts e.g. acid, ionic, ionic/acid containing catalysts such as, e.g. described in U.S. patent No. 9,079,171 and WO 2016/007778. The plate or heating block was placed uncovered in a vacuum oven heated to 50 to 150 °C under a vacuum of 10-800 mbar. The oven vacuum pump was protected by a two-stage condenser consisting of a recirculating chiller trap followed by a dry ice/acetone trap. The plates or blocks are heated for 30 minutes to 6 hours under elevated temperature and reduced pressure without stirring. After a pre-established period of time, the oven was vented to atmospheric pressure, the plates or blocks were cooled to room temperature, and each well or vial was diluted to approximately 50 Brix with deionized water. The solid-phase extraction steps described in Example 2 were performed by elution through sequential wet-packed columns in which the eluent from each column flows immediately into the top of the next column at a rate between 2 and 6 bed volumes/hour using a peristaltic pump or other suitable small pump. The column stack was then rinsed with deionized water and the combined effluents are concentrated by lyophilization to isolate solid powders with residual water content of 1-10% by mass.

### Example 4. Removal of low molecular weight species

Oligomers or polymers were modified so as to remove low molecular weight species.

In one embodiment the separation was achieved by osmotic separation. Approximately 45 cm of 1.0 kD MWCO Biotech CE dialysis tubing (31 mm flat width) from Spectrum Labs was placed into deionized water and soaked for 10 minutes, then one end was sealed with a dialysis tubing clip. A 25 Brix solution of 8 grams dry oligosaccharide was sterile filtered and sealed into the tube with a second clip along with a few mL of air to permit the tube to float. The filled tube was then placed in a 3 gallon tank of deionized water which was stirred with sufficient force to induce slow swirling of the sealed tubes. After 8 hours, the water in the tank was replaced and the tube was allowed to stir for an additional 16 hours. Once the dialysis was complete and the material had a DP2+ yield greater than 95% and a DP3+ yield greater than 90%, the dilute solution was sterile filtered and concentrated in vacuo to a final concentration of approximately 65 Brix or lyophilized to a solid with a residual moisture between 1 and 10%.

In a second embodiment the separation was achieved by tangential flow filtration (TFF). In this case, 100 mL of 25 Brix glycan sample dissolved in deionized water and sterile filtered was placed into the feed bottle of a Spectrum Labs KrosFlo Research IIi TFF system that was prepared according to the manufacturer's recommendation. The sample was then diafiltered through a 1kD mPES MidiKros hollow-fiber filter at a transmembrane pressure of 25 psig. HPLC samples of the feed stock taken every 0.5 diafiltration volumes were used to determine when the material had a DP2+ yield greater than 95% and a DP3+ yield greater than 90% at which point the solution was sterile filtered and concentrated *in vacuo* to a 65 Brix syrup or lyophilized to a solid with residual water content of 1-10% by mass.

In a third embodiment the separation was achieved by ethanol precipitation. In this case, 100 mL of 25 Brix glycan sample was poured into a vigorously stirred beaker containing 900 mL of pure, USP-grade ethanol at a rate no higher than 10 mL/minute. Once the addition was complete, the precipitated solids were subjected to stirring for an additional 15 minutes at or slightly below room temperature. The precipitated solids were isolated by filtration through a fine frit sintered glass funnel under an atmosphere of nitrogen to prevent hydration and gumming. The solids were rinsed once with ethanol, then dissolved in water to a final concentration of 25 Brix and reconcentrated to >65 Brix. This syrup was then diluted back to 25 Brix and concentrated once more to ensure removal of residual ethanol.

### Example 5. Methods for analyzing preparations

### Measurement of concentration by liquid refractometry

This experiment was designed to quantitate the amount of glycan in any given aqueous solution. A Mettler-Toledo Refracto 30GS portable sugar refractometer was calibrated using high-purity reverse-osmosis deionized water. Several drops of the glycan solution were filtered through a 0.2 micron syringe filter directly onto the lens of the refractometer. The measurement was taken at room temperature and reported as Brix. The glycans were routinely concentrated to 50, 60, 70, or 75 Brix without obvious solidification or crystallization at 23 °C. Brix can then be converted to solubility assuming a specific density of water equal to 1.0 g/mL. Thus, 75 Brix (100 grams of solution consisting of 75 grams of glycan and 25 grams of water) equals an aqueous solubility of 3.0 g/mL. As a comparison, the aqueous solubility of D-glucose is reported to be 0.909 g/mL (48 Brix) at 25 °C by Sigma-Aldrich.

### Monomeric composition by hydrolysis and GC-MS

This experiment was designed to quantitate the ratio of monomer content within a given oligosaccharide. Glycosyl composition analysis was performed by combined gas chromatography/mass spectrometry (GC/MS) of the per-O-trimethylsilyl (TMS) derivatives of the monosaccharide methyl glycosides produced from the sample by acidic methanolysis as described previously by Santander et al. (2013) Microbiology 159:1471. Between 100 and 200 µg of sample were lyophilized into a suitable test tube. Inositol (20 µg) was added to the sample as an internal standard, then the sample was heated to 80 °C in 1M HCl/methanol for 18 hours. The resulting monosaccharides were then re-acetylated using pyridine and acetic anhydride in MeOH, and per-*O*-trimethylsilylated with Tri-Sil (Pierce) at 80 °C for 30 minutes. GC/MS analysis of the TMS methyl glycosides was performed on an Agilent 7890A GC interfaced to a 5975C MSD, using a Supelco Equity-1 fused silica capillary column (30 m x 0.25 mm ID). Each peak was assigned to a component sugar based upon comparison to known standards and integration of the respective peaks allowed clean calculation of the relative percentage of monomers within an exemplified glycan. In all enumerated glycans, conditions can be routinely identified in which the monomer composition of a given oligosaccharide matched the input ratio within experimental error and the output composition matched the input composition within the precision of the measurement.

### Molecular weight distribution by size-exclusion chromatography (SEC)

This experiment was designed to quantitate the distribution of molecular weights within a given oligosaccharide. The measurement was made by HPLC using the method described in Monograph of United States Pharmacopeia, 38(6) In-Process Revision: Heparin Sodium (USP37-NF32). Separations were achieved on an Agilent 1200 HPLC system via a GE superpose 12 column using 50 mM ammonium acetate as an eluent at 1.0 mL/min flow rate and an ELSD detector. The column temperature was set at 30 °C and dextran (1 kD, 5 kD, 10 kD weight) were used to draw a standard curve. A 2 mg/ml solution of the samples was prepared and passed through a 0.45 µm spin filter, followed by 40 µl injections into the HPLC. A third-order polynomial curve was constructed based on the logarithmic molecular weights and elution volumes of the listed standards. The weight-average molecular weight (Mw), the number average molecular weight (Mn), and the polydispersity index (PDI) for the sample were calculated by comparison to the standard curve. Figure 1 shows the curve generated during the SEC evaluation of a glu100 sample in which the average molecular weight was determined to be 1212 g/mol or approximately DP7. The upper end of molecular weight of the material as defined by the point of the curve at 10% of maximum absorption leading the curve was determined to be 4559 g/mol or approximately DP28. The lower end of molecular weight of the material as defined by 10% of the maximum absorption trailing the curve was determined to be 200 g/mol or approximately DP1. Similar analysis of a glu50gal50 sample showed a MW, high mass, and low mass of 1195 g/mol (∼DP7), 4331 g/mol (∼DP27), and 221 g/mol (∼DP1) respectively.

### Molecular weight distribution by ion-affinity chromatography (IAC)

The proportion of glycan with DP greater than or equal to 2 (DP2+) and 3(DP3+) may be measured by ion-affinity chromatography. A sample of glycan was diluted out to 50-100 mg/mL and 10 µL of this solution was injected onto an Agilent 1260 BioPure HPLC equipped with a 7.8x300 mm BioRad Aminex HPX-42A column and RI detector. Using pure HPLC-grade water as an eluent, the sample was eluted at 0.6 mL/min through an 80 °C column and an RI detector maintained at 50 °C. The peaks representing DP1-6 are assigned by comparison to reference standards and integrated using the Agilent ChemStation software. Peaks are typically integrated as DP1, DP2, DP3, DP4-7, and DP8+. The DP that is achievable by the reaction described in Example 1 varies from monomer to monomer although it is consistent across batches if the procedure is followed. For example, across 17 batches of glu100, DP2+ values ranged from 77-93% and DP3+ values ranged from 80-90%. Conversely, across 6 batches of ara100, DP2+ values ranged from 63-78% and DP3+ values ranged from 48-71 %. Mixtures of monomers behaved as averages of the individual components.

### Alpha-/betatdistribution by 2D NMR

This experiment was designed to quantitate the ratio of alpha- and beta-glycosidic bonds within a given sample by two-dimensional NMR. Approximately 150 mg of 65 Brix oligosaccharide solution was dried to stable mass in a vacuum oven at 45-95 °C under 400 mbar pressure. The sample was subjected to two cycles of dissolution in D₂O and drying to remove residual H₂O. Once dried, the sample was dissolved in 750 µL D₂O with 0.1% acetone, placed into a 3 mm NMR tube, and analyzed in a Bruker Avance-III operating at 500.13MHz 1H (125.77MHz 13C) equipped with a Bruker BBFO probe operating at 21.1 °C. The sample was analyzed using a heteroatomic single quantum coherence pulse sequence (HSQC) using the standard Bruker pulse sequence. Anomeric protons between 4-6 ppm (1H) and 80-120 ppm (13C) were assigned by analogy to glucose as reported in Roslund, et al. (2008) Carbohydrate Res. 343:101-112. Spectra were referenced to the internal acetone signal: 1H - 2.22 ppm; 13C - 30.8 ppm. Isomers were quantitated by integration of their respective peaks using the MNova software package from Mestrelab Research (Santiago de Compostela, Spain). Figure **2** shows the anomeric region of a representative spectrum. Over 300 samples have been assayed in this fashion and Table 1 lists the distribution across a sample of combinations of monomers showing the alpha-/beta- ratio to be as high as 4:1 as in the case of rha100 and as low as 1:1 as in the case of glu50gal50.

**Table 1: Distribution of alpha- and beta-bonds across batches and types of glycans**

| **glycans** | **alpha-bonds (%)** | **beta-bonds (%)** | **alpha/beta ratio** |
|---|---|---|---|
| Glu100 | 58 | 42 | 1.4 |
| | 61 | 39 | 1.6 |
| | 64 | 36 | 1.8 |
| | 64 | 36 | 1.8 |
| | 62 | 38 | 1.6 |
| | 61 | 39 | 1.6 |
| | 62 | 38 | 1.6 |
| | 63 | 37 | 1.7 |
| | 60 | 40 | 1.5 |
| | 65 | 35 | 1.9 |
| | 65 | 35 | 1.9 |
| | 60 | 40 | 1.5 |
| Gal1 00 | 60 | 40 | 1.5 |
| Gal33man33ara33 | 79 | 21 | 3.8 |
| | 75 | 25 | 3.0 |
| Glu50gal50 | 50 | 50 | 1.0 |
| | 56 | 44 | 1.3 |
| | 61 | 39 | 1.6 |
| | 65 | 35 | 1.9 |
| Glu33gal33fuc33 | 55 | 45 | 1.2 |
| Man100 | 57 | 43 | 1.3 |
| Man52glu29gal19 | 76 | 24 | 3.2 |
| Ara100 | 67 | 33 | 2.0 |
| Rha100 | 80 | 20 | 4.0 |
| Xyl100 | 57 | 43 | 1.3 |
| | 59 | 41 | 1.4 |
| Xyl75gal25 | 56 | 44 | 1.5 |

### Identification of composition by NMR

This experiment was designed to identify the composition of a glycan by 2D-NMR identification of the constituent monomers. Approximately 150 mg of 65 Brix oligosaccharide solution was dried to stable mass in a vacuum oven at 45-95 °C under 400 mbar pressure. The sample was subjected to two cycles of dissolution in D₂O and drying to remove residual H₂O. Once dried, the sample was dissolved in 750 µL D₂O with 0.1% acetone, placed into a 3 mm NMR tube, and analyzed in a Bruker Avance-III operating at 500.13MHz 1H (125.77MHz 13C) equipped with a Bruker BBFO probe operating at 70 °C. The sample was analyzed using a heteroatomic single quantum coherence pulse sequence (HSQC) using the standard Bruker pulse sequence. The anomeric region of each glycan spectra derived from a single sugar monomer was then examined for peaks representing specific glycosidic bonds characteristic to that monomer. For any given glycan, the HSQC spectra allow the identification of peaks that are unique to specific regio- and stereochemical bond arrangement. For example, Figure 5 shows a partial assignment of the spectra of a glu100 preparation demonstrating how these peaks may be used to identify specific glycosidic regio- and stereo-chemistries. Due to the spin-isolated nature of single carbohydrate rings within polysaccharides, the HSQC spectra of a glycan with more than one monomer is predicted to be represented by the sum of the HSQC peaks of each of its constituent sugars. Therefore, each constituent monomer has unique HSQC peaks that will appear in any glycan that contains that monomer irrespective of other constituent monomers and furthermore, the monomers used to synthesize a glycan can be determined by identifying the fingerprint peaks unique to each constituent monomer. For example. Figure 3B shows that the HSQC spectra of glu50gal50 is a hybrid of the spectra of glu100 (Figure 3A) and gal100 (Figure 3C). Table 2 lists the fingerprint peaks for selected glycan units.

**Table 2: Diagnostic HSQC peaks for each component sugar.**

| **Monomer** | **1H shift** | **13C shift** | **Monomer** | **1H shift** | **13C shift** |
|---|---|---|---|---|---|
| Glucose | 5.42 | 92.5 | Xylose | 5.18 | 93.0 |
| | 5.21 | 92.8 | | 5.10 | 94.3 |
| | 5.18 | 93.9 | | 5.34 | 98.2 |
| | 5.08 | 97.0 | | 5.31 | 99.6 |
| | 5.36 | 98.4 | | 5.11 | 100.8 |
| | 5.34 | 99.8 | | 4.91 | 99.4 |
| | 5.38 | 100.3 | | 4.56 | 97.3 |
| | 4.95 | 98.6 | | 4.64 | 104.2 |
| | 4.62 | 96.6 | | 4.54 | 103.4 |
| | 4.70 | 103.6 | | 4.44 | 102.6 |
| | 4.49 | 103.4 | | 4.44 | 104.1 |
| Galactose | 5.37 | 92.9 | Arabinose | 5.22 | 93.2 |
| | 5.24 | 93.1 | | 5.13 | 93.2 |
| | 5.14 | 96.0 | | 5.29 | 96.0 |
| | 4.96 | 99.3 | | 5.26 | 97.2 |
| | 5.31 | 98.7 | | 5.12 | 96.6 |
| | 5.39 | 101.4 | | 5.18 | 99.6 |
| | 5.00 | 101.8 | | 5.06 | 99.2 |
| | 4.80 | 101.3 | | 4.99 | 100.0 |
| | 4.63 | 97.0 | | 5.26 | 101.9 |
| | 4.56 | 97.2 | | 5.06 | 102.1 |
| | 4.53 | 103.1 | | 4.55 | 97.4 |
| | 4.43 | 104.1 | | 4.54 | 105.2 |
| Fucose | 5.18 | 92.9 | | 4.50 | 105.5 |
| | 5.33 | 92.4 | | 4.38 | 103.9 |
| | 5.04 | 96.3 | Rhamnose | 5.21 | 93.2 |
| | 4.90 | 99.7 | | 5.10 | 94.5 |
| | 4.52 | 97.0 | | 4.85 | 94.1 |
| | 4.39 | 103.6 | | 5.01 | 95.8 |
| Mannose | 5.37 | 93.0 | | 5.35 | 100.5 |
| | 5.16 | 94.6 | | 5.15 | 102.2 |
| | 4.88 | 94.2 | | 5.04 | 102.9 |
| | 5.39 | 101.7 | | 4.78 | 97.9 |
| | 5.24 | 101.9 | | 4.71 | 99.0 |
| | 5.13 | 102.8 | | 4.72 | 101.0 |
| | 5.03 | 102.7 | | | |
| | 5.24 | 105.6 | | | |
| | 5.09 | 108.0 | | | |
| | 4.88 | 94.2 | | | |
| | 4.89 | 100.0 | | | |
| | 4.70 | 101.1 | | | |

At least 5 peaks appeared for each glycan unit used as a starting material in the synthesis of glycans containing 3 or fewer distinct glycan units. The HSQC spectra of glycans containing 4 or more distinct glycan units have at least 4 peaks for each constituent glycan unit.

Figure 6A and 6B show the HSQC spectra for man 100 and xyl100, respectively.

### Glycosidic linkage analysis

This experiment was designed to quantitate the distribution of glycosidic regioisomers (branching) within a given oligosaccharide. For glycosyl linkage analysis, the samples were permethylated, depolymerized, reduced, and acetylated; and the resultant partially methylated alditol acetates (PMAAs) analyzed by gas chromatography-mass spectrometry (GC-MS) as described by Heiss et al (2009) Carbohydr. Res. 344:915. The samples were suspended in 200 µl of dimethyl sulfoxide and left to stir for 1 day. Permethylation was affected by two rounds of treatment with sodium hydroxide (15 min) and methyl iodide (45 min). The aqueous solution was hydrolyzed by addition of 2M trifluoroacetic acid and heating to 121 °C for 2 hours. Solids were isolated *in vacuo* and acetylated in acetic acid/trifluoroacetic acid. The resulting PMAAs were analyzed on an Agilent 7890A GC interfaced to a 5975C MSD (mass selective detector, electron impact ionization mode); separation was performed on a 30 m Supelco SP-2331 bonded phase fused silica capillary column. Figure **4** shows three representative GC spectra from this analysis. These analyses show that the glycans had at least 0.1 %, 0.2%, 0.5%, 1%, 2%, 5%, 10% or more of the 1,2-glycoside bond type, e.g. ara100 = 3.8%, gal100 = 7.2%; at least 0.1%, 0.2%, 0.5%, 1%, 2%, 5%, 10% or more of the 1,3-glycoside bond type, e.g. 3-bn-glu100 = 1.7%, glu50gal50 = 10.4%; at least 0.1%, 0.2%, 0.5%), 1%, 2%, 5%, 10% or more of the 1,4-glycoside bond type, e.g. glu50gal50 = 5.9%, gal33man33ara33 = 10.1%; and at least 0.1%, 0.2%, 0.5%, 1%, 2%, 5%, 10%, 15%, 20%, 25% or more of the 1,6-glycoside bond type, e.g. gal33man33ara33 = 13.4%, glu100 = 25.4%. The materials also contained at least 5%, 10%, 15%, 20%, 25%, 30%, 40%, 50%, or more of the branched bond types (including but not limited to 1,3,6-; 1,4,6-; or 1,2,4-glycosides, e.g. Table **3**), a degree of branching (DB) of at least 0.05. Degree of branching is defined as the average number of branched monomers relative to total number of monomer units. For example, a glu100 glycan polymer in which 20% of the glucose monomer units contain glycosidic linkages to three or more other glucose monomers would have a DB of 0.20. The glycans also have about 3-12% of the monomeric units in the furanose form. A glycan originating from a single monomer consisted of at least 12 distinct non-terminal substitution patterns. A glycan originating from two monomers consisted of at least 18 distinct non-terminal substitution patterns, e.g. glu-1,2-glu; glu-1,2-gal; gal-1,2-glu; gal-1,2-gal; glu-1,2(glu),6-glu; glu-1,3-glu; glu-1,3-gal; etc. A glycan originating from three or more monomers consisted of at least 24 distinct non-terminal substitution patterns.

**Table 3. A sample of degree of branching (DB) measurements; sample selected from 54 different preparations characterized as described herein.**

| composition | % branched monomers | |
|---|---|---|
| | highest measure | lowest measure |
| glu100 | 40.4 | 10.4 |
| glu80man20 | 16.1 | |
| glu60man40 | 16.4 | |
| man80glu20 | 18.6 | |
| man60glu40 | 20.5 | |
| glu50gal50 | 22.4 | 12.6 |
| gal100 | 22.2 | |
| glu33gal33fuc33 | 41.8 | |
| ara100 | 16.6 | |
| xyl100 | 63.2 | |
| xyl75ara25 | 26.9 | |
| man52glu29gal19 | 22.7 | 9.8 |
| man100 | 40.0 | |

**Table 4a: Exemplary glycan polymer preparations**

| **Glycan** | **total molar incidence of a bond (%)** | | | | **Misc glycoside sums (%)** | | |
|---|---|---|---|---|---|---|---|
| | total 1,2 | total 1,3 | total 1,4 | total 1,6 | total branching | total furanose | total terminal sugars |
| Glu5Ga15Man90-2 | 19% | 15% | 22% | 43% | 25.9 | 12 | 34.9 |
| Gtu10Gal10Man80-1 | 15% | 16% | 24% | 45% | 22.6 | 6.7 | 33.1 |
| Glu20Gal20Man20Xyl20Ara 20-1 | 16% | 18% | 32% | 34% | 22.0 | 25.1 | 33.1 |
| Glu20Gal20Man20Xyl20Ara 20-2 | 16% | 19% | 1 6% | 48% | 20.1 | 4.8 | 35.3 |
| Gal33Man33Ara33-8 | 17% | 26% | 23 % | 34% | 25.5 | 27.5 | 32.7 |
| Gal57Glu43-1 | 4% | 7% | 73% | 16% | 2 | 2.7 | 50.9 |
| Glu100-87 | 1% | 3% | 93% | 4% | 0 | 0 | 34.7 |
| Gal57Glu43-2 | 2% | 2% | 1% | 94% | 1.3 | 1.5 | 46.6 |
| Glu50Gal50-11 | 15% | 20% | 20% | 45% | 14.8 | 12.2 | 38.3 |
| Glu50Gal50-32 | 15% | 16% | 26% | 43% | 13.1 | 17.9 | 45.2 |
| Glu50Gal50-14 | 13% | 17% | 25% | 44% | 13.5 | 22.4 | 43.3 |
| Glu50Gal50-27 | 15% | 20% | 22% | 43 % | 19.5 | 9.6 | 29.5 |
| Glu50Gal50-23 | 17% | 20% | 20% | 44% | 19.2 | 17.2 | 35.5 |
| Glu50Gal50-2 | 16% | 21% | 18% | 45% | 19.4 | 15.6 | 35.5 |
| Glu100-129 | 20% | 19% | 16% | 46% | 19.1 | 5.3 | 36.3 |
| Glu 100-136 | 19% | 20% | 16% | 46% | 19.6 | 4.7 | 34.8 |
| Glu100-17 | 19% | 20% | 15% | 47% | 19.7 | 3.1 | 31.6 |
| Glu100-64 | 19% | 21% | 15% | 46% | 19.6 | 3.3 | 34.6 |
| Glu100-76 | 18% | 19% | 15% | 47% | 18.5 | 3.8 | 33.4 |
| Glu100-131 | 18% | 18% | 17% | 46% | 16.4 | 7.4 | 39.2 |
| Glu100-83 | 19% | 20% | 1 8% | 44% | 22.2 | 8.7 | 34.5 |
| Glu100-139 | 19% | 20% | 1 5% | 46% | 19.4 | 4.5 | 34.5 |
| Glu100-84 | 19% | 20% | 15% | 46% | 19 | 3.5 | 32.6 |
| Glu100-74 | 19% | 19% | 17% | 45% | 22.2 | 6.7 | 27.9 |
| Glu100-98 | 19% | 19% | 18% | 45% | 18.5 | 6.9 | 36.4 |
| Glu100-141 | 18% | 24% | 16% | 41% | 40.4 | 3.7 | 16.3 |
| Glu100-29 | 19% | 18% | 16% | 46% | 19.5 | 3.8 | 30 |
| Glu100-18 | 20% | 21% | 15% | 45% | 27.5 | 3.4 | 18.9 |
| Glu100-99 | 18% | 20% | 16% | 45% | 20.1 | 6.5 | 35.4 |
| Glu100-72 | 19% | 20% | 17% | 44% | 22.2 | 6.3 | 32.2 |
| Glu 100-82 | 18% | 21% | 17% | 44% | 22 | 6.4 | 30.6 |
| Glu100-130 | 18% | 21% | 17% | 44% | 21.9 | 5.2 | 32.9 |
| Glu100-78 | 18% | 20% | 17% | 44% | 21.6 | 4.5 | 3 2 |
| Glu100-66 | 19% | 20% | 17% | 44% | 22 | 6.6 | 31.1 |
| Glu100-89 | 18% | 19% | 16% | 48% | 18.6 | 6.7 | 35.9 |
| Glu100-133 | 17% | 18% | 18% | 46% | 20.1 | 11.1 | 35.8 |
| Glu100-68 | 18% | 19% | 17% | 46% | 18.7 | 7.4 | 36.3 |
| Glu100-90 | 19% | 20% | 16% | 45% | 16.8 | 4.2 | 38.8 |
| Glu100-94 | 19% | 19% | 1 4% | 47% | 17.7 | 3.1 | 35.1 |
| Glu100-5 | 19% | 19% | 14% | 48% | 16.3 | 3 | 36.6 |
| 3-Obn Glu100-1 | 14% | 5% | 31% | 50% | 34.4 | 5.5 | 22.0 |
| Gal100-30 | 16% | 19% | 24% | 41% | 17.2 | 32.6 | 30.4 |
| Glu33Gal33Fuc33-3 | 15% | 30% | 29% | 27% | 41.8 | 15.2 | 22.5 |
| Ara100-12 | 26% | 42% | 32% | NA | 16.6 | 36.7 | 23.1 |
| Xyl100-8 | 19% | 35% | 46% | NA | 63.2 | 3.8 | 0.3 |
| Xyl75Ara25-3 | 25% | 32% | 43 % | NA | 26.9 | 18.7 | 23.5 |
| Glu80Man20-2 | 15% | 19% | 21% | 45% | 16.1 | 4.6 | 34 |
| Glu60Man40-5 | 10% | 24% | 23 % | 43% | 16.4 | 2.1 | 28.3 |
| Man80Glu20-2 | 8% | 25 % | 17% | 50% | 18.6 | 1.8 | 30.9 |
| Man60Glu40-2 | 8% | 22 % | 26% | 43% | 20.5 | 3.7 | 28.6 |
| Man52Glu29Gal19-2 | 12% | 19% | 27% | 42% | 51.1 | 19 | 8.4 |
| Man52Glu29Gal19-3 | 8% | 18% | 31% | 44% | 37.0 | 26.6 | 23.6 |
| Man 100-17 | 12% | 27% | 25% | 36% | 19.4 | 9.5 | 40.0 |

**Table 4b: Exemplary glycan polymer preparations**

| **Glycan** | **alpha/beta ratio by HSQC NMR** | | **SEC data** | | | | |
|---|---|---|---|---|---|---|---|
| | % alpha | % beta | DP2+ % | Mw | Mη | PD | DPn |
| Glu5Gal5Man90-2 | 80% | 20% | 98% | 1842 | 946 | 1.95 | 11.26 |
| Glu10Gal10Man80-1 | 81% | 19% | 98.60% | 1978 | 1021 | 1.94 | 12.1 |
| Glu20Gal20Man20Xyl20Ara 20-1 | 87% | 13% | 100% | 1278 | 935 | 1.37 | 7.78 |
| Glu20Gal20Man20Xyl20Ara 20-2 | 63% | 37% | 100% | 1845 | 1000 | 1.85 | 11.28 |
| Gal33Man33Ara33-8 | 87% | 1 3% | 98% | 1527 | 834 | 1.83 | 9.31 |
| Gal57Glu43-1 | 33% | 67% | 94% | 374 | 349 | 1.07 | 2.20 |
| Glu100-87 | 69% | 31% | 100% | 416 | 399 | 1.04 | 2.46 |
| Gal57Glu43-2 | 65% | 35% | 98% | 390 | 374 | 1.04 | 2.3 |
| Glu50Gal50-11 | 64% | 36% | 91% | 1456 | 675 | 2.16 | 8.88 |
| Glu50Gal50-32 | 66% | 34% | 96% | 1114 | 790 | 1.41 | 6.77 |
| Glu50Gal50-14 | 70% | 30% | | | | | |
| Glu50Gal50-27 | 61% | 39% | 99% | 1776 | 945 | 1.88 | 10.85 |
| Glu50Gal50-23 | 71% | 29% | 99% | 1497 | 855 | 1.75 | 9.1 3 |
| Glu50Gal50-2 | 65% | 35% | | 1931 | 936 | 2.06 | 11.8 |
| Glu100-129 | 62% | 38% | 99% | 1712 | 1411 | 1.21 | 7.84 |
| Glu100-136 | 64% | 36% | 99% | 1834 | 1577 | 1.16 | 8.76 |
| Glu100-17 | 61% | 39% | 98% | 1797 | 1523 | 1.18 | 8.46 |
| Glu100-64 | 62% | 38% | 98% | 1871 | 1620 | 1.15 | 9.00 |
| Glu100-76 | 62% | 38% | 99% | 1702 | 1410 | 1.21 | 7.83 |
| Glu100-131 | 61% | 39% | 98% | 1520 | 1200 | 1.27 | 6.67 |
| Glu100-83 | 64% | 36% | 99% | 1849 | 1605 | 1.15 | 8.92 |
| Glu100-139 | 64% | 36% | 98% | 1819 | 1542 | 1.18 | 8.57 |
| Glu100-84 | 62% | 38% | 99% | 1726 | 1431 | 1.21 | 7.95 |
| Glu100-74 | 61% | 39% | 98% | 1697 | 1387 | 1.22 | 7.71 |
| Glu100-98 | 62% | 38% | 98% | 1690 | 1383 | 1.22 | 7.68 |
| Glu100-141 | 63% | 37% | 99% | 1898 | 1673 | 1.13 | 9.29 |
| Glu100-29 | 60% | 40% | 98% | 1624 | 1311 | 1.24 | 7.28 |
| Glu100-18 | 65 % | 35% | 99% | 1946 | 1748 | 1.11 | 9.71 |
| Glu100-99 | 64% | 36% | 99% | 1876 | 1641 | 1.14 | 9.12 |
| Glu100-72 | 64% | 3 6% | 99% | 1929 | 171 6 | 1.12 | 9.54 |
| Glu100-82 | 65% | 35% | 99% | 1927 | 1711 | 1.13 | 9.50 |
| Glu100-130 | 63 % | 37% | 99% | 1967 | 1781 | 1.10 | 9.90 |
| Glu100-78 | 63% | 37% | 99% | 1926 | 1719 | 1.12 | 9.55 |
| Glu100-66 | 62% | 38% | 98% | 1763 | 1472 | 1.20 | 8.18 |
| Glu100-89 | 61% | 39% | 98% | 1638 | 1326 | 1.23 | 7.37 |
| Glu100-133 | 65 % | 35% | 97% | 1567 | 1224 | 1.28 | 6.80 |
| Glu100-68 | 60% | 40% | 98% | 1701 | 1394 | 1.22 | 7.74 |
| Glu100-90 | 51% | 49% | 96% | 982 | 674 | 1.46 | 5.90 |
| Glu 100-94 | 54% | 46% | 100% | 1369 | 978 | 1.40 | 8.30 |
| Glu100-5 | 57% | 43% | 100% | 1226 | 902 | 1.36 | 7.40 |
| 3-Obn Glu100-1 | 66% | 34% | 100% | 1014 | 486 | 2.09 | 6.15 |
| Gal100-30 | 74% | 26% | | | | | |
| Glu33Gal33Fuc33-3 | 65% | 35% | | | | | |
| Ara100-12 | 74% | 26% | | | | | |
| Xyl100-8 | 70% | 30% | | | | | |
| Xyl75Ara25-3 | 69% | 31% | | | | | |
| Glu80Man20-2 | 68% | 32% | | | | | |
| Glu60Man40-5 | 79% | 21% | | | | | |
| Man80Glu20-2 | 87% | 13% | | | | | |
| Man60Glu40-2 | 73% | 27% | | | | | |
| Man52Glu29Gal19-2 | 77% | 23% | | | | | |
| Man52Glu29Gal19-3 | 82% | 18% | | | | | |
| Man100-17 | 57% | 43% | | | | | |

### Example 6: Decreases in pathogenic bacteria (including VRE and CRE) in defined community assays in the presence of glycan preparations

Levels of CRE *Klebsiella pneumonia,* CRE *Escherichia coli,* and VRE *Enterococcus faceium* added to a complex microbial community grown in the presence of glycans as a sole carbon source were examined to determine if pathogen levels could be selectively decreased.

A defined community was constructed, composed of 46 strains that belonged to phyla Actinobacteria, Firmicutes, and Bacteroidetes: *Blautia producta, Blautia hansenii, Clostridium celatum, Bacteroiodes cellulosilyticus, Odoribacter splanchnicus, Bifidobacterium catenulatum, Eubacterium hallii, Bacteroides dorei, Bifidobacterium pseudocatenulatum, Bifidobacterium adolescentis*, *Bacteroides coprophilus, Lactobacillus casei, Coprocoecus catus, Bifidobacterium angulatum, Eubacterium ventriosum, Lachnospira multipara, Parabacteroides merdae, Bacteroides finegoldii, Parabacteroides distasonis, Bacteroides thetaiotaomicron, Blautia hydrogenotrophica, Blautia coccoides, Clostridium bolteae, Clostridium scindens, Holdemanella biformis, Bifidobacterium longum sub. Infantalis, Ruminococcus obeum, Dorea formicigenerans, Collinsella aerofaciens, Eubacterium eligens, Faecalibacterium prausnitzii, Bifidobacterium longum, Prevotella copri, Eubacterium rectale, Bacteroides uniformis, Succinivibrio dextrinosolvens, Roseburia intestinalis, Clostridium nexile, Bacteroides caccae, Bacteroides vulgatus, Dorea longicatena, Akkermansia muciniphila, Bacteroides thetaiotaomicron, Bacteroides cellulosilyticus, Clostridium symbiosum,* and *Ruminococcus gnavus.*

Each of the 46 strains was grown separately in standard chopped meat glucose medium (CMG) for 18-48 hours depending on a strain. Optical density (OD600) of each culture was adjusted to 0.2, and equal volumes of each culture were combined into one bottle with 15% final glycerol. 1.5mL aliquots were frozen at -80C. Frozen aliquots were thawed, washed with Clostridial Minimal Media without added carbon source and adjusted to OD=0.01. Each of CRE *Klebsiella pneumonia,* CRE *Escherichia coli,* and VRE *Enterococcus faceium,* obtained from the CDC, were grown aerobically in BHI medium for 12 hours at 37C. Optical density (OD600) of each culture was adjusted to 0.01 and added to a solution of defined community to a final concentration of 12%.

This community was grown for 24 hours in the anaerobic chamber on a medium supplied with the following glycan preparations as sole carbon source: Fru100-9, Gal50Fru50-2, Glu100-104, Glu100-20, Glu50Gal50-27, Gal33Man33Ara33-11, Glu20Gal60Man20-1, Gal33Xyl33Ara33-2, Glu33Gal33Man33-1, Man33Xyl33Ara33-1, Glu33Gal33Xyl33-1, Glu25Gal25Man25Xyl25-1, Glu33Gal33Ara33-3, Glu25Gal25Man25Ara25-2, Gal33Man33Xyl33-2, Glu25Gal25Xyl25Ara25-2, Gal33Man33Ara-33-18, Glu25Man25Xyl25Ara25-1, Water-1, Gal25Man25Xyl25Ara25-2, Glu33Man33Ara33-2, Glu20Gal2OMan20Xyl20Ara20-1, Glu33Xyl33Ara33-1, Glu90Gal5Man5-2, Glu100-103, Glu80Gal10Man10-1, Glu33Gal33Xyl33-3, Glu60Gal20Man20-1, Gal33Man33Xyl33-3, Glu40Gal30Man30-1, Gal33Man33Ara33-13, Glu20Gal40Man40-1, Glu33Man33Xyl33-2, Glu 10Gal45Man45-2, Glu33Man33Ara33-1, Glu5Gal90Man5-1, Gal33Xyl33Ara33-1, Glu10Gal80Man10-1, Man33Xyl33Ara33-2, Glu20Gal60Man20-2, Glu25Gal25Man25Xyl25-2, Glu33Gal33Man33-2, Glu25Gal25Man25Ara25-1, Glu100-77, Glu25Man25Xyl25Ara25-2, Glu5Gal90Man5-2, Gal25Man25Xyl25Ara25-1, Glu10Gal80Man10-2, Glu20Ga120Man20Xyl20Ara20-2, Glu30Ga140Man30-2, Glu90Gal5Man5-1, Glu30Gal40Man30-1, Glu80Gal10Man10-2, Glu40Gal20Man40-2, Glu60Gal20Man20-2, Glu40Gal20Man40-1, Glu40Gal30Man30-2, Glu45Gal10Man45-1, Glu20Gal40Man40-2, Glu45Gal10Man45-2, Glu10Gal45Man45-1, Glu5Gal5Man90-1, Gal100-10, Xyl75Ara25-2, Glu5Gal5Man90-2, Glu45Gal45Man10-1, Glu10Gal10Man80-2, Glu45Gal45Man10-2, Glu10Gal10Man80-1, Ara100-3, Glu20Gal20Man60-1, Man100-6, Glu20Ga120Man60-2, Ara100-4, Glu30Gal30Man40-2, Xyl100-7, Glu30Gal30Man40-1, Man52Glu29Gal19-1, Glu40Gal40Man20-2, Glu50Gal50-39, Glu40Gal40Man20-1, Glu50Gal50-21, Gal100-4, Glu20Gal80-1, Glu100-129, Glu100-130, Glu100-74, Glu100-136, Glu100-98, Glu100-78, Glu100-141, Glu100-66, Glu100-29, Glu100-89, Glu100-18, Glu100-133, Glu100-99, Glu100-68, Glu100-72, Glu100-17, Glu100-82, Glu100-64, Glu100-33, Glu50Gal50-42, Glu100-76, Glu50Gal50-3, Glu100-131, Gal80Xyl20-1, Glu100-83, Gal60Xyl40-1, Glu100-139, Gal40Xyl60-1, Glu100-84, Gal20Xy180-1, Glu50Gal50-22, Gal80Ara20-1, Gal60Ara40-1, Gal40Ara60-1, Glu50Gal50-32, Gal20Ara80-1, Gal100-3, Ara100-6, Man80Xy120-1, Xyl60Ara40-1, Man60Xyl40-1, Glu33Gal33Man33-4, Man40Xyl60-1, Glu33Gal33Xyl33-2, Man20Xyl80-1, Glu33Gal33Ara33-4, Man80Ara20-1, Gal33Man33Xyl33-1, Man60Ara40-1, Gal33Man33Ara33-17, Man40Ara60-1, 3-Obn, Man20Ara80-1, Glu80Gal20-1, Xyl80Ara20-1, Glu60Gal40-1, Ara100-2, Ara100-5, Glu40Gal60-1, Glu60Ara40-1, Gal80Man20-1, Glu40Ara60-1, Gal60Man40-1, Glu20Ara80-1, Gal40Man60-1, Man75Gal25-1, Glu80Xyl20-1, Ara80Xyl20-1, Glu60Xyl40-1, Ara6OXyl40-1, Glu40Xy160-1, Glu80Man20-1, Glu20Xyl80-1, Glu60Man40-1, Glu80Ara20-1, Man80Glu20-1, Ara100-7, Gal100-9, Man80Gal20-3, Glu50Gal50-18, Glu60Man40-2, Glu50Gal50-40, Man60Glu40-1, Glu50Gal50-28, Glu33Gal33Man33-3, Glu50Ga150-41, Glu33Gal33Ara33-1, Glu50Gal50-9, Gal33Man33Ara33-12, Glu50Gal50-43, Glu33Gal33Ara33-5, Glu50Gal50-30, Glu50Gal50-46, Glu50Gal50-33, Man100-7, Gal100-8, Gal100-7, Gal100-2, Gal57Fru43-1, Glu100-114, Glu100-116, Glu100-127, Glu100-87, Man100-15, Glu66Fru33-1, galnac100-1, Gal57Glu43-2, Ara88Gal3Rha2GalA3-1, Gal50Glu25Fru25-1, Gal81Ara14-1, Gal57Glu43-1, GalA60Rha10Ara1Xyl1Gal23-1, Glu100-107, Glu100-123, galnac50GluA50-1, GalA9Rha3Ara6Gal82-1, Ara38Xyl62-1, Glu97-1, Gal22Man78-1, Xyl34Glu45Gal18Ara3-1, Glu100-112, Glu50Gal50-36, Glu33Gal33Fru33-1, Glu100-105, Glu100-100, Gal100-13, Glu100-125, Glu100-138, Xyl100-2, Glu100-134, Glu100-67, Glu100-85, Glu100-79, Glu100-135, Glu100-71, Glu100-21, Glu100-137, Glu-30Gal-70-1, Glu100-16, Glu-50Man-50-1. Glu100-81, Glu-20-Xyl80-1, Glu-20Xyl-80-1, glu50fru50-1, Fuc100-1, Glu100-119, Rib100-1, Man100-10, Glu50Gal50-37, Gal100-14, Sor100-1, Ara100-9, gly100-1, Lara100-1, Neu100-2, Fru100-10, Glun100-1, Xyl100-6, Gala100-2, Rha100-1, Glua100-1, Manni100-1, Glu50Gal50-20, Glu50Gal50-2, Fru100-7, Glu100-65, Glu100-75, Glu100-90, Glu50Lglu50-1, Glu80Lglu20-1, Glu90Lglu10-1, Glu100-140, Glu75Glunac25-1, Glu50Gal50-11, Glu100-6, Tbdps-Gal100-1, Gal33Man33Ara33-9, Glu50Gal50-8, Gal33Man33Ara33-15, Glu50Gal50-45, Gal33Man33Ara33-1, Glu50Gal50-7, Gal33Man33Ara33-4, Glu50Gal50-34, Gal33Man33Ara33-3, Glu50Gal50-17, Gal33Man33Ara33-14, Glu50Gal50-19, Glu90Xyl5Ara5-1, Glu50Gal50-13, Glu80Xyl10Ara10-1, Gal33Man33Ara33-2, Glu60Xyl20Ara20-1, Gal33Man33Ara33-10, Glu40Xy130Ara30-1, Glu20Xyl40Ara40-1, Glu20Xy120Ara60-1, Glu10Xyl45Ara45-1, Glu30Xyl30Ara40-1, Glu5Xyl90Ara5-1, Glu40Xyl40Ara20-1, Glu10Xyl80Ara10-1, Glu45Xyl45Ara10-1, Glu20Xyl60Ara20-1, Gal90Xyl5Ara5-1, Glu30Xyl40Ara30-1, Ga180Xyl10Ara10-1, Glu40Xyl20Ara40-1, Gal60Xyl20Ara20-1, Glu45Xyl10Ara45-1, Ga140Xyl30Ara30-1, Glu5Xyl5Ara90-1, Gal20Xyl40Ara40-1, Glu10Xyl10Ara80-1, Gal10Xyl45Ara45-1, Gal5Xyl90Ara5-1, Ga140Xyl40Ara20-1, Gal10Xyl80Ara10-1, Gal45Xyl45Ara10-1, Gal20Xyl60Ara20-1, 3-Bn, Gal30Xyl40Ara30-1, glu50gal50-22, Gal40Xyl20Ara40-1, glu50gal50-24, Gal45Xyl10Ara45-1, glu50gal50-23, Gal5Xyl5Ara90-1, glu50gal50-15, Gal10Xyl10Ara80-1, glu50gal50-19, Gal20Xyl20Ara60-1, glu50gal50-20, Gal30Xyl30Ara40-1, glu50gal50-13, Glu100-3, glu50gal50, Glu50Gal50-10, glu50gal50, glu50gal50-1, glu50gal50-16, glu50ga150-14, glu50gal50-21, gli50gatl0-18, glu50gal50-11, glu50gal50-12, glu50gal50-17, 6-TBDPS, Glu100-63, 6-TBDPS, Glu100-73, acetylated, gal33man33ara33-3, butyrylated, gal33man33ara33-8, Glu60Man40-4, gal33man33ara33-4, Man80Gal20-2, gal33man33ara33-1, Glu50Gal50-14, gal33man33ara33-2, Glu50Gal50-24, gal33man33ara33-6, Fru100-8, gal33man33ara33-7, gal33man33ara33-5, Glu100-70, Glu100-69, Gal33Man33Ara33-8, Gal100-5, Man66Gal33-3, Glu100-2, Xyl100-3, Glu100-115, Glu100-124, Glu100-106, Gal50Fru50-3, Glu100-117, Glunac100-1, Glunac100-2, Man100-9, Man100-2, Glu100-128, Man100-11, a-1,6-glu100-1, Glu100-94, Man66Gal33-1, Man66Gal33-2, Glu100-143, Glu100-10, Glu100-11, Glu100-26, Glu100-1, Glu100-24, Glu100-9, Glu100-7, butyrylated, Gal85Ara15-10, Gal85Ara15-8, Gal85Ara15-5, Gal85Ara15-6, Glu100-15, Glu100-102, Glu100-22, Glu100-92, Glu100-12, Glu100-4, Gal85Ara15-7, Glu100-8, Gal85Aral5-9, and Glu100-23. "Man", "glu", "gal", "xyl" etc. denotes the sugar; the number immediately following denotes the relative quantity of the sugar in the preparation (e.g., Man80gal20 means the preparation contains 80% mannose and 20% galactose); and the number after the dash denotes a glycan preparation (e.g., -1) that has different characteristics from another glycan preparation (e.g., -3), which differ from each other within the ranges for the glycan preparations described herein.

As a control, water was added to a medium without any added carbon source. The final concentration of each glycan in the assay was 0.5%. Each glycan was represented 3 times within each growth plate and was the sole carbon source for bacteria. Plates were incubated at 37°C in anaerobic chamber AS-580 for a total of 24 hours. At 24 hours, the optical density was determined for each community incubated with a glycan and the resulting measurement was used as a proxy for total anaerobic growth.

To determine level of pathogens in this defined community, 4uL of cultures were diluted in fresh Luria-Bertani (LB) media, and incubated aerobically for 5 hours at 37C. Optical density growth curves were performed using Biotek plate reader and computationally analyzed using the R package, growthcurver. The time to mid-log was calculated and used to determine the total pathogen load at the end of the anaerobic phase of the experiment. Lower time to mid-log values corresponded to higher levels of pathogens at the end of the anaerobic phase of the experiment.

To identify glycans that supported overall community growth, while reducing pathogen growth, both time to midlog (proxy for pathogen growth - higher is a sign of less pathogen growth) and OD600 at the end of the anaerobic phase (higher is a sign of more commensal growth) were normalized within their respective metrics from 0 to 1. These values were multiplied together and subtracted from 1 (1-anerobic growth^{∗}aerobic growth). All glycans were normalized to control without any additional carbon source and glycans that reduced pathogens the most were identified.

Glycans were identified that improved overall growth of the commensal community while also reducing total pathogen growth (Tables 9-11). When combining both commensal community growth and reduction in pathogen growth, a number of glycans improve over control by >25% for CRE *Escherichia coli* (see Figure 7), >5% for VRE *Enterococcus faecium* (see Figure 8), and > 30% for *Klebsiella pneumoneae* (see Figure 9). These results suggest that different glycans can be used to reduce different pathogenic bacteria.

**Table 5. Glycans that reduce CRE Klebsiella pneumoniae when controlling for overall community growth.**

| **Composition** | **Percent *K. pneumoneae* growth (normalized to control)** |
|---|---|
| Gal50Glu25Fru25-1, Gal57Glu43-1 | 60 - < 75 |
| Gal157Glu43-2, Gal57Fru43-1, Glu66Fru33-1, Glu100-87, Glu100-8 | 75-85 |

**Table 6. Glycans that reduce CRE Escherichia coli when controlling for overall community growth.**

| **Composition** | **Percent *E.*coli growth (normalized to control)** |
|---|---|
| Glu100-138 | 70 - <80 |
| Glu100-22 | |
| Glu100-12 | |
| a-1,6-glu100-1 | |
| Glu100-4 | 80 - <85 |
| Glu100-9 | |
| Glu100-85 | |
| Glu100-134 | |
| Glu100-8 | |
| Glu100-100 | |
| Gal85Ara15-7 | |
| Gal57Glu43-2 | |
| Glu100-70 | |
| Glu100-69 | |
| Gal50Glu25Fru25-1 | 80 - <90 |
| Gal85Ara15-9 | |
| Glu 100-15 | |
| Glu-50Man-50-1 | |
| Glu 100-23 | |
| Glu100-2 | |
| Gal85Ara15-5 | |
| Gal57Fru43-1 | |
| Glu50Gal50-39 | |
| Glu100-73 | |
| Glu66Fru33-1 | |
| Glu100-65 | |
| Gal85 Ara 15-6 | |
| Gal57Glu43-1 | |
| Glu100-133 | |
| Glu 100-68 | |

**Table 7. Glycans that reduce VRE Enterococcus faecium when controlling for overall community growth.**

| **Composition** | **Percent *E*. *faecium* growth (normalized to control)** | |
|---|---|---|
| Glu100-12 | 90 - 95 | |
| Glu100-70 | | |
| Glu100-8 | | |
| Glu100-22 | | |
| Glu 100-4 | | |
| Glu100-9 | | |
| Glu1 00-15 | | |

### Example 7: Decreases in pathogenic bacteria in fecal slurries from stem cell transplant patients in the presence of glycan preparations

An assay was performed to assess the ability of a human fecal community collected from patients undergoing a stem cell transplant to utilize different glycans and competitively reduce the growth of pathogenic bacteria. Fecal samples were collected from 5 patients diagnosed with acute myeloid leukemia, Hodgkin's lymphoma, or chronic myelogenous leukemia. All patients went through bone marrow transplant infusion either with a full preparation or non-myeoblative preparation. All patients received Levoflaxacin antibiotic. Clinical characteristics of patients are listed in Table 8.

Human fecal samples were stored at -80°C. To prepare working stocks the fecal samples were transferred into the anaerobic chamber and allowed to thaw. Each fecal sample was prepared to 20% w/v in phosphate buffered saline (PBS) pH 7.4 (P0261, Teknova Inc., Hollister, CA), 15% glycerol and stored at -80°C. The 20% w/v fecal slurry + 15% glycerol was centrifuged at 2,000xg, supernatant was removed, and the pellet was suspended in 900 mg/L sodium chloride, 26 mg/L calcium chloride dihydrate, 20 mg/L magnesium chloride hexahydrate, 10 mg/L manganese chloride tetrahydrate, 40 mg/L ammonium sulfate, 4 mg/L iron sulfate heptahydrate, 1 mg/L cobalt chloride hexahydrate, 300 mg/L potassium phosphate dibasic, 1.5 g/L sodium phosphate dibasic, 5 g/L sodium bicarbonate, 0.125 mg/L biotin, 1 mg/L pyridoxine, 1 m/L pantothenate, 75 mg/L histidine, 75 mg/L glycine, 75 mg/L tryptophan, 150 mg/L arginine, 150 mg/L methionine, 150 mg/L threonine, 225 mg/L valine, 225 mg/L isoleucine, 300 mg/L leucine, 400 mg/L cysteine, and 450 mg/L proline (Theriot CM et al. Nat Commun. 2014; 5:3114) to prepare 1% w/v fecal slurry. CRE *Klebsiella pneumonia was* obtained from the CDC, were grown aerobically in BHI medium for 12 hours at 37C. Optical density (OD600) of each culture was adjusted to 0.01 and added to a solution of defined community to a final concentration of 12%.

Prepared 1% w/v fecal slurries were incubated with a total of 34 glycans and subsequently tested for effective reduction in pathogen growth. Glycans tested included Ara100-11, Gal100-8, Glu50Gal50-24, Fru100-9. Gal50Fru50-2, Gal57Fru43-1, Glu100-114, Glu100-8, galnac100-2. Glu100-94, Glu100-107, Glu100-34, Glu100-3, Ara100-9, Gal85Aral5-5, Glu66Fru33-1, a-1,6-glu100-1, Gal100-13, Glu100-119, Glu100-20, Glu50Gal50-27, Gal50Glu25Fru25-1, galnac50GluA50-1, Glu100-123, Man100-15, Glu100-116, Ga133Man33Ara33-11, Glu100-26, Gal57Glu43-2, Glu100-127, Lara100-1, gly100-1, and Man66Gal33-2 at a final concentration of 0.5% w/v in 96-well deep well microplates, with water included in No Added Glycan controls, with 100 µL final volume per well, at 37°C for 24 hours, anaerobically. "Man", "glu", "gal", "ara" etc. denotes the sugar; the number immediately following denotes the relative quantity of the sugar in the preparation (e.g., Man80gal20 means the preparation contains 80% mannose and 20% galactose); and the number after the dash denotes a glycan preparation (e.g., -1) that has different characteristics from another glycan preparation (e.g., -3), which differ from each other within the ranges for the glycan preparations described herein.

To determine the level of pathogens in this community, 4uL of culture were diluted in fresh Luria-Bertani (LB) media, and incubated aerobically for 5 hours at 37°C. Optical density growth curves were performed using Biotek plate reader and computationally analyzed using the R package, growthcurver. The time to mid-log was calculated and used to determine the total pathogen load at the end of the anaerobic phase of the experiment. Lower time to mid-log values corresponded to higher levels of pathogens at the end of the anaerobic phase of the experiment.

To identify glycans that support overall community growth, while reducing pathogen growth, both time to mid-log (proxy for pathogen growth-higher is a sign of less pathogen growth) and total OD600 at the end of the anaerobic phase (higher is a sign of more commensal growth) were used. A cut-off of total commensal growth was of >0.2 OD600 was used to determine that commensal bacteria grew on a particular glycan. Total growth of pathogens during anaerobic phase was compared between glycan compounds and patient microbiomes by using 1/time to mid-log. Figure 10 depicts the identification of glycans that reduce pathogen growth in all 5 stem cell transplant microbiomes. Reduction of pathogens was improved in patient 3 compared to the other patients. This patient was clinically distinct from the other patients in diagnosis (Hodgkin's Lymphoma vs AML/CML), transplant type (Allogeneic vs. Autologous), and conditioning intensity (non-myeloblative vs. full preparation) (Table 8). This suggests glycans can be used to reduce the growth of pathogenic bacteria in different patient populations.

**Table 8. Clinical characteristics of patients undergoing stem cell transplant that were used to assess glycan-mediated reduction in pathogen growth.**

| **Patient** | **Diagnosis** | **Diagnosis Group** | **Transplant Type** | **Conditioning Intensity** | **Conditioning Regimen** |
|---|---|---|---|---|---|
| 1 | acute myeloid leukemia | AML | Allogeneic | Non-myeloblative | FIu/Cy/TBI |
| 2 | acute myeloid leukemia | AML | Allogeneic | Non-myeloblative | Flu/Mel/Cy/TBI |
| 3 | nodular sclerosing Hodgkin's lymphoma | Hodgkin's Lymphoma | Autologous | Full Preparation | BEAM |
| 4 | chronic myelogenous leukemia, BCR/ABL+ | CML | Allogeneic | Non-myeloblative | Flu/Cv/TBI |
| 5 | acute myeloid leukemia | AML | Allogeneic | Non-myeloblative | Flu/Cy/TBI |

### Example 8. Collection of fecal samples

Fecal samples were collected by providing subjects with the Fisherbrand Commode Specimen Collection System (Fisher Scientific) and associated instructions for use. Collected samples were stored with ice packs or at -80° C until processing (Mclnnes & Cutting, Manual of Procedures for Human Microbiome Project: Core Microbiome Sampling Protocol A, vl2.0, 2010, hmpdacc.org/doc/HMP_MOP_Versionl2_0_O72910.pdf). Alternative collection devices may also be used. For example, samples may be collected into the Faeces Tube 54x28mm (Sarstedt AG, 25ml SC Feces Container w/Scoop), Globe Scientific Screw Cap Container with Spoon (Fisher Scientific) or the OMNIgene-GUT collection system (DNA Genotek, Inc.), which stabilizes microbial DNA for downstream nucleic acid extraction and analysis. Aliquots of fecal samples were stored at -20 °C and -80 °C following standard protocols known to one skilled in the art.

### Example 9. Determining the level of pathogens in subjects.

To determine the titer of pathogens carried in the gastrointestinal tract, fecal samples or rectal swabs are collected by a suitable method. Sample material is cultured on, e.g., i) Cycloserine-Cefoxitin Fructose Agar (available for instance from Anaerobe Systems) cultured anaerobically to selectively and differentially grow *Clostridium difficile*; ii) Eosin Methylene Blue Agar (available for instance from Teknova) cultured aerobically to titer *Escherichia coli* and other Gram-negative enteric bacteria, most of which are opportunistic pathogens; iii) Bile Esculin Agar (BD) cultured aerobically to titer Enterococcus species; iv) phenyl-ethylalcohol blood agar (Becton Dickinson), or Colistin-Nalidixic Acid (CNA) blood agar (for instance, from Hardy Diagnostics) cultured aerobically to grow Enterococcus and/or Streptococcus species; v) Bifidobacterium Selective Agar (Anaerobe Systems) to titer Bifidobacterium species; vi) or MacConkey Agar (Fisher Scientific) to titer *E. coli* and other Gram-negative enteric bacteria. Additional antibiotics can be used as appropriate to select drug-resistant subsets of these bacteria, for instance vancomycin (e.g., for vancomycin-resistant Enterococcus), cefoxitin (e.g., for extended spectrum beta lactamases or Enterococcus), ciprofloxacin (e.g., for fluoroquinolone resistance), ampicillin (e.g., for ampicillin resistant bacteria), and ceftazidime (e.g., for cephalosporin resistant bacteria). Additionally, chromogenic substrates may be added to facilitate the differentiation of pathogens from commensal strains, such as with ChromID plates (Biomerieux) or ChromAgar (Becton Dickinson). Plates are incubated at 35-37°C under aerobic, anaerobic or microaerophilic conditions as appropriate for the pathogen. After 16-48 hours, colonies are counted and used to back-calculate the concentration of viable cells in the original sample.

For quantitative assessment, the subjects sample volume or weight is measured, and serial 1:10 dilutions prepared in phosphate buffered saline or other diluent, followed by plating, growth and counting of colonies to determine the level of a pathogen in a sample.

Alternatively, the quantity of a pathogen is measured by quantitative PCR. For this method, primers specific to one or more of the pathogens (including bacterial pathogens, viral pathogens and pathogenic protozoa) described herein are designed and used in a real-time quantitative PCR (for instance, using a PCR reaction to which a double-stranded-specific fluorescent dye such as Sybr Green, or a sequence-specific Taqman probe (Applied Biosystems/Thermo Scientific). Genomic DNA is extracted from each sample using the Mo Bio Powersoil®-htp 96 Well Soil DNA Isolation Kit (Mo Bio Laboratories, Carlsbad, Calif.) according to the manufacturer's instructions or by bead beating, e.g., performed for 2 minutes using a BioSpec Mini-Beadbeater-96 (BioSpec Products, Bartlesville, Okla.). Alternatively, the genomic DNA is isolated using the Mo Bio Powersoil® DNA Isolation Kit (Mo Bio Laboratories, Carlsbad, Calif.) or the QIAamp DNA Stool Mini Kit (QIAGEN, Valencia, Calif.) according to the manufacturer's instructions. The cycle threshold of a sample of a subject in quantitative PCR is then compared to a standard curve of known quantities of pathogens to determine the level of pathogen in the sample. The development of assays is described (e.g., in "Application of the fluorogenic probe technique (TaqMan PCR) to the detection of Enterococcus spp. and Escherichia coli in water samples", Edith Frahm and Ursala Obst, J. Microbiol. Meth. 2003 Jan;52(1):123-31.). Alternatively, to simplify assay design, analyte-specific reagents are available for many of the pathogens, for instance from Luminex, Inc (www.luminexcorp.com). Alternatively, or in addition, universal ribosomal primers are used to quantitatively measure the total copy number of genomes from pathogens to determine relative instead of absolute abundance of pathogens. If desired, the ratio of pathogen to total copies is calculated. The colony counts can be normalized (e.g., a ratio is calculated) to the total DNA content of the sample, or to the quantitative measure, e.g., determined by a qPCR using universal ribosomal primers.

Alternatively, the colony count of a pathogen, or all pathogens combined, is compared to the total colony count of the sample cultured under non-selective conditions. Samples are cultured on rich media or agar such as Brucella Blood Agar (Anaerobe Systems), Brain Heart Infusion Broth (Teknova), or chocolate agar (Anaerobe Systems). The maximum number of colonies on these media, grown anaerobically are used as the denominator in a normalized ratio of pathogens to commensals as a relative measure.

The amount of pathogen may also be estimated by 16s ribosomal DNA profiling. Genomic DNA is extracted from subject samples (e.g. fecal samples, rectal swabs, skin or mucosal swabs, biopsies or tissue samples), and variable region 4 of the 16S rRNA gene is amplified and sequenced (Earth Microbiome Project protocol www.earthmicrobiome.org/emp-standard-protocols/16s/ and Caporaso JG et al. 2012. Ultra-high-throughput microbial community analysis on the Illumina HiSeq and MiSeq platforms. ISME J.). Operational Taxonomic Units (OTUs) are generated by aligning 16S rRNA sequences at 97% identity, or lower as appropriate. Then the OTUs potentially representing pathogenic species are assessed by aligning the OTUs to known taxonomic structures such as those maintained by NCBI (ncbi.nlm.nih.gov) or the Ribosomal Database Project (https://rdp.cme.msu.edu), and their abundance estimated, for instance as a ratio of number of pathogen sequences to total number of sequences.

### Example 10. Assessment of pathogen level and/or toxicity reduction in the presence of glycans in an animal model

To test the therapeutic potential of a glycan preparation for reducing the level of pathogens and/or pathogen toxicity in a subject (e.g., an ICU patient), various animal models may be used. In one animal model, pathogenic *E. coli* translocation from the intestines to other organs (Green et al., Infect. Immun. (2015) 83(8):3243-3256) are analyzed in a chemotherapy-induced mouse model in the presence or absence of glycans. An extraintestinal pathogenic E. coli (ExPEC) strain (e.g., CP9) is engineered with a resistance marker (rifamipicin) through P1 phage transduction. Mice are administered an oral gavage of innoculates (*E. coli* CP9 or vehicle) at 10⁹ cells in a 0.1 ml volume. Mice are then injected i.p. with 150mg/kg of cyclophosphamide (CTX) or vehicle every other day (q.a.d) for three days. Glycan compound administration is performed via oral gavage daily from day -1 to day 6. Groups not receiving glycans are administered sham gavages on day - 1 through the end of the study. Fecal pellets are collected and bacterial growth (CFUs) is assessed using selection (rifampicin) to measure only CP9 strains. CFUs are counted every day beginning one day after innoculation via oral gavage (Day 1). Animals are assessed daily for health using the following score: rough coat (score of 0 to 1), hunched posture (0 to 1), lethargy (0 to 1), and hyperpnea (0 or 1). Increments of increasing severity of disease will be quantified in intervals of 0.5 except with hyperpnea, which is given a score of 1 (yes) or 0 (no). On day 7, mice are euthanized and organ homogenates are prepared from cecum content, liver, lung, kidneys, and spleen to be used for quantifying bacterial infection by counting colony forming units (CFU) on an antibiotic containing plate to select for the administered *E. coli.* Cecal contents will also be plated on agar to count total aerotolerant bacteria.

In another animal model, re-colonization of gut microbiome with pathogenic bacterial isolates (Caballero et al., PLoS Pathog. (2015) 11 (9):e1005132) is analyzed for the effect of glycans antibiotic resistant bacteria (e.g., VRE *Enterococcus faecium* and CRE *Klebsiella pneumoniae*)*.* Bacterial strains are grown at 37°C in Brain Heart Infusion (VRE) or Luria-Bertani (*K. pneumoniae*) broth to early stationary phase and OD600 recorded. The culture is spun down, washed with PBS, spun down, and then resuspended in phosphate buffered solution (PBS). For the pathogen cocktail of VRE and *K. pneumoniae,* inocula are mixed in a 1:1 ratio prior to administration (100 ul of Kpn: 100 ul VRE). Initial inocula are plated on CHROM_KPC, CHROM_VRE, and MacConkey to determine starting CFUs.

Six to eight-week-old C57BL/6J mice are acclimated for up to 5 days on provided control/matched chow. The animals have free access to food and water. Mice are singly housed throughout the course of the study in a temperature and humidity-controlled room with a 12-hour light cycle. Seven groups of eight animals are utilized for the study. Group 7 contains 5 animals receiving fresh fecal pellets for fecal microbiota transplantation (FMT) dosage. Groups 1-6 receive ampicillin drinking water (0.5 g/L) on day -5 and continue for 8 days. On day 0, groups 1-6 receive the pathogen cocktail, administered through PO dosing. At Day 3, groups 1-6 are taken off antibiotic water. Group 1 receives sham gavages until the end of the study. Group 2 receives glycan treatment (glycan #1) until end of study. Group 3 receives glycan treatment (glycan #2) until end of study. Group 4 receives glycan treatment (glycan #3) until the end of the study. Group 5 is switched to pullulan chow (glycan #4), receiving sham gavages until the end of the study. Group 6 receives FMT treatment for 3 days (days 3-5) and receives sham gavages from days 6-14. CFU fecal sampling occurs on Day 0, 3, 6, 8, 10, 12, and 14. For CFU determination, feces are collected on days 0, 3, 6, 8, 10, 12, and 14. One to two pellets are collected from each animal, weighed and placed in a 14mL round bottom falcon tube with 1 mL of sterile saline. The sample is then homogenized to a uniform consistency and serially diluted. The diluted samples are plated on selective growth media using a spot plate technique. CFUs are enumerated after overnight incubation (37°C).

All fecal samples are plated on CHROM_KPC, CHROM_VRE, and MacConkey plates. In addition, 16S fecal sampling occurs on Day -7, -5,0, 3, 6, 8, 10, 12, and 14. Fecal samples are collected on Days -7, -5, 0, 3, 6, 8, 10, 12, and 14 for 16S analysis. One fecal pellet is placed into a well of a 96-well MoBio plate and kept on dry ice throughout the collection period and then frozen at -80°C. once all collections have been performed.

For the Fecal microbiota transplantation (FMT), 3 fresh fecal pellets in total are collected from 3 untreated C57BL/6 mice each day of FMT dosage. Pellets are resuspended in 3ml of PBS. After allowing for particulate matter to settle (approximately 5 minutes), 200µl of the fecal suspension is administered per mouse via oral gavage.

### Example 11. Ex vivo screening of glycan preparations

A high-throughput screen consisting of 435 total batches of test compositions including over 300 glycan preparations as described herein and commercially available test compounds (e.g., dietary fibers) was performed to identify glycan preparations capable of modulating (*e.g*., reducing) the growth rates of exemplary MDR pathogens, including vancomycin-resistant enterococcus (VRE) and carbepenam-resistant enterobacteriaceae (CRE) strains. Further, the ability of these glycan preparations to support commensal growth, thereby reducing the proportion of pathogens in a community and thus carrier load and the ability to spread pathogens, was also tested.

### Primary screen

The primary screen involved the anaerobic incubation of fecal slurries from healthy subjects (human subjects who were not suffering from a disease) for 24 to 42 hours with or without pathogens (Figure 11). Specifically, populations of seven VRE *E. coli* (incl. strains 131 and 648); ten CRE *K. pneumoniae* (incl. strains 258, 11, 340, and 437); eleven VRE *E. faecium* (incl. strains 78 and 45); and two C. difficile strains were uniquely added to healthy fecal slurries for testing.

Fecal samples were stored at -80°C. To prepare the fecal material for use in the ex *vivo* assay, it was moved into an anaerobic chamber and made into a 20% w/v slurry in phosphate buffered saline with 1 5% glycerol added. Aliquots of this 20% slurry + 15% glycerol solution were stored at -80°C.

On the day of the experiment, an aliquot of the 20% slurry + 15% glycerol was moved into an anaerobic chamber. The aliquot was centrifuged at 2,000xg, the supernatant was removed, and the pellet was resuspended in either a Minimal Medium (MM) containing 10g/L tryptone peptone, 5g/L yeast extract, 4.1mM L-cysteine, 100mM potassium phosphate buffer (pH 7.2), 0.008mM magnesium sulfate, 4.8mM sodium bicarbonate, 1.37mM sodium chloride, 5.8mM vitamin K, 0.8% calcium chloride, 1.44mM iron (II) sulfate heptahydrate, 4mM resazurin, 0.1% histidine-hematin, 1% ATCC trace mineral supplement, 1% ATCC vitamin supplement, 29.7mM acetic acid, 0.9mM isovaleric acid, 8.1mM propionic acid, and 4.4mM N-butyric acid with the pH adjusted to 7 using sodium hydroxide or in Clostridial Minimal Media (CM), which contains 900 mg/L sodium chloride, 26 mg/L calcium chloride dihydrate, 20 mg/L magnesium chloride hexahydrate, 10 mg/L manganese chloride tetrahydrate, 40 mg/L ammonium sulfate, 4 mg/L iron sulfate heptahydrate, 1 mg/L cobalt chloride hexahydrate, 300 mg/L potassium phosphate dibasic, 1.5 g/L sodium phosphate dibasic, 5 g/L sodium bicarbonate, 0.125 mg/L biotin, 1 mg/L pyridoxine, 1 mg/L pantothenate, 75 mg/L histidine, 75 mg/L glycine, 75 mg/L tryptophan, 150 mg/L arginine, 150 mg/L methionine, 150 mg/L threonine, 225 mg/L valine, 225 mg/L isoleucine, 300 mg/L leucine, 400 mg/L cysteine, and 450 mg/L proline (Theriot CM et al. Nat Commun. 2014; 5:3114) which was supplemented with 0.1% peptone and 0.75mM urea. Prior to use in this assay, these media were filter sterilized using a 0.2um filter and stored in an anaerobic chamber to allow any dissolved oxygen to dissipate. The resuspended pellet was then further diluted into a 1% solution in either CM or MM.

One day prior to the start of the experiment, a single strain of carbapenem-resistant Enterobacteriaceae or vancomycin-resistant Enterococcus was grown in isolation overnight in CM or MM with 0.5% D-glucose in an anaerobic chamber. On the day of the experiment, aliquots of the overnight culture were washed with phosphate buffered saline and the optical density (OD600) of the culture was measured. The culture was adjusted to OD 0.01 in CM or MM. The normalized pathogen culture was then added to the 1% fecal slurry so that it made up 4%-12% of the final volume of the culture. A sample of this mixed culture was subjected to 16S sequencing to determine the initial relative abundance of pathogen in the community. The fecal slurry with the spiked pathogen was added to 96 well microplates with a test composition as the sole carbon source in each well. Water was added to CM or MM without any added carbon source as a control. The final concentration of each test composition in the assay was 0.05% or 0.5%. Each test composition was represented 3 times within each assay plate. Plates were incubated at 37°C in an anaerobic chamber for a total of 24-45 hours. At the end of the 24-45 hour incubation, the plates were removed from the anaerobic chamber. The OD600 was measured every 15 minutes generating a growth curve for each well using the Biotek BioStack microplate stacker and Biotek Powerwave HT microplate spectrophotometer. The time to midlog was calculated for the growth curve generated for each strain and test composition combination using the growthcurver library in RStudio.

After initial anaerobic growth of a pathogen in a fecal slurry, test compositions, including glycan preparations, were transferred to the fecal slurries and aerobic growth curves were measured over a 45-hr time course. Commensal strains in the fecal slurry communities are strict. anaerobes and thus do not grow under aerobic conditions. Anaerobic OD600 (Optical density at 600 nm) and time to midlog growth phase, calculated from aerobic growth curves, allowed selection of glycan preparations that exclusively support commensal growth (e.g., high anaerobic OD600 and long time to midlog) (Figure 12). Results from this initial screening effort showed a wide range of pathogen reduction and commensal support for selected test compositions, including glycan preparations. In total, 109 of 435 test compositions, including 55 glycan preparations were selected for further investigation.

### Secondary screen

The 55 glycan preparations selected for further investigation were advanced into a secondary screening phase to determine their ability to support or promote the growth of isolated (single-strain) pathogens in rich or minimal media (depending on the pathogen).

Individual pathogenic bacterial strains including CRE *Escherichia coli,* CRE *Klebsiella pneumoniae,* and *Clostridium difficile,* were grown in clostridial medium and single strain VRE *Enterococcus faecium,* were grown in minimal medium prior to the addition of single glycan preparations.

Individual pathogenic strains including *Escherichia coli* and *Klebsiella pneumoniae* when grown in Clostridial Minimal Media; *Clostridium difficile* when grown in Clostridial Minimal Media; and single strain vancomycin-resistant bacteria, including *Enterococcus faecium* when grown in Minimal Media were exposed to glycan or water (e.g., no carbon control). *Enterococcus faecium* were grown in media (e.g., Minimal Media (MM)) containing 10g/L tryptone peptone, 5g/L yeast extract, 4.1mM L-cysteine, 100mM potassium phosphate buffer (pH 7.2), 0.008mM magnesium sulfate, 4.8mM sodium bicarbonate, 1.37mM sodium chloride, 5.8mM vitamin K, 0.8% calcium chloride, 1.44mM iron (II) sulfate heptahydrate, 4mM resazurin, 0.1% histidine-hematin, 1% ATCC trace mineral supplement, 1% ATCC vitamin supplement, 29.7mM acetic acid, 0.9mM isovaleric acid, 8.1mM propionic acid, 4.4mM N-butyric acid with the pH adjusted to 7 using sodium hydroxide. This media was filter sterilized using a 0.2um filter and stored in an anaerobic chamber prior to use to allow any dissolved oxygen to dissipate.

Single strain carbapenem-resistant bacteria, (e.g., Escherichia coli and Klebsiella pneumoniae) and *Clostridum difficile* when grown in Clostridial Minimal Media, which contains 900 mg/L sodium chloride, 26 mg/L calcium chloride dihydrate, 20 mg/L magnesium chloride hexahydrate, 10 mg/L manganese chloride tetrahydrate, 40 mg/L ammonium sulfate, 4 mg/L iron sulfate heptahydrate, 1 mg/L cobalt chloride hexahydrate, 300 mg/L potassium phosphate dibasic, 1.5 g/L sodium phosphate dibasic, 5 g/L sodium bicarbonate, 0.125 mg/L biotin, 1 mg/L pyridoxine, 1 mg/L pantothenate, 75 mg/L histidine, 75 mg/L glycine, 75 mg/L tryptophan, 150 mg/L arginine, 150 mg/L methionine, 150 mg/L threonine, 225 mg/L valine, 225 mg/L isoleucine, 300 mg/L leucine, 400 mg/L cysteine, and 450 mg/L proline (Theriot CM et al. Nat Commun. 2014; 5:3114) (CM). This media was filter sterilized using a 0.2um filter and stored in an anaerobic chamber prior to use to allow any dissolved oxygen to dissipate.

Single strains of *E. coli* (BAA-2340, BAA-97, 4 strains isolated from patients, and ECO.139), *K. pneumoniae* (ATCC 33259, BAA-1705, BAA-2342, and 7 strains isolated from patients), and *C. difficile* were grown in isolation overnight in CM with 0.5% D-glucose in a COY anaerobic chamber. Single strains of *E. faecium* (ATCC 700221 and 9 strains isolated from patients, and EFM.70), were grown in isolation overnight in MM media with 0.5% D-glucose in a COY anaerobic chamber. 1 mL of each overnight culture was washed with phosphate buffered saline and the optical density (OD600) of each culture was measured. Each culture was adjusted to OD 0.01 in media (e.g., CM or MM).

Inside of the COY anaerobic chamber, the normalized single strain cultures of *E. coli* or *K. pneumoniae* were added to 96 well microplates with one of the following glycan preparations as the sole carbon source in each well: glu50gal50-22, glu50gal50-23, glu33gal33xyl33-3, ga160man40-1, glu10gal45man45-1, man80glu20-1, glu50gal50-33, glu50gal50-41, ara88gal3rha2galA3-3, glu10gal80man10-3, glu10gal80man10-4, glu5gal5man90-5, glu50gal50-1, glu100-11, glu10gal80man10-5, glu50gal50-21, gal85ara15-6, man80glu20-5, glu5gal5man90-3, glu100-94, glu50gal50-46, man52glu29gal19-1, glu100-21, glu50gal50-51, glu50gal50-24, glu60man40-4, glu50gal50-50, glu45gal45man10-5, glu50gal50-15, glu50gal50-29, glu45gal45man10-3, man80glu20-6, glu50gal50-14, glu50gal50-49, gal100-8, glu50gal50-11, and glu50gal50-3.

Inside of the COY anaerobic chamber, the normalized single strain cultures of *C.difficile* were added to 96 well microplates with one of the following glycan preparations as the sole carbon source in each well: ara100-11, glu45gal45man10-21, glu45ga145man10-12, glu10gal80man10-2, glu45gal45man10-15, glu5gal90man5-1, glu45gal45man10-17, glu45gal45man10-23, glu45gal45man10-18, glu33gal33ara33-2, glu45gal45man10-22, glu45gal45man10-20, glu45gal45man10-16, glu33gal33xyl33-3, glu25gal25man25ara25-2, glu45gal45man10-11, gal85ara15-3, glu45gal45man10-13, glu50gal50-55, gal100-8, gal85aral5-6, glu45gal45man10-2, glu45gal45man10-8,glu50gal50-56, glu100-94, glu50gal50-58, water (e.g., no carbon control), glu45gal45man10-19, glu100-20, glu50gal50-23, glu50gal50-24, glu100-11, gal33man33ara33-8, glu10gal80man10-7, glu100-99, glu50gal50-15, glu50gal50-57, glu10gal80man10-1, glu10gal80man10-4, glu100-21, glu50gal50-22, glu10gal80man10-3, glu45gal45man10-14, glu33gal33man33-4, gal33man33ara33-11, glu10gal80man10-8, glu45gal45man10-, glu45gal45man10-1, glu45gal45man10-3, glu50gal50-43, ara88gal3rha2galA3-4, glu100-3, glu10gal45man45-1, xyl100-7, glu10gal80man10-3, ara80xyl20-1, ara88gal3rha2galA3-3, gal60man40-1, man80gal20-2, glu10gal80man10-6, glu50gal50-27, ara88gal3rha2galA3-2, man52glu29gal19-1, glu50gal50-4, glu45gal45man10-9, glu50gal50-32, glu5gal5man90-1, man80glu20-1, glu45gal45man10-10, xyl100-9, glu100-90, glu45gal45man10-5, glu45gal45man10-6, glu50gal50-13, glu45gal45man10-4, glu50gal50-3, glu45gal45man10-7, glu5gal5man90-5, man80glu20-7, glu5gal5man90-4, man80glu20-5, man80glu20-4, glu5gal5man90-6, glu5gal5man90-3, and man80glu20-6.

Inside of the COY anaerobic chamber, the normalized single strain cultures of E. faecium were added to 96 well microplates with one of the following glycan preparations as the sole carbon source in each well: man80glu20-1, glu50gal50-58, glu50gal50-22, glu45gal45man10-8, glu5gal5man90-6, glu45gal45man10-17, glu45gal45man10-12, glu45gal45man10-21, glu45gal45man10-23, glu10gal80man10-7, glu50gal50-23, glu50gal50-56, glu50gal50-57, glu45gal45man10-19, glu45gal45man10-15, glu45gal45man10-22, glu45gal45man10-2, gal60man40-1, glu45gal45man10-16, glu5gal5man90-4, glu10gal80man10-1, glu5gal5man90-5, glu50gal50-24, glu45gal45man10-20, glu10gal80man10-6, man80glu20-4, glu45gal45man10-7, glu45gal45man10-1, man80glu20-5, glu10gal80man10-8, glu45gal45man10-14, glu45gal45man10-18, glu10gal45man45-1, man80glu20-7, glu45gal45man10-13, glu10gal80man10-3, glu5gal5man90-1, glu50gal50-27, glu50gal50-55, glu10gal80man10-4, glu45gal45man10-6, glu45gal45man10-11, glu5gal5man90-3, glu4sgal45man10-9, glu45gal45man10-10, gal33man33ara33-11, glu10gal80man10-5, ara88gal3rha2galA3-3, glu50gal50-27, man80glu20-6, glu45gal45man10-5, glu50gal50-13, glu45gal45man10-3, and glu45gal45man10-4.

Water added to media (*e.g.,* CM or MM) without any carbon source functioned as a control experiment. These microplates were then incubated at 37 °C in the COY anaerobic chamber for a total of 45 hours and the OD600 was measured every 15 minutes to generate a growth curve for each experimental well. Each glycan preparation was tested in three replicates against each bacterial pathogen.

The area under the curve (AUC) was calculated for the growth curve and a time-to-log2 was determined for each experiment (Figures 13A-13C). Desirable glycan preparations did not support pathogen growth of at least one pathogen e.g., the pathogen could not utilize the glycan preparation for fermentation and as a carbon source. Any and all glycan preparations that performed at levels comparable to and better than the water control in at least one of the three experiments (VRE, CRE, or *C. difficile*) are considered to be working and operable glycan preparations (e.g., all Glu50Gal50 preparations, and all Man80Glu20 preparations). Three exemplary glycan preparations (Glu50Ga150 (e.g., Glu50Gal50-22); Glu10Gal80Man10 (e.g., Glu10Gal80Man10-1); and Glu45Gal45Man10, (e.g., Glu45Gal45Man10-1)) were determined to not support VRE and/or CRE pathogen growth and further did not support *C*. *difficile* growth and provided high alpha-diversity. These results suggest that glycan preparations that do not support pathogen growth may be used to disadvantage pathogen growth by selectively favoring growth of commensal bacteria.

### Example 12. Assessment of selected glycan preparation in hospitalized patients

In addition to evaluating the performance of glycan preparations in healthy subject microbiome samples supplemented with pathogens, the ability of selected glycan preparations (*e.g.,* Glu50Gal50; Glu10Gal80Man10; and Glu45Gal45Man10) for their ability to reduce pathogen growth in microbiome samples from fecal slurries of thirteen hospitalized patients receiving antibiotic treatment from an Intensive Care Unit (ICU) facility was assessed.

Fecal samples from ICU patients and healthy subjects were collected and stored at -80 °C. To prepare the fecal material for use in the *ex vivo* assay, it was moved into a AS-580 anaerobic chamber and made into a 30 % w/v slurry in phosphate buffered saline. This slurry was then further diluted into a 1% solution of media (*e.g.,* CM or MM, as used in Example 11). A single strain of CRE *Escherichia coli* was grown in isolation overnight in either CM or MM with 0.5% D-glucose in a AS-580 anaerobic chamber. On the day of the experiment, aliquots of the overnight culture were washed with phosphate buffered saline and the optical density (OD600) of the culture was measured. The culture was adjusted to OD600 of 0.01 in either CM or MM and added to the 1% fecal slurry. A sample of this mixed culture of CRE *Escherichia coli* and fecal slurry was then subjected to 16S sequencing to determine the initial relative abundance of pathogen and commensal bacteria. The mixed culture was then added to 96-well microplates with one of the following carbon sources (final concentration of 0.5% w:v) in each well: maltodextrin, fructooligosaccharide, glu45gal45man10-1, glu10gal80man10-1, gal60man40-1, glu5gal5man90-1, glul0gal45man45-1, and glu50gal50-22. Water added to media (*e.g.,* CM or MM) without any carbon source functioned as a control experiment. These microplates were then incubated at 37 °C in the COY anaerobic chamber for a total of 45 hours and the OD600 was measured every 15 minutes to generate a growth curve for each experimental well. Each glycan preparation was tested in three replicates against each bacterial pathogen.

A single strain of VRE *Enterococcus faecium* was grown in isolation overnight in either CM or MM with 0.5% D-glucose in a AS-580 anaerobic chamber. On the day of the experiment, aliquots of the overnight culture were washed with phosphate buffered saline and the optical density (OD600) of the culture was measured. The culture was adjusted to OD600 of 0.01 in either CM or MM and added to the 1% fecal slurry. A sample of this mixed culture of VRE *Enterococcus faecium* and fecal slurry was then subjected to 16S sequencing to determine the initial relative abundance of pathogen and commensal bacteria. The mixed culture was then added to 96-well microplates with one of the following carbon sources (final concentration of 0.5% w:v) in each well: maltodextrin, fructooligosaccharide, glu45gal45man10-1, glu10gal80man10-1, glu10gal80man10-3, glu10gal80man10-4, ara88gal3rha2galA3-3, gal60man40-1, glu5gal5man90-1, glu10gal45man45-1, man80glu20-1, gal33man33ara33-11, and glu50gal50-27. Water added to media (*e.g*., CM or MM) without any carbon source functioned as a control experiment. These microplates were then incubated at 37 °C in the COY anaerobic chamber for a total of 45 hours and the OD600 was measured every 15 minutes to generate a growth curve for each experimental well. Each glycan preparation was tested in three replicates against each bacterial pathogen.

At the end of the 45-hour incubation, a sample of the culture from each well was subjected to 16S sequencing to determine the final relative abundance of pathogen and commensal bacteria in the community after intervention with glycan preparation.

For the 16S sequencing, genomic DNA was extracted from the fecal slurries and variable region 4 of the 16S rRNA gene was amplified and sequenced (Earth Microbiome Project protocol www.earthmicrobiome.org/emp-standard-protocols/16s/ and Caporaso JG et al. Ultrahigh-throughput microbial community analysis on the Illumina HiSeq and MiSeq platforms. ISME J. (2012) Aug;6(8):1621-4). Raw sequences were demultiplexed, and each sample was processed separately with UNOISE2 (Robert Edgar UNOISE2: improved error-correction for Illumina 16S and ITS amplicon sequencing. bioRxiv (2016) Oct. 15). Reads from 16S rRNA amplicon sequencing data were rarefied to 5000 reads, without replacement, and resulting OTU table used in downstream calculations.

Using 16S data to compare the community differences between healthy and ICU patients, alpha diversity was calculated using the Vegan package in R (box plot to the left) (Figure 14 left panel). The use of alpha diversity metrics were employed to examine the richness and/or evenness of a community. Here, results from the Shannon Index indicated ICU patients have communities that are considerably less even. Community richness, or the number of unique taxa within a sample, was also considerably lower in ICU donors. The use of beta diversity measures were typically used to investigate differences between samples. Here, inter-sample differences were visualized with a multidimensional scaling ordination plot which is based off of a Bray-Curtis dissimilarity calculation (ordination plot to the right). The ordination showed substantial differences between healthy and diseased cohorts (Figure 14, right panel). There was also considerable heterogeneity throughout the ICU cohort (e.g., dots are more spread relative to healthy donors). Collectively, the data shows ICU patients harbor low diversity communities that are different from healthy donors. The distance between these community types offers an opportunity to shift low diversity ICU patients to a healthier, more diverse state free of dominant pathogens.

The fold reduction in abundance of CRE pathogen for each glycan preparation and patient sample relative to control was determined and are shown in Figure 16A (Glu45Gal45Man10), Figure 16B (Glul0Gal80Man10) and Figure 16C (Glu50Gal50). Glycan preparations exhibited higher reduction in CRE abundance than did commercially available control fibers, maltodextrin and fructooligosaccharide (FOS) (Figure 15). This demonstrates that glycan preparations may be capable of reducing or preventing growth of pathogens such as CRE

### E. coli,

The fold reduction in abundance of VRE pathogen for each glycan preparation and patient sample relative to control was determined and are shown in Figure 18A (Glu45Gal45Man10) and Figure 18B (Glu10Gal80Man10). Glycan preparations exhibited higher reduction in VRE abundance than did commercially available control fibers, maltodextrin and fructooligosaccharide (FOS) (Figure 17). This demonstrates that glycan preparations may be capable of reducing or preventing growth of pathogens such as VRE *Enterococcus faecium.*

Further, glycan preparations were able to significantly reduce the relative abundance of pathogens in samples from 10 of the 13 patients. In the three non-responsive patient samples, there were no detectable levels of viable commensal bacteria for which the glycan preparation could support growth (those patients had no viable commensal bacteria in their stool). This suggests that combining the administration of glycan preparations with the administration of commensal strains could benefit patients with depleted microbiomes, such as in patients receiving antibiotics. Such a synbiotic approach could have a synergistic effect on pathogen reduction.

### Example 13. A synbiotic approach to pathogen reduction in non-responding ICU patients

In Example 12, in some fecal communities from pathogen-colonized ICU patients no significant reduction in pathogen abundance was observed in the presence of glycan preparations. It was determined that the fecal samples from these non-responder patients substantially lacked commensal bacteria. Under one theory, commensal bacteria are capable of outcompeting pathogens in growth in the presence of suitable glycan preparations. The ability of commensal bacteria to reduce pathogen abundance in non-responder fecal samples, once added into the community, was examined using an *in vitro* assay (referred to as an *ex vivo* assay in Example 12). These commensals were added to fecal samples with an exemplary glycan preparation to determine whether the presence of a glycan preparation further reduces pathogen levels.

Fecal samples from non-responding ICU patients of Example 12, colonized by Enterobacteriaceae, were stored at -80°C. To prepare the fecal material for use in the *ex vivo* assay, it was transferred into an anaerobic chamber and made into a 20% w/v slurry in phosphate buffered saline with 15% glycerol added. Aliquots of this 20% slurry + 15% glycerol solution were stored at -80°C.

On the day of the experiment, an aliquot of the 20% slurry + 15% glycerol was transferred into an anaerobic chamber. The aliquot was centrifuged at 2,000xg, the supernatant was removed, and the pellet was resuspended in phosphate buffered saline (PBS). The resuspended pellet was then further diluted into a 1% solution in a medium containing 10g/L tryptone peptone, 5g/L yeast extract, 4.1 mM L-cysteine. 100mM potassium phosphate buffer (pH 7.2), 0.008mM magnesium sulfate, 4.8mM sodium bicarbonate, 1.37mM sodium chloride, 5.8mM vitamin K, 0.8% calcium chloride, 1.44mM iron (II) sulfate heptahydrate, 4mM resazurin, 0.1% histidine-hematin, 1% ATCC trace mineral supplement, 1 % ATCC vitamin supplement, 29.7mM acetic acid, 0.9mM isovaleric acid, 8.1mM propionic acid, and 4.4mM N-butyric acid with the pH adjusted to 7 using sodium hydroxide (MM). Prior to use in this assay, media was filter sterilized using a 0.2um filter and stored in an anaerobic chamber to allow any dissolved oxygen to dissipate.

One day prior to the start of the experiment, single strains of *Blautia hansenii, Blautia producta, Clostridium scindens, Parabacteroides distasonis,* and *Bacteroides thetaiotamicron* were grown in isolation overnight in media with 0.5% D-glucose in an anaerobic chamber. Aliquots of the overnight culture were centrifuged and washed with PBS. The optical density (OD600) of each culture was measured. Each culture was adjusted to OD 0.01 in media. A combination of the normalized bacterial cultures was then added to the 1% fecal slurry so that it made up 4% of the final volume of the culture. The combinations included 1) an equal proportion of *Blautia hansenii, Blautia producta,* and *Clostridium scindens* (Bacterial Community A), 2) an equal proportion of *Parabacteroides distasonis* and *Bacteroides thetaiotamicron* (Bacterial Community B), and 3) an equal proportion of *Blautia hansenii, Blautia prociticia, Clostridium scindens, Parabacteroides distasonis,* and *Bacteroides thetaiotamicron* (Bacterial Community A + B). No Bacterial Community was also tested as a negative control for pathogen reduction. A sample of each mixed culture was subjected to 16S sequencing to determine the initial relative abundance of pathogens in the community. The fecal slurry with the spiked commensal bacteria was added to 96 well microplates with a glycan preparation, Man80Glu20, as the sole carbon source in each well. Water was added to media without any added carbon source as a control. The final concentration of glycan preparation in the assay was 0.5%. Glycan preparation was represented 3 times within each assay plate. Plates were incubated at 37°C in an anaerobic chamber for a total of 45 hours. At the end of the 45-hour incubation, the plates were removed from the anaerobic chamber. The remaining culture from each well was subjected to 16S sequencing as described in Example 12 to confirm the final relative abundance of pathogen in the community after glycan preparation intervention.

Figure 19, the left panel shows no significant reduction in pathogen abundance in the presence of glycan in a fecal community from a pathogen-colonized ICU patient non-responder, in agreement with the experiment described in Example 12. The addition of bacterial community A, B and A+B (commensal bacteria) in the absence of a glycan preparation (only water added: "water") lead to a strong reduction in pathogen (Enterobacteriaceae) growth (Fig. 19, second, third, fourth panel from the left). Combining Man80Glu20 with commensal communities A, B and A+B ("Man80Glu20") reduced the abundance of Enterobacteriaceae pathogen even further. The effect became more pronounced as the numbers of commensals in the bacterial community increased. This suggests that glycan preparations may be combined with commensal bacteria to reduce pathogen levels in non-responding patients, e.g., those substantially without commensal bacteria in their gut microbiome, e.g., in a synbiotic approach.

### Example 14: Production of a glu100 glycan preparation at 100g scale from dextrose monohydrate or 70DS corn dextrose syrup

A procedure was developed for the synthesis of glu100 glycan preparations (described in Table 4a and 4b, e.g., Glu 100-94 and Glu 100-5, two batches of the same glu100 preparation) at a 100 gram scale. The procedure was developed to allow for synthesis stalling from either dextrose monohydrate or corn dextrose syrup, as described below. The procedure utilized a multi-neck reaction vessel with the heating mantle configured with an overhead stirrer. A probe thermocouple was disposed in the vessel through a septum, such that the probe tip sat above the stir blade and not in contact with the walls of the reaction vessel.

The procedure utilizes D(+) glucose, either as: Dextrose monohydrate (100 grams, dry solids basis) or 95DE, 70DS Corn dextrose syrup (100 grams, dry solids basis). For production using dextrose monohydrate the condenser was configured initially in a re-flux reaction configuration. For production using 70DS corn dextrose syrup, the apparatus was configured initially for distillation.

The procedure also used an oligomerization catalyst (Dowex Marathon C) (7 grams, dry basis) and de-ionized water for quenching.

According to the procedure, the multi-neck reaction vessel was first charged with 109.9 g dextrose monohydrate powder (or 142.9 g of 70DS 95 DE corn syrup) to provide 100 g dry glucose to the reaction.

The temperature controller was set to 130 °C, and stirring of the contents of the vessel was initiated to promote uniform heat transfer and melting of the sugar solids, as the temperature of the syrup was brought to approximately 130 °C, under ambient (atmospheric) pressure.

When starting with dextrose monohydrate, once at approximately 130 °C, the condenser reflux system was switched to a distillation configuration.

Next, the vessel was charged with 7 grams (dry solids basis) of catalyst to generate the reaction mixture. In some cases, the catalyst was handled in wet form, e.g., at a nominal moisture content of 45 - 50 wt% H₂O. The exact catalyst moisture content was generally determined on a per-experiment basis using, for example, using a moisture analyzing balance (e.g., Mettler-Toledo MJ-33).

Upon addition of the catalyst, the system was maintained at approximately 130 °C under continuous mixing for about 4 hours, determined by following the reaction by HPLC. Next, the heat was turned off while maintaining constant stirring.

The reaction was then quenched by slowly adding approximately 60ml of hot (∼80 °C) deionized (DI) water to dilute and cool the product mixture, to target a final concentration of 70 wt % dissolved solids. Generally, the water addition rate was performed to control the mixture viscosity as the glycan preparation was cooled and diluted.

Following dilution, the glycan preparation was cooled to approximately 60°C. The catalyst was then removed by vacuum filtration through a 100 micron mesh screen or fritted-glass filter, to obtain the final glycan preparation.

### Example 15: Production of glycan preparations at 10 kg scale from dextrose monohydrate

The present example demonstrates the synthesis of glu100 glycan preparations (described in Table 4a and 4b, e.g., Glu100-94 and Glu100-5, two batches of the same glu100 preparation) at 10 kg scale in a 22 L horizontal-mixed reactor.

About 10 kg of food grade dextrose monohydrate was charged into a 22 L horizontal plough mixer (Littleford-Day, Lexington, KY) equipped with a hot-oil jacket. The dextrose was melted by gradually heating to a temperature of about 120°C with continuous mixing at 30 RPM. 1.27 kg (0.70 kg on a dry solid basis) solid acid catalyst (poly-styrene-co-divinylbenzene comprising > 3.0 mmol/g sulfonic acid moieties and < 1.0 mmol/gram cationic moieties) was then added to the reaction mixture to form a mixed suspension. The reaction temperature was gradually increased to about 130 °C at atmospheric pressure over a three hour period with continuous mixing, maintained at 30 RPM. The reaction was maintained at temperature of 130°C for seven hours. Hot de-ionized water was then gradually added to the reaction mixture at a rate of 6 mL/min until the temperature of the reactor contents decreased to 120 °C, then at 150 mL/min until the temperature of the reactor contents decreased to 110 °C, then at 480 mL/min until a total of 6 kg of water was added and the temperature of the reactor contents decreased below 100 °C. The reactor contents were further cooled to below 85 °C, after which the reactor was emptied through a 100 mesh screen to remove the solid acid catalyst from the glycan preparation. Approximately 12 kg of product material were recovered.

The glycan preparation was further diluted to a concentration of about 35 wt% in de-ionized water and then purified by flowing through a cationic exchange resin (Dowex® Monosphere 88H) column, an anionic exchange resin (Dowex® Monosphere 77WBA) column, and a decolorizing polymer resin (Dowex® OptiPore SD-2). The resulting purified material was then concentrated to a final concentration of about 75 wt% solids by vacuum rotary evaporation to yield the purified glycan preparation.

### Example 16: Production of glycan polymer preparations at 10 kg scale from dextrose monohydrate and galactose

To a reaction vessel (22L Littleford-Day horizontal plow mixer) was added 5 kg of dextrose monohydrate, 4.5 kg of galactose and 0.892 kg (0.450 kg on a dry solid basis) solid acid catalyst (poly-styrene-co-divinylbenzene comprising > 3.0 mmol/g sulfonic acid moieties and < 1.0 mmol/gram cationic moieties). The contents were agitated at approximately 30 RPM and the vessel temperature was gradually increased over a two hour period to about 130°C at atmospheric pressure. The mixture was maintained at temperature for one hour, after which the heating was stopped and pre-heated water was gradually added to the reaction mixture at a rate of 6 mL/min until the temperature of the reactor contents decreased to 120°C. then at 150 mL/min until the temperature of the reactor contents decreased to 110°C, then at 480 mL/min until a total of 6 kg of water was added and the temperature of the reactor contents decreased below 100°C. The reaction mixture was drained from the vessel and the solids were removed by filtration, resulting in 12 kg of product material as a syrup.

The glycan composition was further diluted to a concentration of about 35 wt% in de-ionized water and then purified by flowing through a cationic exchange resin (Dowex® Monosphere 88H) column, an anionic exchange resin (Dowex® Monosphere 77WBA) column, and a decolorizing polymer resin (Dowex® OptiPore SD-2). The resulting purified material was then concentrated to a final concentration of about 75 wt % solids by vacuum rotary evaporation to yield the purified glycan composition.

### Example 17: Production of glycan polymer preparations at 10 kg scale from dextrose monohydrate and galactose (e.g., glycan polymer preparation glu50gal50) (10 kg scale) with serial catalyst addition

The present example demonstrates the synthesis of a glycan polymer preparation comprising glucose and galactose sub-units at 10 kg scale (dry glycan polymer preparation) for two replicate batches in a 22L horizontal-mixed reactor.

About 5 kg of food grade dextrose monohydrate and 4.5 kg of food grade galactose were charged into a 22L horizontal plough mixer (Littleford-Day, Lexington, KY) equipped with a hot-oil jacket. The dextrose and galactose mixture was melted by gradually heating to a temperature of about 120°C with continuous mixing at 30 RPM. 0.892 kg (0.450 kg on a dry solid basis) solid acid catalyst (poly-styrene-co-divinylbenzene comprising > 3.0 mmol/g sulfonic acid moieties and < 1.0 mmol/gram cationic moieties) was then added to the reaction mixture to form a mixed suspension. The reaction temperature was gradually increased to about 130°C at atmospheric pressure over a two hour period with continuous mixing, maintained at 30 RPM. Preheated water was then gradually added to the reaction mixture at a rate of 6 mL/min until the temperature of the reactor contents decreased to 120°C, then at 150 mL/min until the temperature of the reactor contents decreased to 110°C, then at 480 mL/min until a total of 6 kg of water was added and the temperature of the reactor contents decreased below 100°C. The reactor contents were further cooled to below 85°C, and filtered to remove the solid acid catalyst from the glycan polymer preparation. Approximately 12 kg of product material were recovered.

The glycan polymer preparation was further diluted to a concentration of about 35 wt% in de-ionized water and then purified by flowing through a cationic exchange resin (Dowex® Monosphere 88H) column, an anionic exchange resin (Dowex® Monosphere 77WBA) column, and a decolorizing polymer resin (Dowex® OptiPore SD-2). The resulting purified material was then concentrated to a final concentration of about 75 wt% solids to yield the purified glycan polymer preparation.

### Example 18: De-monomerization procedure

In one example, the glycan polymer preparation was concentrated on a rotatory evaporator to approximately 50 Brix as measured by a Brix refractometer. The resulting syrup (200 mg) was loaded onto a Teledyne ISCO RediSep Rf Gold Amine column (11 grams stationary phase) using a luer-tip syringe. Other similar columns such as the Biotage SNAP KP-NH Catridges may also be used. The sample was purified on a Biotage Isolera equipped with an ELSD detector using a 20/80 to 50/50 (v/v) deionized water/ACN mobile phase gradient over 55 column volumes. Other flash chromatography systems such as the Teledyne ISCO Rf may also be used. The flow rate was set in accordance with the manufacturer's specifications for the column and system. After the monomer fraction completely eluted at ∼20 column volumes, the mobile phase was set to 100% water until the remainder of the glycan eluted and was collected. The non-monomer containing fractions were concentrated by rotary evaporation to afford the de-monomerized product. (Figure 20).

### EQUIVALENTS AND SCOPE

This application refers to various issued patents, published patent applications, journal articles, and other publications, all of which are incorporated herein by reference. If there is a conflict between any of the incorporated references and the instant specification, the specification shall control. In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art.

Those skilled in the art will recognize or be able to ascertain using no more than routine experimentation many equivalents to the specific embodiments described herein. The scope of the present embodiments described herein is not intended to be limited to the above Description, Figures, or Examples but rather is as set forth in the appended claims. Those of ordinary skill in the art will appreciate that various changes and modifications to this description may be made without departing from the spirit or scope of the present invention, as defined in the following claims.

**Table 9: Genus level bacterial constituents of the GI tract.**

| Phylum | Class | Genus |
|---|---|---|
| Actinobacteria | Actinobacteria | Actinomyces, Adlercreutzia, Atopobium, Bifidobacterium, Collinsella, Corynebacterium, Eggerthella, Mobiluncus, Propionibacterium, Rothia, Slackia |
| Bacteroidetes | Bacteroidia | Alistipes, Bacteroides, Dysgonomonas, Odoribacter, Parabacteroides, Porphyromonas, Prevotella, Tannerella |
| | Flavobacteria | Capnocytophaga |
| Firmicutes | Bacilli | Bacillus, Enterococcus, Gemella, Granulicatella, Lactobacillus, Lactococcus, Staphylococcus, Streptococcus, Turicibacter, Weissella |
| | Clostridia | Acidaminococcus, Anaerococcus, Anaerofilum, Anaerofustis, Anaerostipes, Anaerotruncus, Anaerovorax, Bacteroides, Bacteroides, Blautia, Clostridium, Coprococcus, Dehalobacterium, Dialister, Dorea, Eubacterium, Faecalibacterium, Finegoldia, Lachnobacterium, Lachnospira, Megamonas, Megasphaera, Mitsuokella, Moryella, Oribacterium, Oscillospira, Peptococcus, Peptoniphilus, Peptostreptococcus, Phascolarctobacterium, Pseudobutyrivibrio, Roseburia, Ruminococcus, Ruminococcus, Selenomonas, Subdoligranulum, Veillonella |
| Fusobacteria | Fusobacteria | Fusobacterium, Leptotrichia |
| | Betaproteobacteria | Comamonas, Herbaspirillum, Lautropia, Neisseria, Oxalobacter, Sutterella |
| | Deltaproteobacteria | Bilophila, Desulfovibrio, LE30 |
| | Epsilonproteobacteria | Campylobacter, Helicobacter |
| | Gammaproteobacteria | Actinobacillus, Aggregatibacter, Citrobacter, Escherichia, Haemophilus. Klebsiella, Moraxella, Pseudomonas, Raoultella |
| Spirochaetes | Spirochaetes | Treponema |
| Synergistetes | Synergistetia | Cloacibacillus, Synergistes |
| Tenericutes | Erysipelotrichi | Bulleidia, Catenibacterium, Clostridium, Coprobacillus, Holdemania, RFN20 |
| | Mollicutes | Asteroleplasma, Mycoplasma |
| Verrucomicrobia | Verrucomicrobiae | Akkermansia |
| Eurvarchaeota | Methanobacteria | Methanobrevibacter |

**Table 10: Genus level bacterial constituents predominant in the large intestine (compared to small intestine) in healthy humans.**

| Phylum | Class | Genus |
|---|---|---|
| Bacteroidetes | Bacteroidia | Bacteroides, Butyricimonas, Odoribacter, Parabacteroides, Prevotella |
| Firmicutes | Clostridia | Anaerotruncus, Phascolarctobacterium, Ruminococcus, |
| Proteobacteria | Deltaproteobacteria | Bilophila |
| Verrucomicrobia | Verrucomicrobiae | Akkermansia |

**Table 11: Genus level bacterial constituents predominant in the small intestine (compared to large intestine) in healthy humans.**

| Phylum | Class | Genus |
|---|---|---|
| Actinobacteria | Actinobacteria | Cryocola, Mycobacterium |
| Firmicutes | Bacilli | Enterococcus, Lactococcus, Streptococcus, Turicibacter |
| | Clostridia | Blautia, Coprococcus, Holdemania, Pseudoramibacter Eubacterium |
| Proteobacteria | Alphaproteobacteria | Agrobacterium, Sphingomonas |
| | Betaproteobacteria | Achromobacter, Burkholderia, Ralstonia |

### Embodiments

1. A method of treating an adverse effect of a pathogen (e.g., a drug or antibiotic resistant pathogen, or an MDR pathogen) on a first facility participant in a facility comprising: administering to one or both, the first facility-participant and a second facility participant, an amount of a glycan preparation effective to reduce, prevent, or reduce the risk of, the adverse effect of the pathogen on the first facility participant, thereby reducing, preventing, or reducing the risk of an adverse effect of a pathogen on the first facility participant.
2. The method of embodiment 1, wherein the glycan preparation is administered in an effective amount and/or to a sufficient number of facility-participant(s) to reduce the spread of the pathogen, e.g., from a first facility participant to a second facility participant.
3. The method of embodiment 1, wherein the glycan preparation is administered in an effective amount and/or to a sufficient number of facility participant(s) to reduce the reservoir of pathogen.
4. The method of embodiment 1, wherein the glycan preparation is administered in an effective amount and/or to a sufficient number of facility-participant(s) to reduce the reservoir of drug- or antibiotic-resistance gene, or a MDR gene element.
5. The method of embodiment 1, wherein the glycan preparation is administered in an effective amount and/or to a sufficient number of facility-participant(s) to reduce the spread of drug- or antibiotic-resistance gene, or a MDR element, e.g., from the first facility participant to the second facility participant.
6. The method of embodiment 1, wherein the glycan preparation is administered in an effective amount and/or to a sufficient number of facility-participant(s) to reduce the rate at which a pathogen causes infection.
7. The method of embodiment 1, wherein the glycan preparation is administered in an effective amount and/or to a sufficient number of facility-participant(s) to reduce the severity of pathogen infection.
8. The method of embodiment 1, wherein the glycan preparation is administered in an effective amount and/or to a sufficient number of facility-participant(s) to reduce the rate at which a drugor antibiotic-resistance gene, or an MDR element, is transferred from a donor microbe (e.g., a first pathogen) to a recipient microbe (e.g., a second pathogen or commensal microbe), optionally wherein the microbe is a bacterial taxa.
9. The method of embodiment 1, wherein the glycan preparation is administered in an effective amount and/or to a sufficient number of facility-participant(s) to reduce the expression and/or release by the pathogen of a factor having an adverse effect on the facility participant, e.g., a virulence factor or a toxin, e.g., that causes disease.
10. The method of embodiment 1, wherein the glycan preparation is administered in an effective amount and/or to a sufficient number of facility-participant(s) to reduce dysbiosis of the microbiota in the GI tract (e.g., small intestine, large intestine or colon) of the facility participant by the pathogen.
11. The method of embodiment 1, wherein the glycan preparation is administered in an effective amount and/or to a sufficient number of facility-participant(s) to reduce the spread of the pathogen, e.g., from a first facility participant to an entity which can harbor the pathogen (e.g., another individual or an inanimate object, e.g., facility built surface (e.g. sink, door handle, toilet, faucet) or medical supply (e.g., a package comprising a dressing or device, or a dressing or device itself).
12. The method of embodiment 1, wherein the glycan preparation is administered in an effective amount and/or to a sufficient number of facility-participant(s) to reduce the spread of the pathogen, e.g., from a second facility participant to an entity which can harbor the pathogen (e.g., another individual or an inanimate object, e.g., a facility built surface (e.g. sink, door handle, toilet, faucet) or medical supply (e.g., a package comprising a dressing or device, or a dressing or device itself).
13. The method of any one of embodiments 1-12, wherein the first facility participant is a patient or resident of the facility.
14. The method of any one of embodiments 1-12, wherein the first facility participant is other than a patient or resident of the facility.
15. The method of any one of embodiments 1-12, wherein the first facility participant is a patient or resident of the facility and the second facility participant is a patient or resident of the facility.
16. The method of any one of embodiments 1-15, wherein the first facility participant is a patient or resident of the facility and the second facility participant is other than a patient or resident of the facility.
17. The method of any one of embodiments 1-15 wherein the first facility participant is other than a patient or resident of the facility and the second facility participant is a patient or resident of the facility.
18. The method of any one of embodiments 1-15, wherein the first facility participant is other than a patient or resident of the facility and the second facility participant is other than a patient or resident of the facility.
19. The method of any one of embodiments 11 or 12, wherein the entity which can harbor the pathogen is another individual.
20. The method of any one of embodiments 11 or 12, wherein the entity which can harbor the pathogen is an inanimate object, e.g., a facility built surface (e.g. sink, door handle, toilet, faucet) or a medical supply (e.g., a package comprising a dressing or device, or a dressing or device itself).
21. The method of any of embodiments 1-20, comprising administering the effective glycan preparation to the first facility participant.
22. The method of any of embodiments 1-20, comprising administering the effective glycan preparation to the second facility participant.
23. The method of any of embodiments 1-22, comprising administering the effective glycan preparation to the first facility participant and to the second facility participant.
24. The method of any of embodiments 1-23, wherein the effective glycan preparation administered to the first facility participant and to the second facility participant is the same.
25. The method of any of embodiments 1-23, wherein the effective glycan preparation administered to the first facility participant and to the second facility participant is different, e.g., different in dosage or chemical composition.
26. The method of any of embodiments 1-25, wherein the effective glycan preparation is administered to the first facility participant and to the second facility participant on the same regimen, e.g., for the same number of days.
27. The method of any of embodiments 1-25, wherein the effective glycan preparation is administered to the first facility participant and to the second facility participant on a different regimen, e.g., for a different number of days.
28. The method of any of embodiments 1-27, wherein the effective glycan preparation is administered to a facility participant (e.g., a facility participant who is a patient, resident or staff of the facility) prior to entry or admission to the facility.
29. The method of any of embodiments 1-27, wherein the effective glycan preparation is administered to a facility participant (e.g., a facility participant who is a patient, resident or staff of the facility) while at the facility.
30. The method of any of embodiments 1-27, wherein the effective glycan preparation is administered to a facility participant (e.g., a facility participant who is a patient, resident or staff of the facility) after leaving the facility.
31. The method of any of embodiments 1-30, wherein, independently, the first and second facility participant is selected from:
   a) a patient or resident;
   b) an individual who is a medical care giver, e.g., a medical practitioner, e.g., a physician or nurse,
   c) a housekeeping worker;
   d) a security worker (e.g. a guard);
   e) a maintenance worker;
   f) a food preparation worker;
   g) a laundry worker;
   h) an administrative worker, e.g., an admissions worker;
   i) a social worker;
   j) a visitor or guest;
   k) a facility employee not of (b)-(i) (e.g., a teacher, a soldier, a sailor, an officer);
   l) an individual of b)-k) who has direct contact with the first facility participant of (a), a patient or resident;
   m) an individual of b)-k) who does not have direct contact with the first facility participant of (a), a patient or resident.
32. The method of embodiment 31, comprising administering a glycan preparation to an individual from a plurality of the classes a-m, e.g., at least 2, 3, 4, 5, 6, 7, 8, 9, 10, 11, 12, or all of classes a-m.
33. The method of embodiment 31, wherein at least 50, 60, 70, 80, 90, or 95%, or all of the individuals in a class of a-m are administered a glycan preparation.
34. The method of embodiment 31, wherein at least 50, 60, 70, 80, 90, or 95 %, or all of the individuals from a plurality of the classes a-m, e.g., at least 2, 3, 4, 5, 6, 7, 8, 9, 10, 11, 12, or all of classes a-m are administered a glycan preparation.
35. The method of any of embodiments 1-34, wherein at least 50, 60, 70, 80, 90, or 95 %, or all of the facility participants in predetermined group, e.g., all who visit the room of the first facility participant, or all in a room or segment of the facility housing the first facility participant are administered a glycan preparation.
36. The method of embodiment 35, wherein the effective dose for a first and second class of classes a-m is the same.
37. The method of embodiment 35, wherein the effective dose for a first and second class of claim classes a-m is different.
38. The method of embodiment 35, wherein scheduled doses are administered to an individual from a class of classes a-m.
39. The method of any of embodiment 1-38, wherein a facility participant, is infected with a pathogen, optionally, wherein the facility participant is asymptomatic (e.g., displays no detectable or diagnosable signs of infection).
40. The method of embodiment 3 or 4, wherein the reservoir (e.g., pathogen reservoir or resistance gene reservoir) in the first facility participant is reduced.
41. The method of embodiment 3 or 4, wherein the reservoir (e.g., pathogen reservoir or resistance gene reservoir) in the second facility participant is reduced.
42. The method of embodiment 3 or 4, wherein the reservoir (e.g., pathogen reservoir or resistance gene reservoir) is reduced in a plurality of facility participants.
43. The method of embodiment 8, wherein the donor microbe and the recipient microbe are from the same taxa (e.g., genus, species, or strain).
44. The method of embodiment 8, wherein the donor microbe and the recipient microbe are from a different taxa (e.g., genus, species, or strain).
45. The method of embodiments 43 or 44, wherein the taxa is one or more of: Enterobacteriaciae (e.g., a genus comprising Plesiomonas, Shigella, or Salmonella), Clostridium (e.g., a genus comprising Clostridium difficile), Enterococcus, and Staphylococcus (e.g., a genus comprising Staphylococcus aureus).
46. The method of any one of embodiments 43-45, wherein the recipient microbe is more pathogenic, or has additional or more severe adverse effects on a facility participant (e.g. the first facility participant) compared to the donor microbe.
47. The method of any one of embodiments 43-45, wherein the donor microbe is more pathogenic, or has additional or more severe adverse effects on a facility participant (e.g. the first facility participant) compared to the recipient microbe.
48. The method of any of embodiments 1-47, wherein the adverse effect (e.g., on the first facility participant) is an infection (e.g. a bacterial infection or bacteremia).
49. The method of embodiment 48, wherein the infection is a bloodstream infection, a UTI, or a respiratory infection.
50. The method of embodiment 9, wherein the virulence factor or toxin is one of: Shiga toxin, E. *coli* heat labile toxin, and *Clostridium difficile* Toxin A and B.
51. The method of any one of embodiments 1-50, wherein the drug- or antibiotic-resistance gene, or an MDR element is one of: MecA, KPC, NDM, OXA, SHV, TIM, CTX-M, VIM, AmpC, VanA, VanB, fluoroquinoline resistance genes (e.g., Qnr), trimethoprim resistance genes (e.g. dihydrofolate reductase), sulfamethoxazole resistance genes (e.g., dihydropteroate synthetase), ciprofloxacin resistance genes, and aminoglycoside resistance genes (e.g., ribosomal methyltransferase).
52. The method of any one of embodiments 1-51, wherein the glycan preparation is administered in an amount effective to reduce the level of the pathogen in the gut (e.g., small intestine, large intestine or colon) of a facility-participant.
53. The method of any one of embodiments 1-52, wherein the glycan preparation is administered in an amount effective to modulate (e.g. reduce or inhibit) colonization, or modulate (e.g. increase) decolonization, by the pathogen in a facility-participant, e.g., the first and/or second facility-participant.
54. The method of any one of embodiments 1-53, wherein the glycan preparation is administered in an amount sufficient, to reduce or prevent dysbiosis in the gut (e.g., small intestine, large intestine or colon) of a facility participant, optionally, a facility participant infected with the pathogen.
55. The method of any one of embodiments 1-54, wherein treating comprises reducing an adverse effect of the pathogen on the first facility participant, the second facility participant, or both.
56. The method of any one of embodiments 1-55, wherein treating comprises preventing an adverse effect of the pathogen on the first facility participant, the second facility participant, or both.
57. The method of any one of embodiments 1-55, wherein treating comprises reducing the risk of an adverse effect of the pathogen on the first facility participant, the second facility participant, or both.
58. The method of any one of embodiments 1-57, wherein the glycan preparation is administered in an amount effective to:
   a) modulate (e.g., reduce) pathogen biomass (e.g., the number of pathogens and/or the number of drug- or antibiotic-resistance gene or MDR element carriers);
   b) modulate (e.g., increase) the level of anti-microbial compounds produced by the facility participant (e.g., by the resident gut microbiota and/or the host (e.g., human cells));
   c) modulate the environment of the GI tract (e.g., small intestine, large intestine or colon), e.g. reducing the pH (e.g., by increasing production or levels of lactic acid, e.g. produced by the resident gut microbiota);
   d) modulate (e.g., reduce) the state of competency or a conjugation property of a donor microbe of a drug- or antibiotic-resistance gene or MDR element;
   e) modulate (e.g., reduce) the number of drug- or antibiotic-resistance gene or MDR element recipients;
   f) modulate (e.g., reduce) the copy number of a drug- or antibiotic-resistance gene or MDR element (e.g. total copy number, e.g. in a donor microbe); and/or
   g) modulate (e.g., increase) the fitness deficit (e.g., increase the burden of carrying a drug- or antibiotic-resistance gene or MDR element),
      in the first and/or second facility participant or a plurality of facility participant(s).
59. The method of any one of embodiments 1-58, wherein the glycan preparation is administered in an amount effective to:
   a) decrease in the resident gut microbiota the abundance (e.g. total number or relative number) of pathogens and/or drug- or antibiotic-resistance gene or MDR element carriers; and/or
   b) increase in the resident gut microbiota the abundance (e.g. total number or relative number) of commensals or beneficial bacteria.
60. The method of embodiment 59, wherein the glycan preparation is administered in an amount effective to: increase in the resident gut microbiota the abundance (e.g. total number or relative number) of commensals or beneficial bacteria, thereby reducing the area of colonizable space for the pathogen.
61. The method of embodiment 59, wherein the glycan preparation is administered in an amount effective to: increase in the resident gut microbiota the abundance (e.g. total number or relative number) of commensals or beneficial bacteria, thereby increasing the levels of anti-microbial defense compounds, e.g. bacteriocins, AMPs (anti-microbial peptides), hydrogen peroxide, or acetate (low pH).
62. The method of any of embodiments 1-61 wherein the pathogen is a bacterium.
63. The method of any of embodiments 1-62, wherein the pathogen is a drug or antibiotic resistant pathogen, e.g., bacterium.
64. The method of any of embodiments 1-63 wherein the pathogen is a multiply drug resistant (MDR) carrying pathogen, e.g., bacterium.
65. The method of any of embodiments 1-64, wherein the pathogen is a vancomycin resistant enterococcus (VRE) or carbapenem resistant (CRE) Enterobacteriaceae (e.g. *E. coli, Klebsiella, Enterobacter, Proteus).*
66. The method of any of embodiments 1-65, wherein the pathogen is a vancomycin resistant enterococcus (VRE) *Enterococcus faecium.*
67. The method of any of embodiments 1-65, wherein the pathogen is a carbapenem resistant (CRE) *E. coli.*
68. The method of any of embodiments 1-65, wherein the pathogen is a carbapenem resistant (CRE) *Klebsiella pneumoneae.*
69. The method of any of embodiments 1-64, wherein the pathogen is a gram-positive bacterium.
70. The method of any of embodiments 1-64, wherein the pathogen is a gram-negative bacterium.
71. The method of any of embodiments 1-70, wherein the first and/or second facility participant: i) has received cancer treatment; ii) is a transplant recipient, e.g., a hematopoietic stem cell recipient; iii) has received immunosuppression, and/or iv) has an auto-immune disease (e.g., systemic lupus erythematosus, rheumatoid arthritis, Sjögren's syndrome, or Crohn's disease).
72. The method of any of embodiments 1-71, wherein the first and/or second facility participant has cystic fibrosis.
73. The method of any of embodiments 1-72, wherein the first and/or second facility participant has a defect of the immune system, e.g., i) an acquired defect, e.g., HIV/AIDS, or ii) a hereditary or congenital defect, e.g., SCID, CVID, Bruton's agammaglobulinemia, or an auto-immune disease (e.g., systemic lupus erythematosus, rheumatoid arthritis, Sjögren's syndrome, or Crohn's disease).
74. The method of any of embodiments 1-73, wherein the first and/or second facility participant has a chronic disorder or disease.
75. The method of any of embodiments 1-74, wherein the first and/or second facility participant is i) an infant or ii) an elderly adult.
76. The method of embodiment 75, wherein the facility participant is more than 45, 50, 55, 60, 65, 70, 75, or 80 years of age.
77. The method of embodiment 75, wherein the facility participant is less than 1, 2, 3, 6, 12, 24, or 36 months of age.
78. The method of any of embodiments 1-77, wherein the first and/or second facility participant is immune compromised or has received treatment that reduces immune function.
79. The method of any of embodiments 1-78, wherein the first and/or second facility participant is, has been, or will be administered dialysis treatment.
80. The method of any of embodiments 1-79, wherein the first and/or second facility participant is, has been, or will be administered treatment that is invasive, e.g., breaks the skin, e.g., surgery or the insertion or implantation of a device (e.g., catheter or stent), or connection to a ventilator, drip line, intrusive monitor or food applicator (orally, rectally, enterically, IV, etc.).
81. The method of any of embodiments 1-80, wherein the facility participant in the last year has spent more than 10 days in a facility, or been admitted to a facility more than twice.
82. The method of any of embodiments 1-81, wherein the first facility participant has shared or will share a room or other facility or space with a pathogen infected or colonized second facility participant, optionally, wherein the second facility participant is asymptomatic.
83. The method of any of embodiments 1-82, wherein the first facility participant has come or will come into proximity with a pathogen infected or colonized second facility participant, optionally, wherein the second facility participant is asymptomatic.
84. The method of any of embodiments 1-83, wherein the facility comprises a hospital, a clinic, a rehabilitation facility, a mental health facility, an intensive care facility (ICU), a neonatal facility, a cancer treatment facility, a nursing facility, a drug-treatment facility, a training facility, a clinical trial facility, an inpatient facility, an outpatient facility.
85. The method of any of embodiments 1-84, wherein the facility comprises a long-term care facility, e.g., a home for the elderly.
86. The method of any of embodiments 1-84, wherein the facility comprises a prison or other correctional or penal facility.
87. The method of any of embodiments 1-84, wherein the facility comprises a ship, e.g., a cruise ship or military vessel.
88. The method of any of embodiments 1-84, wherein the facility comprises a military facility, an athletic facility, an educational facility (school, camp), or a leisure facility (e.g., hotel or resort).
89. The method of any of embodiments 1-88, further comprising administering to a facility participant, a second treatment, e.g., an antibiotic.
90. The method of embodiment 1-89, wherein the glycan preparation is administered to the facility participant prior to working at the facility and/or while working at the facility.
91. The method of embodiment 1-90, wherein the glycan preparation is administered to the facility participant i) prior to entering the facility, ii) while at the facility, iii) at or after release from the facility, or any combination of (i), (ii), and (iii).
92. The method of embodiment 1-91, comprising acquiring a level of a pathogen (e.g., a drug or antibiotic resistant pathogen, or an MDR pathogen) from the facility-participant (e.g., by analyzing a sample of the facility participant), optionally, repeating the acquisition two, three, four, or more times.
93. The method of any of embodiments 1-92, wherein the glycan preparation comprises:
   i) glycan polymers that comprise glucose, galactose, arabinose, mannose, fructose, xylose, fucose, or rhamnose glycan units;
   ii) the average degree of branching (DB) of the glycan polymers in the glycan preparation is 0, between 0.01 and 0.6, between 0.05 and 0.5, between 0.1 and 0.4, or between 0.15 and 0.4;
   iii) at least 50% (at least 60%, 65%, 70%, 75%, 80%, or 85%, or less than 50%) of the glycan polymers in the glycan preparation have a degree of polymerization (DP) of at least 3 and less than 30 glycan units, at least 3 and less than 10 glycan units, at least 5 and less than 25 glycan units, or at least 10 and less than 35 glycan units;
   iv) the average DP (mean DP) of the glycan preparation is between about 5 and 8, between about 8 and 13, between about 13 and 25, between about 5 and 15, between about 5 and 20, or between about 5-15;
   v) the ratio of alpha- to beta-glycosidic bonds present in the glycan polymers of the glycan preparation is 0, or between about 0.8:1 to about 5:1, between about 1:1 to about 5:1, between about 1:1 to about 3:1, between about 3:2 to about 2:1, or between about 3:2 to about 3:1,
   vi) the glycan preparation comprises between 15 mol % and 75 mol % (between 20 mol % and 60 mol %, between 25 mol % and 50 mol %, or between 30 mol % and 45 mol %) 1,6 glycosidic bonds;
   vii) the glycan preparation comprises between 1 mol % and 40 mol % (between 1 mol % and 30 mol %, between 5 mol % and 25 mol %, between 10 mol % and 20 mol %) of at least one, two, or three of 1,2; 1,3; and 1,4 glycosidic bonds;
   viii) the glycan preparation has a final solubility limit in water of at least about 50 (at least about 60, 70, at least about 75, or less than 50) Brix at 23 °C; and/or
   ix) the glycan preparation has a dietary fiber content of at least 50% (at least 60%, 70%, 80%, or at least 90%, or less than 50%);
   optionally wherein, the glycan preparation comprises two, three, four, five, six, seven, eight, or nine of the selected properties of i), ii), iii), iv), v), vi), vii), viii), and ix).
94. The method of embodiment 93, wherein the DB is 0, between 0.01 and 0.05, 0.01 and 0.15, 0.01 and 0.2, 0.05 and 0.2, 0.1 and 0.3, 0.1 and 0.4, 0.1 and 0.5, 0.1 and 0.6, 0.1 and 0.7, 0.2 and 0.5, 0.15 and 0.65, or between 0.4 and 0.75.
95. The method of embodiment 93, wherein the DB is between 0.1 and 0.3.
96. The method of embodiment 93, wherein the DB is between 0.3 and 0.6.
97. The method of embodiment 93, wherein DB is between 0.01 and 0.1.
98. The method of any one of embodiments 93-97, wherein at least 50% of the glycans in the glycan polymer preparation have a DP of at least 3 and less than 30 glycan units.
99. The method of any one of embodiments 93-97, wherein at least 60%, 65%, 70%, 75%, 80%, or 85% of the glycans in the glycan polymer preparation have a DP of at least 3 and less than 30 glycan units.
100. The method of any one of embodiments 93-97, wherein less than 50%, of the glycans in the glycan polymer preparation have a DP of at least 3 and less than 30 glycan units.
101. The method of any one of embodiments 93-97, wherein the DP is at least 3 and less than 30 glycan units, at least 3 and less than 10 glycan units, at least 3 and less than 15 glycan units, at least 3 and less than 20 glycan units, at least 3 and less than 25 glycan units, at least 4 and less than 15 glycan units, at least 4 and less than 20 glycan units, at least 4 and less than 25 glycan units, at least 5 and less than 15 glycan units, at least 5 and less than 20 glycan units, at least 5 and less than 25 glycan units, at least 5 and less than 30 glycan units, at least 6 and less than 15 glycan units, at least 6 and less than 20 glycan units, at least 6 and less than 25 glycan units, at least 6 and less than 30 glycan units, at least 8 and less than 15 glycan units, at least 8 and less than 20 glycan units, at least 8 and less than 25 glycan units, at least 8 and less than 30 glycan units, at least 10 and less than 20 glycan units, or at least 10 and less than 30 glycan units.
102. The method of embodiment 101, wherein the DP is 3-8.
103. The method of embodiment 101, wherein the DP is 8-13.
104. The method of embodiment 101, wherein the DP is 13-25.
105. The method of embodiment 101, wherein the DP is 5-25.
106. The method of embodiment 101, wherein the DP is 10-35.
107. The method of any one of embodiments 93-106, wherein the ratio of alpha- to beta-glycosidic bonds present in the glycan polymer preparation is from about 0.8:1 to 5:1.
108. The method of any one of embodiments 93-106, wherein the ratio of alpha- to beta-glycosidic bonds is between about 0.1:1 to about 4:1, 0.2:1 to about 4:1, 0.3:1 to about 4:1, 0.4:1 to about 4:1, 0.5:1 to about 4:1, 0.6:1 to about 4:1, 0.7:1 to about 4:1, 0.8:1 to about 4:1, 0.9:1 to about 4:1, 1:1 to about 2:1, 1:1 to about 3:1, 1:1 to 4:1, about 2:1 to about 4:1, 2:1 to 5:1, about 3:1 to about 5:1, or 4:1 to about 5:1.
109. The method of embodiment 108, wherein the ratio of alpha- to beta-glycosidic bonds is between about 1:1 to about 5:1.
110. The method of embodiment 108, wherein the ratio of alpha- to beta-glycosidic bonds is between about 1:1 to about 3:1.
111. The method of embodiment 108, wherein the ratio of alpha- to beta-glycosidic bonds is between about 3:2 to about 2:1.
112. The method of embodiment 108, wherein the ratio of alpha- to beta-glycosidic bonds is between about 3:2 to about 3:1.
113. The method of any one of embodiment 93 -112, wherein the final solubility limit in water of the glycan polymer preparation is at least 50 Brix.
114. The method of any one of embodiments 93-112, wherein the final solubility limit in water of the glycan polymer preparation is at least about 55, at least about 60, at least about 65, or at least about 70, or at least about 75 Brix at 23 °C.
115. The method of embodiment 114, wherein the final solubility limit in water of the glycan polymer preparation is at least about 70 Brix at 23 °C.
116. The method of any one of embodiments 93-115, wherein the glycan polymer preparation has a total dietary fiber content of at least 50%, 60%, 70%, or at least 80% (as measured by the method AOAC 2009.01).
117. The method of any of embodiments 1-92, wherein the glycan preparation comprises:
   i) glycan polymers that comprise glucose, galactose, or mannose glycan units;
   ii) the average degree of branching (DB) of the glycan polymers in the glycan preparation is between 0.05 and 0.5;
   iii) at least 50% of the glycan polymers in the glycan preparation have a degree of polymerization (DP) of at least 3 and less than 30 glycan units;
   iv) the average DP (mean DP) of the glycan preparation is between about 5 and 20;
   v) the ratio of alpha- to beta-glycosidic bonds present in the glycan polymers of the glycan preparation is between about 0.8:1 to about 5:1;
   vi) the glycan preparation comprises between 15 mol % and 75 mol % 1,6 glycosidic bonds;
   vii) the glycan preparation comprises between 1 mol % and 30 mol % of at least one, two, or three of 1,2; 1,3; and 1,4 glycosidic bonds;
   viii) the glycan preparation has a final solubility limit in water of at least about 70 Brix at 23 °C; and/or
   ix) the glycan preparation has a dietary fiber content of at least 70%;
   optionally wherein, the glycan preparation comprises two, three, four, five, six, seven, eight, or nine of the selected properties of i), ii), iii), iv), v), vi), vii), viii), and ix).
118. The method of any of embodiments 1-92, wherein the glycan preparation comprises:
   i) glycan polymers that comprise glucose, galactose or mannose glycan units;
   ii) the average degree of branching (DB) of the glycan polymers in the glycan preparation is between 0.05 and 0.5;
   iii) at least 50% of the glycan polymers in the glycan preparation have a degree of polymerization (DP) at least 3 and less than 30 glycan units;
   iv) the average DP (mean DP) of the glycan preparation is between about 5 and 15;
   v) the ratio of alpha- to beta-glycosidic bonds present in the glycan polymers of the glycan preparation is between about 0.8:1 to about 5:1;
   vi) the glycan preparation comprises between 15 mol % and 75 mol % 1,6 glycosidic bonds;
   vii) the glycan preparation comprises between 1 mol % and 30 mol % of at least one, two, or three of 1,2; 1,3; and 1,4 glycosidic bonds;
   viii) the glycan preparation has a final solubility limit in water of at least about 70 Brix at 23 °C; and/or
   ix) the glycan preparation has a dietary fiber content of at least 70%;
   optionally wherein, the glycan preparation comprises two, three, four, five, six, seven, eight, or nine of the selected properties of i), ii), iii), iv), v), vi), vii), viii), and ix).
119. The method of any of embodiments 1-92, wherein the glycan preparation comprises:
   i) glycan polymers that comprise glucose or galactose glycan units;
   ii) the average degree of branching (DB) of the glycan polymers in the glycan preparation is between 0.1 and 0.4;
   iii) at least 50% of the glycan polymers in the glycan preparation have a degree of polymerization (DP) at least 3 and less than 10 glycan units;
   iv) the average DP (mean DP) of the glycan preparation is between about 5 and 8;
   v) the ratio of alpha- to beta-glycosidic bonds present in the glycan polymers of the glycan preparation is between about 1:1 to about 3:1;
   vi) the glycan preparation comprises between 20 mol % and 60 mol % 1,6 glycosidic bonds;
   vii) the glycan preparation comprises between 5 mol % and 25 mol % of at least one, two, or three of 1,2; 1,3; and 1,4 glycosidic bonds;
   viii) the glycan preparation has a final solubility limit in water of at least about 70 Brix at 23 °C; and/or
   ix) the glycan preparation has a dietary fiber content of at least 70%;
   optionally wherein, the glycan preparation comprises two, three, four, five, six, seven, eight, or nine of the selected properties of i), ii), iii), iv), v), vi), vii), viii), and ix).
119. The method of any of embodiments 1-92, wherein the glycan preparation comprises:
   i) glycan polymers that comprise glucose glycan units;
   ii) the average degree of branching (DB) of the glycan polymers in the glycan preparation is between 0.1 and 0.4;
   iii) at least 50% of the glycan polymers in the glycan preparation have a degree of polymerization (DP) at least 3 and less than 10 glycan units;
   iv) the average DP (mean DP) of the glycan preparation is between about 5 and 8;
   v) the ratio of alpha- to beta-glycosidic bonds present in the glycan polymers of the glycan preparation is between about 1:1 to about 3:1;
   vi) the glycan preparation comprises between 20 mol % and 60 mol % 1,6 glycosidic bonds;
   vii) the glycan preparation comprises between 5 mol % and 25 mol % of at least one, two, or three of 1,2; 1,3; and 1,4 glycosidic bonds;
   viii) the glycan preparation has a final solubility limit in water of at least about 70 Brix at 23 °C; and/or
   ix) the glycan preparation has a dietary fiber content of at least 70%;
   optionally wherein, the glycan preparation comprises two, three, four, five, six, seven, eight, or nine of the selected properties of i), ii), iii), iv), v), vi), vii), viii), and ix).
120. The method of any one of embodiments 1-119, further comprising, administering to the first and/or second facility participant a second treatment.
121. The method of embodiment 120, wherein the second treatment comprises administering an antibiotic.
122. A method of reducing the acquisition by a first facility participant of a pathogen, e.g., a drug or antibiotic resistant pathogen, or an MDR pathogen, in a facility comprising: administering to a second facility participant an amount of a glycan preparation effective to reduce the acquisition of the pathogen by the first facility participant, thereby reducing the acquisition by a first facility participant of the pathogen in a facility.
123. A method of reducing the reservoir of a pathogen, e.g., a drug or antibiotic resistant pathogen, or an MDR pathogen, in a facility comprising: administering to a facility-participant an amount of a glycan preparation effective to reduce the acquisition of a pathogen, e.g., a drug or antibiotic resistant pathogen, or an MDR pathogen, by a facility-participant, thereby reducing the reservoir of a MDR organism in a facility.
124. A method of monitoring or evaluating any of a) pathogen load, b) antibiotic resistance gene load and c) response to a therapeutic preparation in a facility participant comprising: acquiring a value (e.g., by analyzing a suitable sample, e.g., a fecal sample from the facility participant) for a) pathogens, and/or b) antibiotic resistance genes and/or microbiome analysis (e.g. the presence of one or more microbes related to response to a particular therapeutic preparation), thereby monitoring or evaluating the facility participant.
125. The method of embodiment 124, wherein the facility participant is a worker or prospective worker in the facility, and responsive to the result, evaluating whether to grant the facility participant access to the facility.
126. The method of embodiment 124, wherein the facility participant is a patient or resident, or prospective patient or resident of the facility, and responsive to the result, evaluating whether to grant the facility participant admission to the facility.
127. The method of embodiment 124, wherein the facility participant is a patient or resident, or prospective patient or resident of the facility, and responsive to the result evaluating the risk of acquiring a pathogen, optionally, evaluating further treatment and/or changes or adjustments to the treatment regimen or care.
128. The method of any of embodiments 1-127, comprising reducing ammonia levels in a human subject, comprising:
   administering to the human subject an effective amount of a glycan preparation according to any one of claims 1-127.
129. The method of embodiment 128, wherein the ammonia levels are systemic ammonia levels in the human subject.
130. The method of embodiment 128-129, wherein the reduction in ammonia levels is at least a 20% reduction compared with the ammonia levels prior to treatment.
131. The method of embodiments 128-130, wherein the human subject has or has been diagnosed as having liver damage (e.g., liver cirrhosis).
132. The method of embodiments 128-131, wherein the subject has or has been diagnosed as having hyperammonemia.
133. The method of embodiments 128-132, wherein the subject has or has been diagnosed as having overt hepatic encephalopathy (OHE).

## Claims

1. A glycan preparation for use in a method of treating an adverse effect of a pathogen on a first facility participant in a facility, said method comprising:
administering to one or both, the first facility-participant and a second facility participant, the glycan preparation in an amount effective to reduce the rate at which the pathogen causes infection and/or reduce the severity of pathogen infection of the first facility participant, thereby treating the adverse effect of the pathogen on the first facility participant,
wherein the first facility participant is a transplant recipient.

2. The glycan preparation for use of claim 1, wherein the glycan preparation is administered in an effective amount and/or to a sufficient number of facility-participant(s) to:
reduce the spread of the pathogen,
reduce the reservoir of the pathogen;
reduce the reservoir of a drug- or antibiotic-resistance gene, or a MDR gene element; or
reduce dysbiosis of the microbiota in the GI tract of the first or second facility participant.

3. The glycan preparation for use of claim 1 or 2, wherein the second facility participant is selected from:
a) a patient or resident;
b) an individual who is a medical care giver, e.g., a medical practitioner, e.g., a physician or nurse,
c) a housekeeping worker;
d) a security worker (e.g. a guard);
e) a maintenance worker;
f) a food preparation worker;
g) a laundry worker;
h) an administrative worker, e.g., an admissions worker;
i) a social worker;
j) a visitor or guest;
k);
l) an individual of b)-k) who has direct contact with the first facility participant.

4. The glycan preparation for use of any of claims 1-3, wherein the first and/or second facility participant, is infected with a pathogen, and wherein the facility participant is asymptomatic

5. The glycan preparation for use of any of claims 1 - 4, wherein the infection is a bloodstream infection, a UTI, or a respiratory infection.

6. The glycan preparation for use of any of claims 1-5, wherein the pathogen is a drug or antibiotic resistant pathogenic bacterium.

7. The glycan preparation for use of any of claims 1-6, wherein the pathogen is a multiply drug resistant (MDR) carrying pathogenic bacterium.

8. The glycan preparation for use of any of claims 1-7, wherein the pathogen is a vancomycin resistant enterococcus (VRE) or carbapenem resistant (CRE) Enterobacteriaceae (e.g. *E*. *coli, Klebsiella, Enterobacter, Proteus).*

9. The glycan preparation for use of any one of claims 1-8, comprising acquiring a level of a pathogen from the first facility-participant and optionally the second facility participant (e.g., by analyzing a sample of the facility participant), optionally, repeating the acquisition two, three, four, or more times.

10. The glycan preparation for use of any of claims 1-9, wherein the glycan preparation comprises one, two, three, four, five, six, seven, eight, or nine of the selected properties of i), ii), iii), iv), v), vi), vii), viii), and ix):
i) glycan polymers that comprise glucose, galactose, arabinose, mannose, fructose, xylose, fucose, or rhamnose glycan units;
ii) the average degree of branching (DB) of the glycan polymers in the glycan preparation is 0, between 0.01 and 0.6, between 0.05 and 0.5, between 0.1 and 0.4, or between 0.15 and 0.4;
iii) at least 50% (at least 60%, 65%, 70%, 75%, 80%, or 85%, or less than 50%) of the glycan polymers in the glycan preparation have a degree of polymerization (DP) of at least 3 and less than 30 glycan units, at least 3 and less than 10 glycan units, at least 5 and less than 25 glycan units, or at least 10 and less than 35 glycan units;
iv) the average DP (mean DP) of the glycan preparation is between about 5 and 8, between about 8 and 13, between about 13 and 25, between about 5 and 15, between about 5 and 20, or between about 5-15;
v) the ratio of alpha- to beta-glycosidic bonds present in the glycan polymers of the glycan preparation is 0, or between about 0.8:1 to about 5:1, between about 1:1 to about 5:1, between about 1:1 to about 3:1, between about 3:2 to about 2:1, or between about 3:2 to about 3:1,
vi) the glycan preparation comprises between 15 mol % and 75 mol % (between 20 mol % and 60 mol %, between 25 mol % and 50 mol %, or between 30 mol % and 45 mol %) 1,6 glycosidic bonds;
vii) the glycan preparation comprises between 1 mol % and 40 mol % (between 1 mol % and 30 mol %, between 5 mol % and 25 mol %, between 10 mol % and 20 mol %) of at least one, two, or three of 1,2; 1,3; and 1,4 glycosidic bonds; viii) the glycan preparation has a final solubility limit in water of at least about 50 (at least about 60, 70, at least about 75, or less than 50) Brix at 23 °C; and/or
ix) the glycan preparation has a dietary fiber content of at least 50% (at least 60%, 70%, 80%, or at least 90%, or less than 50%).

11. The glycan preparation for use of any one of claims 1-10, further comprising, administering to the first and/or second facility participant a second treatment comprising administering an antibiotic.

12. The glycan preparation for use of any one of claims 1-11, wherein the first facility participant is
a hematopoietic stem cell transplant (HSCT) recipient, or
an organ transplant recipient, preferably wherein the organ is liver or bone marrow.

13. The glycan preparation for use any one of claims 1-12, wherein the glycan preparation comprises glycan polymers comprising:
i) glucose, galactose, and mannose glycan units,
ii) glucose and galactose glycan units, or
iii) galactose and arabinose glycan units.

14. The glycan preparation for use of any one of claims 1-13, wherein the first facility participant has received cancer treatment; has received immunosuppression, and/or has an auto-immune disease.

15. The glycan preparation for use of any one of claims 1-12, wherein the transplant recipient received pre-transplant immune system ablation.

16. A glycan preparation for use in managing an infection in a subject, comprising:
administering the glycan preparation in an amount effective and for a time sufficient to treat the infection, wherein the glycan preparation comprises one, two, three, four, five, six, seven, eight, or nine of the selected properties of i), ii), iii), iv), v), vi), vii), viii), and ix):
i) glycan polymers that comprise glucose, galactose, arabinose, mannose, fructose, xylose, fucose, or rhamnose glycan units;
ii) the average degree of branching (DB) of the glycan polymers in the glycan preparation is between 0.05 and 0.5;
iii) at least 50% of the glycan polymers in the glycan preparation have a degree of polymerization (DP) at least 3 and less than 30 glycan units;
iv) the average DP (mean DP) of the glycan preparation is between about 5 and 15;
v) the ratio of alpha- to beta-glycosidic bonds present in the glycan polymers of the glycan preparation is between about 0.8:1 to about 5:1;
vi) the glycan preparation comprises between 15 mol % and 75 mol % 1,6 glycosidic bonds;
vii) the glycan preparation comprises between 1 mol % and 30 mol % of at least one, two, or three of 1,2; 1,3; and 1,4 glycosidic bonds;
viii) the glycan preparation has a final solubility limit in water of at least about 70 Brix at 23 °C; and/or
ix) the glycan preparation has a dietary fiber content of at least 70%,
wherein the subject is a transplant recipient, preferably wherein the subject is a hematopoietic stem cell transplant (HSCT) recipient, or
an organ transplant recipient, preferably wherein the organ is liver or bone marrow.

17. The glycan preparation for use of claim 16, wherein the glycan preparation comprises glycan polymers comprising
glucose, galactose, and mannose glycan units,
glucose and galactose glycan units, or
galactose and arabinose glycan units.

18. A kit comprising a unit dosage form of a glycan preparation for treating an adverse effect of a pathogen on a first facility participant in a facility, the kit comprising a glycan preparation comprising one, two, three, four, five, six, seven, eight, or nine of the selected properties of i), ii), iii), iv), v), vi), vii), viii), and ix):
i) glycan polymers that comprise glucose, galactose, arabinose, mannose, fructose, xylose, fucose, or rhamnose glycan units;
ii) the average degree of branching (DB) of the glycan polymers in the glycan preparation is between 0.05 and 0.5;
iii) at least 50% of the glycan polymers in the glycan preparation have a degree of polymerization (DP) at least 3 and less than 30 glycan units;
iv) the average DP (mean DP) of the glycan preparation is between about 5 and 15;
v) the ratio of alpha- to beta-glycosidic bonds present in the glycan polymers of the glycan preparation is between about 0.8:1 to about 5:1;
vi) the glycan preparation comprises between 15 mol % and 75 mol % 1,6 glycosidic bonds;
vii) the glycan preparation comprises between 1 mol % and 30 mol % of at least one, two, or three of 1,2; 1,3; and 1,4 glycosidic bonds;
viii) the glycan preparation has a final solubility limit in water of at least about 70 Brix at 23 °C; and/or
ix) the glycan preparation has a dietary fiber content of at least 70%,
wherein the first facility participant is a transplant recipient, preferably wherein a hematopoietic stem cell transplant (HSCT) recipient or
an organ transplant recipient, preferably wherein the organ is liver or bone marrow.

19. The glycan preparation for use of any one of claims 1-17, the method further comprising administering a second agent in combination with the glycan preparation, preferably wherein said second agent is charcoal or an antibiotic-degrading enzyme, a synbiotic, or a prebiotic.
